# EUROPEAN PATENT SPECIFICATION

(11) **EP 1 237 847 B1**
(45) Date of publication and mention of the grant of the patent: **17.05.2006**
(21) Application number: 00980881.7
(22) Date of filing: 30.11.2000
(51) Int. Cl.: C07C 227/32, C07C 229/28, C07C 57/46, C07C 255/31, C07C 255/41, C07C 265/08, C07C 271/12, C07C 69/616

(54) **METHOD FOR THE STEREOSELECTIVE SYNTHESIS OF CYCLIC AMINO ACIDS**
VERFAHREN ZUR STEREOSELEKTIVEN SYNTHESE ZYKLISCHER AMINOSÄUREN
METHODE DE SYNTHESE STEREOSELECTIVE D'ACIDES AMINES CYCLIQUES

(30) Priority: 08.12.1999 US 169602 P
(43) Date of publication of application: 11.09.2002
(73) Proprietor: Warner-Lambert Company LLC, Morris Plains, NJ 07950 (US)
(72) Inventor: BRYANS, Justin, Stephen, Balsham CB1 6DZ (GB); BLAKEMORE, David, Clive, Cambridge CB1 8TH (GB); WILLIAMS, Sophie, Caroline, Cambridge CB1 7TX (GB)
(74) Representative: Tesch, Rudolf
(86) International application number: PCT/US2000/032570
(87) International publication number: WO 2001/042190

(56) References cited:
- WO-A-99/21824
- WERNER HERZ: "Azulenes. VII. A Novel Rearrangement in the Synthesis of Azulenes" JOURNAL OF THE AMERICAN CHEMICAL SOCIETY., vol. 78, no. 7, 5 April 1956 (1956-04-05), pages 1485-1494, XP002161288 AMERICAN CHEMICAL SOCIETY, WASHINGTON, DC., US ISSN: 0002-7863

## Description

### BACKGROUND OF THE INVENTION

Compounds of formula 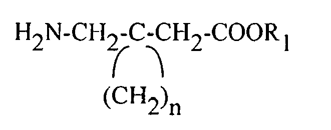 wherein R₁ is hydrogen or a lower alkyl radical and n is 4, 5, or 6 are known in United States Patent Number 4,024,175 and its divisional United States Patent Number 4,087,544. The uses disclosed are: protective effect against cramp induced by thiosemicarbazide; protective action against cardiarole cramp; the cerebral diseases, epilepsy, faintness attacks, hypokinesia, and cranial traumas; and improvement in cerebral functions. The compounds are useful in geriatric patients. The patents are hereby incorporated by reference.

United States Serial Number 09/485,382 filed February 8, 2000 and WO 9921824 teach in part compounds of Formula I 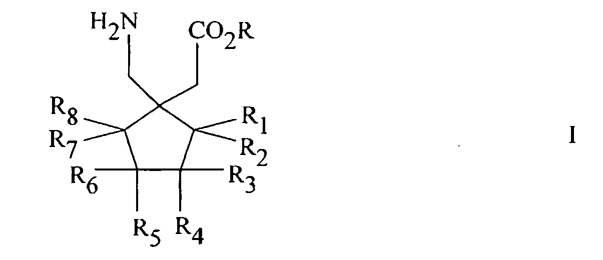 or a pharmaceutically acceptable salt thereof wherein R is hydrogen or a lower alkyl; and R₁ to R₈ are each independently selected from hydrogen, straight or branched alkyl of from 1 to 6 carbon atoms, phenyl, benzyl, fluorine, chlorine, bromine, hydroxy, hydroxymethyl, amino, aminomethyl, trifluoromethyl, -CO₂H, -CO₂R₁₅, -CH₂CO₂H, -CH₂CO₂R₁₅, -OR₁₅ wherein R₁₅ is a straight or branched alkyl of from 1 to 6 carbons, phenyl, or benzyl, and R₁ to R₈ are not simultaneously hydrogen.

United States Patent Number 5,929,116 describes endothelin antagonists of formulas 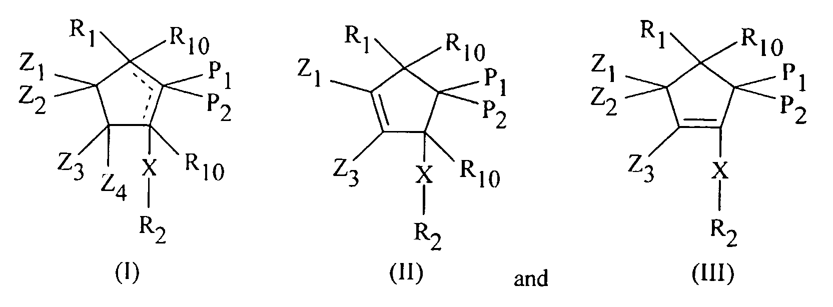 wherein
- R₁: is -X(CH₂)ₙAr;
- R₂: is Ar;
- P₁: is -X(CH₂)ₙR₈;
- P₂: is -X(CH₂)ₙR₈, or -XR₉Y;
- R₃ and R₅: are independently hydrogen, R₁₁, OH, C₁₋₈ alkoxy, S(O)_{q}R₁₁, N(R₆)₂, Br, F, I, Cl, CF₃, NHCOR₆, -R₁₁CO₂R₇, -XR₉-Y, or -X(CH₂)ₙR₈ wherein each methylene group within -X(CH₂)ₙR₈ may be unsubstituted or substituted by one or two -(CH₂)ₙAr groups;
- R₄: is hydrogen, R₁₁, 01-1, C₁₋₅ alkoxy, S(O)_{q}R₁₁, N(R₆)₂, -X(R₁₁), Br, F, I, Cl, or NHCOR₆ wherein the C₁₋₅ alkoxy may be unsubstituted or substituted by OH, methoxy, or halogen;
- R₆: is independently hydrogen or C₁₋₄ alkyl;
- R₇: is independently hydrogen, C₁₋₆ alkyl, or (CH₂)ₙAr;
- R₈: is hydrogen, R₁₁, CO₂R₇ PO₃H₂, SO₂NR₇R₁₁, NR₇SO₂R₁₁, P(O)(OH)R₇, CN, -C(O)N(R₆)₂, tetrazole, or OR₆;
- R₉: is C₁₋₁₀ alkyl, C₂₋₁₀ alkenyl, or phenyl, all of which may be unsubstituted or substituted by one or more OH, N(R₆)₂, COOH, >C=O, halogen, or XC₁₋₅ alkyl;
- R₁₀: is R₃ or R₄;
- R₁₁: is C₁₋₈ alkyl, C₂₋₈ alkenyl, C₂₋₈ alkynyl, all of which may be unsubstituted or substituted by one or more OH, CH₂OH, N(R₆)₂, or halogen;
- X: is (CH₂)ₙ, O, NR₆, or S(O)_{q};
- Y: is CH₃ or X(CH₂)ₙAr;
- Ar: is: 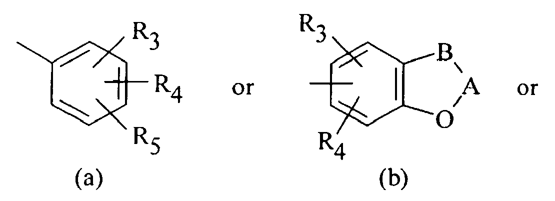 naphthyl, indolyl, pyridyl, thienyl, oxazolidinyl, oxazolyl, thiazolyl, isothiazolyl, pyrazolyl, triazolyl, tetrazolyl, imidazolyl, imidazolidinyl, thiazolidinyl, isoxazolyl, oxadiazolyl, thiadiazolyl, morpholinyl, piperidinyl, piperazinyl, pyrrolyl, or pyrimidyl; all of which may be unsubstituted or substituted by one or more R₃ or R₄ groups;
- A: is >C=O, or [C(R₆)₂]ₘ;
- B: is -CH₂- or -O-;
- Z₁, Z₂, Z₃, and Z₄: are independently hydrogen, C₁₋₈ alkyl, C₂₋₈ alkenyl, C₂₋₈ alkynyl, OH, C₁₋₈ alkoxy, S(O)_{q}C₁₋₈ alkyl, N(R₆)₂, Br, F, I, Cl, NHCOR₆, -X(CH₂)ₙR₈, XR₉Y, phenyl, benzyl, or C₃₋₆ cycloalkyl wherein the C₁₋₈ alkyl, C₂₋₈ alkenyl, or C₂₋₈ alkynyl may be optionally substituted by COOH, OH, CO(CH₂)ₙCH₃, CO(CH₂)ₙCH₂N(R₆)₂, or halogen;
- q: is zero, one, or two;
- n: is an integer from 0 to 6;
- m: is 1, 2, or 3;
and the dotted line in Formula (I) indicates the optional presence of a double bond; or a pharmaceutically acceptable salt thereof; provided that
- when the optional double bond is present, there is only one R₁₀, there is no P₁, and P₂ is not NR₆R₉Y;
- X is not NR₆, and Z₃ is not OH or N(R₆)₂ in Formula (III);
- Z₁ and Z₃ are not OH, N(R₆)₂, or Iodine in Formula (II);
- when the optional double bond is present in Formula (I) and X-R₂ is attached to the double bond, X is not NR₆;
- when the optional double bond is present in Formula (I) and R₁ is attached directly to the double bond, R₁ is not NR₆Ar;
- when R₃, R₅, Z₁, Z₂, or Z₃ is X(CH₂)ₙR₈ and n is not zero, X is oxygen or NR₆ when R₈ is OR₆ or CO₂H.

Also included in the invention are pharmaceutically acceptable salts of the active compounds.

Most or all of the desired pharmacological activity of a compound comprised of two or more stereoisomers frequently resides in just one of the stereoisomers. The other stereoisomer(s) typically is inactive at best or exhibits undesirable side effects such as, for example, toxicity. Therefore where a compound is comprised of two or more stereoisomers, it is important, and sometimes mandatory, to develop a method of selectively preparing the beneficial stereoisomer in a form that is free from, or almost free from, contamination by the other inactive or harmful stereoisomer(s). However, usually it is very difficult to discover a method for the preparation of a beneficial stereoisomer in a form that is free from, or almost free from, contamination by the other inactive or harmful stereoisomer(s). Unexpectedly, we have invented novel preparations of certain important 3-substituted cyclopentyl-containing, amino acid analogs of gabapentin, a marketed anticonvulsant, which provide the desirable stereoisomers with a high degree of stereochemical purity.

None of the above teach the synthesis of the instant invention.

### SUMMARY OF THE INVENTION

The instant invention encompasses novel synthetic routes for the preparation of important 3-substituted cyclopentyl-based analogs of gabapentin and pharmaceutically acceptable salts thereof. Gabapentin, marketed under the trade name Neurontin® for the treatment of seizure disorders, particularly epilepsy, provides well-known medical benefits to patients in need of such treatment. The instant invention encompasses novel synthetic routes for the preparation of 3-substituted cyclopentyl-based analogs of gabapentin and pharmaceutically acceptable salts thereof that enable the synthesis of each stereoisomer of these analogs with a high degree of stereochemical purity. These routes provide access to pure stereoisomers of Formulas I, II, III, and IV 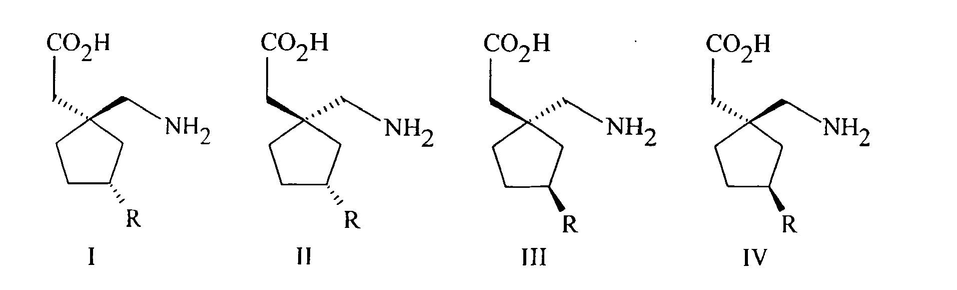 wherein R is C₁-C₁₀ alkyl or C₃-C₁₀ cycloalkyl.

Further, the invention encompasses the key intermediates of formulas (6) and (26). Still further, the invention provides novel synthetic routes for the preparation of compounds of formulas (6) and (26). The routes enable the synthesis of each stereoisomer of compounds of formulas (6) and (26) with a high degree of stereochemical purity. These routes provide access to pure stereoisomers of formulas (6) and (26) wherein R is C₁-C₁₀ alkyl or C₃-C₁₀ cycloalkyl.

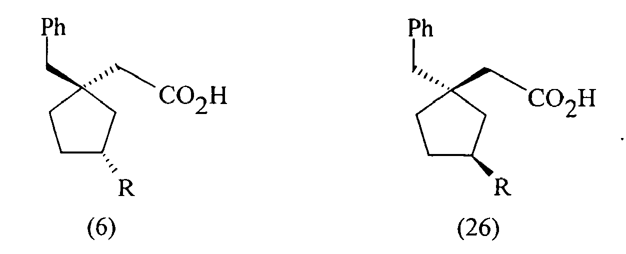

The invention provides a process for the preparation of a compound of Formula I 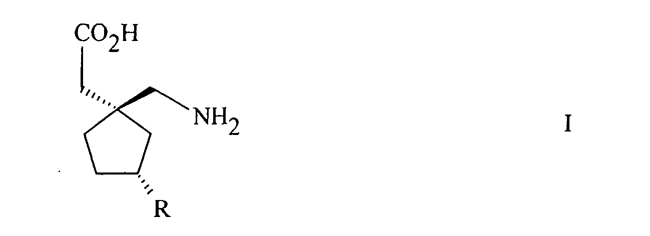 wherein R is C₁-C₁₀ alkyl or C₃-C₁₀ cycloalkyl, and pharmaceutically acceptable salts thereof, which comprises:
a) adding a cyanoacetate of formula (A) 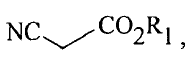 wherein R₁ is alkyl or benzyl, to a mixture of a chiral cyclopentanone of formula (1) 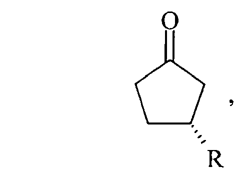 a solvent, a carboxylic acid, and a Knoevenagel reaction catalyst, and stirring the mixture in the presence of a means of removing water to produce the alkene of formula (2) 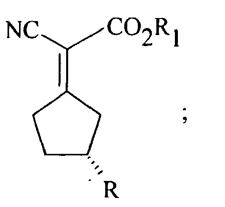
b) adding the product of Step a) above to a mixture of benzylmagnesium chloride, benzylmagnesium bromide, or benzylmagnesium iodide, in a solvent to produce the addition products of formulas (3a) 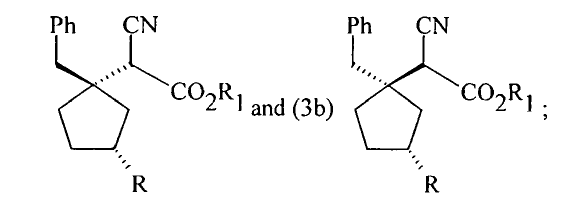
c) adding the products of Step b) above to a mixture of a base selected from potassium hydroxide, sodium hydroxide, lithium hydroxide, or cesium hydroxide in a solvent and stirring, and then acidifying to produce the carboxylic acids of formulas (4a) 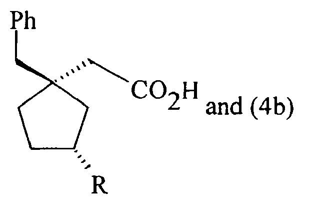 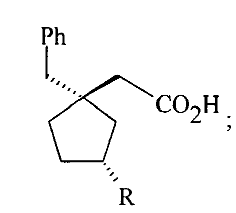 or adding the products of Step b) above to an acid mixture and stirring to produce the carboxylic acids of formulas (4a) 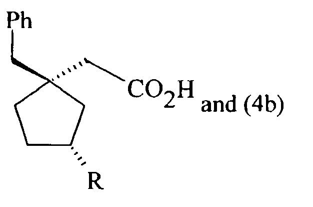 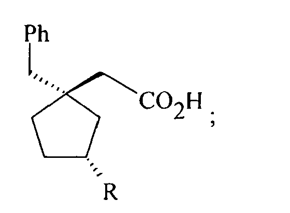
d) contacting the products of Step c) above with an amine in a solvent, and recrystallizing the salt so formed to produce the enriched diastereomer of formula (5) 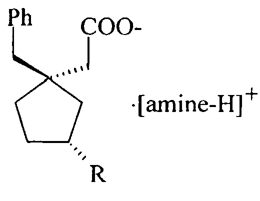 as the amine salt;
e) converting the product of Step d) to a carboxylic acid of formula (6) 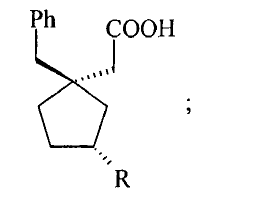
f) adding the product of Step e) to a mixture of iodomethane, a solvent, and a base, and stirring to produce the ester of formula (7) 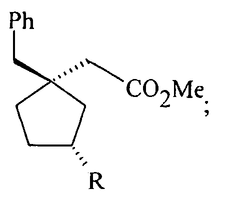 or adding the product of Step e) to methanol and an acid to produce the ester of formula (7) 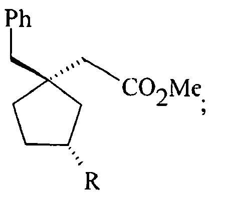 or adding the product of Step e) above to trimethylsilyldiazo-methane and methanol in a solvent to produce the ester of formula (7) 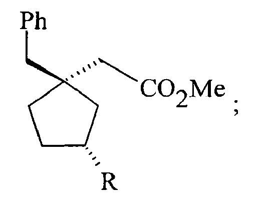 or adding the product of Step e) to a solution of diazomethane or trimethylsilyl-diazomethane in a solvent to produce ester of formula (7) 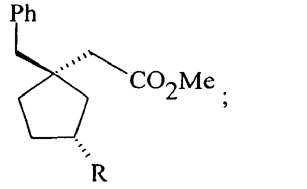
g) adding the product of Step f) to a mixture of carbon tetrachloride or ethyl acetate, and acetonitrile, water, sodium periodate, and ruthenium(III) chloride, and stirring to produce the carboxylic acid of formula (8) 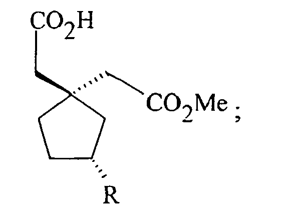
h) adding the product of Step g) to a mixture of a tertiary amine base, a solvent, and diphenylphosphoryl azide (DPPA), and stirring to produce the isocyanate of formula (9) 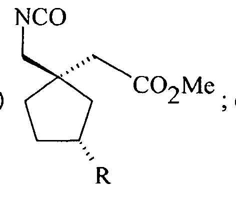 or adding the product of Step g) above to ethyl chloroformate or isobutyl chloroformate and a base in a solvent at a temperature of from -40°C to 78°C, followed by adding a solution of sodium azide in water and tetrahydrofuran or acetone, followed by adding toluene or benzene, and refluxing to produce the isocyanate of formula (9) 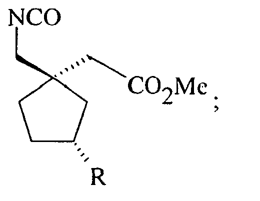
i) adding the product of Step h) to a mixture of a solvent and methanol, and stirring to produce the carbamate of formula (10) 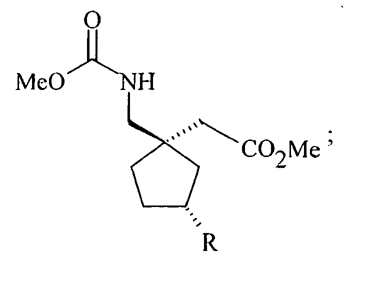
j) adding the product of Step i) to a mixture of a solvent and aqueous hydrochloric acid, and stirring to produce a compound of formula (la) 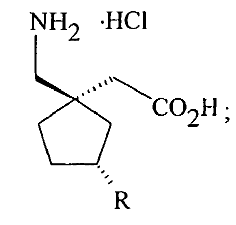 and
k) converting the product of Step j) to a compound of formula (I) 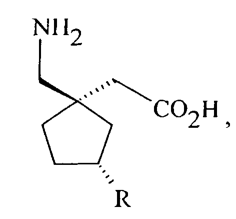 and further converting, if desired, to a pharmaceutically acceptable salt by known means.

This process is outlined in Scheme 1.

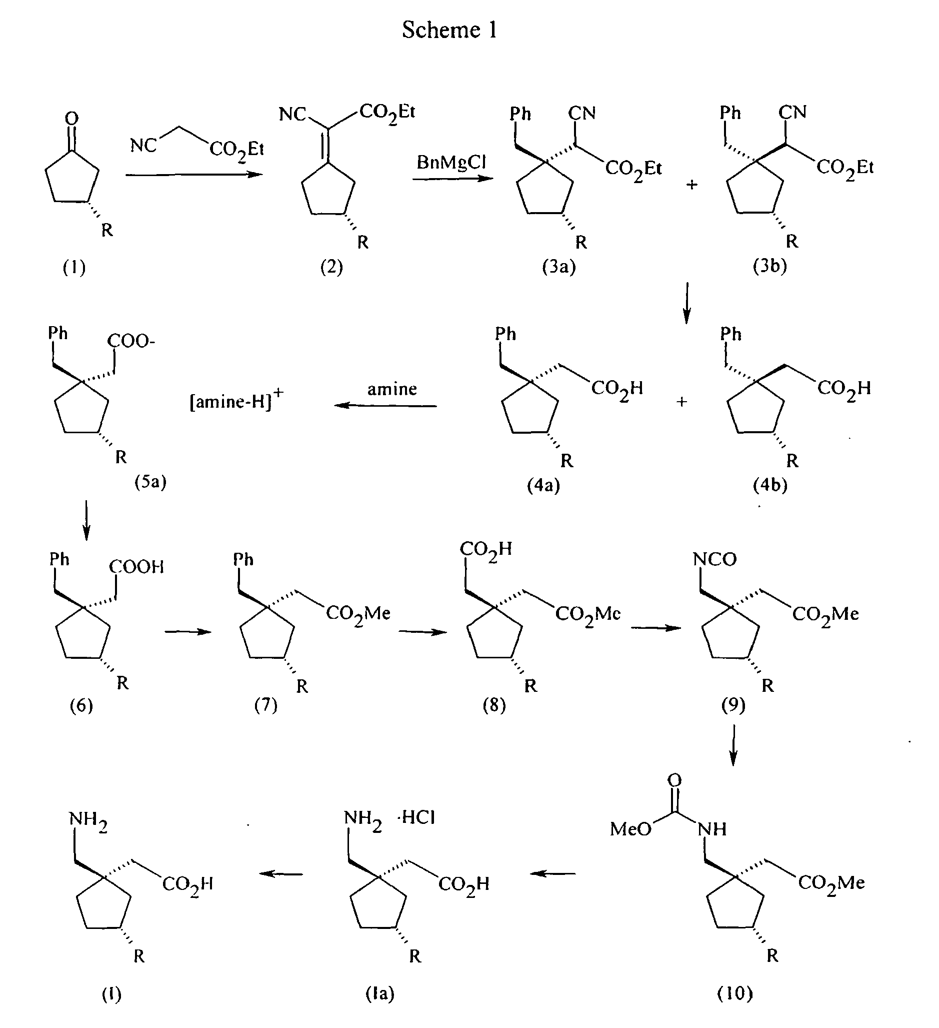

Preferred is a process for the preparation of a compound of Formula I wherein R is C₁-C₁₀ alkyl or C₃-C₁₀ cycloalkyl, and pharmaceutically acceptable salts thereof, which comprises:
a) adding a cyanoacetate of formula (A) 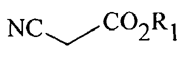 wherein R₁ is selected from methyl, ethyl, *n*-propyl, *iso*-propyl, *n*-butyl, *iso*-butyl, *sec*-butyl, *tert*-butyl, and benzyl to a mixture of a chiral cyclopentanone of formula (1) 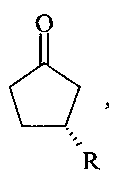 a solvent selected from tetrahydrofuran, 1,4-dioxane, *tert*-butylmethylether, chloroform, dichloromethane, acetonitrile, ethyl ether, ethyl acetate, hexanes, N,N-dimethylformamide, dimethylsulfoxide, ethanol, *tert*-butanol, toluene, benzene, xylenes, and *n*-heptane, acetic acid, and a Knoevenagel reaction catalyst selected from β-alanine, ammonium acetate, and piperidine, and stirring the mixture in the presence of a means of removing water selected from azeotropic distillation, activated molecular sieves, anhydrous magnesium sulfate, anhydrous sodium sulfate, anhydrous sodium carbonate, anhydrous potassium carbonate, anhydrous cesium carbonate, trimethyl orthoformate, and triethyl orthoformate to produce the alkene of formula (2) 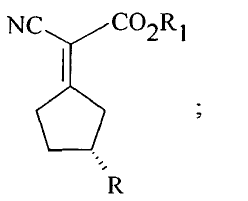
b) adding the product of Step a) above to a mixture of benzylmagnesium chloride, benzylmagnesium bromide, or benzylmagnesium iodide in a solvent selected from tetrahydrofuran, benzene, 1,4-dioxane, hexanes, *n*-heptane, toluene, diethyl ether, and *tert*-butyl methyl ether to produce the addition products of formulas (3a) 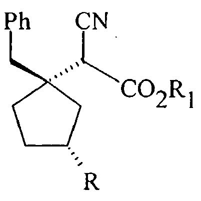 and 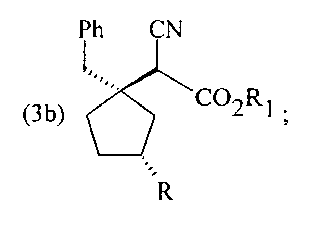
c) adding the products of Step b) above to a mixture of a base selected from potassium hydroxide, sodium hydroxide, lithium hydroxide, and cesium hydroxide in a solvent selected from ethylene glycol, 2-methoxyethyl ether, 1,4-dioxane, and diethylene glycol, and stirring the mixture, and then acidifying to produce the carboxylic acids of formulas (4a) 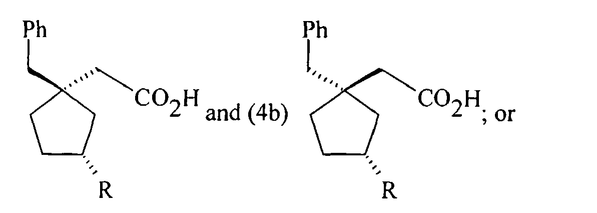 adding the products of Step b) above to an acid mixture selected from 6-12 M HCl, 12 M H₂SO₄, 10%-48% wt/wt hydrobromic acid, and HBr in aqueous acetic acid, and stirring to produce the carboxylic acids of formulas (4a) 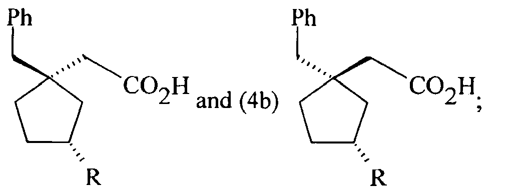
d) contacting the products of Step c) above with an amine selected from (S)-α-methyl-benzylamine, (R)-α-methyl-benzylamine, (R)-(+)-1-(naphthyl)ethylamine, (S)-(+)-1-(naphthyl)ethylamine, triethylamine, diisopropylethylamine, dicyclohexylamine, benzylamine, dibenzylamine, morpholine, N-methylmorpholine, piperidine, N-methylpiperidine, and pyridine in a solvent selected from N,N-dimethylformamide, chloroform, benzene, xylenes, hexanes, acetone, ethanol, methanol, *iso*-propanol, diethyl ether, dichloromethane, benzene, toluene, n-pentane, n-hexane, n-heptane, ethyl acetate, acetonitrile, *tert*-butyl methyl ether, tetrahydrofuran, and 1,4-dioxane, and recrystallizing the salt so formed to produce the enriched diastereomer of formula (5) 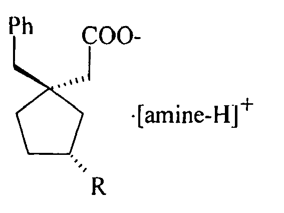 as the amine salt;
e) adding the product of Step d) to a mixture selected from aqueous hydrochloric acid, aqueous sulfuric acid, aqueous acetic acid, hydrochloric acid dissolved in acetic acid, or hydrochloric acid dissolved in acetic acid to which water is added and stirring to produce the carboxylic acid of formula (6) 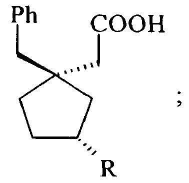 or partitioning the product of Step d) between a mixture of aqueous hydrochloric acid and a solvent selected from chloroform, dichloromethane, ethyl acetate, ethyl ether, tetrahydrofuran, 1,4-dioxane, toluene, and *tert*-butylmethylether, and drying and evaporating the organic layer to produce the carboxylic acid of formula (6) 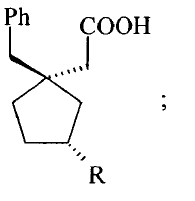
f) adding the product of Step e) above to a mixture of iodomethane, a solvent selected from dichloromethane, chloroform, tetrahydrofuran, toluene, and 1,4-dioxane, and a base selected from 1,8-diazabicyclo[5.4.0]undec-7-ene (DBU), diisopropylethylamine, triethylamine, and 1,5-diazabicyclo[4.3.0]non-5-ene (DBN), and stirring at a temperature of from -40°C to 110°C to produce the ester of formula (7) 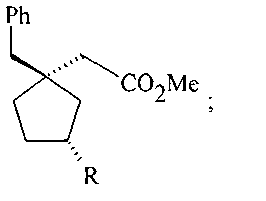 or adding the product of Step e) above to a mixture of methanol and concentrated sulphuric acid, concentrated hydrochloric acid, or hydrogen chloride at a temperature of from 0°C to 100°C to produce the ester of formula (7) 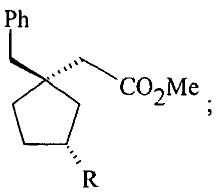 or adding the product of Step e) above to trimethylsilyldiazomethane and methanol in benzene or toluene at a temperature of from -40°C to 100°C to produce the ester of formula (7) 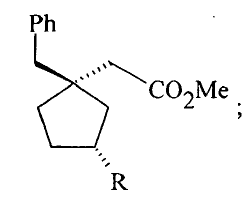 or adding the product of Step e) above to diazomethane or trimethylsilyldiazomethane in a solvent selected from benzene, toluene, dichloromethane, and diethyl ether at a temperature of from -40°C to 40°C to give a compound of formula (7) 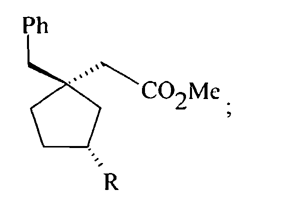
g) adding the product of Step f) to a mixture of carbon tetrachloride or ethyl acetate, and acetonitrile, water, sodium periodate, and ruthenium(III) chloride, and stirring at a temperature from -40°C to 80°C to produce the carboxylic acid of formula (8) 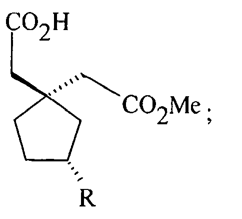
h) adding the product of Step g) above to a mixture of a base selected from triethylamine and diisopropylethylamine, a solvent selected from toluene, benzene, xylenes, tetrahydrofuran, diethyl ether and *n*-heptane, and diphenylphosphoryl azide (DPPA), and stirring at a temperature of from 0°C to 150°C to produce the isocyanate of formula (9) 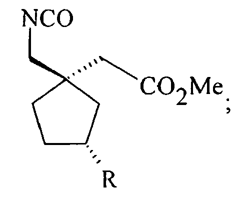 or adding the product of Step g) above to ethyl chloroformate or isobutyl chloroformate, a base selected from triethylamine and diisopropylethylamine, and a solvent selected from tetrahydrofuran, acetone, and diethyl ether at a temperature of from -40°C to 78°C, followed by adding a solution of sodium azide in water and tetrahydrofuran or acetone, followed by adding toluene or benzene, and refluxing to produce the isocyanate of formula (9) 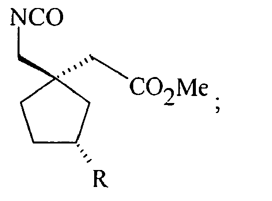
i) adding the product of Step h) to a mixture of a solvent selected from toluene, benzene, xylenes and *n*-heptane, and methanol, and stirring at a temperature from 0°C to 150°C to produce the carbamate of formula (10) 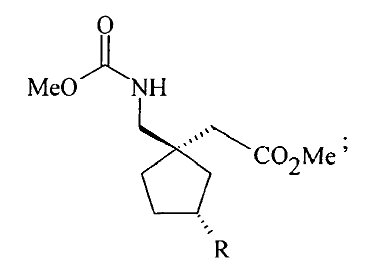
j) adding the product of Step i) to a mixture of a solvent selected from water, acetic acid, and 1,4-dioxane, and aqueous hydrochloric acid at a concentration of from 0.01 M to 12 M, and stirring at a temperature from 0°C to 115°C to produce a compound of formula Ia 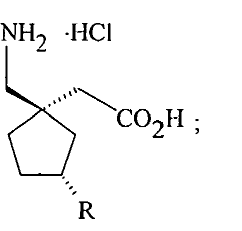 and
k) converting the product of Step j) to a compound of Formula I 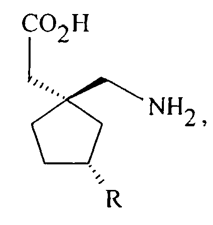 and further converting, if desired, to a pharmaceutically acceptable salt by known means.

More preferred is a process for the preparation of a compound of Formula I wherein R is C₁-C₁₀ alkyl or C₃-C₁₀ cycloalkyl, and pharmaceutically acceptable salts thereof, which comprises:
a) adding a cyanoacetate of formula (A) 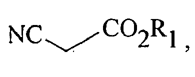 wherein R₁ is ethyl, to a mixture of a chiral cyclopentanone of formula (1) 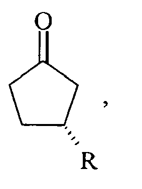 toluene, acetic acid, and a Knoevenagel reaction catalyst which is ammonium acetate, and heating the mixture at reflux over a Dean-Stark trap to produce the alkene of formula (2) 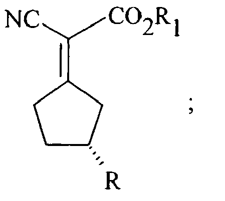
b) adding the product of Step a) above to a mixture of benzylmagnesium chloride in dry tetrahydrofuran at -100°C to 25°C to produce the addition products of formulas (3a) 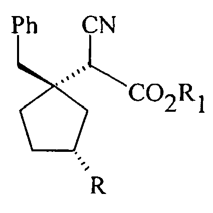 and (3b) 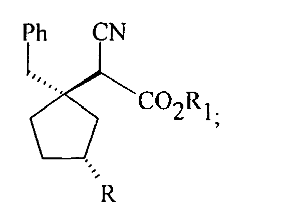
c) adding the products of Step b) above to a mixture of potassium hydroxide in ethylene glycol, and heating the mixture at 100°C to 200°C, and then acidifying to produce the hydrolysis products of formulas (4a) 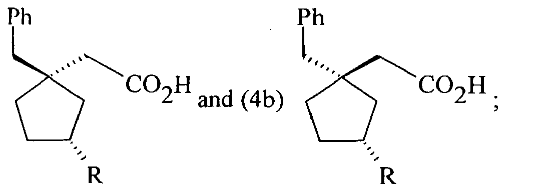
d) contacting the products of Step c) above with (S)-α-methyl-benzylamine in ethyl acetate, and recrystallizing the salt so formed from ethyl acetate to produce the enriched diastereomer of formula (5) 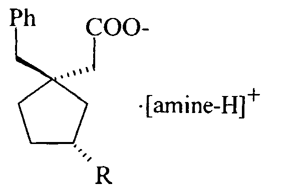 as the (S)-α-methyl-benzylamine salt;
e) adding the product of Step d) to aqueous hydrochloric acid and stirring to produce the carboxylic acid of formula (6) 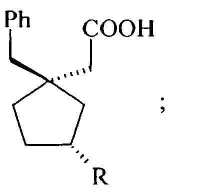
f) adding the product of Step e) to a mixture of iodomethane, dichloromethane, and 1,8-diazabicyclo[5.4.0]undec-7-ene (DBU), and stirring to produce the ester of formula (7) 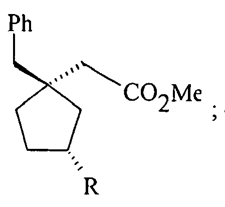 or adding the product of Step e) to methanol and concentrated sulfuric acid to produce the ester of formula (7) 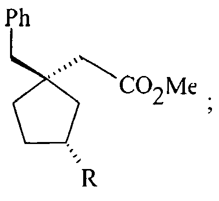 or adding the product of Step e) to a solution of diazomethane or trimethylsilyldiazomethane in dichloromethane to produce the ester of formula (7) 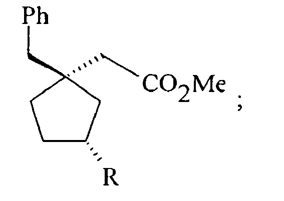
g) adding the product of Step f) to a mixture of carbon tetrachloride or ethyl acetate, and acetonitrile, water, sodium periodate, and ruthenium(III) chloride, and stirring to produce the carboxylic acid of formula (8) 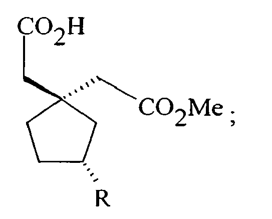
h) adding the product of Step g) to a mixture of triethylamine, toluene, and diphenylphosphoryl azide (DPPA), and refluxing to produce the isocyanate of formula (9) 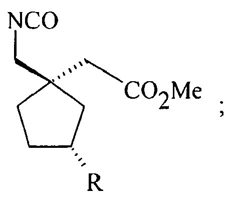 or adding the product of Step g) above to ethyl chloroformate or isobutyl chloroformate and triethylamine in tetrahydrofuran at a temperature of from -40°C to 78°C, followed by adding a solution of sodium azide in water and tetrahydrofuran, followed by adding toluene or benzene, and refluxing to produce ester of formula (9) 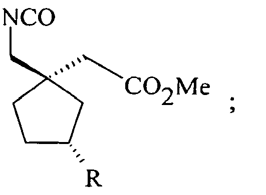
i) adding the product of Step h) to a mixture of methanol and toluene, and refluxing to produce the carbamate of formula (10) 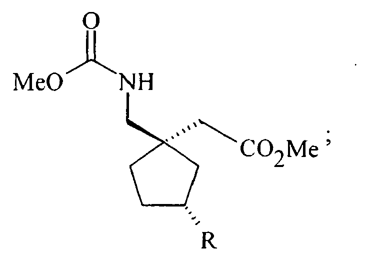
j) adding the product of Step i) to a mixture of 1,4-dioxane and aqueous hydrochloric acid at a concentration of 6 M, and stirring to produce a compound of formula Ia 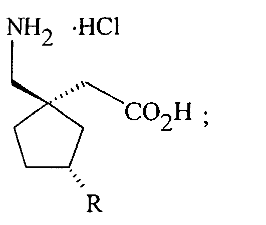
k) converting the product of Step j) to a compound of Formula I 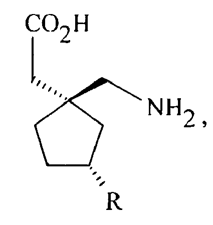 and further converting, if desired, to a pharmaceutically acceptable salt by known means.

Also preferred is a process for the preparation of a compound of Formula I as described above, further characterized in that the intermediate product (9) 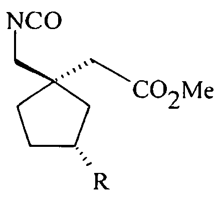 formed is reacted, without isolation, with methanol to produce the carbamate of formula (10)

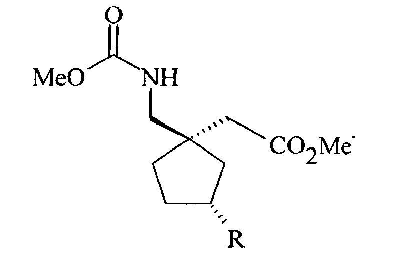

Further, the invention provides a process for the preparation of a compound of Formula II 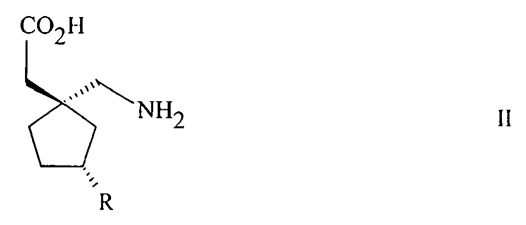 wherein R is C₁-C₁₀ alkyl or C₃-C₁₀ cycloalkyl, and pharmaceutically acceptable salts thereof, which comprises:
a) adding a cyanoacetate of formula (A) 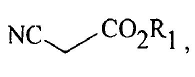 wherein R₁ is alkyl or benzyl, to a mixture of a chiral cyclopentanone of formula (1) 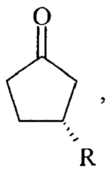 a solvent, a carboxylic acid, and a Knoevenagel reaction catalyst, and stirring the mixture in the presence of a means of removing water to produce the alkene of formula (2) 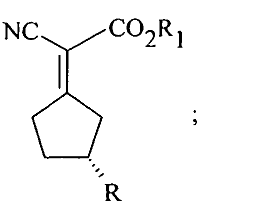
b) adding the product of Step a) above to a mixture of benzylmagnesium chloride, benzylmagnesium bromide, or benzylmagnesium iodide, in a solvent to produce the addition products of formulas (3a) 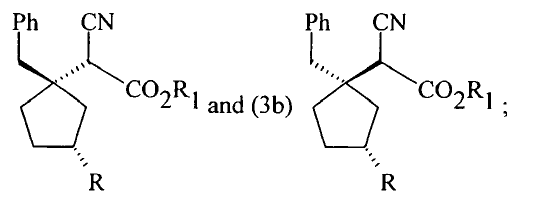
c) adding the products of Step b) above to a mixture of a base selected from potassium hydroxide, sodium hydroxide, lithium hydroxide, and cesium hydroxide and a solvent, and stirring, and then acidifying to produce the carboxylic acids of formulas (4a) 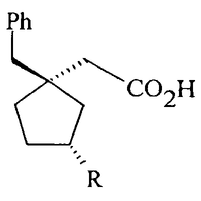 and (4b) 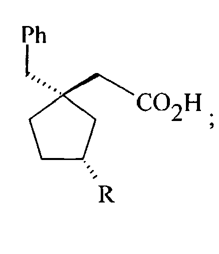 or adding the products of Step b) above to an acid mixture and stirring to produce the carboxylic acids of formulas (4a) 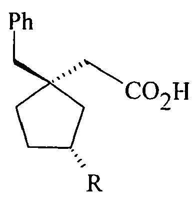 and (4b) 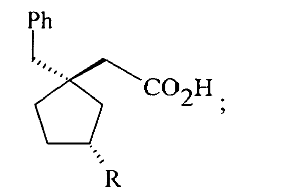
d) contacting the products of Step c) above with an amine in a solvent, and recrystallizing the salt so formed to produce the enriched diastereomer of formula (5) 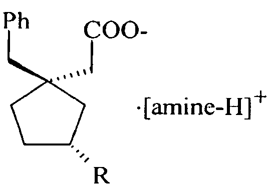 as the amine salt; and
e) converting the product of Step d) to a carboxylic acid of formula (6) 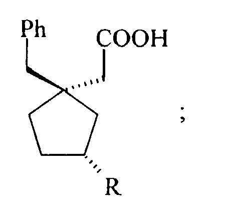
f) adding the product of Step e) to a mixture of a tertiary amine base, a solvent, and diphenylphosphoryl azide (DPPA), and stirring to produce the isocyanate of formula (11) 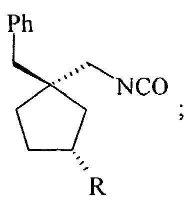 or adding the product of Step e) above to ethyl chloroformate or isobutyl chloroformate and a base in a solvent at a temperature of from -40°C to 78°C, followed by adding a solution of sodium azide in water and tetrahydrofuran or acetone, followed by adding toluene or benzene, and refluxing to produce isocyanate of formula (11) 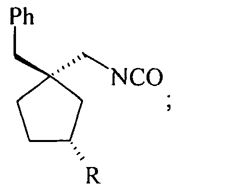
g) adding the product of Step f) to a mixture of a solvent and methanol, and stirring to produce the carbamate of formula (12) 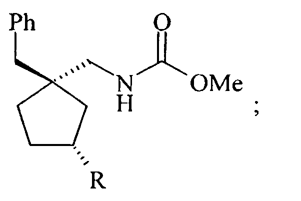
h) adding the product of Step g) to a mixture of carbon tetrachloride or ethyl acetate, and acetonitrile, water, sodium periodate, and ruthenium(III) chloride, and stirring to produce the carboxylic acid of formula (13) 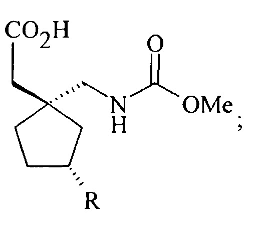
i) adding the product of Step h) to a mixture of a solvent and aqueous hydrochloric acid, and stirring to produce a compound of formula (IIa) 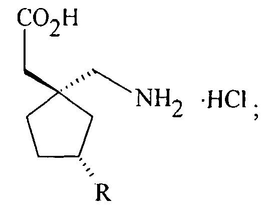
j) converting the product of Step i) to a compound of formula (II) 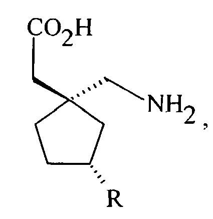 and further converting, if desired, to a pharmaceutically acceptable salt by known means.

This process is outlined below in Scheme 2.

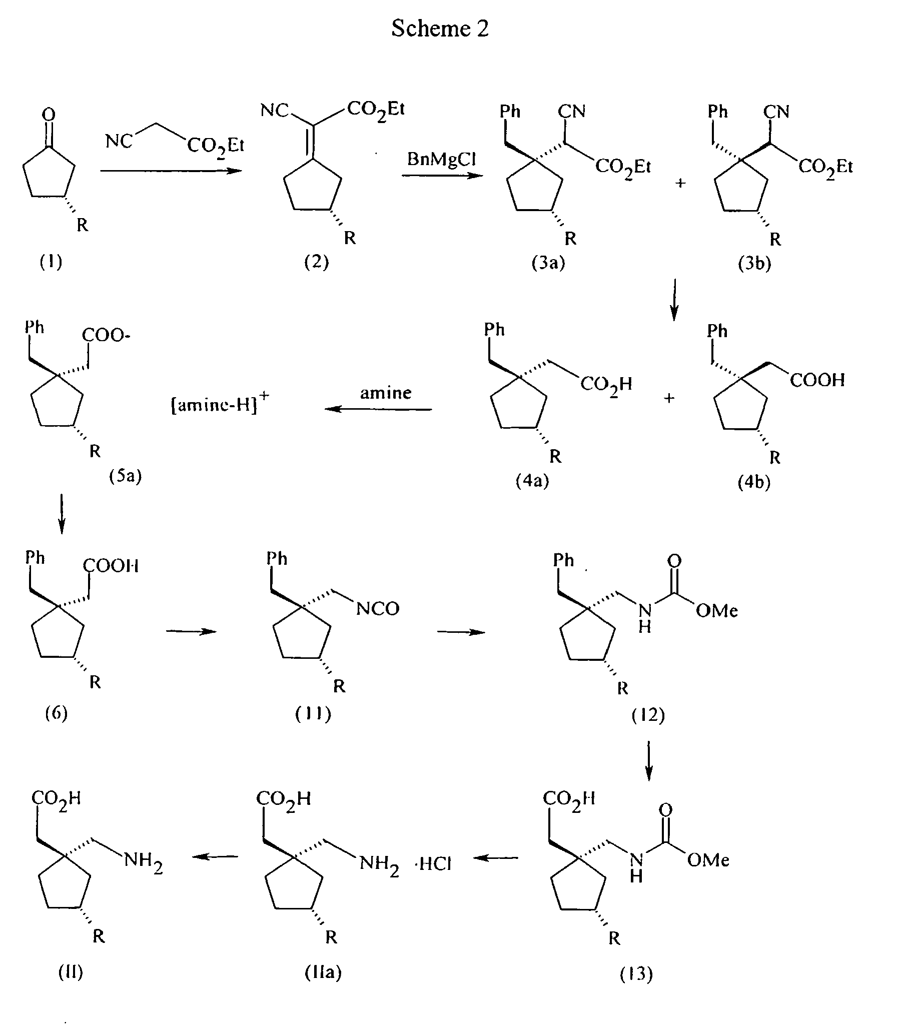

Preferred is a process for the preparation of a compound of Formula II wherein R is C₁-C₁₀ alkyl or C₃-C₁₀ cycloalkyl, and pharmaceutically acceptable salts thereof, which comprises:
a) adding a cyanoacetate of formula (A) 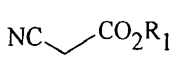 wherein R₁ is selected from methyl, ethyl, *n*-propyl, *iso*-propyl, *n*-butyl, *iso*-butyl, *sec*-butyl, *tert*-butyl, and benzyl to a mixture of a chiral cyclopentanone of formula (1) 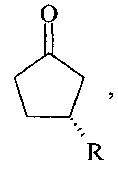 a solvent selected from tetrahydrofuran, 1,4-dioxane, *tert*-butylmethylether, chloroform, dichloromethane, acetonitrile, ethyl ether, ethyl acetate, hexanes, N,N-dimethylformamide, dimethylsulfoxide, ethanol, *tert*-butanol, toluene, benzene, xylenes, and *n*-heptane, acetic acid, and a Knoevenagel reaction catalyst selected from β-alanine, ammonium acetate, and piperidine, and stirring the mixture in the presence of a means of removing water selected from azeotropic distillation, activated molecular sieves, anhydrous magnesium sulfate, anhydrous sodium sulfate, anhydrous sodium carbonate, anhydrous potassium carbonate, anhydrous cesium carbonate, trimethyl orthoformate, and triethyl orthoformate to produce the alkene of formula (2) 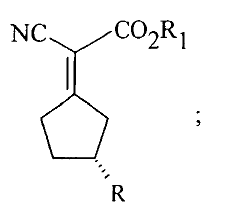
b) adding the product of Step a) above to a mixture of benzylmagnesium chloride, benzylmagnesium bromide, or benzylmagnesium iodide in a solvent selected from tetrahydrofuran, benzene, 1,4-dioxane, hexanes, *n*-heptane, toluene, diethyl ether, and *tert*-butyl methyl ether to produce the addition products of formulas (3a) 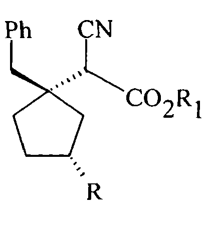 and 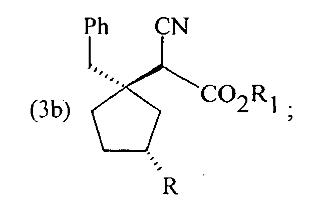
c) adding the products of Step b) above to a mixture of a base selected from potassium hydroxide, sodium hydroxide, lithium hydroxide, and cesium hydroxide in a solvent selected from ethylene glycol, 2-methoxyethyl ether, 1,4-dioxane, and diethylene glycol, and stirring the mixture and then acidifying to produce the carboxylic acids of formulas (4a) 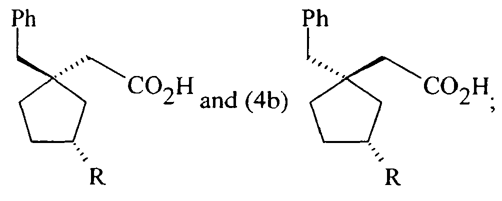 or adding the products of Step b) above to an acid mixture selected from 6-12 M HCl, 12 M H₂SO₄, 10%-48% wt/wt hydrobromic acid, and HBr in aqueous acetic acid, and stirring to produce the carboxylic acids of formulas (4a) 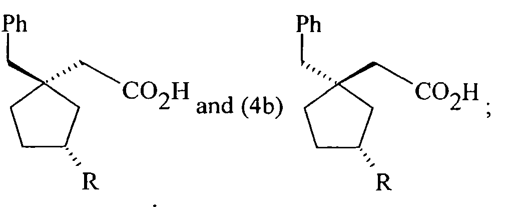
d) contacting the products of Step c) above with an amine selected from (S)-α-methyl-benzylamine, (R)-α-methyl-benzylamine, (R)-(+)-1-(naphthyl)ethylamine, (S)-(+)-1-(naphthyl)ethylamine, triethylamine, diisopropylethylamine, dicyclohexylamine, benzylamine, dibenzylamine, morpholine, N-methylmorpholine, piperidine, N-methylpiperidine, and pyridine in a solvent selected from N,N-dimethylformamide, chloroform, benzene, xylenes, hexanes, acetone, ethanol, methanol, *iso-*propanol, diethyl ether, dichloromethane, benzene, toluene, n-pentane, n-hexane, n-heptane, ethyl acetate, acetonitrile, *tert*-butyl methyl ether, tetrahydrofuran, and 1,4-dioxane, and recrystallizing the salt so formed to produce the enriched diastereomer of formula (5) 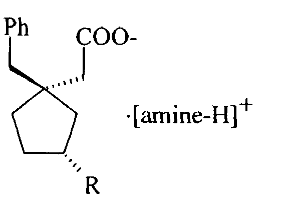 as the amine salt;
e) adding the product of Step d) to a mixture selected from aqueous hydrochloric acid, aqueous sulfuric acid, aqueous acetic acid, hydrochloric acid dissolved in acetic acid, and hydrochloric acid dissolved in acetic acid and water, and stirring to produce the carboxylic acid of formula (6) 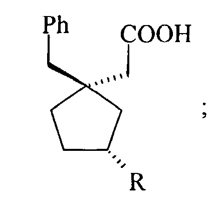 or partitioning the product of Step d) between a mixture of aqueous hydrochloric acid and a solvent selected from chloroform, dichloromethane, ethyl acetate, ethyl ether, tetrahydrofuran, 1,4-dioxane, toluene, and *tert*-butylmethylether, and drying and evaporating the organic layer to produce the carboxylic acid of formula (6) 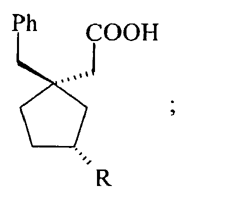
f) adding the product of Step e) above to a mixture of a base selected from triethylamine and diisopropylethylamine, a solvent selected from toluene, benzene, xylenes, tetrahydrofuran, diethyl ether and *n*-heptane, and diphenylphosphoryl azide (DPPA), and stirring at a temperature of from 0°C to 150°C to produce the isocyanate of formula (11) 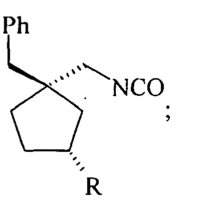 or adding the product of Step e) above to ethyl chloroformate or isobutyl chloroformate and a base selected from triethylamine and diisopropylethylamine, and a solvent selected from tetrahydrofuran, acetone, and diethyl ether at a temperature of from -40°C to 78°C, followed by adding a solution of sodium azide in water and tetrahydrofuran or acetone, followed by adding toluene or benzene, and refluxing to produce the isocyanate of formula (11) 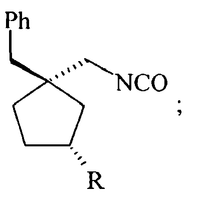
g) adding the product of Step f) to a solvent selected from toluene, benzene, xylenes, and *n*-heptane, and methanol, and stirring at a temperature from 0°C to 150°C to produce the carbamate of formula (12) 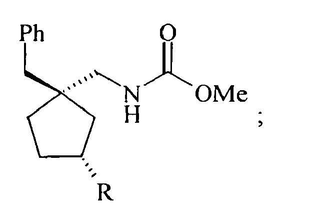
h) adding the product of Step g) to a mixture of carbon tetrachloride or ethyl acetate, and acetonitrile, water, sodium periodate, and ruthenium(III) chloride, and stirring at a temperature from -40°C to 80°C to produce the carboxylic acid of formula (13) 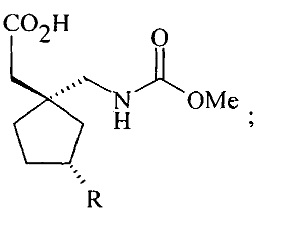
i) adding the product of Step h) to a mixture of a solvent selected from water, acetic acid, and 1,4-dioxane, and aqueous hydrochloric acid at a concentration of from 0.01 M to 12 M, and stirring at a temperature from 0°C to 115°C to produce a compound of formula IIa 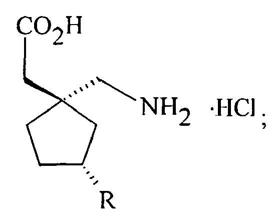 and
j) converting the product of Step i) to a compound of Formula II 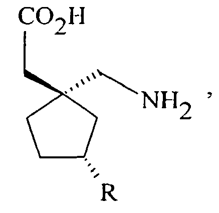 and further converting, if desired, to a pharmaceutically acceptable salt by known means.

More preferred is a process for the preparation of a compound of Formula II wherein R is C₁-C₁₀ alkyl or C₃-C₁₀ cycloalkyl, and pharmaceutically acceptable salts thereof, which comprises:
a) adding a cyanoacetate of formula (A) 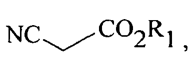 wherein R₁ is ethyl, to a mixture of a chiral cyclopentanone of formula (1) 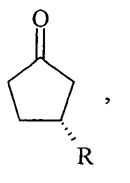 toluene, acetic acid, and a Knoevenagel reaction catalyst which is ammonium acetate, and heating the mixture at reflux over a Dean-Stark trap to produce the alkene of formula (2) 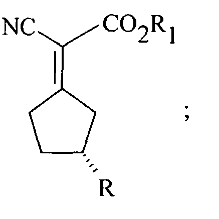
b) adding the product of Step a) above to a mixture ofbenzylmagnesium chloride in dry tetrahydrofuran at -100°C to 25°C to produce the addition products of formulas (3a) 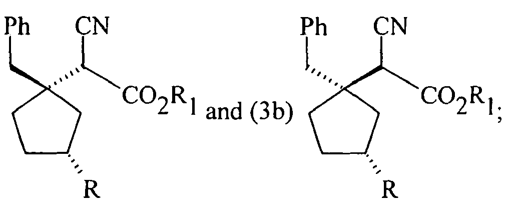
c) adding the products of Step b) above to a mixture of potassium hydroxide in ethylene glycol, and heating the mixture at 100°C to 200°C, and then acidifying to produce the hydrolysis products of formulas (4a) 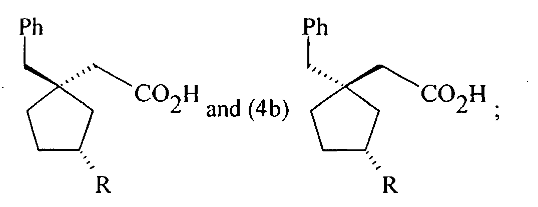
d) contacting the products of Step c) above with (S)-α-methyl-benzylamine in ethyl acetate, and recrystallizing the salt so formed from ethyl acetate to produce the enriched diastereomer of formula (5) 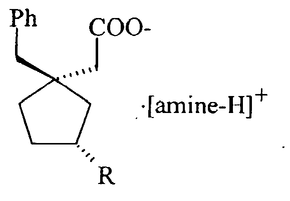 as the (S)-α-methyl-benzylamine salt;
c) adding the product of Step d) to aqueous hydrochloric acid and stirring to produce the carboxylic acid of formula (6) 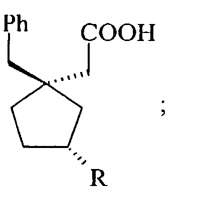
f) adding the product of Step e) to a mixture of triethylamine, toluene, and diphenylphosphoryl azide (DPPA), and refluxing to produce the isocyanate of formula (11) 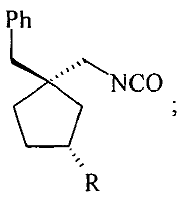 or adding the product of Step e) above to ethyl chloroformate or isobutyl chloroformate and triethylamine in tetrahydrofuran at a temperature of from -40°C to 78°C, followed by adding a solution of sodium azide in water and tetrahydrofuran or acetone, followed by adding toluene or benzene, and refluxing to produce isocyanate of formula (11) 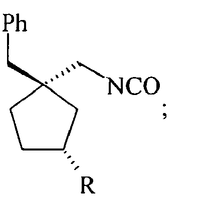
g) adding the product of Step f) to a mixture of methanol and toluene, and refluxing to produce the carbamate of formula (12) 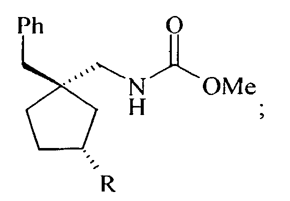
h) adding the product of Step g) to a mixture of carbon tetrachloride or ethyl acetate, and acetonitrile, water, sodium periodate, and ruthenium(III) chloride, and stirring to produce the carboxylic acid of formula (13) 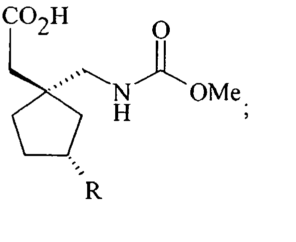
i) adding the product of Step h) to a mixture of 1,4-dioxane and aqueous hydrochloric acid at a concentration of 6 M, and stirring to produce a compound of formula IIa 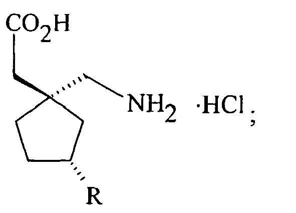
j) converting the product of Step i) to a compound of Formula II 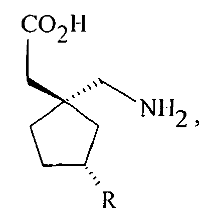 and further converting, if desired, to a pharmaceutically acceptable salt by known means.

Also preferred is a process for the preparation of a compound of Formula II as described above, further characterized in that the intermediate product (11) 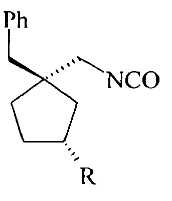 formed is further reacted, without isolation, with methanol to produce the carbamate of formula (12)

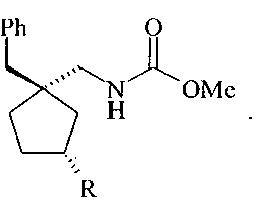

Still further, the invention provides a process for the preparation of a compound of Formula II 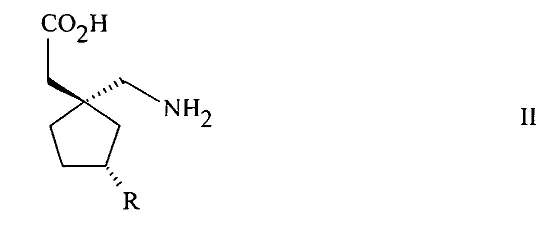 wherein R is C₁-C₁₀ alkyl or C₃-C₁₀ cycloalkyl, and pharmaceutically acceptable salts thereof, which comprises:
a) adding a cyanoacetate of formula (A) 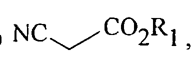 wherein R₁ is alkyl or benzyl, to a mixture of a chiral cyclopentanone of formula (1) 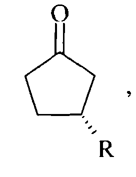 a solvent, a carboxylic acid, and a Knoevenagel reaction catalyst, and stirring the mixture in the presence of a means of removing water to produce the alkene of formula (2) 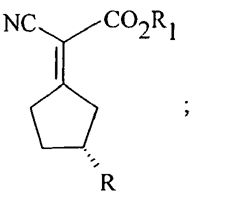
b) adding the product of Step a) above to a mixture of benzylmagnesium chloride, benzylmagnesium bromide, or benzylmagnesium iodide, in a solvent to produce the addition products of formulas (3a) 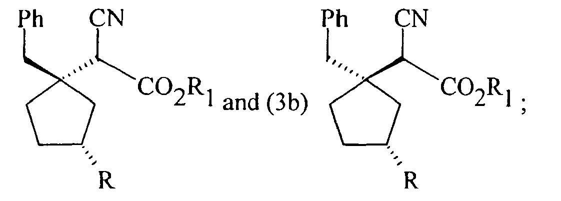
c) adding the products of Step b) above to a mixture of a base selected from potassium hydroxide, sodium hydroxide, lithium hydroxide, and cesium hydroxide, in a solvent, and stirring, and then acidifying to produce the carboxylic acids of formulas (4a) 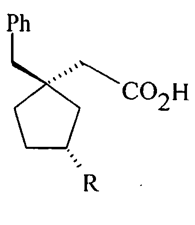 and (4b) 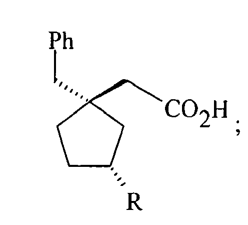 or adding the products of Step b) above to an acid mixture and stirring to produce the carboxylic acids of formulas (4a) 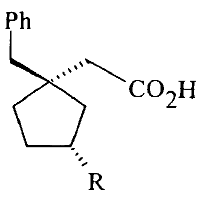 and (4b) 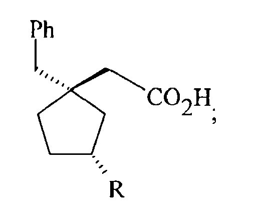
d) contacting the products of Step c) above with an amine in a solvent, and recrystallizing the salt so formed to produce the enriched diastereomer of formula (5) 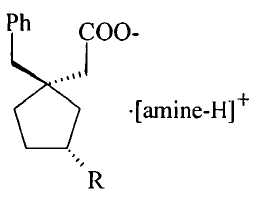 as the amine salt;
e) converting the product of Step d) to a carboxylic acid of formula (6) 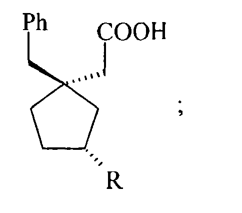
f) adding oxalyl chloride to a mixture of the product of Step e), a solvent, and N,N-dimethylformamide (DMF), and stirring to produce the acid chloride of formula (14) 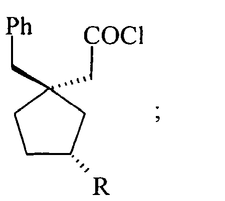
g) adding the product of Step f) to a mixture of *tert*-butyl alcohol, a solvent, and a tertiary amine base, and stirring to produce the ester of formula (15) 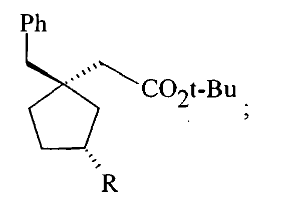
h) adding the product of Step g) to a mixture of carbon tetrachloride or ethyl acetate, and acetonitrile, water, sodium periodate, and ruthenium(III) chloride, and stirring to produce the carboxylic acid of formula (16) 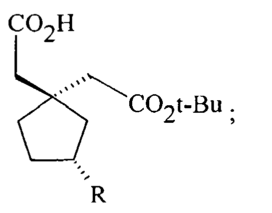
i) adding the product of Step h) to a mixture of a solvent, methanol, and (trimethylsilyl)diazomethane, and stirring to produce the bis ester of formula (17) 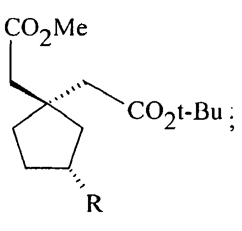 or adding the product of Step h) to a mixture of iodomethane, a solvent, and a base, and stirring to produce the bis ester of formula (17) 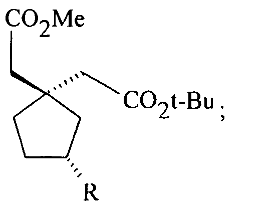
j) adding an acid to a mixture of the product from Step i) and a solvent, and stirring to produce the carboxylic acid of formula (18) 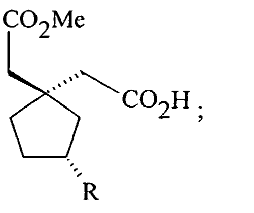
k) adding the product of Step j) to a mixture of a tertiary amine base, a solvent, and diphenylphosphoryl azide (DPPA) is added, and stirring to produce the isocyanate of formula (19) 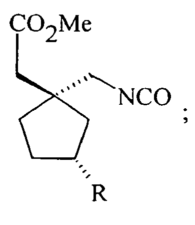 or adding the product of Step j) above to ethyl chloroformate or isobutyl chloroformate and a base in a solvent at a temperature of from -40°C to 78°C, followed by adding a solution of sodium azide in water and tetrahydrofuran or acetone, followed by adding toluene or benzene, and refluxing to produce isocyanate of formula (19) 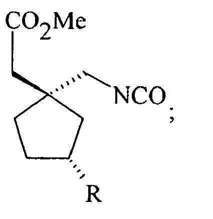
l) adding the product of Step k) to a mixture of a solvent and methanol, and stirring to produce the carbamate of formula (20) 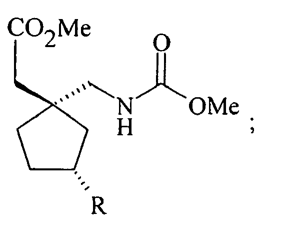
m) adding the product of Step I) to a mixture of a solvent and aqueous hydrochloric acid is added, and stirring to produce a compound of formula (IIa) 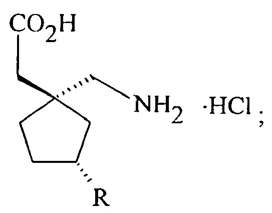 and
n) converting the product of Step m) to a compound of formula (II) 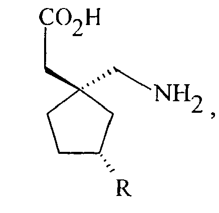 and further converting, if desired, to a pharmaceutically acceptable salt by known means.

This process is outlined in Scheme 3.

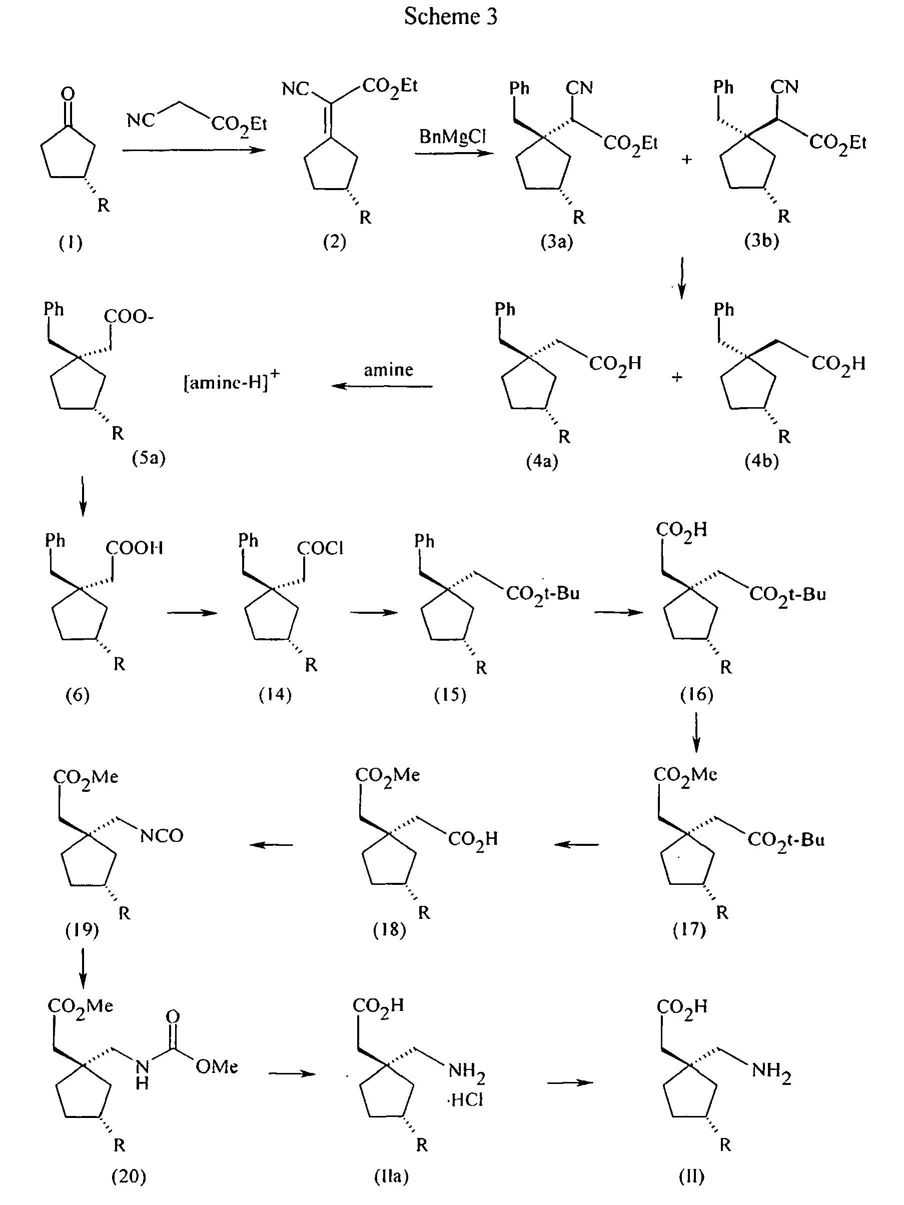

Preferred is a process for the preparation of a compound of Formula II wherein R is C₁-C₁₀ alkyl or C₃-C₁₀ cycloalkyl, and pharmaceutically acceptable salts thereof, which comprises:
a) adding a cyanoacetate of formula (A) 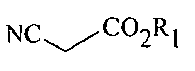 wherein R₁ is selected from methyl, ethyl, *n*-propyl, *iso*-propyl, *n*-butyl, *iso*-butyl, *sec*-butyl, *tert-*butyl, and benzyl to a mixture of a chiral cyclopentanone of formula (1) 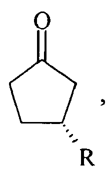 a solvent selected from tetrahydrofuran, 1,4-dioxane, *tert*-butylmethylether, chloroform, dichloromethane, acetonitrile, ethyl ether, ethyl acetate, hexanes, N,N-dimethylformamide, dimethylsulfoxide, ethanol, *tert*-butanol, toluene, benzene, xylenes, and *n*-heptane, acetic acid, and a Knoevenagel reaction catalyst selected from β-alanine, ammonium acetate, and piperidine, and stirring the mixture in the presence of a means of removing water selected from azeotropic distillation, activated molecular sieves, anhydrous magnesium sulfate, anhydrous sodium sulfate, anhydrous sodium carbonate, anhydrous potassium carbonate, anhydrous cesium carbonate, trimethyl orthoformate, and triethyl orthoformate to produce the alkene of formula (2) 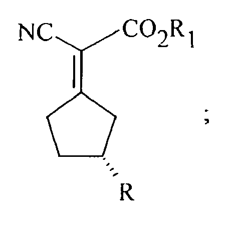
b) adding the product of Step a) above to a mixture of benzylmagnesium chloride, benzylmagnesium bromide, or benzylmagnesium iodide in a solvent selected from tetrahydrofuran, benzene, 1,4-dioxane, hexanes, *n*-heptane, toluene, diethyl ether, and *tert*-butyl methyl ether to produce the addition products of formulas (3a) 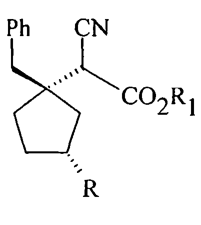 and (3b) 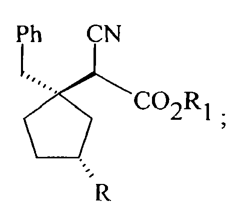
c) adding the products of Step b) above to a mixture of a base selected from potassium hydroxide, sodium hydroxide, lithium hydroxide, and cesium hydroxide in a solvent selected from ethylene glycol, 2-methoxyethyl ether, 1,4-dioxane, and diethylene glycol, and stirring the mixture and then acidifying to produce the carboxylic acids of formulas (4a) 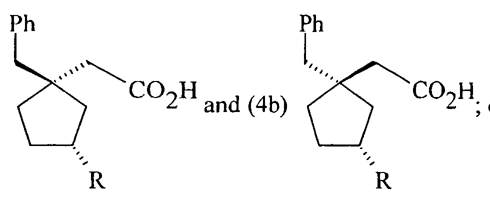 or adding the products of Step b) above to an acid mixture selected from 6-12 M HCl, 12 M H₂SO₄, 10%-48% wt/wt hydrobromic acid, and HBr in aqueous acetic acid, and stirring to produce the carboxylic acids of formulas (4a) 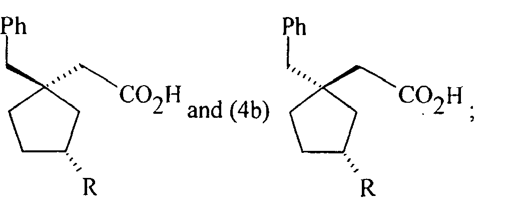
d) contacting the products of Step c) above with an amine selected from (S)-α-methyl-benzylamine, (R)-α-methyl-benzylamine, (R)-(+)-1-(naphthyl)ethylamine, (S)-(+)-1-(naphthyl)ethylamine, triethylamine, diisopropylethylamine, dicyclohexylamine, benzylamine, dibenzylamine, morpholine, N-methylmorpholine, piperidine, N-methylpiperidine, and pyridine in a solvent selected from N,N-dimethylformamide, chloroform, benzene, xylenes, hexanes, acetone, ethanol, methanol, *iso*-propanol, diethyl ether, dichloromethane, benzene, toluene, n-pentane, n-hexane, n-heptane, ethyl acetate, acetonitrile, *tert*-butyl methyl ether, tetrahydrofuran, and 1,4-dioxane, and recrystallizing the salt so formed to produce the enriched diastereomer of formula (5) 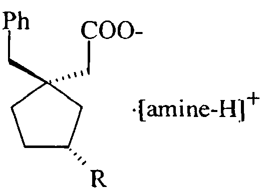 as the amine salt;
e) adding the product of Step d) to a mixture selected from aqueous hydrochloric acid, aqueous sulfuric acid, aqueous acetic acid, hydrochloric acid dissolved in acetic acid, or hydrochloric acid dissolved in acetic acid and water, and stirring to produce the carboxylic acid of formula (6) 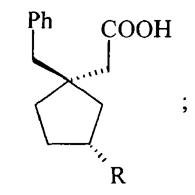 or partitioning the product of Step d) between a mixture of aqueous hydrochloric acid and a solvent selected from chloroform, dichloromethane, ethyl acetate, ethyl ether, tetrahydrofuran, 1,4-dioxane, toluene, and *tert*-butylmethylether, and drying and evaporating the organic layer to produce the carboxylic acid of formula (6) 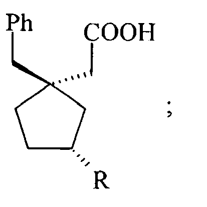
f) adding oxalyl chloride to a mixture of the product of Step e), a solvent selected from dichloromethane, chloroform, ethyl ether, toluene, and *tert-*butyl methyl ether, and 0.01 to 10 mole percent of N,N-dimethylformamide (DMF), and stirring at a temperature from -40°C to 110°C to produce the acid chloride of formula (14) 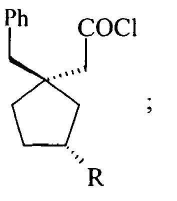
g) adding the product of Step f) to a mixture of *tert-*butyl alcohol, a solvent selected from dichloromethane, chloroform, ethyl ether, toluene, and *tert-*butyl methyl ether, and *N,N*-diisopropylethylamine (DIPEA) or triethylamine, and stirring at a temperature from -40°C to 110°C to produce the ester of formula (15) 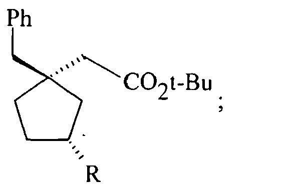
h) adding the product of Step g) to a mixture of carbon tetrachloride or ethyl acetate, and acetonitrile, water, sodium periodate, and ruthenium(III) chloride, and stirring at a temperature from -40°C to 80°C to produce the carboxylic acid of formula (16) 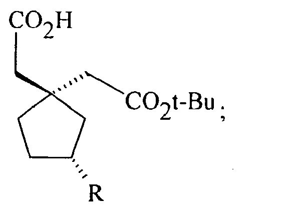
i) adding the product of Step h) to a solvent selected from toluene, benzene, xylenes, and *n*-heptane, methanol, and (trimethylsilyl)diazomethane, and stirring at a temperature from 0°C to 150°C to produce the bis ester of formula (17) 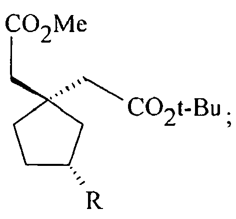 or adding the product of Step h) to a mixture of iodomethane, a solvent selected from dichloromethane, chloroform, tetrahydrofuran, toluene and 1,4-dioxane, and a base selected from 1,8-diazabicyclo[5.4.0]undec-7-ene (DBU), diisopropylethylamine, triethylamine, or 1,5-diazabicyclo[4.3.0]non-5-ene (DBN), and stirring at a temperature of from -40°C to 110°C to produce the bis ester of formula (17) 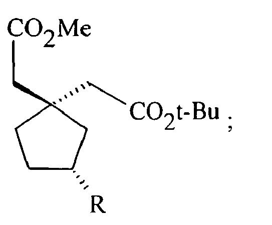
j) adding hydrochloric acid or trifluoroacetic acid (TFA) to a mixture of the product from Step i) and a solvent selected from dichloromethane, chloroform, 1,4-dioxane, tetrahydrofuran, ethyl ether, and *tert*-butyl methyl ether, and stirring at a temperature from -40°C to 110°C to produce the carboxylic acid of formula (18) 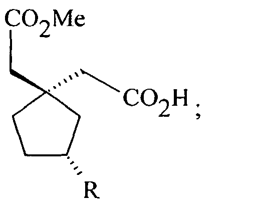
k) adding the product of Step j) to a mixture of a base selected from triethylamine and diisopropylethylamine, a solvent selected from toluene, benzene, xylenes, and *n*-heptane, and diphenylphosphoryl azide (DPPA), and stirring at a temperature from 0°C to 150°C to produce the isocyanate of formula (19) 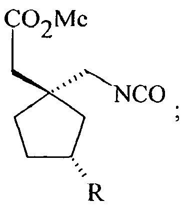 or adding the product of Step j) above to ethyl chloroformate or isobutyl chloroformate, a base selected from triethylamine and diisopropylethylamine, and a solvent selected from tetrahydrofuran, acetone, and diethyl ether at a temperature of from -40°C to 78°C, followed by adding a solution of sodium azide in water and tetrahydrofuran or acetone, followed by adding toluene or benzene, and refluxing to produce isocyanate of formula (19) 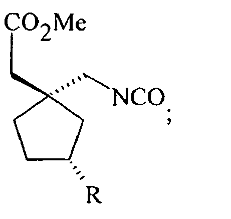
l) adding the product of Step k) to a mixture of a solvent selected from toluene, benzene, xylenes, and *n*-heptane, and methanol, and stirring at a temperature from 0°C to 150°C to produce the carbamate of formula (20) 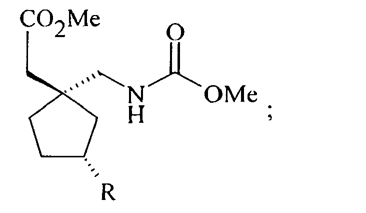
m) adding the product of Step 1) to a mixture of a solvent selected from water, acetic acid, and 1,4-dioxane, and aqueous hydrochloric acid at a concentration of from 0.01 M to 12 M, and stirring at a temperature from 0°C to 115°C to produce a compound of formula IIa 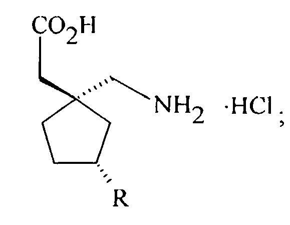 and
n) converting the product of Step m) to a compound of Formula II 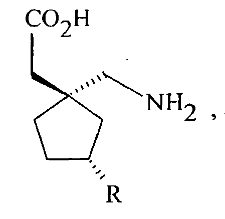 and further converting, if desired, to a pharmaceutically acceptable salt by known means.

More preferred is a process for the preparation of a compound of Formula II wherein R is C₁-C₁₀ alkyl or C₃-C₁₀ cycloalkyl, and pharmaceutically acceptable salts thereof, which comprises:
a) adding a cyanoacetate of formula (A) 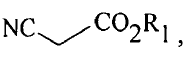 wherein R₁ is ethyl, to a mixture of a chiral cyclopentanone of formula (1) 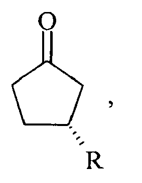 toluene, acetic acid, and a Knoevenagel reaction catalyst which is ammonium acetate, and heating the mixture at reflux over a Dean-Stark trap to produce the alkene of formula (2) 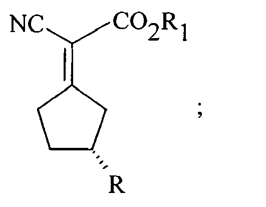
b) adding the product of Step a) above to a mixture of benzylmagnesium chloride in dry tetrahydrofuran at -100°C to 25°C to produce the addition products of formulas (3a) 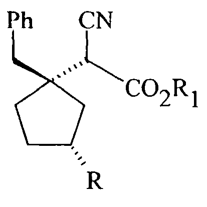 and 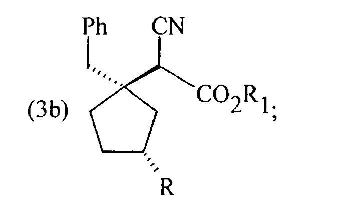
c) adding the products of Step b) above to a mixture of potassium hydroxide in ethylene glycol and heating the mixture at 100°C to 200°C, and then acidifying to produce the hydrolysis products of formulas (4a) 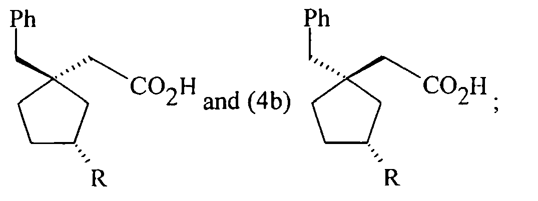
d) contacting the products of Step c) above with (S)-α-methyl-benzylamine in ethyl acetate, and recrystallizing the salt so formed from ethyl acetate to produce the enriched diastereomer of formula (5) 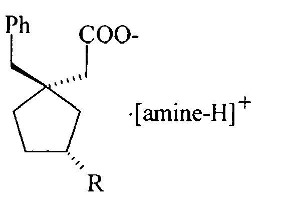 as the (S)-α-methyl-benzylamine salt;
e) adding the product of Step d) to aqueous hydrochloric acid and stirring to produce the carboxylic acid of formula (6) 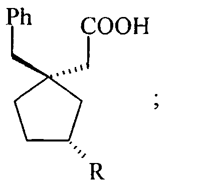
f) adding oxalyl chloride to a mixture of the product of Step e), dichloromethane, and a catalytic amount of N,N-dimethylformamide (DMF), and stirring to produce the acid chloride of formula (14) 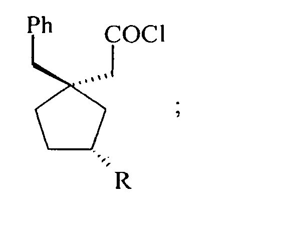
g) adding the product of Step f) to a mixture of *tert*-butyl alcohol, dichloromethane, and *NN*-diisopropylethylamine (DIPEA), and stirring to produce the ester of formula (15) 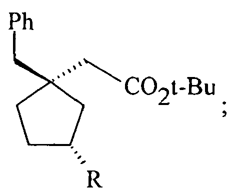
h) adding the product of Step g) to a mixture of carbon tetrachloride or ethyl acetate, and acetonitrile, water, sodium periodate, and ruthenium(III) chloride, and stirring to produce the carboxylic acid of formula (16) 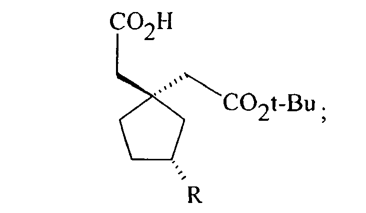
i) adding the product of Step h) to a mixture of methanol, toluene, and (trimethylsilyl)diazomethane, and stirring to produce the bis ester of fonnula (17) 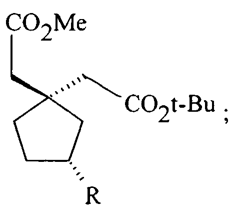 or adding the product of Step h) to a mixture of iodomethane, dichloromethane, triethylamine, and stirring to produce the bis ester of formula (17) 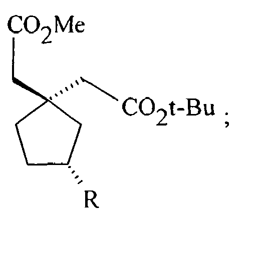
j) adding hydrochloric acid or trifluoroacetic acid (TFA) to a mixture of the product from Step i) and dichloromethane, and stirring to produce the carboxylic acid of formula (18) 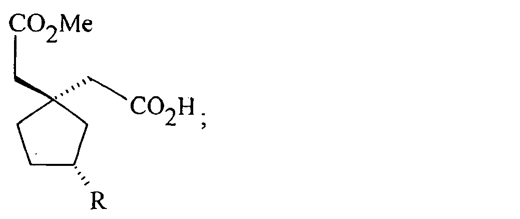
k) adding the product of Step j) to a mixture of triethylamine, toluene, and diphenylphosphoryl azide (DPPA), and refluxing to produce the isocyanate of formula (19) 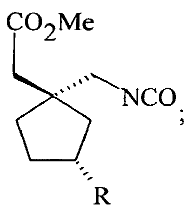 or adding the product of Step j) above to ethyl chloroformate or isobutyl chloroformate, triethylamine, and tetrahydrofuran at a temperature of from -40°C to 78°C, followed by adding a solution of sodium azide in water and tetrahydrofuran or acetone, followed by adding toluene or benzene, and refluxing to produce isocyanate of formula (19) 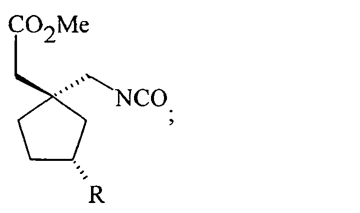
l) adding the product of Step k) to a mixture of methanol and toluene, and refluxing to produce the carbamate of formula (20) 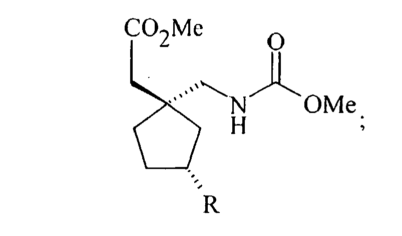
m) adding the product of Step 1) to a mixture of 1,4-dioxane and aqueous hydrochloric acid at a concentration of 6 M, and stirring to produce a compound of formula IIa 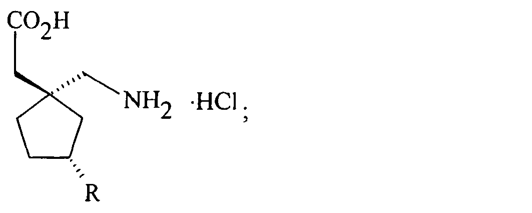
n) converting the product of Step m) to a compound of Formula II 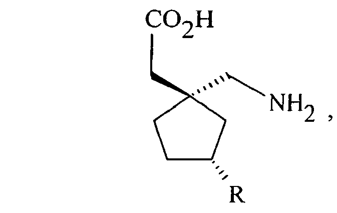 and further converting, if desired, to a pharmaceutically acceptable salt by known means.

Also preferred is a process for the preparation of a compound of Formula II, further characterized in that the intermediate product (14) 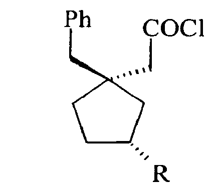 formed is further reacted, without isolation, with *tert*-butyl alcohol to produce the ester of formula (15)

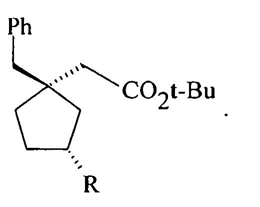

Also preferred is a process for the preparation of a compound of Formula 11, further characterized in that the intermediate product (19) 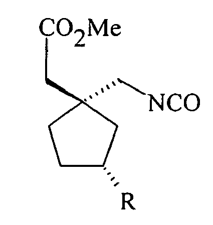 formed is further reacted, without isolation, with methanol to produce the carbamate of formula (20)

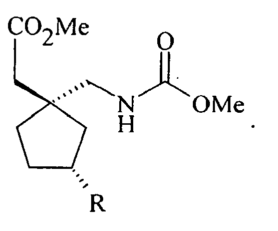

Also preferred is a process for the preparation of a compound of Formula II, further characterized in that the intermediate product (14) 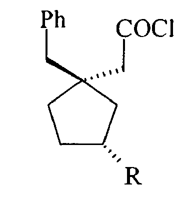 formed is further reacted, without isolation, with *tert*-butyl alcohol to produce the ester of Formula (15) 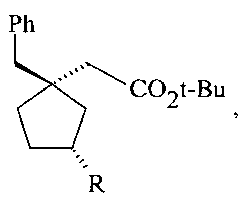 and the intermediate product (19) 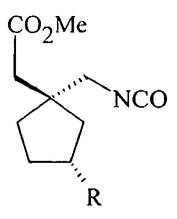 formed is further reacted, without isolation, with methanol to produce the carbamate of formula (20)

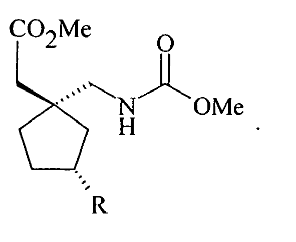

Further, the invention provides a process for the preparation of a compound of Formula III 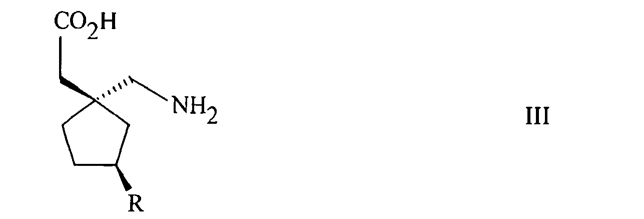 wherein R is C₁-C₁₀ alkyl or C₃-C₁₀ cycloalkyl, and pharmaceutically acceptable salts thereof, which comprises:
a) adding a cyanoacetate of formula (A) 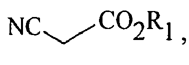 wherein R₁ is alkyl or benzyl, to a mixture of a chiral cyclopentanone of formula (21) 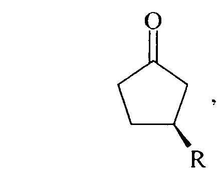 a solvent, a carboxylic acid, and a Knoevenagel reaction catalyst, and stirring the mixture in the presence of a means of removing water to produce the alkene of formula (22) 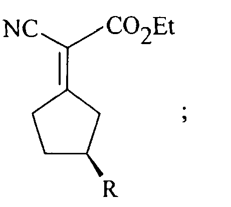
b) adding the product of Step a) above to a mixture of benzylmagnesiuin chloride or benzylmagnesium iodide, in a solvent to produce the addition of products of formulas (23a) 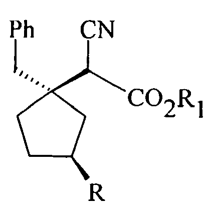 and (23b) 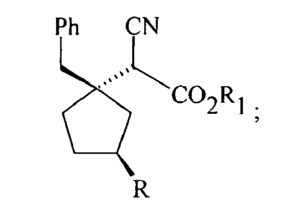 adding the products of Step b) above to a mixture of a base selected from potassium hydroxide, sodium hydroxide, lithium hydroxide, and cesium hydroxide, in a solvent, and stirring, and then acidifying to produce the carboxylic acids of formulas (24a) 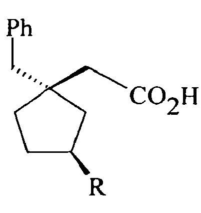 and (24b) 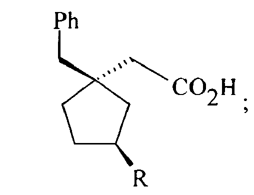 or adding the products of Step b) above to an acid mixture, and stirring to produce the carboxylic acids of formulas (24a) 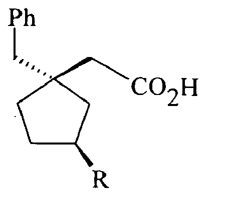 and (24b) 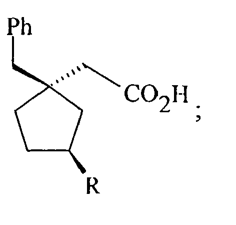
d) contacting the products of Step c) above with an amine in a solvent, and recrystallizing the salt so formed to produce the enriched diastereomer of formula (25) 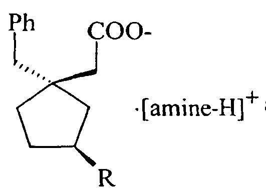 as the amine salt; and
e) converting the product of Step d) to a carboxylic acid of formula (26) 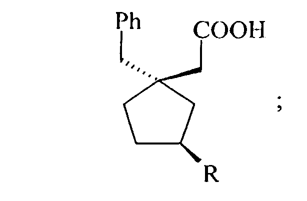
f) adding the product of Step e) to a mixture of iodomethane, a solvent, and 1,8-diazabicyclo[5.4.0]undec-7-ene (DBU), and stirring to produce the ester of formula (27) 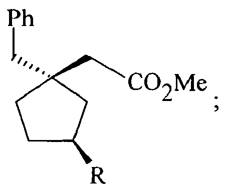 or adding the product of Step e) to methanol and an acid to produce the ester of formula (27) 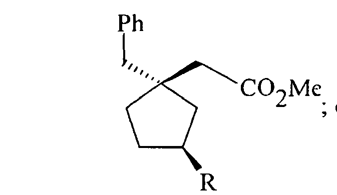 or adding the product of Step e) to a solution of diazomethane or trimethylsilyl-diazomethane in a solvent to produce ester of formula (27) 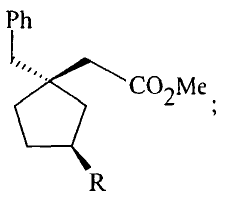
g) adding the product of Step f) to a mixture of carbon tetrachloride or ethyl acetate, and acetonitrile, water, sodium periodate, and ruthenium(III) chloride, and stirring to produce the carboxylic acid of formula (28) 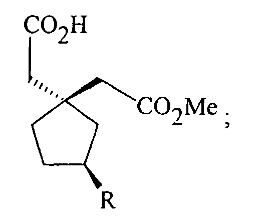
h) adding the product of Step g) to a mixture of a tertiary amine base, a solvent, and diphenylphosphoryl azide (DPPA), and stirring to produce the isocyanate of formula (29) 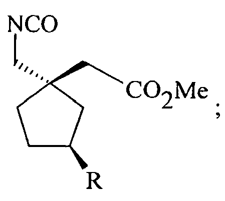 or adding the product of Step g) above to ethyl chloroformate or isobutyl chloroformate and a base in a solvent at a temperature of from -40°C to 78°C, followed by adding a solution of sodium azide in water and tetrahydrofuran or acetone, followed by adding toluene or benzene, and refluxing to produce isocyanate of formula (29) 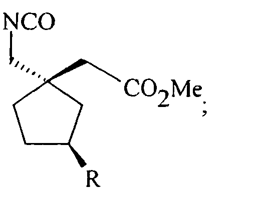
i) adding the product of Step h) to a mixture of a solvent and methanol, and stirring to produce the carbamate of formula (30) 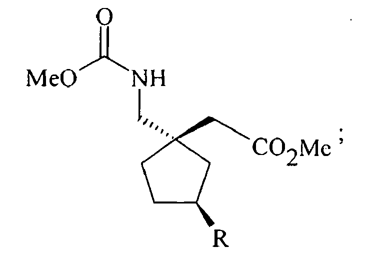
j) adding the product of Step i) to a mixture of a solvent and aqueous hydrochloric acid, and stirring to produce a compound of formula (IIIa) 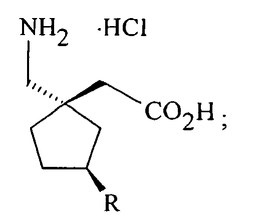
k) converting the product of Step j) to a compound of formula (III) 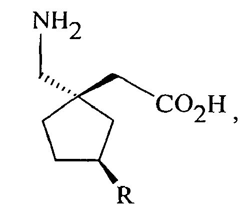 and further converting, if desired, to a pharmaceutically acceptable salt by known means.

This process is outlined in Scheme 4.

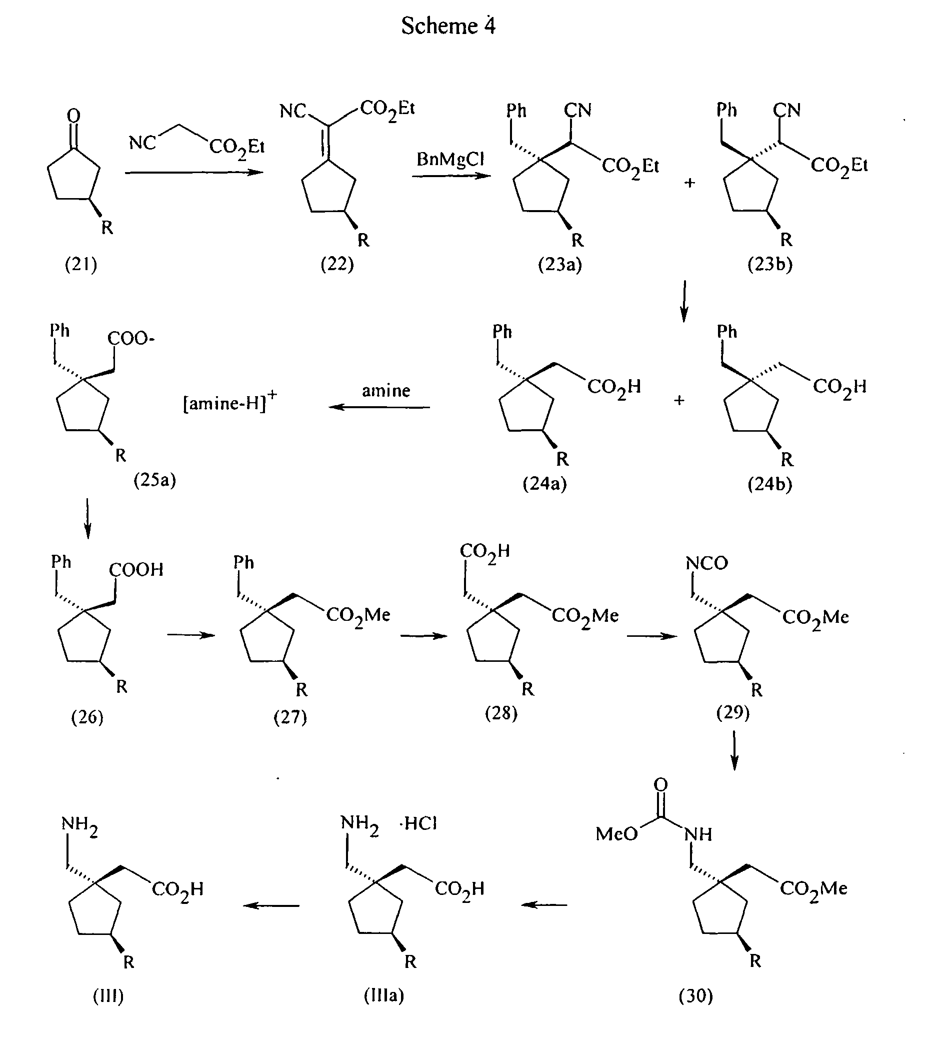

Preferred is a process for the preparation of a compound of Formula III wherein R is C₁-C₁₀ alkyl or C₃-C₁₀ cycloalkyl, and pharmaceutically acceptable salts thereof, which comprises:
a) adding ethyl cyanoacetate to a mixture of a chiral cyclopentanone of formula (21) in a solvent selected from toluene, benzene, xylenes, or *n*-heptane to which acetic acid and β-alanine or ammonium acetate were added, and stirring the mixture at a temperature from 0°C to 150°C to produce the alkene of formula (22);
b) adding the product of Step a) above to a mixture of benzylmagnesium chloride in a dry solvent selected from tetrahydrofuran, 1,4-dioxane, *n*-heptane, toluene, ethyl ether, or *tert*-butyl methyl ether at a temperature from -100°C to 110°C to produce the addition products of formulas (23a) and (23b);
c) adding the products of Step b) above to a mixture of a base selected from potassium hydroxide, sodium hydroxide, lithium hydroxide, or cesium hydroxide in a solvent selected from ethylene glycol, 2-methoxyethyl ether, 1,4-dioxane, or diethylene glycol and stirring the mixture at a temperature from 25°C to 250°C to produce the carboxylic acids of formulas (24a) and (24b);
d) contacting the products of Step c) above with (R)-α-methyl-benzylamine in a solvent selected from ethyl acetate, acetonitrile, tetrahydrofuran, or 1,4-dioxane at a temperature from -40°C to 105°C, and recrystallizing the salt so formed from a solvent selected from ethyl acetate, acetonitrile, tetrahydrofuran, 1,4-dioxane, *tert*-butyl methyl ether, toluene, or *n*-heptane to produce the enriched diastereomer of formula (25a) as the (R)-α-methylbenzylamine salt;
e) adding the product of Step d) to a mixture selected from aqueous hydrochloric acid, hydrochloric acid dissolved in acetic acid, or hydrochloric acid dissolved in acetic acid to which water was added and stirring at a temperature from -40°C to 115°C to produce the carboxylic acid of formula (26);
f) adding the product of Step e) to a mixture of iodomethane in a solvent selected from dichloromethane, chloroform, tetrahydrofuran, toluene, or 1,4-dioxane to which 1,8-diazabicyclo[5.4.0]undec-7-ene (DBU) was added, and stirring at a temperature from -40°C to 110°C to produce the ester of formula (27);
g) adding the product of Step f) to a mixture of carbon tetrachloride and acetonitrile to which water, sodium periodate, and ruthenium(III) chloride were added, and stirring at a temperature from -40°C to 80°C to produce the carboxylic acid of formula (28);
h) adding the product of Step g) to a mixture of a base selected from triethylamine or diisopropylethylamine and a solvent selected from toluene, benzene, xylenes, or *n*-heptane to which diphenylphosphoryl azide (DPPA) was added, and stirring at a temperature from 0°C to 150°C to produce the isocyanate of formula (29);
i) adding the product of Step h) to a solvent selected from toluene, benzene, xylenes, or *n*-heptane to which methanol was added and stirring at a temperature from 0°C to 150°C to produce the carbamate of formula (30);
j) adding the product of Step i) to a solvent selected from water, acetic acid, or 1,4-dioxane to which aqueous hydrochloric acid at a concentration of from 0.01 M to 12 M was added, and stirring at a temperature from 0°C to 115°C to produce a compound of Formula llla;
k) converting the product of Step j) to a compound of Formula III, and further converting, if desired, to a pharmaceutically acceptable salt by known means.

More preferred is a process for the preparation of a compound of Formula III wherein R is C₁-C₁₀ alkyl or C₃-C₁₀ cycloalkyl, and pharmaceutically acceptable salts thereof, which comprises:
a) adding ethyl cyanoacetate to a mixture of a chiral cyclopentanone of formula (21) in toluene to which acetic acid and ammonium acetate were added, and heating the mixture at reflux to produce the alkene of formula (22);
b) adding the product of Step a) above to a mixture of benzylmagnesium chloride in dry tetrahydrofuran at -100°C to -20°C to produce the addition products of formulas (23a) and (23b);
c) adding the products of Step b) above to a mixture of potassium hydroxide in ethylene glycol, and heating the mixture at 100°C to 200°C to produce the hydrolysis products of formulas (24a) and (24b);
d) contacting the products of Step c) above with (R)-α-methyl-benzylamine in ethyl acetate, and recrystallizing the salt so formed from ethyl acetate to produce the enriched diastereomer of formula (25a) as the (R)-α-methylbenzylamine salt;
e) adding the product of Step d) to aqueous hydrochloric acid and stirring to produce the carboxylic acid of formula (26);
f) adding the product of Step e) to a mixture of iodomethane in dichloromethane to which 1,8-diazabicyclo[5.4.0]undec-7-ene (DBU) was added, and stirring to produce the ester of formula (27);
g) adding the product of Step f) to a mixture of carbon tetrachloride and acetonitrile to which water, sodium periodate, and ruthenium(III) chloride were added, and stirring to produce the carboxylic acid of formula (28);
h) adding the product of Step g) to a mixture of triethylamine and toluene to which diphenylphosphoryl azide (DPPA) was added, and refluxing to produce the isocyanate of formula (29);
i) adding the product of Step h) to a mixture of methanol and toluene, and refluxing to produce the carbamate of formula (30);
j) adding the product of Step i) to 1,4-dioxane to which aqueous hydrochloric acid at a concentration of 6 M was added, and stirring to produce a compound of Formula IIIa;
k) converting the product of Step j) to a compound of Formula III, and further converting, if desired, to a pharmaceutically acceptable salt by known means.

Also preferred is a process for the preparation of a compound of Formula III, further characterized in that the intermediate product (29) 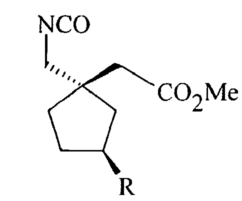 formed is further reacted, without isolation, with methanol to produce the carbamate of formula (30)

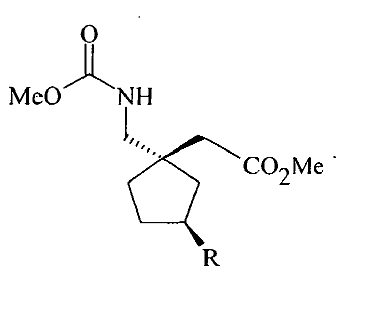

Further, the invention provides a process for the preparation of a compound of Formula IV 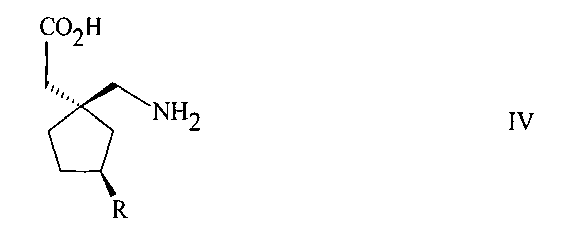 wherein R is C₁-C₁₀ alkyl or C₃-C₁₀ cycloalkyl, and pharmaceutically acceptable salts thereof, which comprises:
a) adding a cyanoacetate of formula (A) 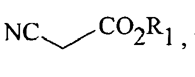 wherein R is alkyl or benzyl, to a mixture of chiral cyclopentanone of formula (21) 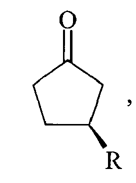 a solvent, a carboxylic acid, and a Knoevenagel reaction catalyst, and stirring the mixture in the presence of a means of removing water to produce the alkene of formula (22) 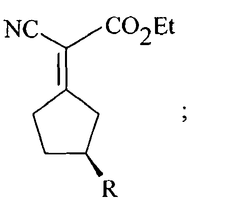
b) adding the product of Step a) above to a mixture of benzylmagnesium chloride or benzylmagnesium iodide, in a solvent to produce the addition of products of formulas (23a) 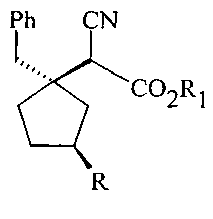 and (23b) 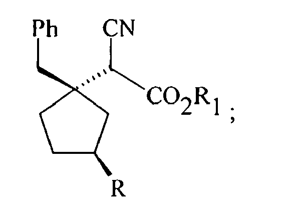
c) adding the products of Step b) above to a mixture of a base selected from potassium hydroxide, sodium hydroxide, lithium hydroxide, and cesium hydroxide, in a solvent, and stirring, and then acidifying to produce the carboxylic acids of formulas (24a) 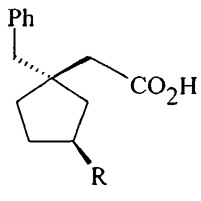 and (24b) 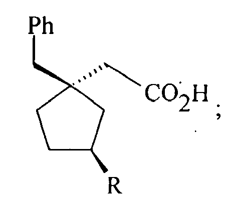 or adding the products of Step b) above to an acid mixture, and stirring to produce the carboxylic acids of formulas (24a) 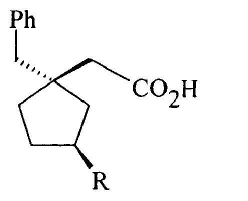 and (24b) 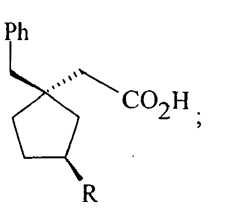
d) contacting the products of Step c) above with an amine in a solvent, and recrystallizing the salt so formed to produce the enriched diastereomer of formula (25) 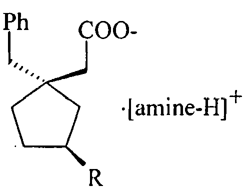 as the amine salt; and
e) converting the product of Step d) to a carboxylic acid of formula (26) 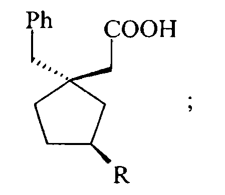
f) adding the product of Step e) to a mixture of a tertiary amine base, a solvent, and diphenylphosphoryl azide (DPPA) is added, and stirring to produce the isocyanate of formula (31) 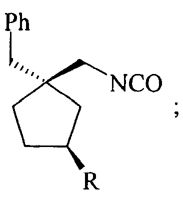 or adding the product of Step g) above to ethyl chloroformate or isobutyl chloroformate and a base in a solvent at a temperature of from -40°C to 78°C, followed by adding a solution of sodium azide in water and tetrahydrofuran or acetone, followed by adding toluene or benzene, and refluxing to produce the isocyanate of formula (31) 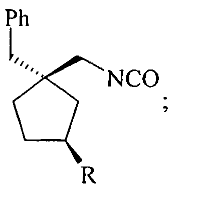
g) adding the product of Step f) to a mixture of a solvent and methanol, and stirring to produce the carbamate of formula (32) 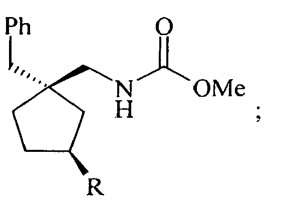
h) adding the product of Step g) to a mixture of carbon tetrachloride or ethyl acetate, and acetonitrile, water, sodium periodate, and ruthenium(III) chloride, and stirring to produce the carboxylic acid of formula (33) 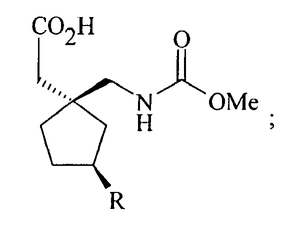
i) adding the product of Step h) to a mixture of a solvent and aqueous hydrochloric acid, and stirring to produce a compound of formula (IVa) 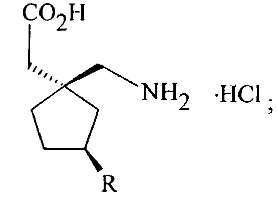 and
j) converting the product of Step i) to a compound of formula (IV) 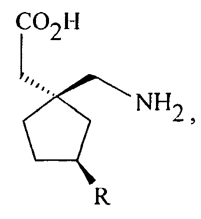 and further converting, if desired, to a pharmaceutically acceptable salt by known means.

This process is outlined in Scheme 5.

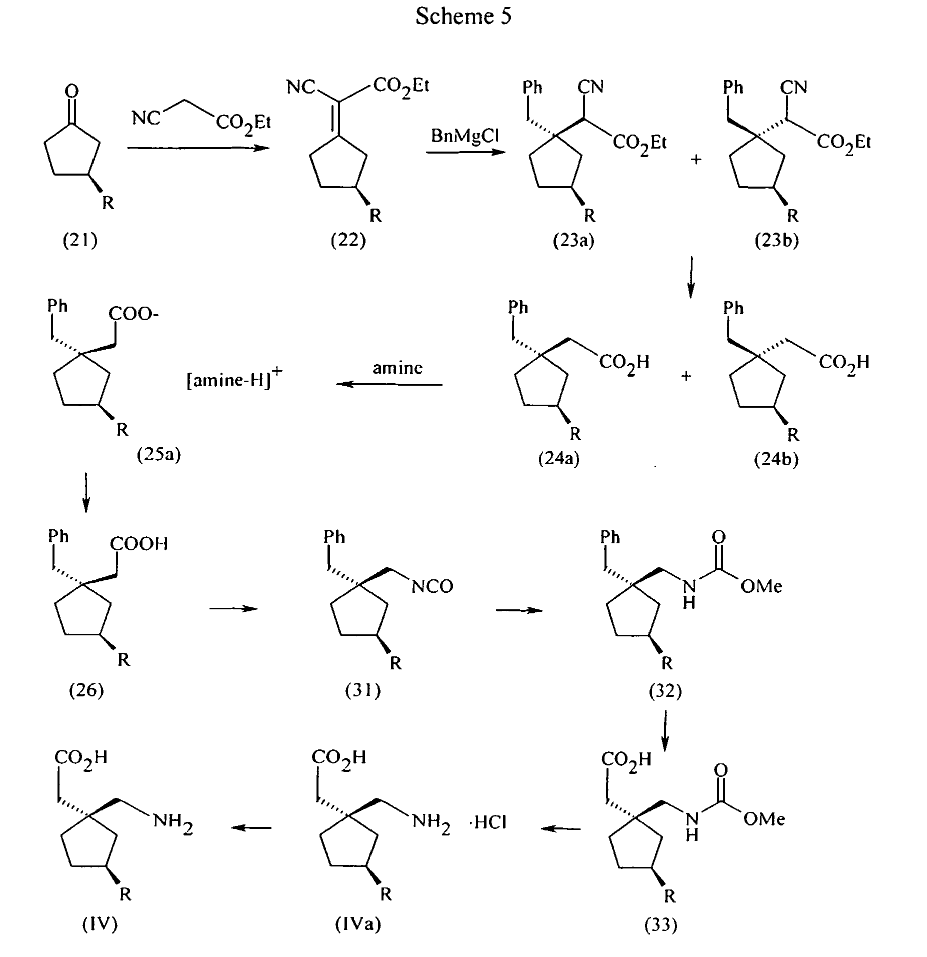

Preferred is a process for the preparation of a compound of Formula IV wherein R is C₁-C₁₀ alkyl or C₃-C₁₀ cycloalkyl, and pharmaceutically acceptable salts thereof, which comprises:
a) adding ethyl cyanoacetate to a mixture of a chiral cyclopentanone of formula (21) in a solvent selected from toluene, benzene, xylenes, or *n*-heptane to which acetic acid and β-alanine or ammonium acetate were added, and stirring the mixture at a temperature from 0°C to 150°C to produce the alkene of formula (22);
b) adding the product of Step a) above to a mixture of benzylmagnesium chloride in a dry solvent selected from tetrahydrofuran, 1,4-dioxane, *n*-heptane, toluene, ethyl ether, or *tert*-butyl methyl ether at a temperature from -100°C to 110°C to produce the addition products of formulas (23a) and (23b);
c) adding the products of Step b) above to a mixture of a base selected from potassium hydroxide, sodium hydroxide, lithium hydroxide, or cesium hydroxide in a solvent selected from ethylene glycol, 2-methoxyethyl ether, 1,4-dioxane, or diethylene glycol and stirring the mixture at a temperature from 25°C to 250°C to produce the carboxylic acids of formulas (24a) and (24b);
d) contacting the products of Step c) above with (R)-α-methyl-benzylamine in a solvent selected from ethyl acetate, acetonitrile, tetrahydrofuran, or 1,4-dioxane at a temperature from -40°C to 105°C, and recrystallizing the salt so formed from a solvent selected from ethyl acetate, acetonitrile, tetrahydrofuran, 1,4-dioxane, *tert*-butyl methyl ether, toluene, or *n*-heptane to produce the enriched diastereomer of formula (25a) as the (R)-α-methylbenzylamine salt;
e) adding the product of Step d) to a mixture selected from aqueous hydrochloric acid, hydrochloric acid dissolved in acetic acid, or hydrochloric acid dissolved in acetic acid to which water was added and stirring at a temperature from -40°C to 115°C to produce the carboxylic acid of formula (26);
f) adding the product of Step e) to a mixture of a base selected from triethylamine or diisopropylethylamine and a solvent selected from toluene, benzene, xylenes, or *n*-heptane to which diphenylphosphoryl azide (DPPA) was added, and stirring at a temperature from 0°C to 150°C to produce the isocyanate of formula (31);
g) adding the product of Step f) to a solvent selected from toluene, benzene, xylenes, or *n*-heptane to which methanol was added and stirring at a temperature from 0°C to 150°C to produce the carbamate of formula (32);
h) adding the product of Step g) to a mixture of carbon tetrachloride and acetonitrile to which water, sodium periodate, and ruthenium(III) chloride were added, and stirring at a temperature from -40°C to 80°C to produce the carboxylic acid of formula (33);
i) adding the product of Step h) to a solvent selected from water, acetic acid, or 1,4-dioxane to which aqueous hydrochloric acid at a concentration of from 0.01 M to 12 M was added, and stirring at a temperature from 0°C to 115°C to produce a compound of Formula IVa;
j) converting the product of Step i) to a compound of Formula IV, and further converting, if desired, to a pharmaceutically acceptable salt by known means.

More preferred is a process for the preparation of a compound of Formula IV wherein R is C₁-C₁₀ alkyl or C₃-C₁₀ cycloalkyl, and pharmaceutically acceptable salts thereof, which comprises:
a) adding ethyl cyanoacetate to a mixture of a chiral cyclopentanone of formula (21) in toluene to which acetic acid and ammonium acetate were added, and heating the mixture at reflux to produce the alkene of formula (22);
b) adding the product of Step a) above to a mixture of benzylmagnesium chloride in dry tetrahydrofuran at -100°C to -20°C to produce the addition products of formulas (23a) and (23b);
c) adding the products of Step b) above to a mixture of potassium hydroxide in ethylene glycol, and heating the mixture at 100°C to 200°C to produce the hydrolysis products of formulas (24a) and (24b);
d) contacting the products of Step c) above with (R)-α-methyl-benzylamine in ethyl acetate, and recrystallizing the salt so formed from ethyl acetate to produce the enriched diastereomer of formula (25a) as the (R)-α-methylbenzylamine salt;
e) adding the product of Step d) to aqueous hydrochloric acid and stirring to produce the carboxylic acid of formula (26);
f) adding the product of Step e) to a mixture of triethylamine and toluene to which diphenylphosphoryl azide (DPPA) was added, and refluxing to produce the isocyanate of formula (31);
g) adding the product of Step f) to a mixture of methanol and toluene, and refluxing to produce the carbamate of formula (32);
h) adding the product of Step g) to a mixture of carbon tetrachloride and acetonitrile to which water, sodium periodate, and ruthenium(III) chloride were added, and stirring to produce the carboxylic acid of formula (33);

i) adding the product of Step h) to 1,4-dioxane to which aqueous hydrochloric acid at a concentration of 6 M was added, and stirring to produce a compound of Formula IVa;
j) converting the product of Step i) to a compound of Formula IV, and further converting, if desired, to a pharmaceutically acceptable salt by known means.

Also preferred is a process for the preparation of a compound of Formula IV, further characterized in that the intermediate product (31) 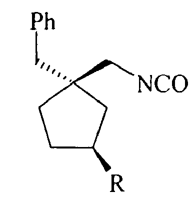 formed is further reacted, without isolation, with methanol to produce the carbamate of formula (32)

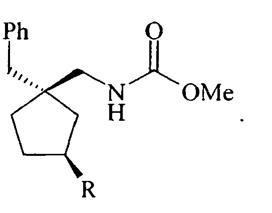

Still further, the invention provides a process for the preparation of a compound of Formula IV 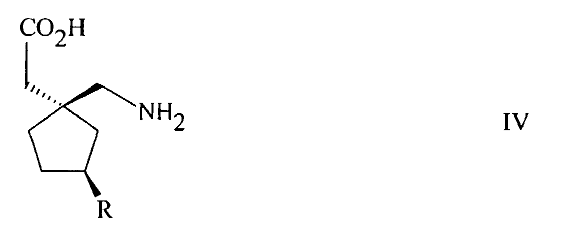 wherein R is C₁-C₁₀ alkyl or C₃-C₁₀ cycloalkyl, and pharmaceutically acceptable salts thereof, which comprises:
a) adding a cyanoacetate of formula (A) 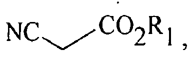 wherein R₁ is alkyl or benzyl, to a mixture of a chiral cyclopentanone of formula (21) 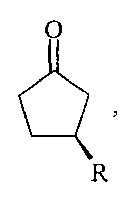 a solvent, a carboxylic acid, and a Knoevenagel reaction catalyst, and stirring the mixture in the presence of a means of removing water to produce the alkene of formula (22) 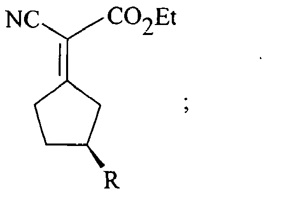
b) adding the product of Step a) above to a mixture of benzylmagnesium chloride or benzylmagnesium iodide, in a solvent to produce the addition of products of formulas (23a) 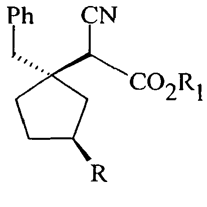 and (23b) 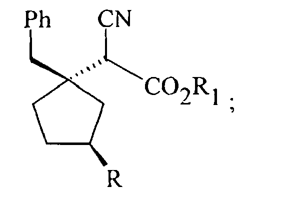
c) adding the products of Step b) above to a mixture of a base selected from potassium hydroxide, sodium hydroxide, lithium hydroxide, and cesium hydroxide, in a solvent, and stirring, and then acidifying to produce the carboxylic acids of formulas (24a) 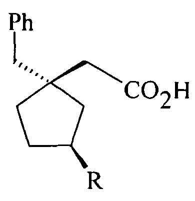 and (24b) 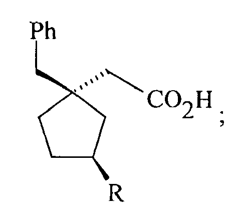 or adding the products of Step b) above to an acid mixture, and stirring to produce the carboxylic acids of formulas (24a) 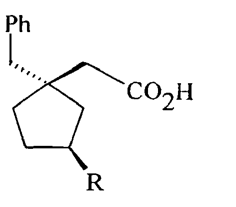 and (24b) 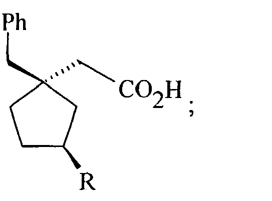
d) contacting the products of Step c) above with an amine in a solvent, and recrystallizing the salt so formed to produce the enriched diastereomer of formula (25) 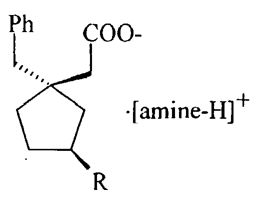 as the amine salt; and
e) converting the product of Step d) to a carboxylic acid of formula (26) 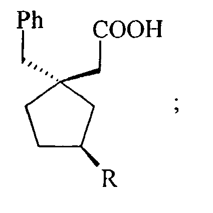
f) adding oxalyl chloride to a mixture of the product of Step e), a solvent, and N,N-dimethylformamide (DMF), and stirring to produce the acid chloride of formula (34) 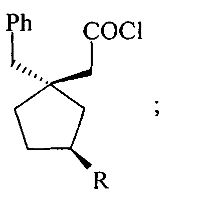
g) adding the product of Step f) to a mixture of *tert-*butyl alcohol, a solvent, and a tertiary amine base, and stirring to produce the ester of formula (35) 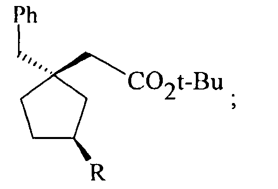
h) adding the product of Step g) to a mixture of carbon tetrachloride or ethyl acetate, and acetonitrile, water, sodium periodate, and ruthenium(III) chloride, and stirring to produce the carboxylic acid of formula (36) 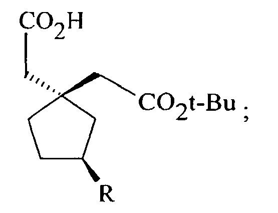
i) adding the product of Step h) to a mixture of a solvent, methanol, and (trimethylsilyl)diazomethane, and stirring to produce the bis ester of formula (37) 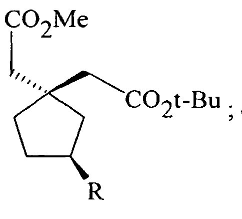 or adding the product of Step h) to a mixture of iodomethane, a solvent, and a base, and stirring to produce the bis ester of formula (37) 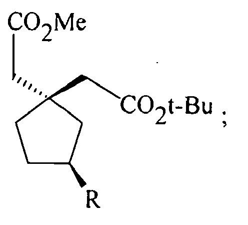
j) adding an acid to a mixture of the product from Step i) and a solvent and stirring to produce the carboxylic acid of formula (38) 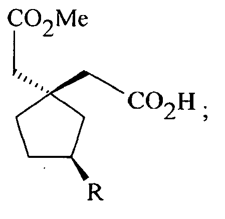
k) adding the product of Step j) to a mixture of a tertiary amine base, a solvent, and diphenylphosphoryl azide (DPPA), and stirring to produce the isocyanate of formula (39) 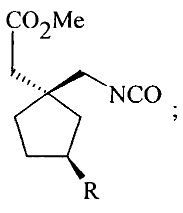 or adding the product of Step j) above to ethyl chloroformate or isobutyl chloroformate and a base in a solvent at a temperature of from -40°C to 78°C, followed by adding a solution of sodium azide in water and tetrahydrofuran or acetone, followed by adding toluene or benzene, and refluxing to produce isocyanate of formula (39) 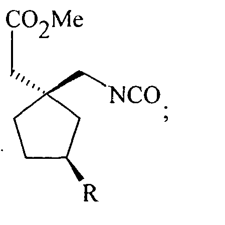
l) adding the product of Step k) to a mixture of a solvent and methanol, and stirring to produce the carbamate of formula (40) 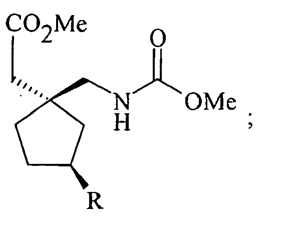
m) adding the product of Step 1) to a mixture of a solvent and hydrochloric acid, and stirring to produce a compound of formula (IVa) 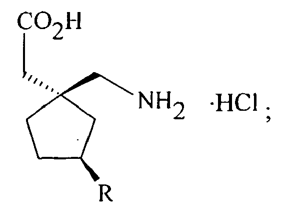
n) converting the product of Step m) to a compound of Formula IV 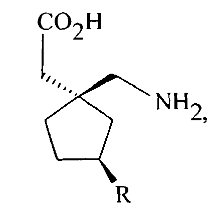 and further converting, if desired, to a pharmaceutically acceptable salt by known means.

This process is outlined in Scheme 6.

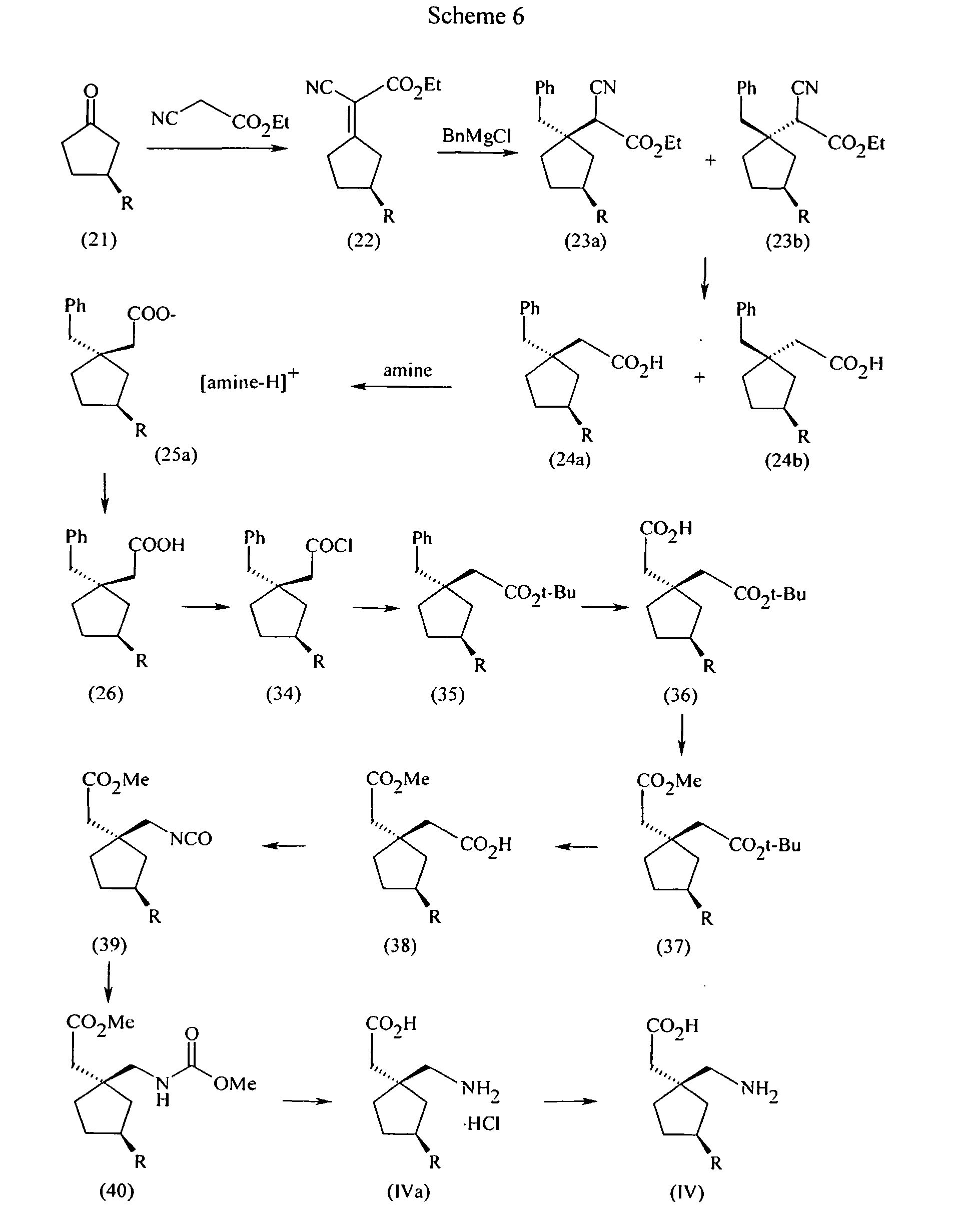

Preferred is a process for the preparation of a compound of Formula IV wherein R is C₁-C₁₀ alkyl or C₃-C₁₀ cycloalkyl, and pharmaceutically acceptable salts thereof, which comprises:
a) adding ethyl cyanoacetate to a mixture of a chiral cyclopentanone of formula (21) in a solvent selected from toluene, benzene, xylenes, or *n*-heptane to which acetic acid and β-alanine or ammonium acetate were added, and stirring the mixture at a temperature from 0°C to 150°C to produce the alkene of formula (22);
b) adding the product of Step a) above to a mixture of benzylmagnesium chloride in a dry solvent selected from tetrahydrofuran, 1,4-dioxane, *n-*heptane, toluene, ethyl ether, or *tert*-butyl methyl ether at a temperature from - 100°C to 110°C to produce the addition products of formulas (23a) and (23b);
c) adding the products of Step b) above to a mixture of a base selected from potassium hydroxide, sodium hydroxide, lithium hydroxide, or cesium hydroxide in a solvent selected from ethylene glycol, 2-methoxyethyl ether, 1,4-dioxane, or diethylene glycol and stirring the mixture at a temperature from 25°C to 250°C to produce the carboxylic acids of formulas (24a) and (24b);
d) contacting the products of Step c) above with (R)-α-methyl-benzylamine in a solvent selected from ethyl acetate, acetonitrile, tetrahydrofuran, or 1,4-dioxane at a temperature from -40°C to 105°C, and recrystallizing the salt so formed from a solvent selected from ethyl acetate, acetonitrile, tetrahydrofuran, 1,4-dioxane, *tert*-butyl methyl ether, toluene, or *n*-heptane to produce the enriched diastereomer of formula (25a) as the (R)-α-methylbenzylamine salt;
e) adding the product of Step d) to a mixture selected from aqueous hydrochloric acid, hydrochloric acid dissolved in acetic acid, or hydrochloric acid dissolved in acetic acid to which water was added and stirring at a temperature from -40°C to 115°C to produce the carboxylic acid of formula (26);
f) adding oxalyl chloride to a mixture of the product of Step e) and a solvent selected from dichloromethane, chloroform, ethyl ether, toluene, or *tert-*butyl methyl ether to which 0.01 mol percent to 10 mol percent of N,N-dimethylformamide (DMF) was added, and stirring at a temperature from -40°C to 110°C to produce the acid chloride of formula (34);
g) adding the product of Step f) to a mixture of *tert*-butyl alcohol in a solvent selected from dichloromethane, chloroform, ethyl ether, toluene, or *tert-*butyl methyl ether to which *N,N*-diisopropylethylamine (DIPEA) or triethylamine was added, and stirring at a temperature from -40°C to 110°C to produce the ester of formula (35);
h) adding the product of Step g) to a mixture of carbon tetrachloride and acetonitrile to which water, sodium periodate, and ruthenium(III) chloride were added, and stirring at a temperature from -40°C to 80°C to produce the carboxylic acid of formula (36);
i) adding the product of Step h) to a solvent selected from toluene, benzene, xylenes, or *n*-heptane to which methanol and (trimethylsilyl)diazomethane were added, and stirring at a temperature from 0°C to 150°C to produce the bis ester of formula (37);
j) adding trifluoroacetic acid (TFA) to a mixture of the product from Step i) and a solvent selected from dichloromethane, chloroform, 1,4-dioxane, tetrahydrofuran, ethyl ether, or *tert*-butyl methyl ether and stirring at a temperature from -40°C to 110°C to produce the carboxylic acid of formula (38);
k) adding the product of Step j) to a mixture of a base selected from triethylamine or diisopropylethylamine and a solvent selected from toluene, benzene, xylenes, or *n*-heptane to which diphenylphosphoryl azide (DPPA) was added, and stirring at a temperature from 0°C to 150°C to produce the isocyanate of formula (39);
l) adding the product of Step k) to a solvent selected from toluene, benzene, xylenes, or *n*-heptane to which methanol was added and stirring at a temperature from 0°C to 150°C to produce the carbamate of formula (40);
m) adding the product of Step 1) to a solvent selected from water, acetic acid, or 1,4-dioxane to which aqueous hydrochloric acid at a concentration of from 0.01 M to 12 M was added, and stirring at a temperature from 0°C to 115°C to produce a compound of Formula IVa;
n) converting the product of Step m) to a compound of Formula IV, and further converting, if desired, to a pharmaceutically acceptable salt by known means.

More preferred is a process for the preparation of a compound of Formula IV wherein R is C₁-C₁₀ alkyl or C₃-C₁₀ cycloalkyl, and pharmaceutically acceptable salts thereof, which comprises:
a) adding ethyl cyanoacetate to a mixture of a chiral cyclopentanone of formula (21) in toluene to which acetic acid and ammonium acetate were added, and heating the mixture at reflux to produce the alkene of formula (22);
b) adding the product of Step a) above to a mixture of benzylmagnesium chloride in dry tetrahydrofuran at -100°C to -20°C to produce the addition products of formulas (23a) and (23b);
c) adding the products of Step b) above to a mixture of potassium hydroxide in ethylene glycol, and heating the mixture at 100°C to 200°C to produce the hydrolysis products of formulas (24a) and (24b);
d) contacting the products of Step c) above with (R)-α-methyl-benzylamine in ethyl acetate, and recrystallizing the salt so formed from ethyl acetate to produce the enriched diastereomer of formula (25a) as the (R)-α-methylbenzylamine salt;
e) adding the product of Step d) to aqueous hydrochloric acid and stirring to produce the carboxylic acid of formula (26);
f) adding oxalyl chloride to a mixture of the product of Step e) and dichloromethane to which a catalytic amount of N,N-dimethylformamide (DMF) was added, and stirring to produce the acid chloride of formula (34);
g) adding the product of Step f) to a mixture of *tert*-butyl alcohol in dichloromethane to which *N,N* diisopropylethylamine (DIPEA) was added, and stirring to produce the ester of formula (35);
h) adding the product of Step g) to a mixture of carbon tetrachloride and acetonitrile to which water, sodium periodate, and ruthenium(III) chloride were added, and stirring to produce the carboxylic acid of formula (36);

i) adding the product of Step h) to a mixture of methanol and toluene to which (trimethylsilyl)diazomethane was added, and stirring to produce the bis ester of formula (37);
j) adding trifluoroacetic acid (TFA) to a mixture of the product from Step i) and dichloromethane, and stirring to produce the carboxylic acid of formula (38);
k) adding the product of Step j) to a mixture of triethylamine and toluene to which diphenylphosphoryl azide (DPPA) was added, and refluxing to produce the isocyanate of formula (39);
l) adding the product of Step k) to a mixture of methanol and toluene, and refluxing to produce the carbamate of formula (40);
m) adding the product of Step 1) to 1,4-dioxane to which aqueous hydrochloric acid at a concentration of 6 M was added, and stirring to produce a compound of Formula IVa;
n) converting the product of Step m) to a compound of Formula IV, and further converting, if desired, to a pharmaceutically acceptable salt by known means.

Also preferred is a process for the preparation of a compound of Formula IV, further characterized in that the intermediate product (34) 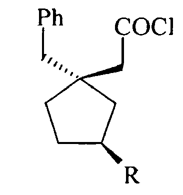 formed is further reacted, without isolation, with *tert*-butyl alcohol to produce the ester of formula (35)

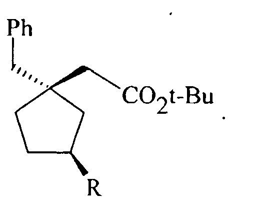

Also preferred is a process for the preparation of a compound of Formula IV, further characterized in that the intermediate product (39) 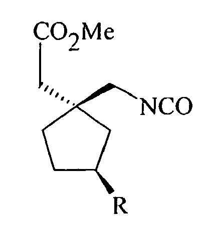 formed is further reacted, without isolation, with methanol to produce the carbamate of formula (40)

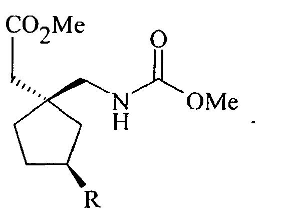

Also preferred is a process for the preparation of a compound of Formula IV, further characterized in that the intermediate product (34) 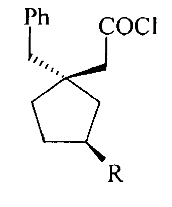 formed is further reacted, without isolation, with *tert-*butyl alcohol to produce the ester of formula (35) 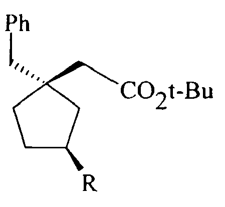 and the intermediate product (39) 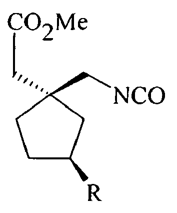 formed is further reacted, without isolation, with methanol to produce the carbamate of formula (40)

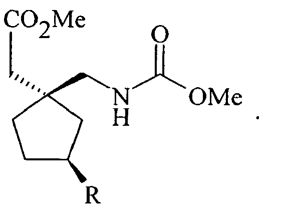

Further, the invention provides a process for the preparation of a compound of formula (6) 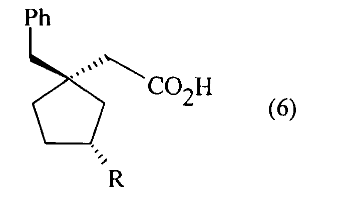 wherein R is C₁-C₁₀ alkyl or C₃-C₁₀ cycloalkyl, and pharmaceutically acceptable salts thereof, which comprises:
a) adding a cyanoacetate of formula (A) 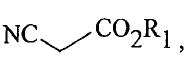 wherein R₁ is alkyl or benzyl, to a mixture of a chiral cyclopentanone of formula (1) 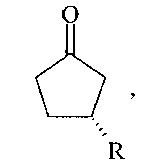 a solvent, a carboxylic acid, and a Knoevenagel reaction catalyst, and stirring the mixture in the presence of a means of removing water to produce the alkene of formula (2) 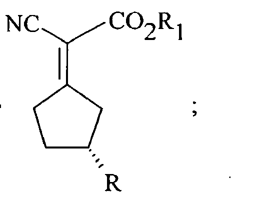
b) adding the product of Step a) above to a mixture of benzylmagnesium chloride, benzylmagnesium bromide, or benzylmagnesium iodide, in a solvent to produce the addition products of formulas (3a) 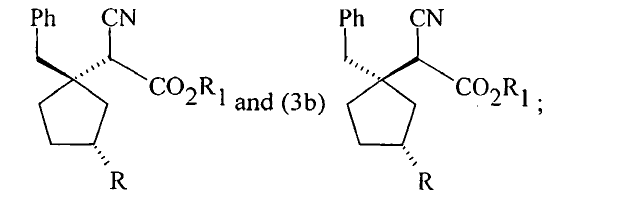
c) adding the products of Step b) above to a mixture of a base selected from potassium hydroxide, sodium hydroxide, lithium hydroxide, and cesium hydroxide, and a solvent, and stirring, and then acidifying to produce the carboxylic acids of formulas (4a) 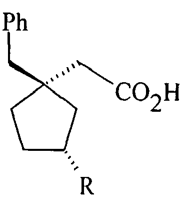 and (4b) 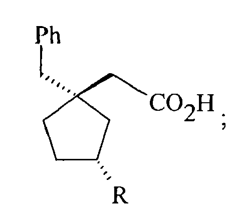 or adding the products of Step b) above to an acid mixture and stirring to produce the carboxylic acids of formulas (4a) 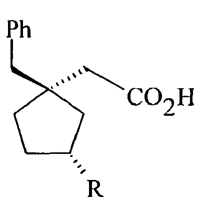 and (4b) 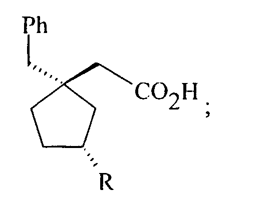
d) contacting the products of Step c) above with an amine in a solvent, and recrystallizing the salt so formed to produce the enriched diastereomer of formula (5) 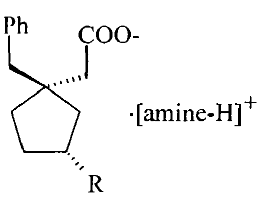 as the amine salt; and
e) converting the product of Step d) to a carboxylic acid of formula (6)

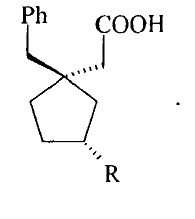

This process is outlined in Scheme 7.

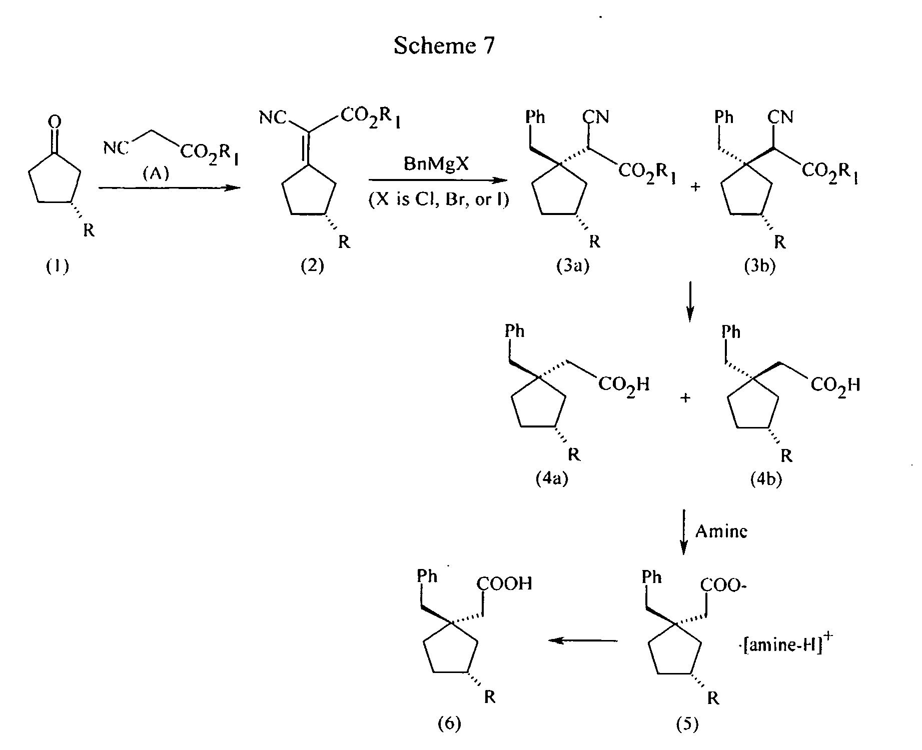

Preferred is a process for the preparation of a compound of formula (6) wherein R is C₁-C₁₀ alkyl or C₃-C₁₀ cycloalkyl, and pharmaceutically acceptable salts thereof, which comprises:
a) adding a cyanoacetate of formula (A) 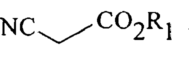 wherein R₁ is selected from methyl, ethyl, *n*-propyl, *iso*-propyl, *n*-butyl, *iso*-butyl, *sec*-butyl, *tert-*butyl, and benzyl to a mixture of a chiral cyclopentanone of formula (1) 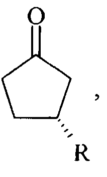 a solvent selected from tetrahydrofuran, 1,4-dioxane, *tert*-butylmethylether, chloroform, dichloromethane, acetonitrile, ethyl ether, ethyl acetate, hexanes, N,N-dimethylformamide, dimethylsulfoxide, ethanol, *tert*-butanol, toluene, benzene, xylenes, and *n*-heptane, acetic acid, and a Knoevenagel reaction catalyst selected from β-alanine, ammonium acetate, and piperidine, and stirring the mixture in the presence of a means of removing water selected from azeotropic distillation, activated molecular sieves, anhydrous magnesium sulfate, anhydrous sodium sulfate, anhydrous sodium carbonate, anhydrous potassium carbonate, anhydrous cesium carbonate, trimethyl orthoformate, and triethyl orthoformate to produce the alkene of formula (2) 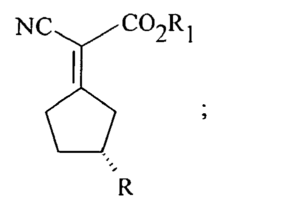
b) adding the product of Step a) above to a mixture of benzylmagnesium chloride, benzylmagnesium bromide, or benzylmagnesium iodide in a solvent selected from tetrahydrofuran, benzene, 1,4-dioxane, hexanes, *n*-heptane, toluene, diethyl ether, and *tert*-butyl methyl ether to produce the addition products of formulas (3a) 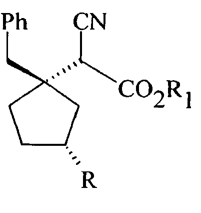 and (3b) 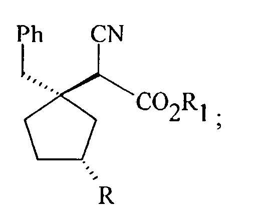
c) adding the products of Step b) above to a mixture of a base selected from potassium hydroxide, sodium hydroxide, lithium hydroxide, and cesium hydroxide in a solvent selected from ethylene glycol, 2-methoxyethyl ether, 1,4-dioxane, and diethylene glycol, and stirring the mixture, and then acidifying to produce the carboxylic acids of formulas (4a) 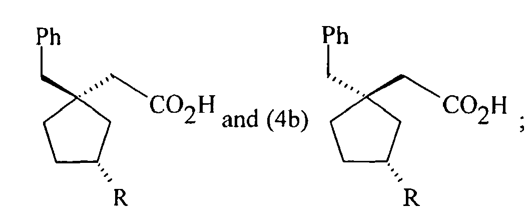 or adding the products of Step b) above to an acid mixture selected from 6-12 M HCl, 12 M H₂SO₄, 10%-48% wt/wt hydrobromic acid, and HBr in aqueous acetic acid, and stirring to produce the carboxylic acids of formulas (4a) 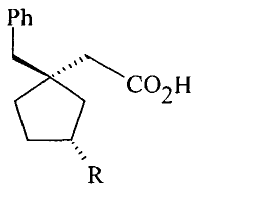 and (4b) 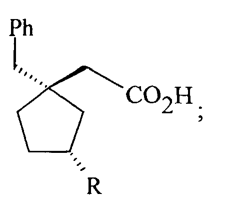
d) contacting the products of Step c) above with an amine selected from (S)-α-methyl-benzylamine, (R)-α-methyl-benzylamine, (R)-(+)-1-(naphthyl)ethylamine, (S)-(+)-1-(naphthyl)ethylamine, triethylamine, diisopropylethylamine, dicyclohexylamine, benzylamine, dibenzylamine, morpholine, N-methylmorpholine, piperidine, N-methylpiperidine, and pyridine in a solvent selected from N,N-dimethylformamide, chloroform, benzene, xylenes, hexanes, acetone, ethanol, methanol, *iso*-propanol, diethyl ether, dichloromethane, benzene, toluene, n-pentane, n-hexane, n-heptane, ethyl acetate, acetonitrile, *tert*-butyl methyl ether, tetrahydrofuran, and 1,4-dioxane, and recrystallizing the salt so formed to produce the enriched diastereomer of formula (5) 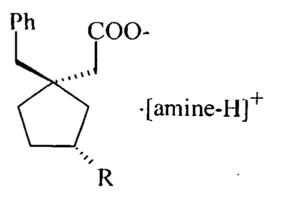 as the amine salt; and
e) adding the product of Step d) to a mixture selected from aqueous hydrochloric acid, aqueous sulfuric acid, aqueous acetic acid, hydrochloric acid dissolved in acetic acid, and hydrochloric acid dissolved in acetic acid and water, and stirring to produce the carboxylic acid of formula (6) 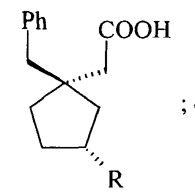 or partitioning the product of Step d) between a mixture of aqueous hydrochloric acid and a solvent selected from chloroform, dichloromethane, ethyl acetate, ethyl ether, tetrahydrofuran, 1,4-dioxane, toluene, and *tert*-butylmethylether, and drying and evaporating the organic layer to produce the carboxylic acid of formula (6)

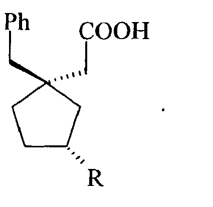

More preferred is a process for the preparation of a compound of formula (6) wherein R is C₁-C₁₀ alkyl or C₃-C₁₀ cycloalkyl, and pharmaceutically acceptable salts thereof, which comprises:
a) adding a cyanoacetate of formula (A) 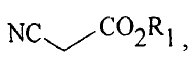 wherein R₁ is ethyl, to a mixture of a chiral cyclopentanone of formula (1) 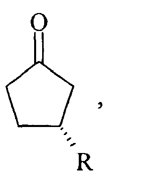 toluene, acetic acid, and a Knoevenagel reaction catalyst which is ammonium acetate, and heating the mixture at reflux over a Dean-Stark trap to produce the alkene of formula (2) 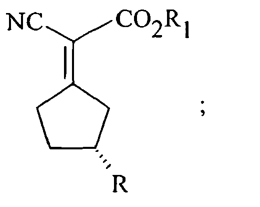
b) adding the product of Step a) above to a mixture of benzylmagnesium chloride in dry tetrahydrofuran at -100°C to 25°C to produce the addition products of formulas (3a) 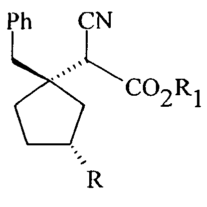 and (3b) 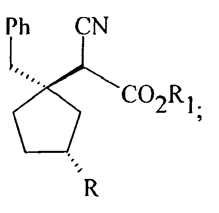
c) adding the products of Step b) above to a mixture of potassium hydroxide in ethylene glycol, and heating the mixture at 100°C to 200°C, and then acidifying to produce the hydrolysis products of formulas (4a) 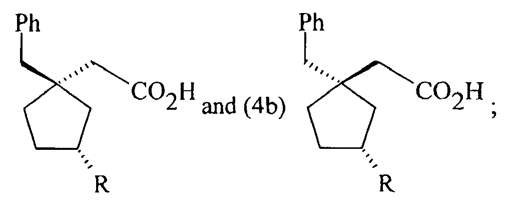
d) contacting the products of Step c) above with (S)-α-methyl-benzylamine in ethyl acetate, and recrystallizing the salt so formed from ethyl acetate to produce the enriched diastereomer of formula (5) 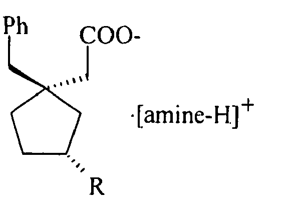 as the (S)-α-methyl-benzylamine salt;
e) adding the product of Step d) to aqueous hydrochloric acid and stirring to produce the carboxylic acid of formula (6)

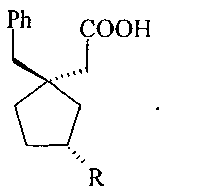

Further, the invention provides a process for the preparation of a compound of formula (26) 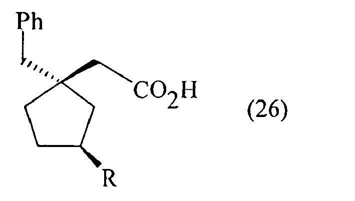 wherein R is C₁-C₁₀ alkyl or C₃-C₁₀ cycloalkyl, and pharmaceutically acceptable salts thereof, which comprises:
a) adding a cyanoacetate of formula (A) 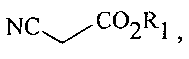 wherein R₁ is alkyl or benzyl, to a mixture of a chiral cyclopentanone of formula (21) 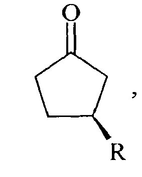 a solvent, a carboxylic acid, and a Knoevenagel reaction catalyst, and stirring the mixture in the presence of a means of removing water to produce the alkene of formula (22) 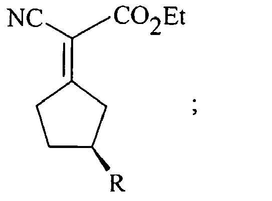
b) adding the product of Step a) above to a mixture of benzylmagnesium chloride or benzylmagnesium iodide, in a solvent to produce the addition of products of formulas (23a) 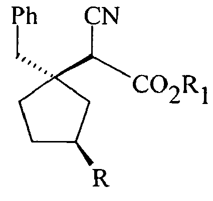 and (23b) 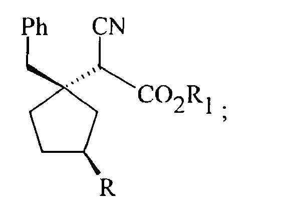
c) adding the products of Step b) above to a mixture of a base selected from potassium hydroxide, sodium hydroxide, lithium hydroxide, and cesium hydroxide, and a solvent, and stirring, and then acidifying to produce the carboxylic acids of formulas (24a) 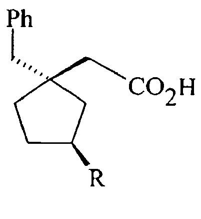 and (24b) 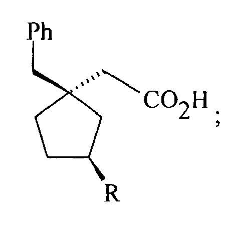 or adding the products of Step b) above to an acid mixture, and stirring to produce the carboxylic acids of formulas (24a) 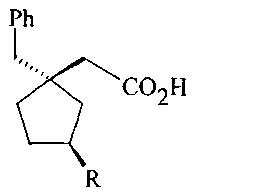 and (24b) 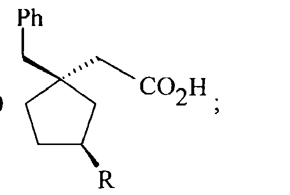
d) contacting the products of Step c) above with an amine in a solvent, and recrystallizing the salt so formed to produce the enriched diastereomer of formula (25) 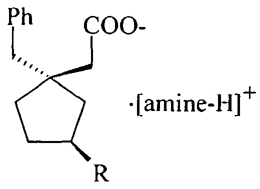 as the amine salt; and
e) converting the product of Step d) to a carboxylic acid of formula (26)

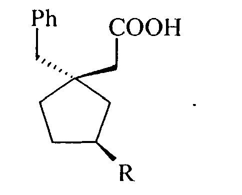

This process is outlined in Scheme 8.

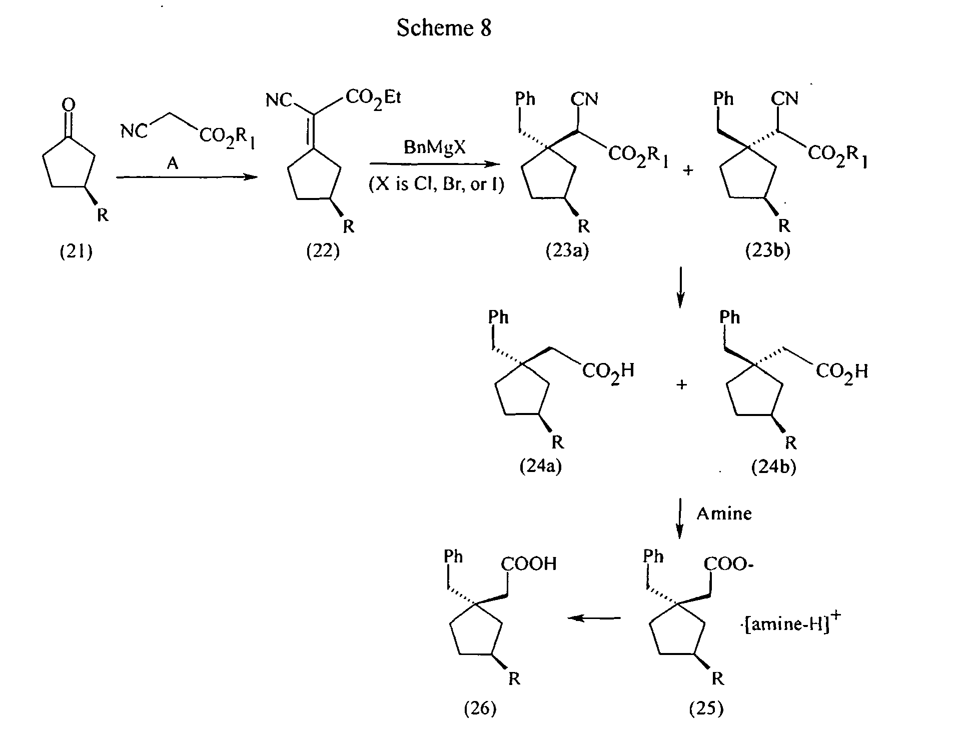

Preferred is a process for the preparation of a compound of formula (26) wherein R is C₁-C₁₀ alkyl or C₃-C₁₀ cycloalkyl, and pharmaceutically acceptable salts thereof, which comprises:
a) adding a cyanoacetate of formula (A) 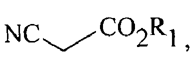 wherein R₁ is selected from methyl, ethyl, *n*-propyl, *iso*-propyl, *n*-butyl, *iso*-butyl, *sec*-butyl, *tert*-butyl, and benzyl, to a mixture of a chiral cyclopentanone of formula (21) 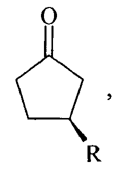 a solvent selected from tetrahydrofuran, 1,4-dioxane, *tert*-butylmethylether, chloroform, dichloromethane, acetonitrile, ethyl ether, ethyl acetate, hexanes, N,N-dimethylformamide, dimethylsulfoxide, ethanol, *tert*-butanol, toluene, benzene, xylenes, and *n*-heptane, acetic acid, and a Knoevenagel reaction catalyst selected from β-alanine, ammonium acetate, and piperidine, and stirring the mixture in the presence of a means of removing water selected from azeotropic distillation, activated molecular sieves, anhydrous magnesium sulfate, anhydrous cesium carbonate, trimethyl orthoformate, and triethyl orthoformate to produce the alkene of formula (22) 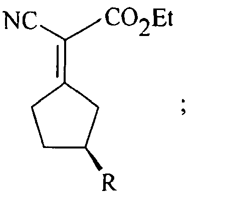
b) adding the product of Step a) above to a mixture of benzylmagnesium chloride, benzylmagnesium bromide, or benzylmagnesium iodide in a solvent selected from tetrahydrofuran, 1,4-dioxane, hexanes, *n*-heptane, toluene, diethyl ether, and *tert*-butyl methyl ether to produce the addition products of formulas (23a) 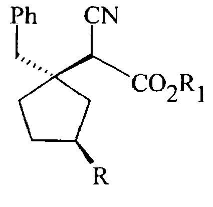 and (23b) 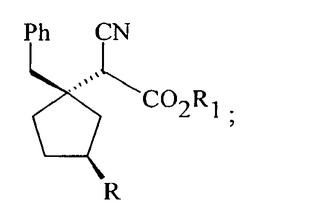
c) adding the products of Step b) above to a mixture of a base selected from potassium hydroxide, sodium hydroxide, lithium hydroxide, and cesium hydroxide in a solvent selected from ethylene glycol, 2-methoxycthyl ether, 1,4-dioxane, and diethylene glycol, and stirring the mixture, and then acidifying to produce the carboxylic acids of formulas (24a) 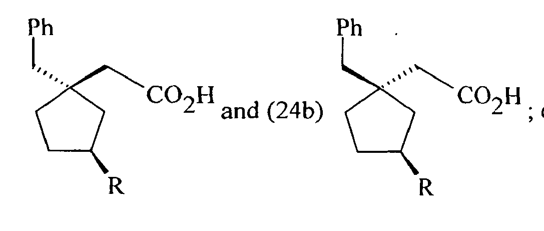 or adding the products of Step b) above to an acid mixture selected from 6-12 M HCl, 12 M H₂SO₄, 10%-48% wt/wt hydrobromic acid, and HBr in aqueous acetic acid, and stirring to produce the carboxylic acids of formulas (24a) 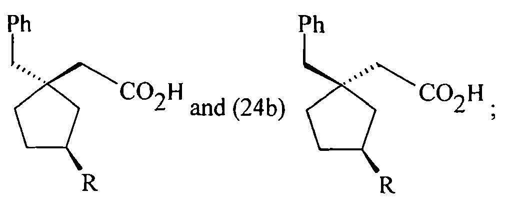
d) contacting the products of Step c) above with an amine selected from (S)-α-methyl-benzylamine, (R)-α-methyl-benzylamine, (R)-(+)-1-(naphthyl)ethylamine, (S)-(+)-1-(naphthyl)ethylamine, triethylamine, diisopropylethylamine, dicyclohexylamine, benzylamine, dibenzylamine, morpholine, N-methylmorpholine, piperidine, N-methylpiperidine, and pyridine in a solvent selected from N,N-dimethylformamide, chloroform, hexanes, acetone, ethanol, methanol, *iso*-propanol, diethyl ether, dichloromethane, benzene, toluene, n-pentane, n-hexane, n-heptane, ethyl acetate, acetonitrile, *tert*-butyl methyl ether, tetrahydrofuran, and 1,4-dioxane, and recrystallizing the salt so formed to produce the enriched diastereomer of formula (25) 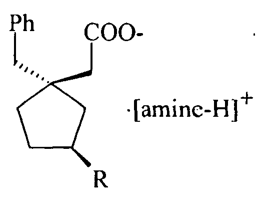 as the amine salt; and
e) adding the product of Step d) to a mixture selected from aqueous hydrochloric acid, aqueous sulfuric acid, aqueous acetic acid, hydrochloric acid dissolved in acetic acid, and hydrochloric acid dissolved in acetic acid and water, and stirring to produce the carboxylic acid of formula (26) 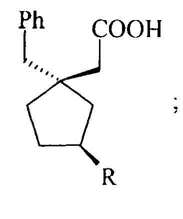 or partitioning the product of Step d) between a mixture of aqueous hydrochloric acid and a solvent selected from chloroform, dichloromethane, ethyl acetate, ethyl ether, tetrahydrofuran, 1,4-dioxane, toluene, and *tert*-butylmethylether, and drying and evaporating the organic layer to produce the carboxylic acid of formula (26)

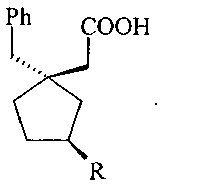

More preferred is a process for the preparation of a compound of formula (26) wherein R is C₁-C₁₀ alkyl or C₃-C₁₀ cycloalkyl, and pharmaceutically acceptable salts thereof, which comprises:
a) adding a cyanoacetate of formula (A) 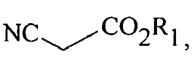 wherein R₁ is ethyl, to a mixture of a chiral cyclopentanone of formula (21) 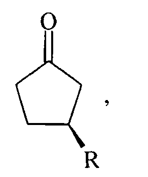 toluene, acetic acid, and a Knoevenagel reaction catalyst which is ammonium acetate, and heating the mixture at reflux over a Dean-Stark trap to produce the alkene of formula (22) 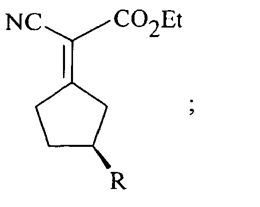
b) adding the product of Step a) above to a mixture of benzylmagnesium chloride in dry tetrahydrofuran at -100°C to -20°C to produce the addition products of formulas (23a) 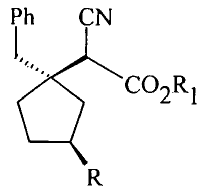 and (23b) 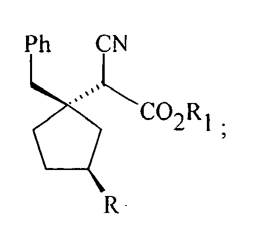
c) adding the products of Step b) above to a mixture of potassium hydroxide in ethylene glycol, and heating the mixture at 100°C to 200°C, and then acidifying to produce the hydrolysis products of formulas (24a) 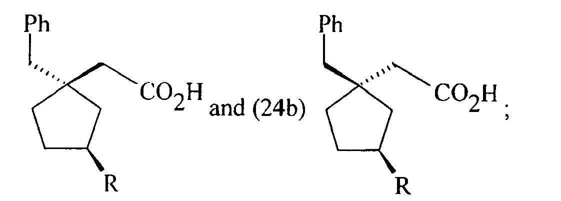
d) contacting the products of Step c) above with (R)-α-methyl-benzylamine in ethyl acetate, and recrystallizing the salt so formed from ethyl acetate to produce the enriched diastereomer of formula (25) 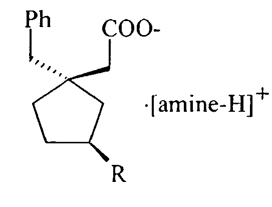 as the (R)-α-methyl-benzylamine salt; and
e) adding the product of Step d) to aqueous hydrochloric acid and stirring to produce the carboxylic acid of formula (26)

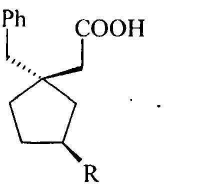

Further, the invention provides a key intermediate of formula (6) 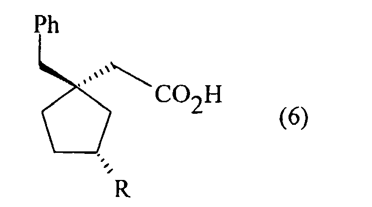 wherein R is C₁-C₁₀ alkyl or C₃-C₁₀ cycloalkyl, and pharmaceutically acceptable salts thereof.

Preferred is a compound of formula (6) and pharmaceutically acceptable salts thereof wherein R is C₁-C₁₀ alkyl.

More preferred is a compound of formula (6) and pharmaceutically acceptable salts thereof wherein R is selected from methyl, ethyl, and n-propyl.

Still more preferred is a compound of formula (6) named ((1S,3R)-1-benzyl-3-methyl-cyclopentyl)-acetic acid.

Further, the invention provides a key intermediate of formula (26) 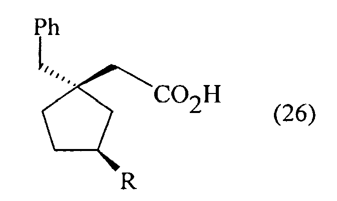 wherein R is C₁-C₁₀ alkyl or C₃-C₁₀ cycloalkyl and pharmaceutically acceptable salts thereof.

Preferred is a compound of formula (26) and pharmaceutically acceptable salts thereof wherein R is C₁-C₁₀ alkyl.

More preferred is a compound of formula (26) and pharmaceutically acceptable salts thereof wherein R is selected from methyl, ethyl, and n-propyl.

Still more preferred is a compound of formula (26) named ((1R,3S)-1-benzyl-3-methyl-cyclopentyl)-acetic acid.

Further, the invention provides a compound of Formula I, wherein R is as defined above, prepared according to any one of the processes for the preparation of a compound of Formula I described above.

Preferred is a compound of Formula I, wherein R is C₁-C₁₀ alkyl, prepared according to any one of the processes for the preparation of a compound of Formula I described above.

More preferred is a compound of Formula I, wherein R is selected from methyl, ethyl, and *n*-propyl, prepared according to any one of the processes for the preparation of a compound of Formula I described above.

Still more preferred is a compound of Formula I selected from:
((1R,3S)-1-aminomethyl-3-methyl-cyclopentyl)-acetic acid; and
((1R,3S)-1-aminomethyl-3-methyl-cyclopentyl)-acetic acid hydrochloride, prepared according to any one of the processes for the preparation of a compound of Formula I described above.

Further, the invention provides a compound of Formula II, wherein R is as defined above, prepared according to any one of the processes for the preparation of a compound of Formula II described above.

Preferred is a compound of Formula II, wherein R is C₁-C₁₀ alkyl, prepared according to any one of the processes for the preparation of a compound of Formula II described above.

More preferred is a compound of Formula II, wherein R is selected from methyl, ethyl, and *n*-propyl, prepared according to any one of the processes for the preparation of a compound of Formula II described above.

Still more preferred is a compound of Formula II selected from:
((1S,3R)-1-aminomethyl-3-methyl-cyclopentyl)-acetic acid; and
((1S,3R)-1-aminomethyl-3-methyl-cyclopentyl)-acetic acid hydrochloride, prepared according to any one of the processes for the preparation of a compound of Formula II described above.

Further, the invention provides a compound of Formula III, wherein R is as defined above, prepared according to any one of the processes for the preparation of a compound of Formula III described above.

Further, the invention provides a compound of Formula IV, wherein R is as defined above, prepared according to any one of the processes for the preparation of a compound of Formula IV described above.

Further, the invention provides a compound of formula (6), wherein R is as defined above, prepared according to any one of the processes for the preparation of a compound of formula (6) described above.

Preferred is a compound of formula (6), wherein R is C₁-C₁₀ alkyl, prepared according to any one of the processes for the preparation of a compound of formula (6) described above.

More preferred is a compound of formula (6), wherein R is selected from methyl, ethyl, and *n*-propyl, prepared according to any one of the processes for the preparation of a compound of formula (6) described above.

Still more preferred is a compound of formula (6) named ((1S,3R)-1-benzyl-3-methyl-cyclopentyl)-acetic acid, prepared according to any one of the processes for the preparation of a compound of formula (6) described above.

Further, the invention provides a compound of formula (26), wherein R is as defined above, prepared according to any one of the processes for the preparation of a compound of formula (26) described above.

Preferred is a compound of formula (26), wherein R is C₁-C₁₀ alkyl, prepared according to any one of the processes for the preparation of a compound of formula (26) described above.

More preferred is a compound of formula (26), wherein R is selected from methyl, ethyl, and *n*-propyl, prepared according to any one of the processes for the preparation of a compound of formula (26) described above.

Still more preferred is a compound of formula (26) named ((1R,3S)-I-benzyl-3-methyl-cyclopentyl)-acetic acid, prepared according to any one of the processes for the preparation of a compound of formula (26) described above.

Further, the invention provides a compound of Formula I selected from:
((1R,3R)-1-aminomethyl-3-methyl-cyclopentyl)-acetic acid;
((1R,3R)-1-aminomethyl-3-methyl-cyclopentyl)-acetic acid hydrochloride;
((1R,3R)-1-aminomethyl-3-ethyl-cyclopentyl)-acetic acid;
((1R,3R)-1-aminomethyl-3-ethyl-cyclopentyl)-acetic acid hydrochloride;
((1R,3R)-1-aminomethyl-3-propyl-cyclopentyl)-acetic acid; and
((1R,3R)-1-aminomethyl-3-propyl-cyclopentyl)-acetic acid hydrochloride.

Further, the invention provides a compound of Formula II selected from:
((1S,3R)-1-aminomethyl-3-methyl-cyclopentyl)-acetic acid;
((1S,3R)-1-aminomethyl-3-methyl-cyclopentyl)-acetic acid hydrochloride;
((1S,3R)-1-aminomethyl-3-ethyl-cyclopentyl)-acetic acid;
((1S,3R)-1-aminomethyl-3-ethyl-cyclopentyl)-acetic acid hydrochloride;
((1S,3R)-1-aminomethyl-3-propyl-cyclopentyl)-acetic acid; and
((1S,3R)-1-aminomethyl-3-propyl-cyclopentyl)-acetic acid hydrochloride.

Further, the invention provides compounds selected from:
E-Cyano-((R)-3-methyl-cyclopentylidene)-acetic acid ethyl ester;
Z-Cyano-((R)-3-methyl-cyclopentylidene)-acetic acid ethyl ester;
(R)-((1S,3R)-)-Benzyl-3-methyl-cyclopentyl)-cyano-acetic acid ethyl ester;
(S)-((1S,3R)-1-Benzyl-3-methyl-cyclopentyl)-cyano-acetic acid ethyl ester;
(R)-((1R,3R)-1-Benzyl-3-methyl-cyclopentyl)-cyano-acetic acid ethyl ester;
(S)-((1R,3R)-1-Benzyl-3-methyl-cyclopentyl)-cyano-acetic acid ethyl ester;
((1S,3R)-1-Isocyanatomethyl-3-methyl-cyclopentylmethyl)-benzene;
((1S,3R)-1-Benzyl-3-methyl-cyclopentylmethyl)-carbamic acid methyl ester;
[(1S,3R)-1-(Methoxycarbonylamino-methyl)-3-methyl-cydopentyl]-acetic acid;
((1S,3R)-1-Benzyl-3-methyl-cyclopentyl)-acetic acid methyl ester;
(1S,3R)-1-Methoxycarbonylmethyl-3-methyl-cyclopentyl)-acetic acid;
((1R,3R)-1-Isocyanatomethyl-3-methyl-cyclopentyl)-acetic acid methyl ester;
[(1R,3R)-1-(Methoxycarbonylamino-methyl)-3-methyl-cyclopentyl]-acetic acid methyl ester;
((1S,3R)-1-Benzyl-3-methyl-cyclopentyl)-acetic acid *tert*-butyl ester;
[(1S,3R)-1-Carboxymethyl-3-methyl-cyciopentyl]-acetic acid *tert*-butyl ester;
[(1S,3R)-1-Methoxycarbonylmethyl-3-methyl-cyclopentyl]-acetic acid *tert*-butyl ester;
((1R,3K)-1-Methoxycarbonylmethyl-3-methyl-cyclopentyl)-acetic acid;
((1S,3R)-1-Isocyanatomethyl-3-methyl-cyclopentyl)-acetic acid methyl ester; and
[(1S,3R)-1-(Methoxycarbonylamino-methyl)-3-methyl-cyclopentyl]-acetic acid methyl ester.

More preferred is a process for the preparation of a compound of formula (4a) 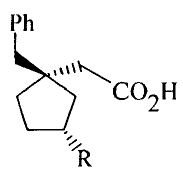 wherein R is C₁-C₁₀ alkyl or C₃-C₁₀ cycloalkyl, comprising, hydrolyzing a compound of formula (3a) 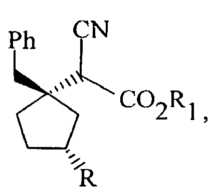 wherein R₁ is H, alkyl, or benzyl.

More preferred is a process for the preparation of a compound of formula (24a) 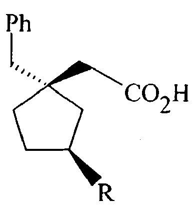 wherein R is C₁-C₁₀ alkyl or C₃-C₁₀ cycloalkyl, comprising, hydrolyzing a compound of formula (23a) 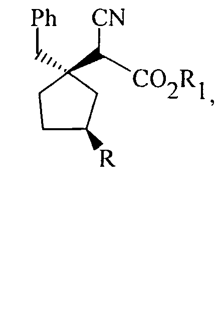 wherein R₁ is H, alkyl, or benzyl.

More preferred is a process for the preparation of a compound of formula (6) 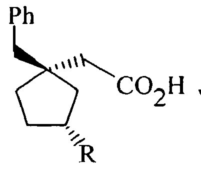 wherein R is C₁-C₁₀ alkyl or C₃-C₁₀ cycloalkyl, comprising, resolving a mixture containing compounds of formulas (4a) 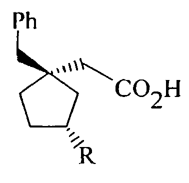 and (4b) 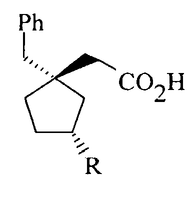 wherein R is C₁-C₁₀ alkyl or C₃-C₁₀ cycloalkyl.

More preferred is a process for the preparation of a compound of formula (26) 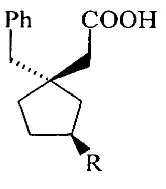 wherein R is C₁-C₁₀ alkyl or C₃-C₁₀ cycloalkyl, comprising, resolving a mixture containing compounds of formulas (24a) 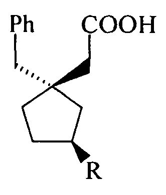 and (24b) 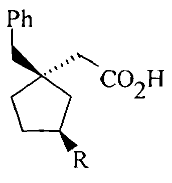 wherein R is C₁-C₁₀ alkyl or C₃-C₁₀ cycloalkyl.

More preferred is a process for the preparation of a compound of Formula I 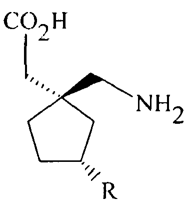 wherein R is C₁-C₁₀ alkyl or C₃-C₁₀ cycloalkyl, and pharmaceutically acceptable salt thereof, comprising, hydrolyzing a compound of formula (41) 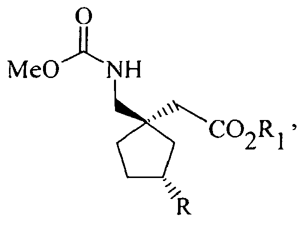 wherein R₁ is H, alkyl, or benzyl, and contacting the product, if desired, with an acid or a base.

More preferred is a process for the preparation of a compound of Formula II 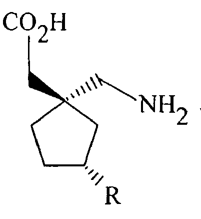 wherein R is C₁-C₁₀ alkyl or C₃-C₁₀ cycloalkyl, and pharmaceutically acceptable salt thereof, comprising, hydrolyzing a compound of formula (42) 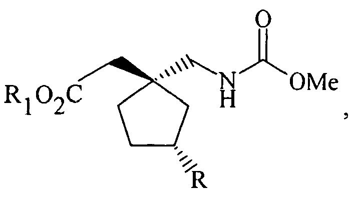 wherein R₁ is H, alkyl, or benzyl, and contacting the product, if desired, with an acid or a base.

More preferred is a process for the preparation of a compound of Formula III 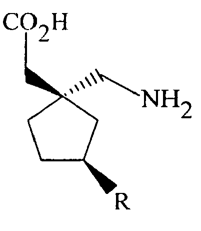 wherein R is C₁-C₁₀ alkyl or C₃-C₁₀ cycloalkyl, and pharmaceutically acceptable salt thereof, comprising, hydrolyzing a compound of formula (43) 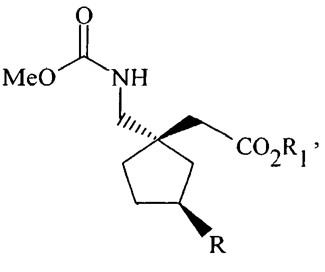 wherein R₁ is H, alkyl, or benzyl, and contacting the product, if desired, with an acid or a base.

More preferred is a process for the preparation of a compound of Formula IV 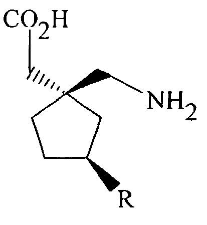 wherein R is C₁-C₁₀ alkyl or C₃-C₁₀ cycloalkyl, and pharmaceutically acceptable salt thereof, comprising, hydrolyzing a compound of formula (44) 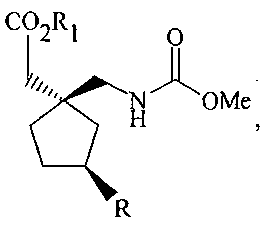 wherein R₁ is H, alkyl, or benzyl, and contacting the product, if desired, with an acid or a base.

### DETAILED DESCRIPTION OF THE INVENTION

The instant invention is an important process as it permits the synthesis of single isomers; it is a route to stereospecific 3-substituted 5-membered rings of formula

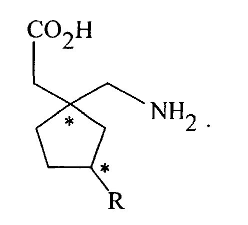

A key feature of the invention is the stereoselective preparation of a compound of formula (6) by selective fractional crystallization of a salt of formula (5) from a mixture of compounds of formulas (4a) and (4b), and conversion of a salt of formula (5) to a compound of formula (6). Another feature of the invention is the conversion of a compound of formula (2) to a mixture of compounds of formulas (3a) and (3b) wherein the yield of a compound of formula (3a) over a diastereomer of formula (3b) may be enhanced by optimizing certain reaction parameters such as, for example, temperature. Reaction of a compound of formula (2) at a relatively low temperature generally provides for a relatively higher yield of a compound of formula (3a) over (3b) than when the reaction is run at a higher temperature. The invention also provides for translation of the stereochemistry at the two chiral carbons of the cyclopentane ring of the resulting pure enantiomer of formula (6) into enantiomerically pure compounds of Formulas I or II with little or no racemization.

Another key feature of the invention is the stereoselective preparation of a compound of formula (26) by selective fractional crystallization of salt of formula (25) from a mixture of compounds of formulas (24a) and (24b), and conversion of a salt of formula (25) to a compound of formula (26). Another feature of the invention is the conversion of a compound of formula (22) to a mixture of compounds of formulas (23a) and (23b) wherein the yield of a compound of formula (23a) over a diastereomer of formula (23b) may be enhanced by optimizing certain reaction parameters such as, for example, temperature. Reaction of a compound of formula (22) at a relatively low temperature generally provides for a relatively higher yield of a compound of formula (23a) over (23b) than when the reaction is run at a higher temperature. The invention also provides for translation of the stereochemistry at the two chiral carbons of the cyclopentane ring of the resulting pure enantiomer of formula (26) into enantiomerically pure compounds of Formulas III or IV with little or no racemization.

The final products are useful as agents in the treatment of epilepsy, faintness attacks, hypokinesia, cranial disorders, neurodegenerative disorders, depression, anxiety, panic, pain, neuropathological disorders, gastrointestinal disorders such as irritable bowel syndrome (IBS), inflammation especially arthritis, sleep disorders, premenstrual syndrome, and hot flashes.

The following experimental procedures provide a novel route to be used to stereoselectively synthesize 3-substituted cyclopentyl-based analogs of gabapentin and pharmaceutically acceptable salts thereof. These routes provide access to pure stereoisomers of Formulas I, II, III, and IV 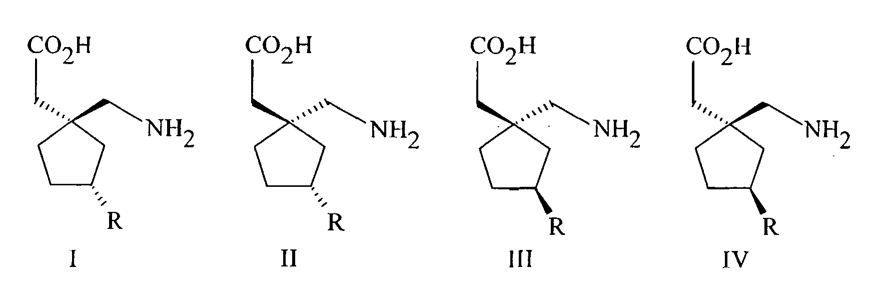 wherein R is C₁-C₁₀ alkyl or C₃-C₁₀ cycloalkyl.

Examples 1 and 3 below each show a synthesis of a compound of Formula II wherein R is methyl.

Example 2 below shows a synthesis of a compound of Formula I wherein R is methyl.

It is understood that compounds of Formulas I, II, III, or IV, or a pharmaceutically acceptable salt thereof, produced by a hydrolysis reaction such as, for example, step j) in the above process for the preparation of a compound of Formula I, or a pharmaceutically acceptable salt thereof, or the process described above wherein a compound of formula (41) is hydrolyzed, may be formed as an acid or base salt thereof, which salt may be optionally converted to a free amino acid form or a pharmaceutically acceptable salt form thereof by methods well known to a skilled person in the pharmaceutical or chemical arts.

The following terms are defined as used herein.

As used herein the term "C₁-C₁₀ alkyl" means a straight or branched alkyl group or radical containing from 1 to 10 carbon atoms. Illustrative examples of C₁-C₁₀ alkyl include methyl, ethyl, 1-propyl, 2-propyl, 1-butyl, 2-butyl, 2-methyl-1-propyl, 1,1-dimethylethyl, 1-pentyl, 2-pentyl, 3-pentyl, 2,2-dimethylpropyl, 1-hexyl, 2-hexyl, 3-hexyl, 4-methyl-1-pentyl, 1-heptyl, 2-heptyl, 3-heptyl, 4-heptyl, 5-methyl-1-hexyl, 1-octyl, 2-octyl, 3-octyl, 4-octyl, 6-methyl-1-heptyl, 5,5-dimethylhexyl, 1-nonyl, 2-nonyl, 1-decyl, and 2-decyl.

The term "C₃-C₁₀ cycloalkyl" means a cycloalkyl group or radical having from 3 to 10 carbon atoms. Illustrative examples of a C₃-C₁₀ cycloalkyl include cyclopropyl, cyclobutyl, cyclopentyl, cyclohexyl, cycloheptyl, cyclooctyl, cyclononyl and cyclodecyl.

The term "stereoisomer" means any of a group of isomers in which identical atoms are linked in the same order but differ in their spatial arrangement.

| | |
|---|---|
| AcOH | Acetic acid |
| Alkali hydroxide | LiOH, NaOH, KOH, or CsOH |
| NH₄OAc | Ammonium acetate |
| BnMgCl or PhCH₂MgCl | Benzylmagnesium chloride |
| *t*-BuOH or *tert*-butyl alcohol | 1,1-Dimethylethanol |
| ^{*t*}Butyl | 1,1-Dimethylethyl |
| CH₂Cl₂ | Dichloromethane |
| CCl₄ | Carbon tetrachloride |
| CDCl₃ | Deuterochloroform |
| (CH₃))SiCHN₂ | Trimethylsilyldiazomethane |
| CN | Carbon-nitrogen triple bond (nitrile) |
| (COCl)₂ | Oxalyl chloride |
| CsOH | Cesium hydroxide |
| de | Diastereomeric excess |
| DBU | 1,8-Diazabicyclo[5.4.0]undec-7-ene |
| DMF | N,N-Dimethylformamide |
| DMSO | Dimethylsulfoxide |
| DMSO-*d*₆ | Deuterated dimethylsulfoxide |
| ee | Enantiomeric excess |
| Et | Ethyl |
| EtOAc | Ethyl acetate |
| Et₃N | Triethylamine |
| HCl | Hydrogen chloride |
| HCl (aq) | Hydrochloric acid |
| 6*N* HCl 6 | normal hydrochloric acid |
| HCl (g) | Hydrogen chloride (gaseous) |
| ¹H-NMR | Proton (nuclear) magnetic resonance |
| | spectroscopy |
| IR | Infrared spectroscopy |
| *J* | Coupling constant in Hz |
| KOH | Potassium hydroxide |
| LCMS | Liquid chromatography-mass spectrometry |
| LiOH | Lithium hydroxide |
| Me | Methyl |
| MeO | Methoxy |
| MeCN | Acetonitrile |
| Mel | Iodomethane |
| MeOH | Methanol |
| MgSO₄ | Magnesium sulfate |
| MS (ES⁺) | Positive ion electrospray mass spectrometry |
| MS (ES-) | Negative ion electrospray mass spectrometry |
| MS (CI⁺) | Positive ion chemical ionization mass spectrometry |
| MS (CI⁻) | Negative ion chemical ionization mass spectrometry |
| *m*/*z* | mass per unit charge |
| NCCH₂CO₂Et | Ethyl cyanoacetate |
| NaIO₄ | Sodium periodate |
| NaOH | Sodium hydroxide |
| ODS | Octadecyl-functionalized silica gel |
| Ph | Phenyl |
| (*i*-Pr)₂NEt | Diisopropylethylamine |
| R_{f} | R_{f} value |
| RuCl₃ | Ruthenium(III) chloride |
| SOCl₂ | Thionyl chloride |
| TFA or CF₃CO₂H | Trifluoroacetic acid |
| THF | Tetrahydrofuran |

| | |
|---|---|
| * Asterisk symbol points out an enantiomerically enriched chiral carbon atom | |

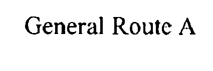

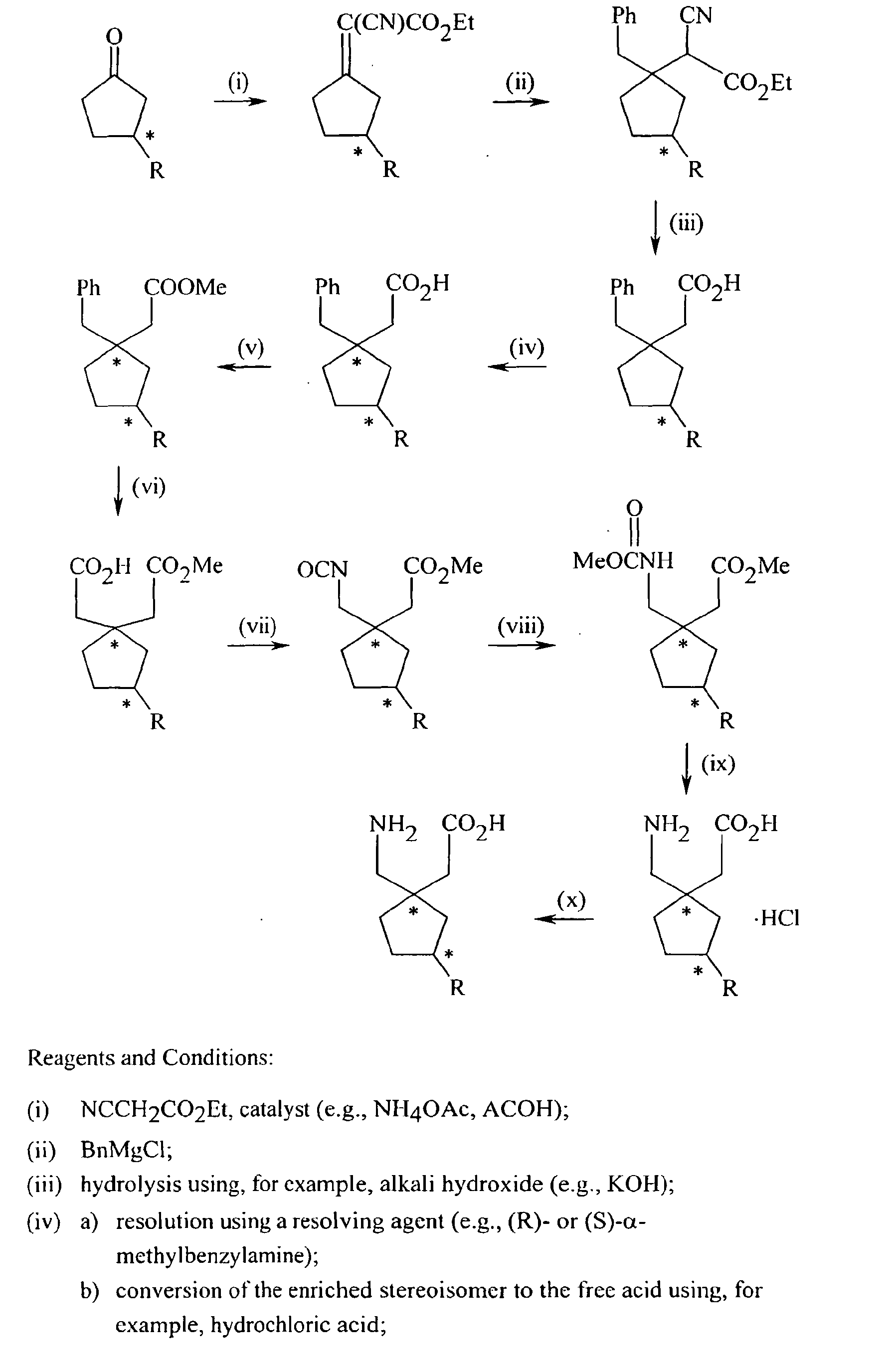

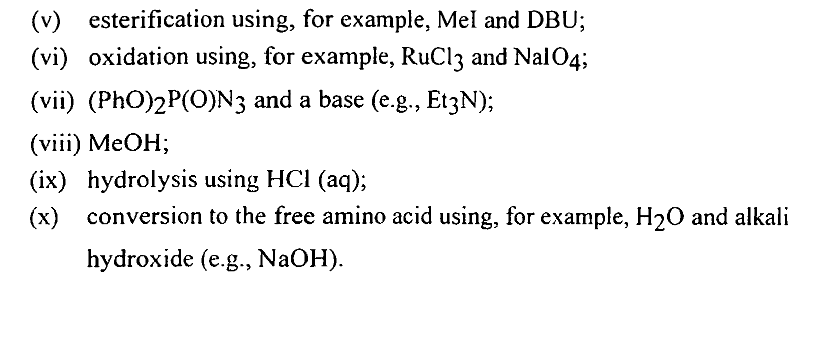

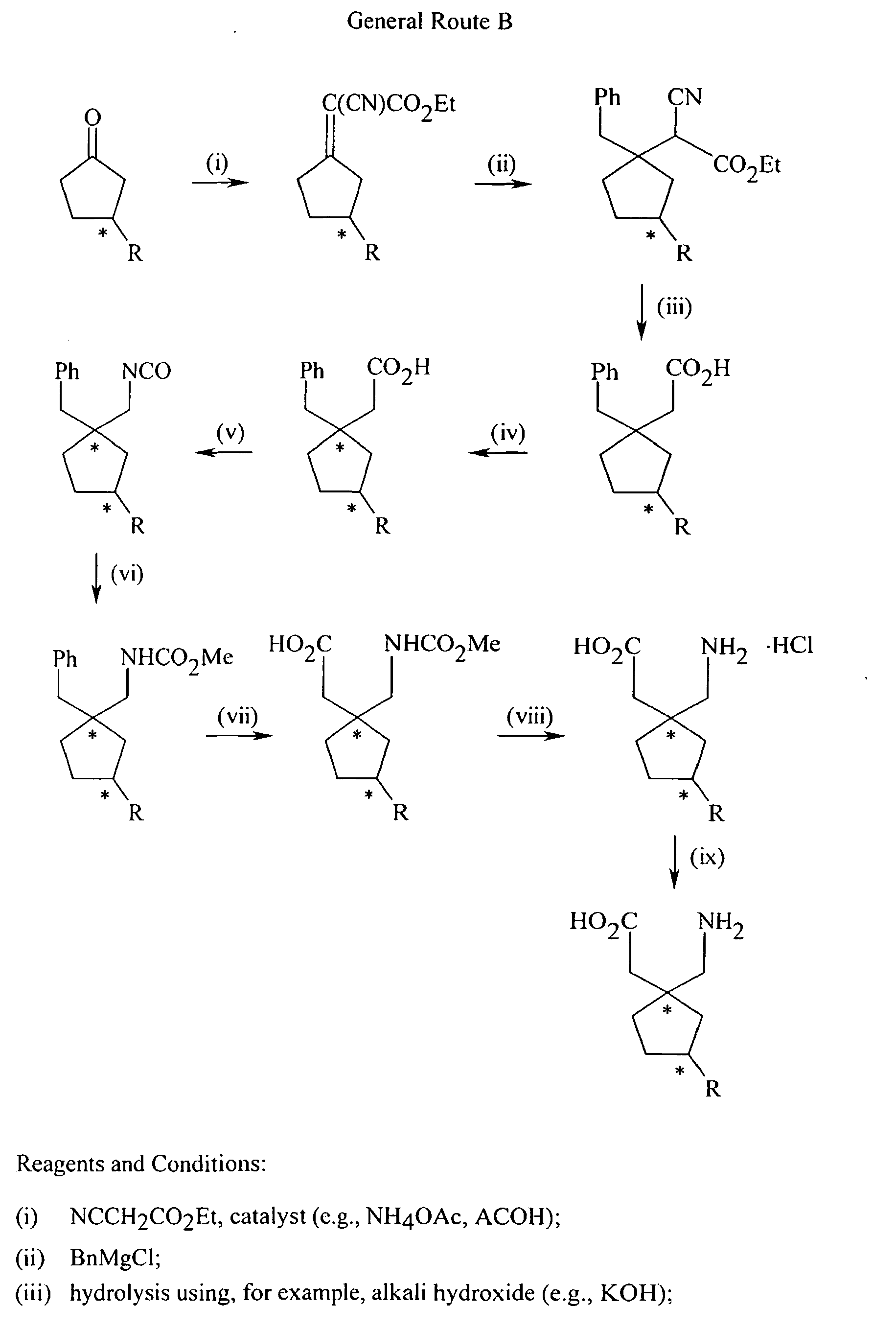

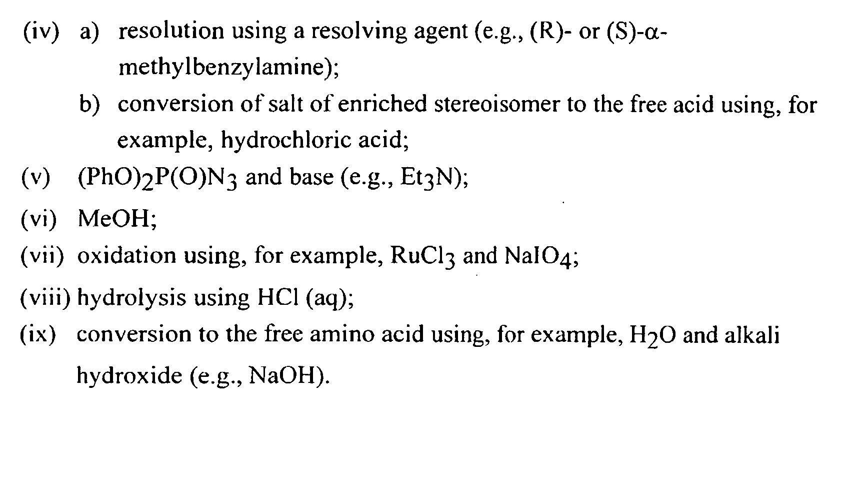

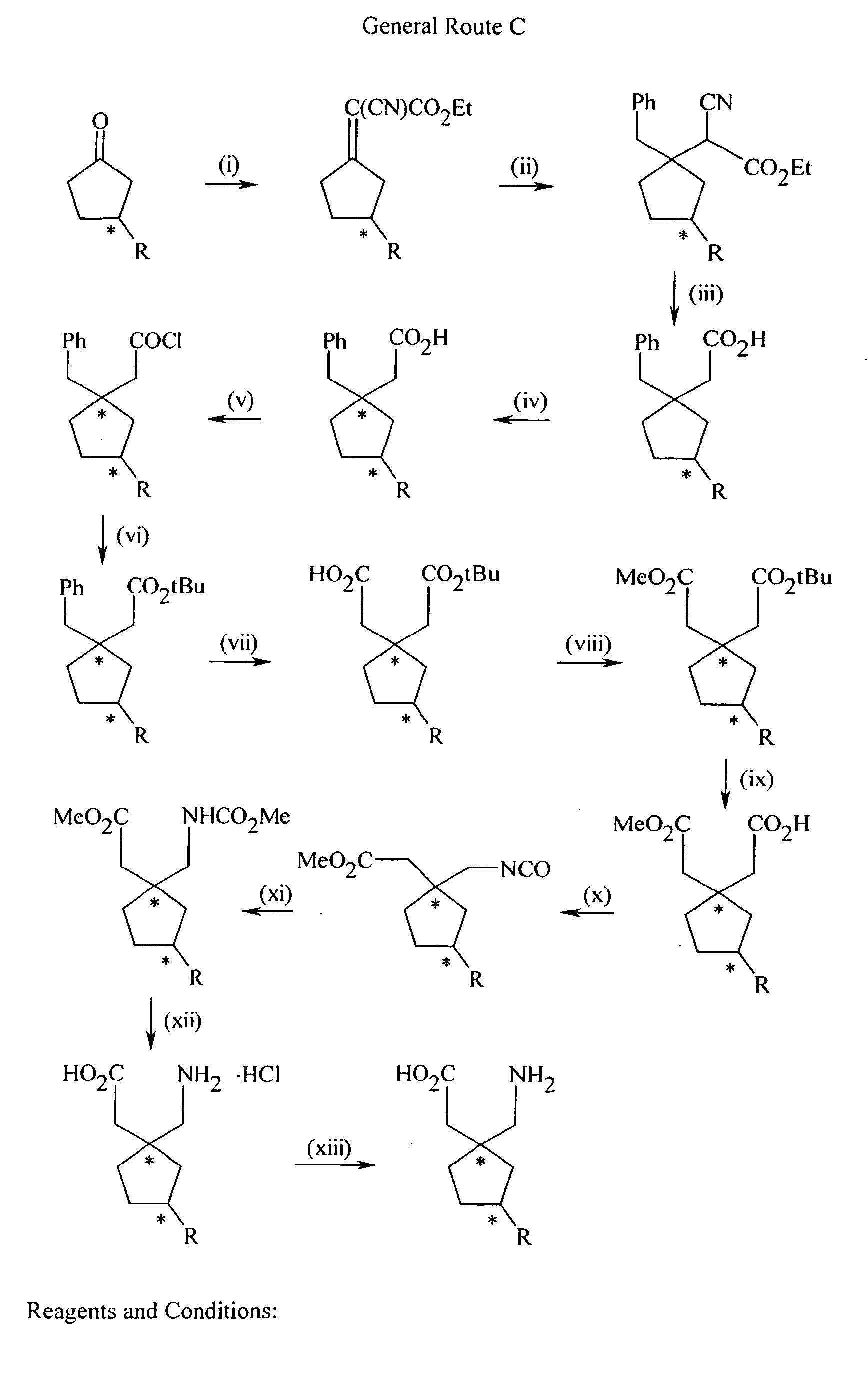

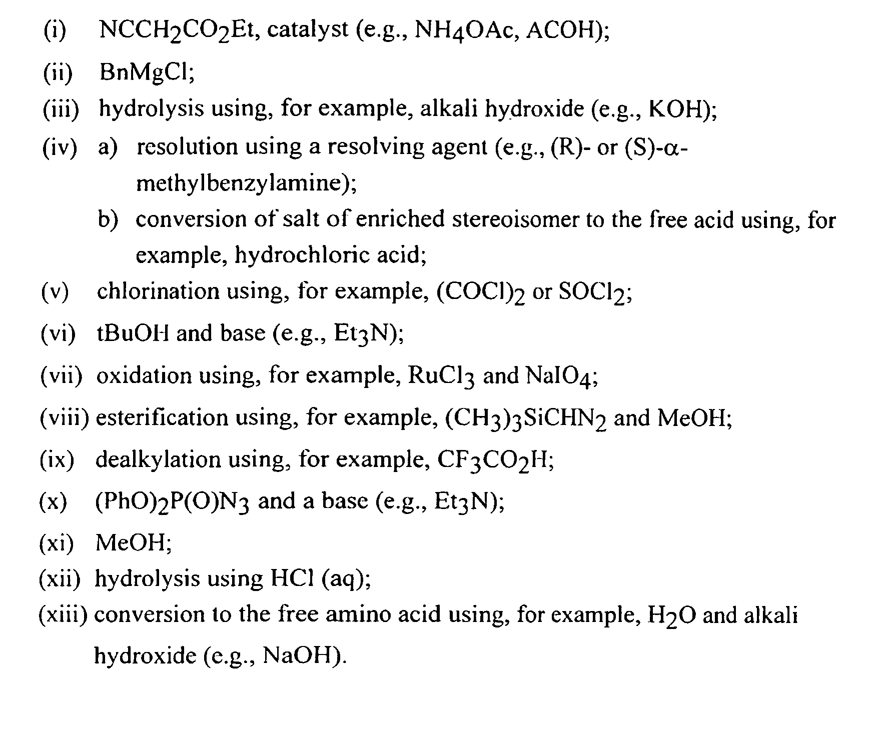

### EXAMPLE 1

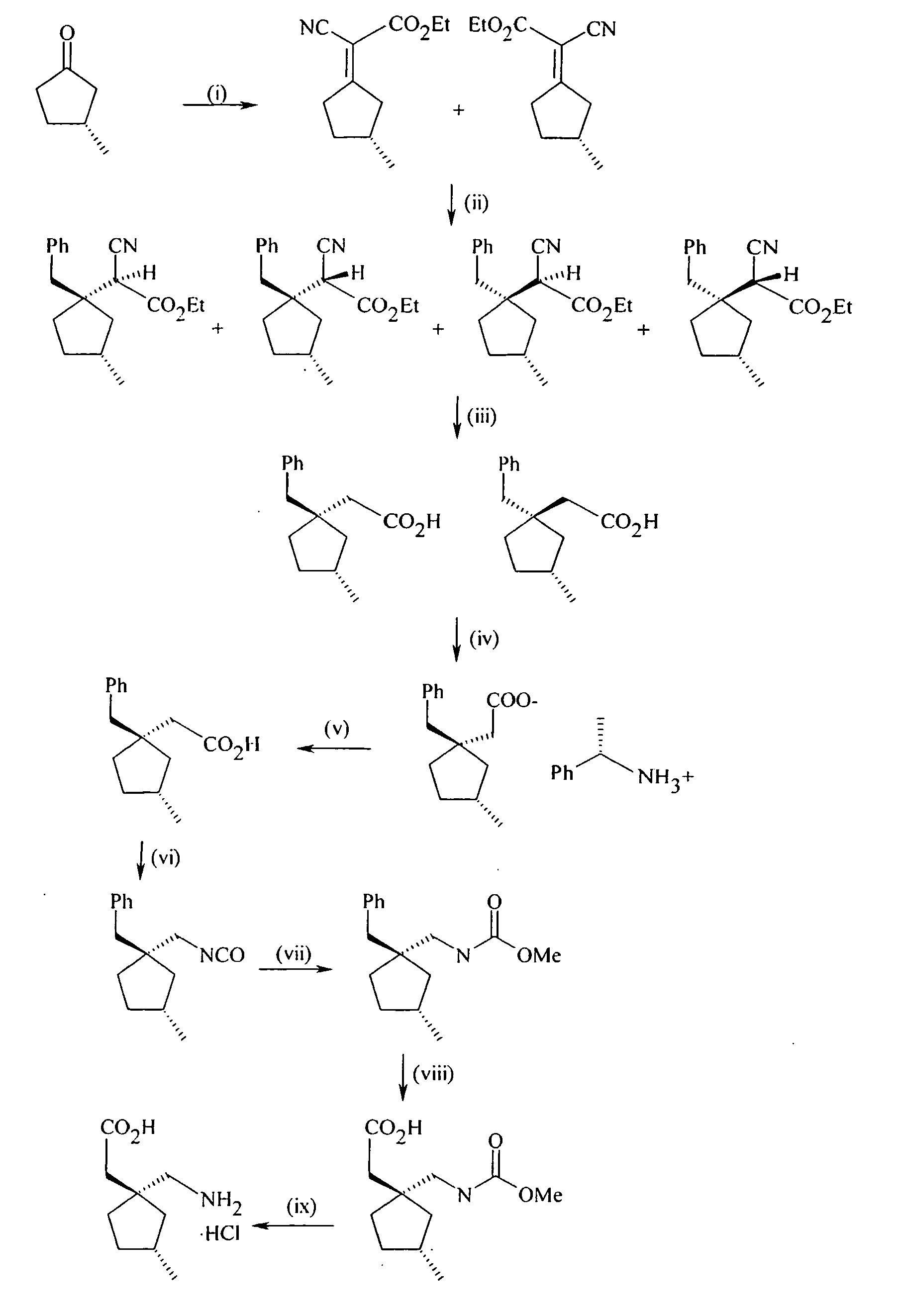

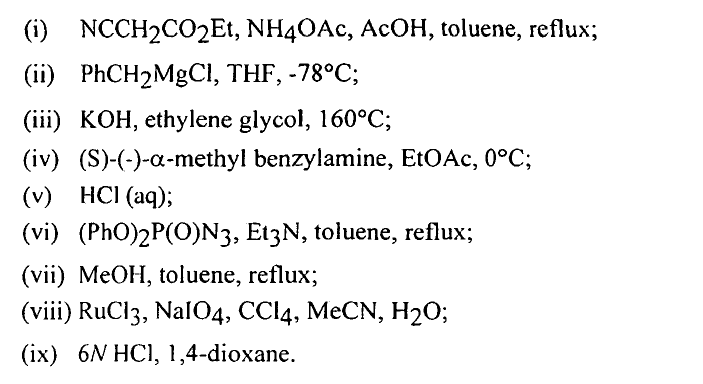

### (E and Z)-Cyano-((R)-3-methyl-cyclopentylidene)-acetic acid ethyl ester

(R)-(+)-3-Methylcyclopentanone (5 g, 51.0 mmol), ethyl cyanoacetate (5.42 mL, 51.0 mmol), ammonium acetate (0.4 g, 5.1 mmol), and glacial acetic acid (0.58 mL, 10.2 mmol) were refluxed in toluene (30 mL) using a Dean-Stark trap. After 6 hours, the mixture was allowed to cool and diluted with ethyl acetate (100 mL), washed with water (3 x 80 mL), brine, and dried (MgSO₄). The solvent was evaporated under reduced pressure. The residue was chromatographed (silica gel, heptane/ethyl acetate, 9:1) to give 8.87 g (90%) of a 1:1 mixture of (E and Z)-cyano-((R)-3-methyl-cyclopentylidene)-acetic acid ethyl ester;
R_{f} (heptane-ethyl acetate, 9:1) 0.28;
IR thin film(cm⁻¹) 2225 (CN), 1724 (C=O), 1617 (C=C);
¹H-NMR (400 MHz; CDCl₃): δ 4.27 (2H, q, *J* 7.2, CO₂C*H*_{*2*}Me), 4.26 (2H, q, *J* 7.2, CO₂*CH*_{*2*}Mc), 3.35 (1H, dt, *J* 7.1, 1.6), 3.30 (1H, dt, *J* 7.1, 1.6), 3.23 (1H, ddd, *J*8*.*1, 3.5, 1.7), 3.18 (1H, ddd, *J*8.1, 3.4, 1.7), 3.05-2.67 (4H, m), 2.50-2.32 (2H, m), 2.29-1.96 (4H, m), 1.50-1.35 (2H, m), 1.34 (3H, t, *J7.2,* CO₂CH₂*Me*), 1.33 (3H, t, *J* 7.1, CO₂CH₂*Me*), 1.10 (3H, d, *J* 6.6, Me), 1.08 (3H, d, *J* 6.6, Me);
MS (ES-): *m*/*z* 192 (M-H, 100%).

### (R and S)-((1S,3R)-1-Benzyl-3-methyl-cyclopentyl)-cyano-acetic acid ethyl ester and (R and S)-((1R,3R)-1-Benzyl-3-methyl-cyclopentyl)-cyano-acetic acid ethyl ester

A mixture of (E and Z)-cyano-((R)-3-methyl-cyclopentylidene)-acetic acid ethyl ester (4.13 g, 21.4 mmol) in THF (30 mL) was added over 1 hour to a stirring solution of benzylmagnesium chloride (27.7 mL of a 1 M solution in ether, 27.7 mmol) in THF (50 mL) at -78°C under argon. After stirring for a further 1 hour, the mixture was quenched by addition of saturated ammonium chloride solution (15 mL). The mixture was allowed to warm to room temperature, diluted with ether (30 mL), and dilute hydrochloric acid (20 mL) was added. The organic layer was separated, and the aqueous layer was further extracted with ether (2 × 40 mL). The combined ether layers were washed with brine, dried (MgSO₄), and the solvent was evaporated under reduced pressure. The residue was chromatographed (silica gel, heptane-ethyl acetate, 95:5) to give 5.8 g (100%) of a 7:7:3:3 mixture of diastereomeric (R and S)-((1S,3R)-1-benzyl-3-methylcyclopentyl)-cyano-acetic acid ethyl ester and (R and S)-((1R,3R)-1-benzyl-3-methyl-cyclopentyl)-cyano-acetic acid ethyl ester;
R_{f} (heptane-ethyl acetate, 9:1) 0.32;
IR thin film (cm⁻¹) 2246 (CN), 1740 (C=O), 1603 (C=C);
MS (ES-) *m*/*z* 284 (M-H, 100%).

### ((1S,3R)-1-Benzyl-3-methyl-cyclopentyl)-acetic acid and ((1R,3R)-1-Benzyl-3-methyl-cyclopentyl)-acetic acid

The mixture of (R and S)-((1S,3R)-1-benzyl-3-methyl-cyclopentyl)-cyano-acetic acid ethyl ester and (R and S)-((1R,3R)-1-benzyl-3-methyl-cyclopentyl)-cyanoacetic acid ethyl ester (1 g, 3.5 mmol) and potassium hydroxide (1.2 g, 21.4 mmol) were heated to 160°C in ethylene glycol (5 mL) for 16 hours. After this time, the mixture was allowed to cool and dilute hydrochloric acid (150 mL) was added carefully. The mixture was extracted with ethyl acetate (3 x 50 mL), and the combined organic fractions were washed with brine, dried (MgSO₄), and the solvent was evaporated under reduced pressure. The residue was chromatographed (silica gel, heptane/ethyl acetate, 98:2) to give 0.65 g (80%) of a 7:3 mixture of ((1S,3R)-1-benzyl-3-methyl-cyclopentyl)-acetic acid and ((1R,3R)-1-benzyl-3-methyl-cyclopentyl)-acetic acid as an oil;
R_{f} (heptane-ethyl acetate, 98:2) 0.36;
IR thin film (cm⁻¹) 1702 (C=O);
¹H-NMR (400 MHz; CDCl₃) major isomer ((1S,3R)-1-benzyl-3-methylcyclopentyl)-acetic acid: δ 7.31-7.21 (5H, m, Ph), 2.82 (1H, d, *J* 13.4, *CH*_{*A*}H_{B}CO₂H), 2.76 (1H, d,*J* 13.4, CH_{A}*H*_{*B*}CO₂H), 2.33 (2H, br s, C*H*₂Ph), 2.19-1.66 (m), 1.62-1.52 (m), 1.11 (1H, dd, *J* 13.0, 9.9), 1.01 (3H, d, *J* 6.6, Me); minor isomer ((1R,3R)-1-benzyl-3-methyl-cyclopentyl)-acetic acid: δ 7.31-7.21 (5H, m, Ph), 2.89 (1H, d, *J* 13.2, C*H*_{*A*}H_{B}CO₂H), 2.84 (1H, d, *J* 13.4, CH_{A}*H*_{*B*}CO₂H), 2.28 (2H, br s, C*H*_{*2*}Ph), 2.19-1.66 (m), 1.62-1.52 (m), 1.30-1.17 (m), 1.00 (3H, d, *J* 6.6, Me);
MS (CI⁻): *m*/*z* 231 (M-H, 100%).

### ((1S,3R)-1-Benzyl-3-methyl-cyclopentyl)-acetic acid

(s)-(-)-α-Methyl benzylamine (8.8 g, 72.7 mmol) was added to a stirring solution of the diastereomeric mixture of ((1S,3R)-1-benzyl-3-methyl-cyclopentyl)-acetic acid and ((1R,3R)-1-benzyl-3-methyl-cyclopentyl)-acetic acid (16.9 g, 72.7 mmol) dissolved in the minimum quantity of ethyl acetate. The mixture was placed in the fridge and left for 1 hour. After this time, the acid salt had crystallised out and this was filtered off. The salt was recrystallized several times from ethyl acetate (to 95% de). The salt was taken up in ethyl acetate, washed with dilute hydrochloric acid, brine and dried (MgSO₄). The solvent was evaporated under reduced pressure to give 6.8 g (40%) of ((1S,3R)-1-benzyl-3-methyl-cyclopentyl)-acetic acid; LCMS (Prodigy® (Phenomenex, Ltd.) ODS 3 50 mm x 4.6 mm id column, 5-50% Acetonitrile/water) Retention Time = 2.01 min, 98% purity.

### ((1S,3R)-1-Isocyanatomethyl-3-methyl-cyclopentylmethyl)-benzene

Diphenylphosphoryl azide (4.48 g, 16 mmol), triethylamine (1.69 g, 16.8 mmol), and acid ((1S,3R)-1-benzyl-3-methyl-cyclopentyl)-acetic acid (3.74 g, 16 mmol) were refluxed in toluene (40 mL) for 17 hours. The mixture was allowed to cool and then taken up in ethyl acetate (150 mL), washed with saturated aqueous sodium hydrogen carbonate (200 mL), brine (150 mL), and dried (MgSO₄). The solvent was removed under reduced pressure to give 3.69 g (100%) of ((1S,3R)-1-isocyanatomethyl-3-methyl-cyclopentylmethyl)-benzene, which was used without further purification;
R_{f} (heptane-ethyl acetate, 8:2) 0.36;
IR thin film (cm⁻¹) 2262 (CN).

### ((1S,3R)-1-Benzyl-3-methyl-cyclopentylmethyl)-carbamic acid methyl ester

((1S,3R)-1-Isocyanatomethyl-3-methyl-cyclopentylmethyl)-benzene (3.69 g, 16 mmol) was refluxed in methanol (10 mL) and toluene (20 mL) for 16 hours and then allowed to cool to room temperature. The solvent was removed under reduced pressure, and the residue was purified by chromatography (silica gel, heptane-ethyl acetate 9:1) to give 2.66 g (63%) of ((1S,3R)-1-benzyl-3-methylcyclopentylmethyl)-carbamic acid methyl ester;
R_{f} (heptane-ethyl acetate, 8:2) 0.28;
IR thin film (cm⁻¹) 1709 (C=O);
¹H-NMR (400 MHz; CDCl₃) δ 7.32-7.16 (5H, m, Ph), 4.60 (1H, bs, *NH*), 3.68 (3H, s, OMe), 3.18-3.00 (2H, m, C*H*_{*2*}NH), 2.62-2.60 (2H, s, C*H*_{*2*}Ph), 0.99 (3H, d, *J* 6.8, Me), 2.05-1.92, 1.87-1.72, 1.60-1.40, 1.00-0.89 (7H, m); MS (ES⁺) *m*/*z* 262 (M+H, 90%), 302 (M+CH₃CN+H,100%);
LCMS (Prodigy® ODS 350 mm × 4.6 mm id column, 5-50% Acetonitrile (0.05% formic acid)/water (0.05% formic acid)) Retention Time = 2.11, 94% de.

### [(1S,3R)-1-(Methoxycarbonylamino-methyl)-3-methyl-cyclopentyl]-aceticacid

((1S,3R)-1-Benzyl-3-methyl-cyclopentylmethyl)-carbamic acid methyl ester (2.6 g, 9.9 mmol) and sodium periodate (29.8 g, 140 mmol) were stirred together in carbon tetrachloride (30 mL), acetonitrile (30 mL), and water for 6 hours. The mixture was cooled to 0°C, and ruthenium(III) chloride (0.04 g, 0.2 mmol) was added to the reaction mixture. The reaction was allowed to warm to room temperature and stirred for 20 hours. Diethyl ether (50 mL) was added, and the mixture was then extracted with saturated aqueous sodium hydrogen carbonate (200 mL). The aqueous layer was acidified to pH 1 with 4N hydrochloric acid and re-extracted with ethyl acetate (200 mL), dried (MgSO₄), and the solvent was evaporated under reduced pressure. The residue was purified by chromatography (silica gel, eluting with a gradient of heptane to 1:1 heptane:ethyl acetate) to give 0.32 g (14%) of [(1S,3R)-1-(methoxycarbonylamino-methyl)-3-methylcyclopentyl]-acetic acid;
R_{f}(heptane-ethyl acetate, 8:2) 0.30;
IR thin film (cm⁻¹) 3338 (NH), 1712 (C=O);
¹H-NMR (400 MHz; CDCl₃): δ 9.29 (1H, s, COOH), 5.17 (1H, bs, NH), 3.71 (3H, s, OMe), 3.30 (1H, dd, *J* 14.4, 7.1, CH_{A}*H*_{*B*}NH₂), 3.17 (1H, dd, *J* 14.4, 6.6, C*H*_{*A*}H_{B}NH₂), 2.37 (2H, s, C*H*_{*2*}COOH), 2.20-1.00 (7H, m), 1.01 (3H, d, *J* 6.4, CH*Me*);
MS (ES⁺) *mlz* 230 (M+H, 63%), 481 (M+Na.100).

### ((1S,3R)-1-Aminomethyl-3-methyl-cyclopentyl)-acetic acid hydrochloride

[(1S,3R)-1-(Methoxycarbonylamino-methyl)-3-methyl-cyclopentyl]-acetic acid (0.32 g, 1.4 mmol) was refluxed in a mixture of 1,4-dioxane (3 mL) and 6N Hydrochloric acid (8 mL) for 4 hours. The mixture was allowed to cool, diluted with water (200 mL), and washed with dichloromethane (2 × 200 mL). The aqueous layer was evaporated under reduced pressure, and the residue was recrystallized from ethyl acetate/methanol to give 0.17 g (59%) of ((1S,3R)-1-aminomethyl-3-methyl-cyclopentyl)-acetic acid hydrochloride;
IR thin film (cm⁻¹) 1710 (C=O);
¹H-NMR (400 MHz; DMSO-*d*_{*6*}): δ 2.96 (1H, d, *J* 12.8, C*H*_{*A*}H_{B}NH₂), 2.90 (1H, d, *J* 12.8, CH_{A}*H*_{*B*}NH₂), 2.40 (2H, s, C*H*_{*2*}COOH), 2.04 (1H, m, C*H*Me), 1.81-1.61, 1.51-1.43, 1.21-1.11 (5H, m), 1.06 (1H, dd, *J* 12.8, 10.4), 0.97 (3H, d, *J* 6.35, Me);
MS (ES⁺) *m*/*z* 173 (M+H, 100%), 196 (M+Na, 10%);

LCMS (Prodigy® ODS 3 50 mm × 4.6 mm id column, 5% for 2 min, 5-50% over 1.5 min of Acetonitrile (0.05% formic acid)/water (0.05% formic acid)) Retention Time = 0.92, 94% de.

### EXAMPLE 2

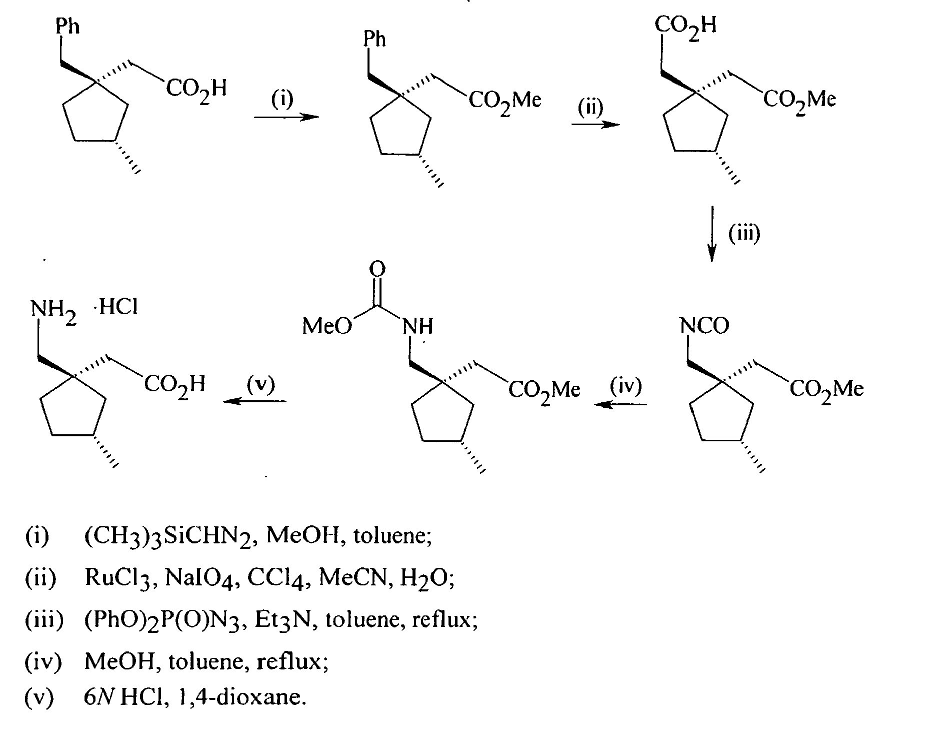

### (1S,3R)-1-Benzyl-3-methyl-cyclopentyl)-acetic acid methyl ester

Trimethylsilyldiazomethane (31.5 mL of a 2 M solution in hexanes, 63 mmol) was added dropwise to a stirring solution of ((1S,3R)-1-benzyl-3-methyl-cyclopentyl)-acetic acid (10 g, 43 mmol) in toluene (80 mL) and methanol (20 mL) at 0°C under argon, and the mixture was allowed to warm to room temperature. The mixture was stirred for 1 hour, and then the solvent was evaporated under reduced pressure. The residue was taken up in ethyl acetate (50 mL), washed with saturated sodium hydrogen carbonate solution, dilute hydrochloric acid, dried (MgSO₄), and the solvent removed *in vacuo* to give 10.6 g (100%) of ((1S,3R)-1-benzyl-3-methyl-cyclopentyl)-acetic acid methyl ester;
R_{f} (heptane-ethyl acetate, 9:1) 0.40;
IR thin film (cm⁻¹) 1736 (C=O);
¹H-NMR (400 MHz; CDCl₃): δ 7.30-7.18 (5H, m, Ph), 3.69 (3H, s, OMe), 2.78 (1H, d, *J* 13.4, C*H*_{*A*}H_{B}CO₂Me), 2.72 (1H, d, *J* 13.4, CH_{*A*}*H*_{*B*}CO₂Me), 2.28 (2H, s, CH₂Ph), 2.16-1.50 (5H, m), 1.30-1.03 (2H, m), 1.00 (3H, d, *J* 6.6, Me).

### ((1S,3R)-1-Methoxycarbonylmethyl-3-methyl-cyclopentyl)-acetic acid

((1S,3R)-1-Benzyl-3-methyl-cyclopentyl)-acetic acid methyl ester (10.5 g, 43 mmol) and sodium periodate (128.0 g, 598 mmol) were stirred together in carbon tetrachloride (120 mL), acetonitrile (120 mL), and water (210 mL) for 1 hour. The mixture was cooled to 10°C, and ruthenium(III) chloride (0.177 g, 0.86 mmol) was added to the reaction mixture. The reaction was allowed to warm to room temperature and stirred for 20 hours. Diethyl ether (100 mL) was added, and the mixture was acidified to pH 1 with concentrated hydrochloric acid and then extracted with ether (2 × 200 mL). The organic layer was extracted with saturated aqueous sodium hydrogen carbonate (2 × 200 mL) which was then acidified to pH 1 with 4N hydrochloric acid and re-extracted with ethyl acetate, dried (MgSO₄) and concentrated *in vacuo.* The residue was purified by chromatography (silica gel, eluting with a gradient of heptane to 1:1 heptane:ethyl acetate) to give 8.02 g (87.7%) of((1S,3R)-1-methoxycarbonylmethyl-3-methylcyclopentyl)-acetic acid;
R_{f} (heptane-ethyl acetate, 1: 1) 0.46;
IR thin film (cm⁻¹) 3100 (OH), 1737 (C=O), 1705 (C=O);
¹H-NMR (400 MHz; CDCl₃): δ 3.68 (3H, s, OMe), 2.67-2.51 (4H, m), 2.06 (1H, m), 1.97-1.79 (2H, m), 1.76-1.59 (2H, m), 1.29-1.08 (2H, m), 1.01 (3H, d, *J* 6.6, Me);
MS (ES⁺) *m*/*z* 215 (M+H), 278 (M+Na, 100), 451 (2M+Na, 80%).

### ((1R,3R)-1-Isocyanatomethyl-3-methyl-cyclopentyl)-acetic acid methyl ester

Diphenylphosphoryl azide (8.07 mL, 37.4 mmol), triethylamine (5.36 mL, 39 mmol), and ((1S,3R)-1-methoxycarbonylmethyl-3-methyl-cyclopentyl)-acetic acid (7.93 g, 37 mmol) were refluxed in toluene (80 mL) for 17 hours. The mixture was allowed to cool and then taken up in ethyl acetate (250 mL), washed with saturated aqueous sodium hydrogen carbonate (250 mL), brine (100 mL), and dried (MgSO₄). The solvent was removed under reduced pressure to give 7.82 g (100%) of ((1R,3R)-1-isocyanatomethyl-3-methyl-cyclopentyl)-acetic acid methyl ester which was used without further purification;
IR thin film (cm⁻¹) 2264 (CN), 1732 (C=O).

### [(1R,3R)-1-(Methoxycarbonylamino-methyl)-3-methyl-cyclopentyl]-acetic acid methyl ester

((1R,3R)-1-Isocyanatomethyl-3-methyl-cyclopentyl)-acetic acid methyl ester (7.82 g, 37 mmol) was refluxed in methanol (30 mL) and toluene (80 mL) for 17 hours and then allowed to cool to room temperature. The solvent was removed under reduced pressure, and the residue was purified by chromatography (silica gel, heptane to heptane:ether 8:2) to give 2.60 g (29%) of [(1R,3R)-1-(methoxycarbonylamino-methyl)-3-methyl-cyclopentyl]-acetic acid methyl ester;
R_{f} (heptane-ethyl acetate, 1:1) 0.52;
IR thin film (cm⁻¹) 1728 (C=O), 1716 (C=O);
¹H-NMR (400 MHz; CDCl₃): δ 3.67 (6H, s, OMe, NHCO₂*Me*), 3.21 (1H, dd, *J* 7.08, 14.2, C*H*_{*A*}H_{B}NHCO₂Me), 3.11 (1H, dd, *J* 6.10, 13.9, *C*H_{A}*H*_{*B*}NHCO₂Me), 2.36 (2H, s, C*H*_{*2*}CO₂Me), 2.05 (1H, m, C*H*Me), 1.86-1.46 & 1.29-1.18 (5H, m), 0.99 (3H, d, *J* 6.59, Me).

### ((1R,3R)-1-Aminomethyl-3-methyl-cyclopentyl)-acetic acid hydrochloride

[(1R,3R)-1-(Methoxycarbonylamino-methyl)-3-methyl-cyclopentyl]-acetic acid methyl ester (2.60 g, 37 mmol) was refluxed in a mixture of 1,4-dioxane (15 mL) and 6N Hydrochloric acid (30 mL) for 16 hours. The mixture was allowed to cool, diluted with water (80 mL), and washed with dichloromethane (2 x 200 mL). The aqueous layer was evaporated under reduced pressure, and the residue was recrystallized from ethyl acetate/methanol (95:5) to give 0.55 g (25%) of ((1R,3R)-1-aminomethyl-3-methyl-cyclopentyl)-acetic acid hydrochloride;
1R thin film (cm⁻¹) 1724 (C=O);
¹H-NMR (400 MHz; DMSO-*d*₆): δ 2.92 (1H, d, *J* 12.9, C*H*_{*A*}H_{B}N), 2.87 (1H, d, *J* 12.9, CH_{A}*H*_{*B*}N), 2.45 (1H, d, *J* 15.9, C*H*_{*A*}H_{B}COOH), 2.40 (1H, d, *J* 15.9, CH_{A}*H*_{*B*}COOH), 1.95 (1H, m), 1.84-1.72 (2H, m), 1.60-1.48 (2H, m), 1.20 (1H, m), 1.04 (1H, m), 0.96 (3H, d, *J* 6.8, Me).

### EXAMPLE 3

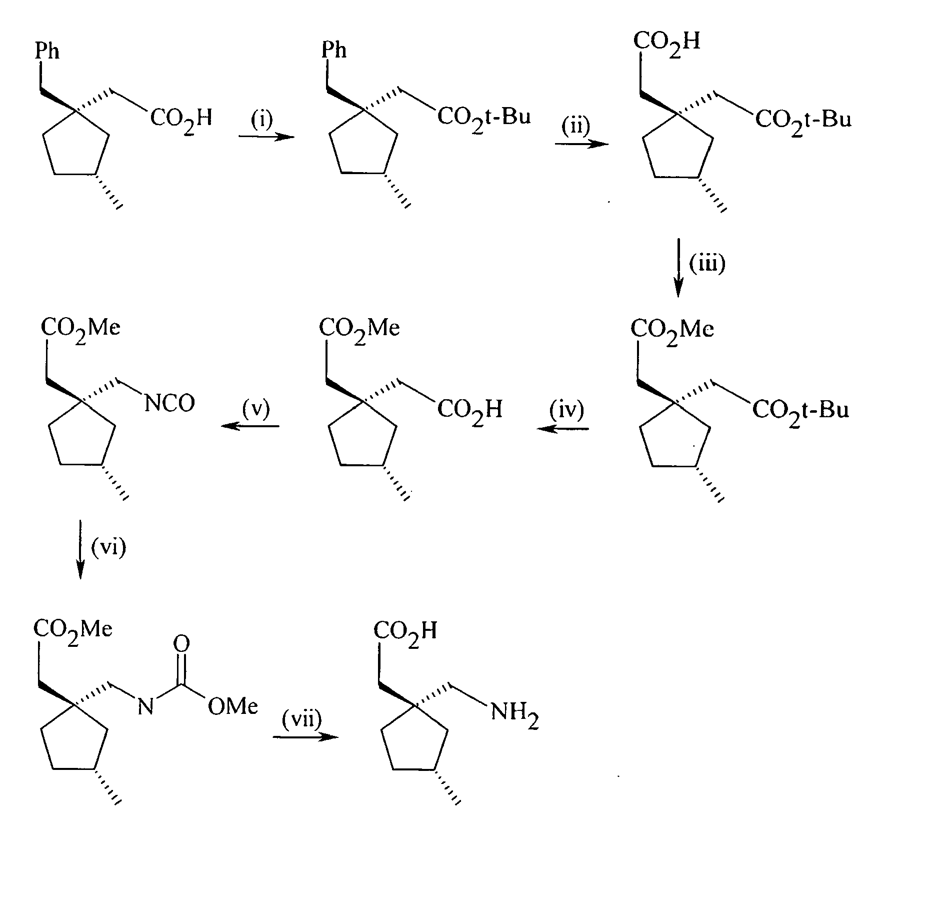

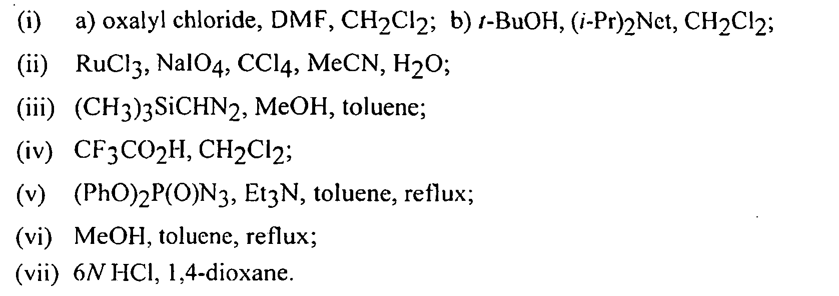

### ((1S,3R)-1-Benzyl-3-methyl-cyclopentyl)-acetic acid tert-butyl ester

Oxalyl chloride (4.14 mL, 47 mmol) was added dropwise to a stirring solution of ((1S,3R)-1-benzyl-3-methyl-cyclopentyl)-acetic acid (10 g, 43 mmol) in dichloromethane under argon at room temperature. The reaction mixture was cooled to 5°C, dimethylformamide (1 mL) was carefully added, and the mixture was allowed to warm to room temperature and stirred for a further 2 hours. The solvent was removed *in vacuo* and the residue diluted with dichloromethane (60 mL). 1,1-Dimethylethanol (15 mL) was carefully added to the reaction mixture under argon followed by diisopropylethylamine (11.5 mL, 65 mmol). The mixture was stirred for 17 hours and then taken up in ethyl acetate, washed with saturated aqueous sodium hydrogen carbonate (2 × 200 mL), and dried (MgSO₄). The solvent was removed under reduced pressure, and the residue was purified by chromatography (silica gel, eluting with a gradient of heptane to 9:1 heptane:ethyl acetate) to give 10.92 g (88%) of ((1S,3R)-1-benzyl-3-methyl-cyclopentyl)-acetic acid *tert*-butyl ester;
R_{f} (heptane-ethyl acetate, 9:1) 0.64;
1R thin film (cm⁻¹) 1724 (C=O);
¹H-NMR (400 MHz;CDCl₃): δ 7.29-7.17 (5H, m, Ph), 2.77 (1H, d, *J* 13.6, C*H*_{*A*}H_{B}Ph), 2.71 (1H, d, *J* 13.6, CH_{A}*H*_{*B*}Ph), 2.18 (1H, s, C*H*_{*A*}H_{B}CO₂^{t}Butyl), 2.17 (1H, s,CH_{A}*H*_{*B*}CO₂^{t}Butyl), 1.49 (9H, s, CMe₃), 2.17-1.5 & 1.30-1.00 (7H, m), 1.00 (3H, d, *J* 6.8, CH*Me*).

### [(1S,3R)-1-Carboxymethyl-3-methyl-cyclopentyl]-acetic acid tert-butyl ester

((1S,3R)-1-Benzyl-3-methyl-cyclopentyl)-acetic acid *tert*-butyl ester (10.72 g, 37.2 mmol) and sodium periodate (124.77 g, 0.583 mol) were stirred together in carbon tetrachloride (120 mL), acetonitrile (120 mL), and water (210 mL) for 2 hours. The mixture was cooled to 0°C, and ruthenium(III) chloride (0.173 g, 0.83 mmol) was added to the reaction mixture. The reaction was allowed to warm to room temperature and stirred for 48 hours. Diethyl ether (60 mL) was added, and the mixture was then acidified to pH 2 by the addition of dilute hydrochloric acid. The mixture was extracted with ethyl acetate (2 × 200 mL), dried (MgSO₄) and concentrated *in vacuo.* The residue was purified by chromatography (silica gel, eluting with a gradient of heptane to 1:1 heptane:ethyl acetate) to give 7.01 g (73.5%) of [(1S,3R)-1-carboxymethyl-3-methyl-cyclopentyl]-acetic acid *tert*-butyl ester;
R_{f}(heptane-ethyl acetate, 1:1) 0.58;
IR thin film (cm⁻¹) 2953 (OH), 1726 (C=O) 1705 (C=O);
¹H-NMR (400 MHz; CDCl₃): δ 2.51 (2H, s, CH₂CO), 2.46 (2H, s, CH₂CO), 1.47 (9H, s, CMe₃), 2.05-2.15, 1.95-1.80, 1.75-1.60, 1.30-1.03 (7H, m), 1.01 (3H, d, *J* 6.4, Me).

### [(1S,3R)-1-Methoxycarbonylmethyl-3-methyl-cyclopenthyl]-acetic acid tert-butyl ester

Trimethylsilyldiazomethane (14 mL of a 2 M solution in hexanes, 26.9 mmol) was added dropwise to a stirring solution of [(1S,3R)-1-carboxymethyl-3-methylcyclopentyl]-acetic acid *tert*-butyl ester (6.9 g, 26.9 mmol) in toluene (60 mL) and methanol (15 mL) at 10°C under argon, and the mixture was allowed to warm to room temperature. The mixture was stirred for 2 hours, and then the solvent was evaporated under reduced pressure. The residue was taken up in ethyl acetate (200 mL), washed with saturated sodium hydrogen carbonate solution, dilute hydrochloric acid, dried (MgSO₄), and the solvent removed *in vacuo.* The residue was purified by chromatography (silica gel, eluting with a gradient of heptane to 95:5 heptane:ethyl acetate) to give 6.73 g (92.4%) of [(1S,3R)-1-methoxycarbonylmethyl-3-methyl-cydopentyl]-acetic acid *tert*-butyl ester;
R_{f}(heptane-ethyl acetate, 9:1) 0.36;
IR thin film (cm⁻¹) 1738 (C=O) 1732 (C=O);
¹H-NMR (400 MHz; CDCl₃): δ 3.65 (3H, s, OMe), 2.52 (2H, m, CH₂CO₂), 2.45 (1H, d, *J* 4.8, CH₂CO₂), 1.44 (9H, s, CMe₃), 2.05-1.5, 1.30-1.10 (7H, m), 1.00 (3H, d, *J* 6.8, Me).

### ((1R,3R)-1-Methoxycarbonylmethyl-3-methyl-cyclopentyl)-acetic acid

[(1S,3R)-1-Methoxycarbonylmethyl-3-methyl-cyclopentyl]-acetic acid *tert-*butyl ester (6.64 g, 24.6 mmol) and trifluoroacetic acid (10 mL) were stirred together in dichloromethane (30 mL) for 17 hours at room temperature. The mixture was carefully poured into aqueous sodium carbonate and extracted with ethyl acetate (200 mL). The aqueous was acidified to pH 1 with concentrated hydrochloric acid and re-extracted with ethyl acetate (3 × 200 mL), dried (MgSO₄), and concentrated *in vacuo.* The residue was purified by chromatography (silica gel, eluting with a gradient of heptane to 1:1 heptane:ethyl acetate) to give 5.26 g (100%) of [(1R,3R)-1-methoxycarbonylmethyl-3-methyl-cyclopentyl]-acetic acid;
R_{f} (heptane-ethyl acetate, 1:1) 0.46;
IR thin film (cm⁻¹) 2952 (OH), 1737 (C=O), 1706(C=O);
¹H-NMR (400 MHz; CDCl₃): δ 3.68 (3H, s, OMe), 2.67 (1H, d, *J* 15.0, C*H*_{*A*}H_{B}CO₂), 2.61 (1H, d, *J* 14.9, CH_{A}*H*_{*B*}CO₂), 2.58 (1H, d, *J* 14.8, C*H*_{*A*}H_{B}CO₂), 2.53 (1H, d, *J* 14.8, CH_{A}*H*_{*B*}CO₂), 1.93-1.81, 1.75-1.59, 1.75-1.63 (6H, m), 1.16(1H, dd, *J* 19.5, 9.3), 1.01 (3H, d, *J* 6.35, Me).

### ((1S,3R)-1-Isocyanatomethyl-3-methyl-cyclopentyl)-acetic acid methyl ester

Diphenylphosphoryl azide (5.35 mL, 24.8 mmol), triethylamine (3.55 mL, 25.6 mmol), and [(1R,3R)-1-methoxycarbonylmethyl-3-methyl-cyclopentyl]-acetic acid (5.26 g, 24.5 mmol) were refluxed in toluene (80 mL) for 17 hours. The mixture was allowed to cool and then taken up in ethyl acetate (300 mL), washed with saturated aqueous sodium hydrogen carbonate solution (250 mL), brine (200 mL), and dried (MgSO₄). The solvent was removed under reduced pressure to give 5.19 g (100%) of ((1S,3R)-1-isocyanatomethyl-3-methylcyclopentyl)-acetic acid methyl ester which was used without further purification;
IR thin film (cm⁻¹) 2262 (NCO), 1732 (C=O).

### [(1S,3R)-1-(Methoxycarbonylamino-methyl)-3-methyl-cyclopentyl]-acetic acid methyl ester

((1S,3R)-1-Isocyanatomethyl-3-methyl-cyclopentyl)-acetic acid methyl ester (5.19 g, 24.5 mmol) was refluxed in methanol (30 mL) and toluene (80 mL) for 17 hours and then allowed to cool to room temperature. The solvent was removed under reduced pressure, and the residue was purified by chromatography (silica gel, heptane-ethyl acetate 9:1) to give 4.62 g (77%) of [(1S,3R)-1-(methoxycarbonylamino-methyl)-3-methyl-cyclopenlyl]-acetic acid methyl ester;
R_{f} (heptane-ethyl acetate, 1:1) 0.59;
IR thin film (cm⁻¹) 1730 (C=O);
¹H-NMR (400 MHz; CDCl₃): δ 3.68 (6H, s, O*Me*, NHCO₂*Me*), 3.27 (1H, dd, *J* 13.7, 6.8, C*H*_{*A*}H_{B}NHCO₂Me), 3.13 (1H, dd, *J* 13.9, 6.4, CH_{A}*H*_{*B*}NHCO₂Me), 2.37 (1H, d, *J* 13.9, *CH*_{*A*}H_{B}CO₂), 2.33 (1H, d, *J* 13.9, CH_{A}*H*_{*B*}CO₂), 2.09-1.99 (1H, m, C*H*Me), 1.88-1.76, 1.69-1.43, 1.28-1.19 (6H, m), 1.01 (3H, d, *J* 6.4, Me);
*m*/*z* (Cl⁺) 244 (M+H, 100%).

### ((1S,3R)-1-Aminomethyl-3-methyl-cyclopentyl)-acetic acid hydrochloride

[(1S,3R)-1-(Methoxycarbonylamino-methyl)-3-methyl-cyclopentyl]-acetic acid methyl ester (2.84 g, 11.7 mmol) was refluxed in a mixture of 1,4-dioxane (15 mL) and 6N Hydrochloric acid (30 mL) for 17 hours. The mixture was allowed to cool, diluted with water (200 mL), and washed with dichloromethane (2 x 100 mL). The aqueous layer was evaporated under reduced pressure, and the residue was recrystallized from ethyl acetate/methanol (95:5) to give 1.28 g (53%) of ((1S,3R)-1-aminomethyl-3-methyl-cyclopentyl)-acetic acid hydrochloride;
IR thin film (cm⁻¹) 1710 (C=O);
¹H-NMR (400 MHz; DMSO-*d*_{*6*}): δ 2.96 (1H, d, *J* 12.8, C*H*_{*A*}H_{B}NH₂), 2.90 (1H, d, *J* 12.8, CH_{A}*H*_{*B*}NH₂), 2.40 (2H, s, C*H*_{*2*}COOH), 2.09-1.98 (1H, m, C*H*Me), 1.81-1.61, 1.51-1.43, 1.21-1.11(5H, m), 1.04 (1H, dd, *J* 13.2, 10.4), 0.97 (3H, d, *J* 6.35, Me);
MS (ES⁺) *m*/*z* 173 (M+H, 100%), 196 (M+Na, 10%);
LCMS (Prodigy® ODS 3 50 mm x 4.6 mm id column, 5% for 2 min, 5-50% over 1.5 min of acetonitrile (0.05% formic acid)/water (0.05% formic acid)) Retention Time = 0.92, 94% de; (Found: C, 49.5; H, 8.78; N, 6.37.
C₉H₁₇NO₂ 1HCl 0.6H₂O requires C, 49.5; H, 8.86; N, 6.41).

## Claims

1. A process for the preparation of a compound of Formula I 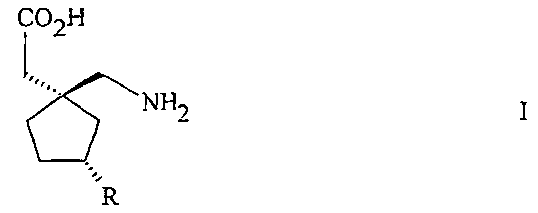 wherein R is C₁-C₁₀ alkyl or C₃-C₁₀ cycloalkyl, and pharmaceutically acceptable salts thereof, which comprises:
a) adding a cyanoacetate of formula (A) 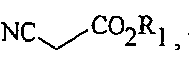 wherein R₁ is alkyl or benzyl, to a mixture of a chiral cyclopentanone of formula (1) 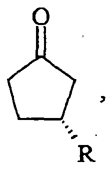 a solvent, a carboxylic acid, and a Knoevenagel reaction catalyst, and stirring the mixture in the presence of a means of removing water to produce the alkene of formula (2) 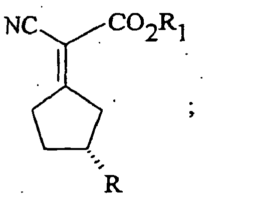
b) adding the product of Step a) above to a mixture of benzylmagnesium chloride, benzylmagnesium bromide, or benzylmagnesium iodide, in a solvent to produce the addition products of formulas (3a) 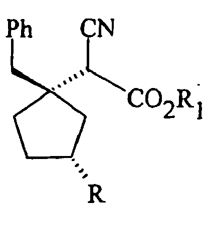 and (3b) 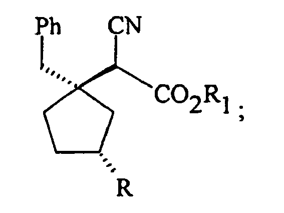
c) adding the products of Step b) above to a mixture of a base selected from potassium hydroxide, sodium hydroxide, lithium hydroxide, or cesium hydroxide in a solvent and stirring, and then acidifying to produce the carboxylic acids of formulas (4a) 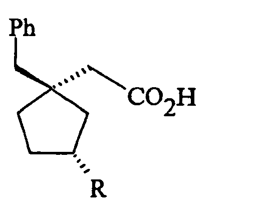 and (4b) 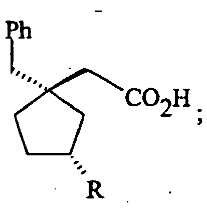 or adding the products of Step b) above to an acid mixture and stirring to produce the carboxylic acids of formulas (4a) 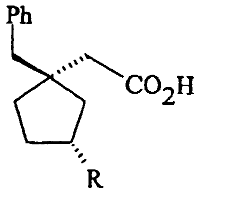 and (4b) 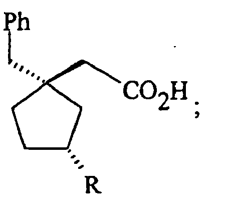
d) contacting the products of Step c) above with an amine in a solvent, and recrystallizing the salt so formed to produce the enriched diastereomer of formula (5) 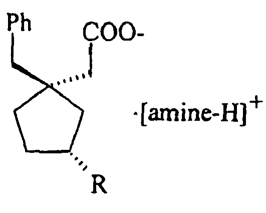 as the amine salt;
e) converting the product of Step d) to a carboxylic acid of formula (6) 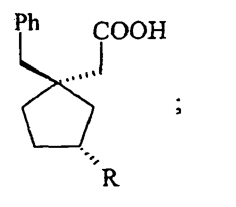
f) adding the product of Step e) to a mixture of iodomethane, a solvent, and a base, and stirring to produce the ester of formula (7) 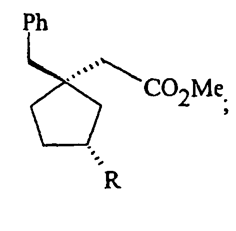 or adding the product of Step e) to methanol and an acid to produce the ester of formula (7) 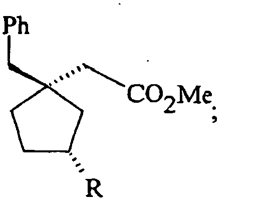 or adding the product of Step e) above to trimethylsilyldiazomethane and methanol in a solvent to produce the ester of formula (7) 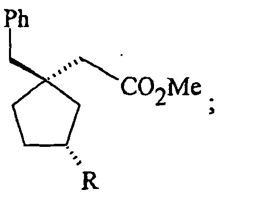 or adding the product of Step e) to a solution of diazomethane or trimethylsilyl-diazomethane in a solvent to produce ester of formula (7) 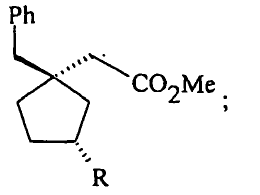
g) adding the product of Step f) to a mixture of carbon tetrachloride or ethyl acetate, and acetonitrile, water, sodium periodate, and ruthenium(III) chloride, and stirring to produce the carboxylic acid of formula (8) 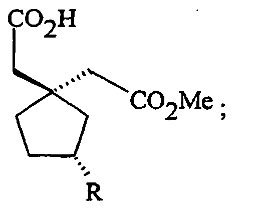
h) adding the product of Step g) to a mixture of a tertiary amine base, a solvent, and diphenylphosphoryl azide (DPPA), and stirring to produce the isocyanate of formula (9) 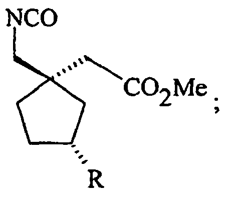 or adding the product of Step g) above to ethyl chloroformate or isobutyl chloroformate and a base in a solvent at a temperature of from -40°C to 78°C, followed by adding a solution of sodium azide in water and tetrahydrofuran or acetone, followed by adding toluene or benzene, and refluxing to produce the isocyanate of formula (9) 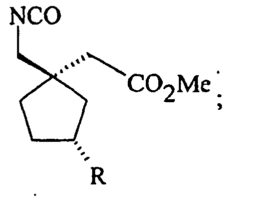
i) adding the product of Step h) to a mixture of a solvent and methanol, and stirring to produce the carbamate of formula (10) 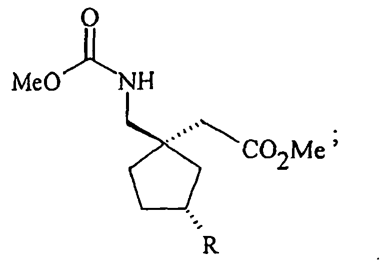
j) adding the product of Step i) to a mixture of a solvent and aqueous hydrochloric acid, and stirring to produce a compound of formula (Ia) 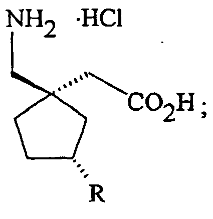 and
k) converting the product of Step j) to a compound of formula (I) 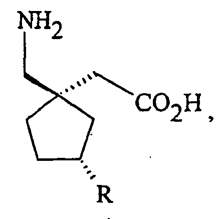 and further converting, if desired, to a pharmaceutically acceptable salt by known means.

2. A process according to Claim 1 which comprises:
a) adding a cyanoacetate of formula (A) 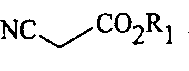 wherein R₁ is selected from methyl, ethyl, *n*-propyl, *iso*-propyl, *n*-butyl, *iso*-butyl, *sec*-butyl, *tert*-butyl, and benzyl to a mixture of a chiral cyclopentanone of formula (1) 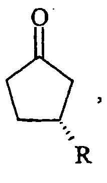 a solvent selected from tetrahydrofuran, 1,4-dioxane, *tert*-butylmethylether, chloroform, dichloromethane, acetonitrile, ethyl ether, ethyl acetate, hexanes, N,N-dimethylformamide, dimethylsulfoxide, ethanol, *tert*-butanol, toluene, benzene, xylenes, and *n*-heptane, acetic acid, and a Knoevenagel reaction catalyst selected from β-alanine, ammonium acetate, and piperidine, and stirring the mixture in the presence of a means of removing water selected from azeotropic distillation, activated molecular sieves, anhydrous magnesium sulfate, anhydrous sodium sulfate, anhydrous sodium carbonate, anhydrous potassium carbonate, anhydrous cesium carbonate, trimethyl orthoformate, and triethyl orthoformate to produce the alkene of formula (2) 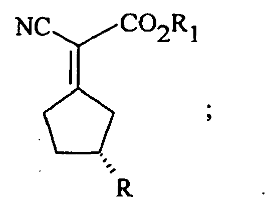
b) adding the product of Step a) above to a mixture of benzylmagnesium chloride, benzylmagnesium bromide, or benzylmagnesium iodide in a solvent selected from tetrahydrofuran, benzene, 1,4-dioxane, hexanes, *n*-heptane, toluene, diethyl ether, and *tert*-butyl methyl ether to produce the addition products of formulas (3a) 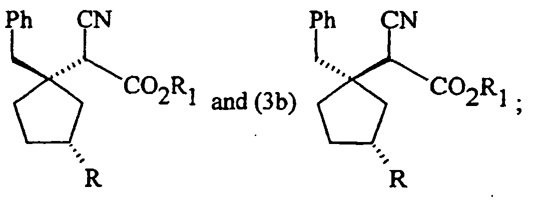
c) adding the products of Step b) above to a mixture of a base selected from potassium hydroxide, sodium hydroxide, lithium hydroxide, and cesium hydroxide in a solvent selected from ethylene glycol, 2-methoxyethyl ether, 1,4-dioxane, and diethylene glycol, and stirring the mixture, and then acidifying to produce the carboxylic acids of formulas (4a) 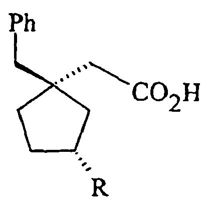 and (4b) 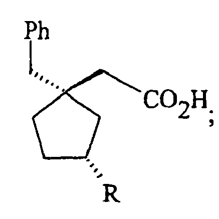 or adding the products of Step b) above to an acid mixture selected from 6-12 M HCl, 12 M H₂SO₄, 10%-48% wt/wt hydrobromic acid, and HBr in aqueous acetic acid, and stirring to produce the carboxylic acids of formulas (4a) 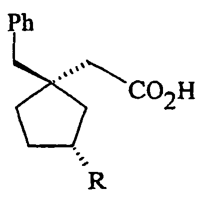 and (4b) 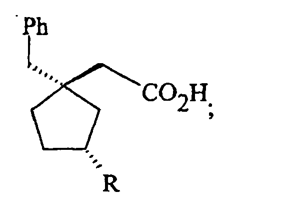
d) contacting the products of Step c) above with an amine selected from (S)-α-methyl-benzylamine, (R)-α-methyl-benzylamine, (R)-(+)-1-(naphthyl)ethylamine, (S)-(+)-1-(naphthyl)ethylamine, triethylamine, diisopropylethylamine, dicyclohexylamine, benzylamine, dibenzylamine, morpholine, N-methylmorpholine, piperidine, N-methylpiperidine, and pyridine in a solvent selected from N,N-dimethylformamide, chlorofonn, benzene, xylenes, hexanes, acetone, ethanol, methanol, *iso*-propanol, diethyl ether, dichloromethane, benzene, toluene, n-pentane, n-hexane, n-heptane, ethyl acetate, acetonitrile, *tert-*butyl methyl ether, tetrahydrofuran, and 1,4-dioxane, and recrystallizing the salt so formed to produce the enriched diastereomer of formula (5) 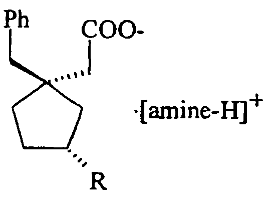 as the amine salt;
e) adding the product of Step d) to a mixture selected from aqueous hydrochloric acid, aqueous sulfuric acid, aqueous acetic acid, hydrochloric acid dissolved in acetic acid, or hydrochloric acid dissolved in acetic acid to which water is added and stirring to produce the carboxylic acid of formula (6) 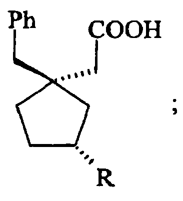 or partitioning the product of Step d) between a mixture of aqueous hydrochloric acid and a solvent selected from chloroform, dichloromethane, ethyl acetate, ethyl ether, tetrahydrofuran, 1,4-dioxane, toluene, and *tert*-butylmethylether, and drying and evaporating the organic layer to produce the carboxylic acid of formula (6) 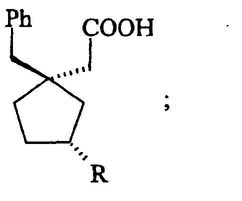
f) adding the product of Step e) above to a mixture of iodomethane, a solvent selected from dichloromethane, chloroform, tetrahydrofuran, toluene, and 1,4-dioxane, and a base selected from 1,8-diazabicyclo[5.4.0]undec-7-ene (DBU), diisopropylethylamine, triethylamine, and 1,5-diazabicyclo[4.3.0]non-5-ene (DBN), and stirring at a temperature of from -40°C to 110°C to produce the ester of formula (7) 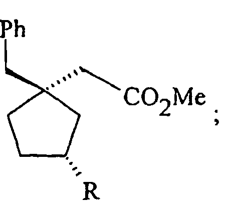 or adding the product of Step e) above to a mixture of methanol and concentrated sulphuric acid, concentrated hydrochloric acid, or hydrogen chloride at a temperature of from 0°C to 100°C to produce the ester of formula (7) 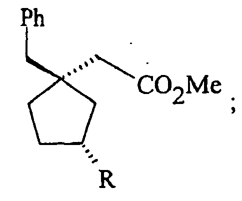 or adding the product of Step e) above to trimethylsilyldiazomethane and methanol in benzene or toluene at a temperature of from -40°C to 100°C to produce the ester of formula (7) 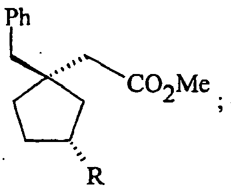 or adding the product of Step e) above to diazomethane or trimethylsilyldiazomethane in a solvent selected from benzene, toluene, dichloromethane, and diethyl ether at a temperature of from -40°C to 40°C to give a compound of formula (7) 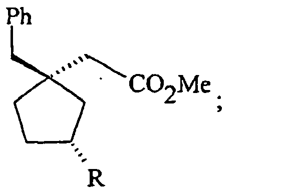
g) adding the product of Step f) to a mixture of carbon tetrachloride or ethyl acetate, and acetonitrile, water, sodium periodate, and ruthenium(III) chloride, and stirring at a temperature from -40°C to 80°C to produce the carboxylic acid of formula (8) 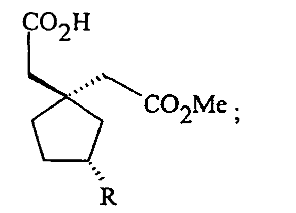
h) adding the product of Step g) above to a mixture of a base selected from triethylamine and diisopropylethylamine, a solvent selected from toluene, benzene, xylenes, tetrahydrofuran, diethyl ether and *n*-heptane, and diphenylphosphoryl azide (DPPA), and stirring at a temperature of from 0°C to 150°C to produce the isocyanate of formula (9) 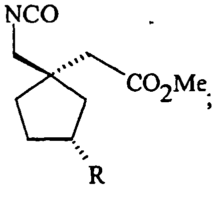 or adding the product of Step g) above to ethyl chloroformate or isobutyl chloroformate, a base selected from triethylamine and diisopropylethylamine, and a solvent selected from tetrahydrofuran, acetone, and diethyl ether at a temperature of from -40°C to 78°C, followed by adding a solution of sodium azide in water and tetrahydrofuran or acetone, followed by adding toluene or benzene, and refluxing to produce the isocyanate of formula (9) 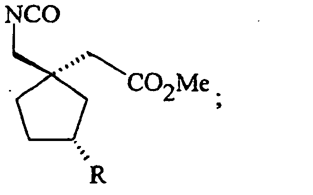
i) adding the product of Step h) to a mixture of a solvent selected from toluene, benzene, xylenes and *n*-heptane, and methanol, and stirring at a temperature from 0°C to 150°C to produce the carbamate of formula (10) 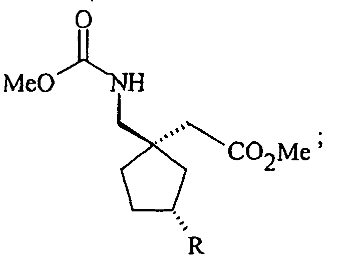
j) adding the product of Step i) to a mixture of a solvent selected from water, acetic acid, and 1,4-dioxane, and aqueous hydrochloric acid at a concentration of from 0.01 M to 12 M, and stirring at a temperature from 0°C to 115°C to produce a compound of formula Ia 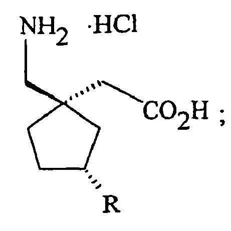 and
k) converting the product of Step j) to a compound of Formula I 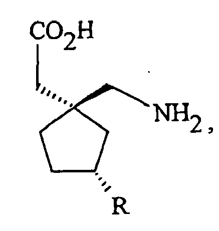 and further converting, if desired, to a pharmaceutically acceptable salt by known means.

3. A process according to Claim 1 which comprises:
a) adding a cyanoacetate of formula (A) 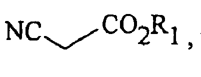 wherein R₁ is ethyl, to a mixture of a chiral cyclopentanone of formula (1) 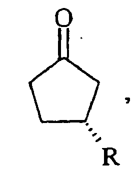 toluene, acetic acid, and a Knoevenagel reaction catalyst which is ammonium acetate, and heating the mixture at reflux over a Dean-Stark trap to produce the alkene of formula (2) 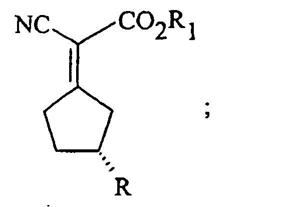
b) adding the product of Step a) above to a mixture of benzylmagnesium chloride in dry tetrahydrofuran at -100°C to 25°C to produce the addition products of formulas (3a) 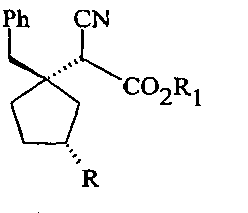 and (3b) 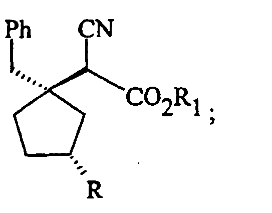
c) adding the products of Step b) above to a mixture of potassium hydroxide in ethylene glycol, and heating the mixture at 100°C to 200°C, and then acidifying to produce the hydrolysis products of formulas (4a) 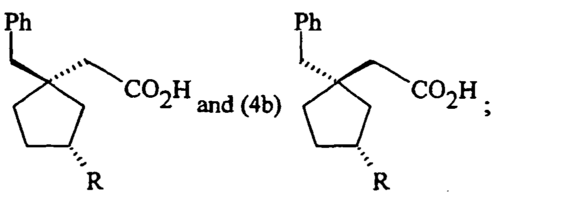
d) contacting the products of Step c) above with (S)-α-methylbenzylamine in ethyl acetate, and recrystallizing the salt so formed from ethyl acetate to produce the enriched diastereomer of formula (5) 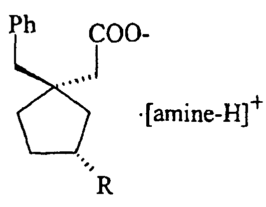 as the (S)-α-methylbenzylamine salt;
e) adding the product of Step d) to aqueous hydrochloric acid and stirring to produce the carboxylic acid of formula (6) 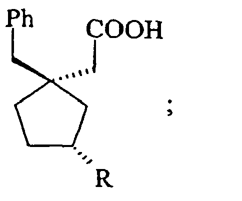
f) adding the product of Step e) to a mixture of iodomethane, dichloromethane, and 1,8-diazabicyclo[5.4.0]undec-7-ene (DBU), and stirring to produce the ester of formula (7) 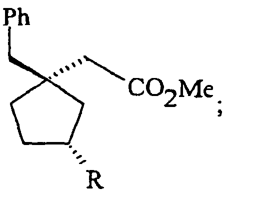 or adding the product of Step e) to methanol and concentrated sulfuric acid to produce the ester of formula (7) 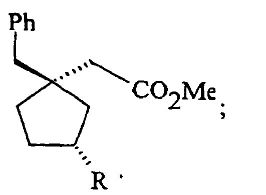 or adding the product of Step e) to a solution of diazomethane or trimethylsilyl-diazomethane in dichloromethane to produce the ester of formula (7) 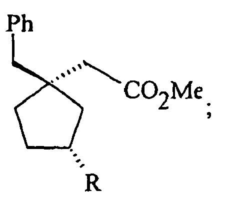
g) adding the product of Step f) to a mixture of carbon tetrachloride or ethyl acetate, and acetonitrile, water, sodium periodate, and ruthenium(III) chloride, and stirring to produce the carboxylic acid of formula (8) 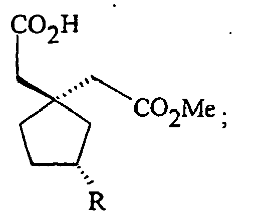
h) adding the product of Step g) to a mixture of triethylamine, toluene, and diphenylphosphoryl azide (DPPA), and refluxing to produce the isocyanate of formula (9) 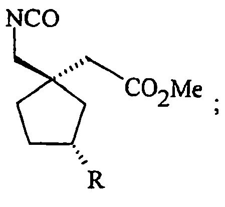 or adding the product of Step g) above to ethyl chloroformate or isobutyl chloroformate and triethylamine in tetrahydrofuran at a temperature of from -40°C to 78°C, followed by adding a solution of sodium azide in water and tetrahydrofuran, followed by adding toluene or benzene, and refluxing to produce ester of formula (9) 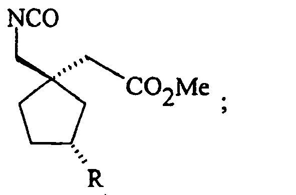
i) adding the product of Step h) to a mixture of methanol and toluene, and refluxing to produce the carbamate of formula (10) 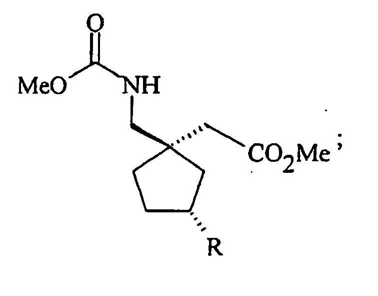
j) adding the product of Step i) to a mixture of 1,4-dioxane and aqueous hydrochloric acid at a concentration of 6 M, and stirring to produce a compound of formula la 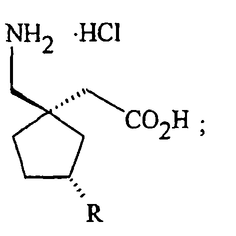
k) converting the product of Step j) to a compound of Formula I 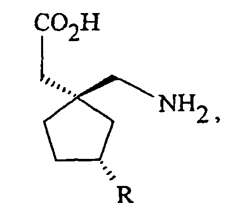 and further converting, if desired, to a pharmaceutically acceptable salt by known means.

4. A process according to Claim 1, further **characterized in that** the intermediate product (9) 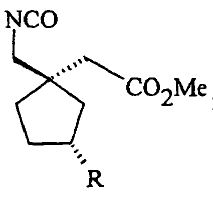 formed is reacted, without isolation, with methanol to produce the carbamate of formula (10)

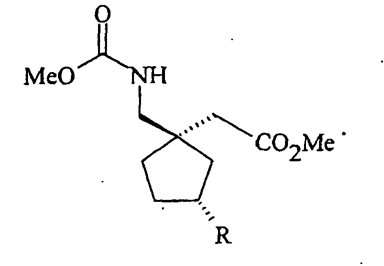

5. A process for the preparation of a compound of Formula II 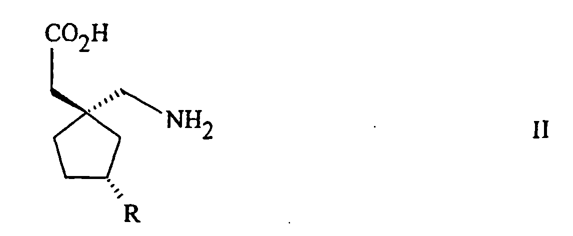 wherein R is C₁-C₁₀ alkyl or C₃-C₁₀ cycloalkyl, and pharmaceutically acceptable salts thereof, which comprises:
a) adding a cyanoacetate of formula (A) 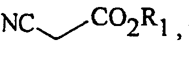 wherein R₁ is alkyl or benzyl, to a mixture of a chiral cyclopentanone of formula (1) 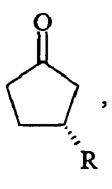 a solvent, a carboxylic acid, and a Knoevenagel reaction catalyst, and stirring the mixture in the presence of a means of removing water to produce the alkene of formula (2) 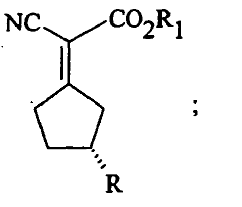
b) adding the product of Step a) above to a mixture of benzylmagnesium chloride, benzylmagnesium bromide, or benzylmagnesium iodide, in a solvent to produce the addition products of formulas (3a) 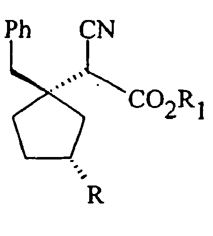 and (3b) 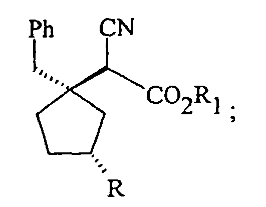
c) adding the products of Step b) above to a mixture of a base selected from potassium hydroxide, sodium hydroxide, lithium hydroxide, and cesium hydroxide and a solvent, and stirring, and then acidifying to produce the carboxylic acids of formulas (4a) 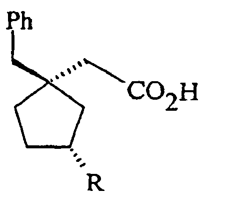 and (4b) 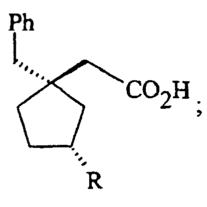 or adding the products of Step b) above to an acid mixture and stirring to produce the carboxylic acids of formulas (4a) 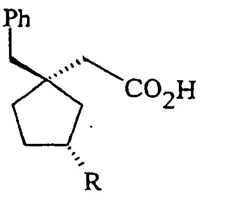 and (4b) 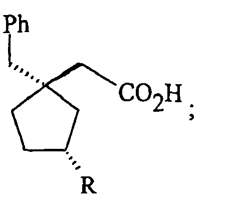
d) contacting the products of Step c) above with an amine in a solvent, and recrystallizing the salt so formed to produce the enriched diastereomer of formula (5) 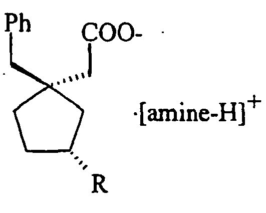 as the amine salt; and
e) converting the product of Step d) to a carboxylic acid of formula (6) 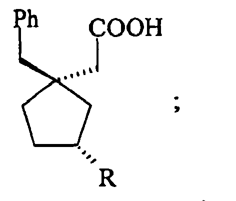
f) adding the product of Step e) to a mixture of a tertiary amine base, a solvent, and diphenylphosphoryl azide (DPPA), and stirring to produce the isocyanate of formula (11) 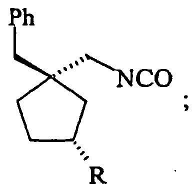 or adding the product of Step e) above to ethyl chloroformate or isobutyl chloroformate and a base in a solvent at a temperature of from -40°C to 78°C, followed by adding a solution of sodium azide in water and tetrahydrofuran or acetone, followed by adding toluene or benzene, and refluxing to produce isocyanate of formula (11) 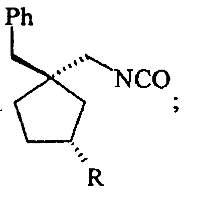
g) adding the product of Step f) to a mixture of a solvent and methanol, and stirring to produce the carbamate of formula (12) 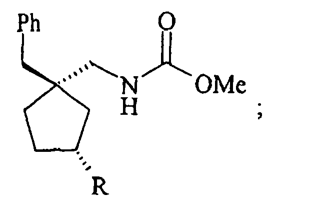
h) adding the product of Step g) to a mixture of carbon tetrachloride or ethyl acetate, and acetonitrile, water, sodium periodate, and ruthenium(III) chloride, and stirring to produce the carboxylic acid of formula (13) 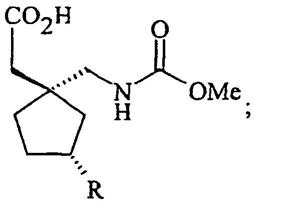
i) adding the product of Step h) to a mixture of a solvent and aqueous hydrochloric acid, and stirring to produce a compound of formula (IIa) 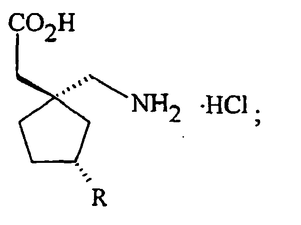
j) converting the product of Step i) to a compound of formula (II) 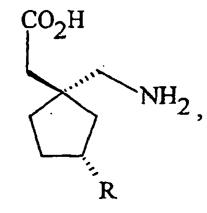 and further converting, if desired, to a pharmaceutically acceptable salt by known means.

6. A process according to Claim 5 which comprises:
a) adding a cyanoacetate of formula (A) 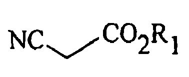 wherein R₁ is selected from methyl, ethyl, *n*-propyl, *iso*-propyl, *n*-butyl, *iso*-butyl, *sec*-butyl, *tert*-butyl, and benzyl to a mixture of a chiral cyclopentanone of formula (1) 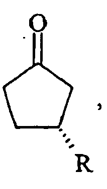 a solvent selected from tetrahydrofuran, 1,4-dioxane, *tert*-butylmethylether, chloroform, dichloromethane, acetonitrile, ethyl ether, ethyl acetate, hexanes, N,N-dimethylformwnide, dimethylsulfoxide, ethanol, *tert*-butanol, toluene, benzene, xylenes, and *n*-heptane, acetic acid, and a Knoevenagel reaction catalyst selected from β-alanine, ammonium acetate, and piperidine, and stirring the mixture in the presence of a means of removing water selected from azeotropic distillation, activated molecular sieves, anhydrous magnesium sulfate, anhydrous sodium sulfate, anhydrous sodium carbonate, anhydrous potassium carbonate, anhydrous cesium carbonate, trimethyl orthoformate, and triethyl orthoformate to produce the alkene of formula (2) 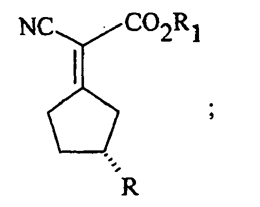
b) adding the product of Step a) above to a mixture of benzylmagnesium chloride, benzylmagnesium bromide, or benzylmagnesium iodide in a solvent selected from tetrahydrofuran, benzene, 1,4-dioxane, hexanes, *n*-heptane, toluene, diethyl ether, and *tert*-butyl methyl ether to produce the addition products of formulas (3a) 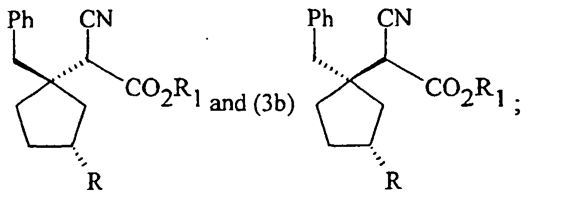
c) adding the products of Step b) above to a mixture of a base selected from potassium hydroxide, sodium hydroxide, lithium hydroxide, and cesium hydroxide in a solvent selected from ethylene glycol, 2-methoxyethyl ether, 1,4-dioxane, and diethylene glycol, and stirring the mixture and then acidifying to produce the carboxylic acids of formulas (4a) 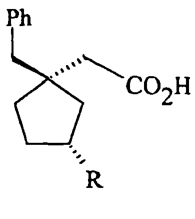 and (4b) 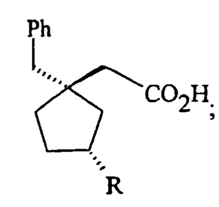 or adding the products of Step b) above to an acid mixture selected from 6-12 M HCl, 12 M H₂SO₄, 10%-48% wt/wt hydrobromic acid, and HBr in aqueous acetic acid, and stirring to produce the carboxylic acids of formulas (4a) 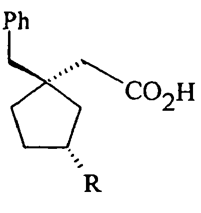 and (4b) 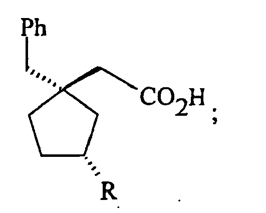
d) contacting the products of Step c) above with an amine selected from (S)-α-methyl-benzylamine, (R)-α-methyl-benzylamine, (R)-(+)-1-(naphthyl)ethylamine, (S)-(+)-1-(naphthyl)ethylamine, triethylamine, diisopropylethylamine, dicyclohexylamine, benzylamine, dibenzylamine, morpholine, N-methylmorpholine, piperidine, N-methylpiperidine, and pyridine in a solvent selected from N,N-dimethylformamide, chloroform, benzene, xylenes, hexanes, acetone, ethanol, methanol, *iso*-propanol, diethyl ether, dichloromethane, benzene, toluene, n-pentane, n-hexane, n-heptane, ethyl acetate, acetonitrile, *tert-*butyl methyl ether, tetrahydrofuran, and 1,4-dioxane, and recrystallizing the salt so formed to produce the enriched diastereomer of formula (5) 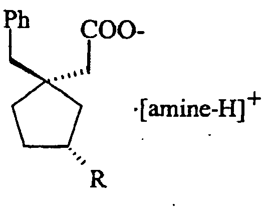 as the amine salt;
e) adding the product of Step d) to a mixture selected from aqueous hydrochloric acid, aqueous sulfuric acid, aqueous acetic acid, hydrochloric acid dissolved in acetic acid, and hydrochloric acid dissolved in acetic acid and water, and stirring to produce the carboxylic acid of formula (6) 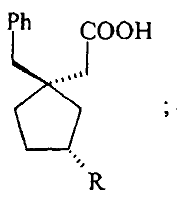 or partitioning the product of Step d) between a mixture of aqueous hydrochloric acid and a solvent selected from chloroform, dichloromethane, ethyl acetate, ethyl ether, tetrahydrofuran, 1,4-dioxane, toluene, and *tert*-butylmethylether, and drying and evaporating the organic layer to produce the carboxylic acid of formula (6) 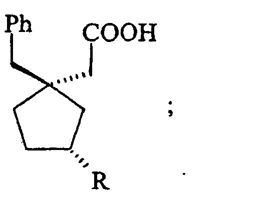
f) adding the product of Step e) above to a mixture of a base selected from triethylamine and diisopropylethylamine, a solvent selected from toluene, benzene, xylenes, tetrahydrofuran, diethyl ether and *n*-heptane, and diphenylphosphoryl azide (DPPA), and stirring at a temperature of from 0°C to 150°C to produce the isocyanate of formula (11) 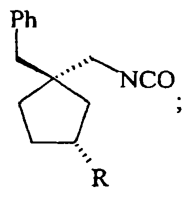 or adding the product of Step e) above to ethyl chloroformate or isobutyl chloroformate and a base selected from triethylamine and diisopropylethylamine, and a solvent selected from tetrahydrofuran, acetone, and diethyl ether at a temperature of from -40°C to 78°C, followed by adding a solution of sodium azide in water and tetrahydrofuran or acetone, followed by adding toluene or benzene, and refluxing to produce the isocyanate of formula (11) 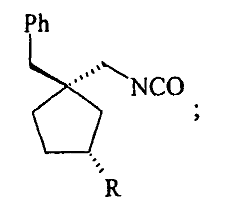
g) adding the product of Step f) to a solvent selected from toluene, benzene, xylenes, and *n*-heptane, and methanol, and stirring at a temperature from 0°C to 150°C to produce the carbamate of formula (12) 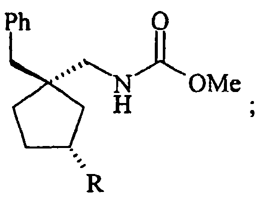
h) adding the product of Step g) to a mixture of carbon tetrachloride or ethyl acetate, and acetonitrile, water, sodium periodate, and ruthenium(III) chloride, and stirring at a temperature from -40°C to 80°C to produce the carboxylic acid of formula (13) 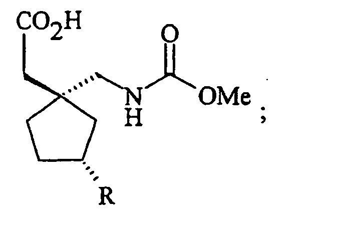
i) adding the product of Step h) to a mixture of a solvent selected from water, acetic acid, and 1,4-dioxane, and aqueous hydrochloric acid at a concentration of from 0.01 M to 12 M, and stirring at a temperature from 0°C to 115°C to produce a compound of formula IIa 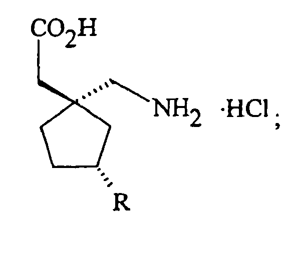 and
j) converting the product of Step i) to a compound of Formula II 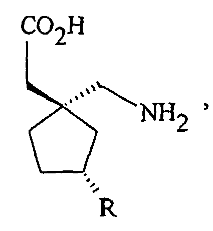 and further converting, if desired, to a pharmaceutically acceptable salt by known means.

7. A process according to Claim 5 which comprises:
a) adding a cyanoacetate of formula (A) 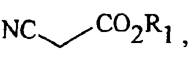 wherein R₁ is ethyl, to a mixture of a chiral cyclopentanone of formula (1) 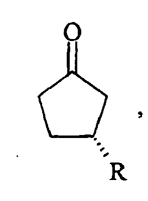 toluene, acetic acid, and a Knoevenagel reaction catalyst which is ammonium acetate, and heating the mixture at reflux over a Dean-Stark trap to produce the alkene of formula (2) 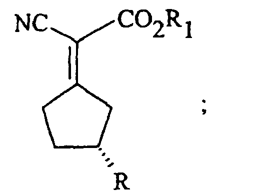
b) adding the product of Step a) above to a mixture of benzylmagnesium chloride in dry tetrahydrofuran at -100°C to 25°C to produce the addition products of formulas (3a) 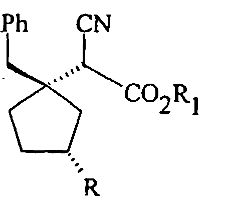 and (3b) 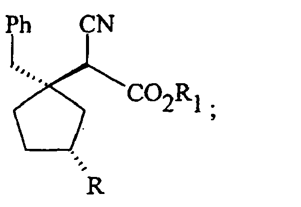
c) adding the products of Step b) above to a mixture of potassium hydroxide in ethylene glycol, and heating the mixture at 100°C to 200°C, and then acidifying to produce the hydrolysis products of formulas (4a) 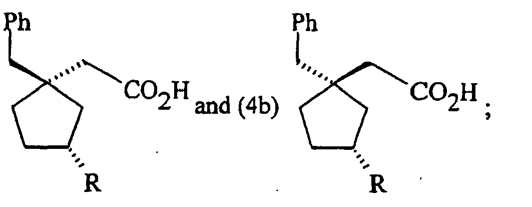
d) contacting the products of Step c) above with (S)-α-methylbenzylamine in ethyl acetate, and recrystallizing the salt so formed from ethyl acetate to produce the enriched diastereomer of formula (5) 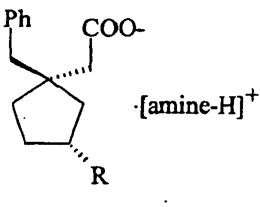 as the (S)-α-methylbenzylamine salt;
e) adding the product of Step d) to aqueous hydrochloric acid and stirring to produce the carboxylic acid of formula (6) 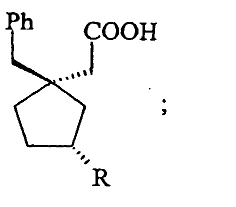
f) adding the product of Step e) to a mixture of triethylamine, toluene, and diphenylphosphoryl azide (DPPA), and refluxing to produce the isocyanate of formula (11) 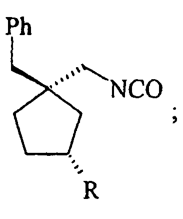 or adding the product of Step e) above to ethyl chloroformate or isobutyl chloroformate and triethylamine in tetrahydrofuran at a temperature of from -40°C to 78°C, followed by adding a solution of sodium azide in water and tetrahydrofuran or acetone, followed by adding toluene or benzene, and refluxing to produce isocyanate of formula (11) 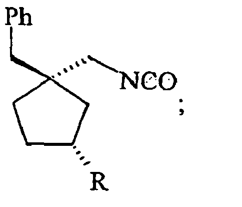
g) adding the product of Step f) to a mixture of methanol and toluene, and refluxing to produce the carbamate of formula (12) 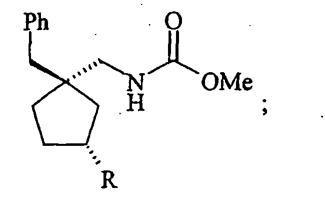
h) adding the product of Step g) to a mixture of carbon tetrachloride or ethyl acetate, and acetonitrile, water, sodium periodate, and ruthenium(III) chloride, and stirring to produce the carboxylic acid of formula (13) 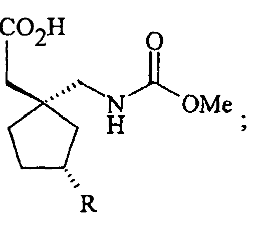
i) adding the product of Step h) to a mixture of 1,4-dioxane and aqueous hydrochloric acid at a concentration of 6 M, and stirring to produce a compound of formula Ila 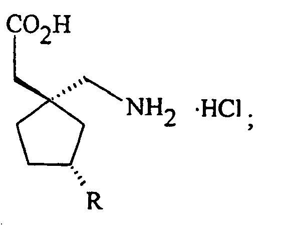
j) converting the product of Step i) to a compound of Formula II 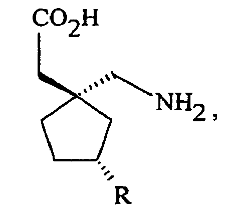 and further converting, if desired, to a pharmaceutically acceptable salt by known means.

8. A process according to Claim 5, further **characterized in that** the intermediate product (11) 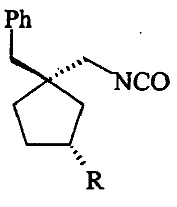 formed is further reacted, without isolation, with methanol to produce the carbamate of formula (12)

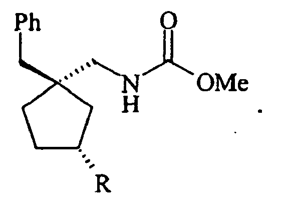

9. A process for the preparation of a compound of Formula II 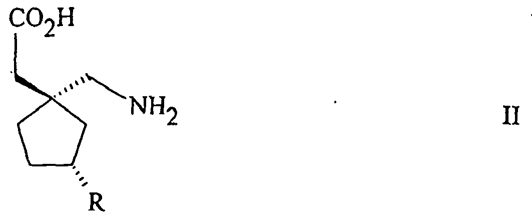 wherein R is C₁-C₁₀ alkyl or C₃-C₁₀ cycloalkyl, and pharmaceutically acceptable salts thereof, which comprises:
a) adding a cyanoacetate of formula (A) 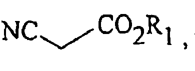 wherein R₁ is alkyl or benzyl, to a mixture of a chiral cyclopentanone of formula (1) 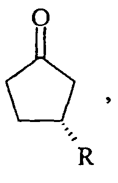 a solvent, a carboxylic acid, and a Knoevenagel reaction catalyst, and stirring the mixture in the presence of a means of removing water to produce the alkene of formula (2) 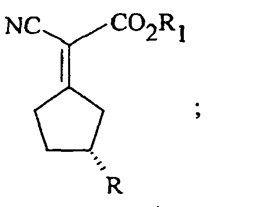
b) adding the product of Step a) above to a mixture of benzylmagnesium chloride, benzylmagnesium bromide, or benzylmagnesium iodide, in a solvent to produce the addition products of formulas (3a) 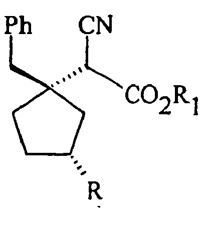 and (3b) 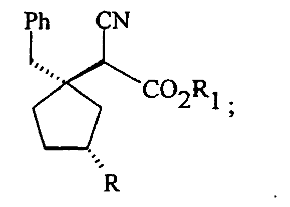
c) adding the products of Step b) above to a mixture of a base selected from potassium hydroxide, sodium hydroxide, lithium hydroxide, and cesium hydroxide, in a solvent, and stirring, and then acidifying to produce the carboxylic acids of formulas (4a) 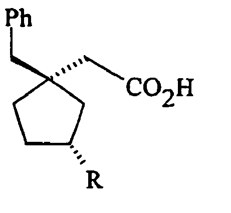 and (4b) 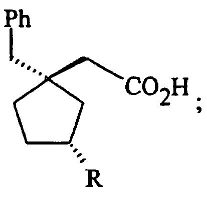 or adding the products of Step b) above to an acid mixture and stirring to produce the carboxylic acids of formulas (4a) 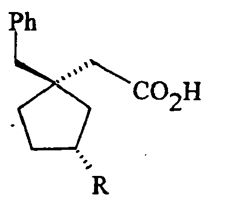 and (4b) 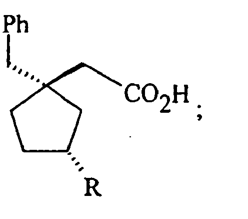
d) contacting the products of Step c) above with an amine in a solvent, and recrystallizing the salt so formed to produce the enriched diastereomer of formula (5) 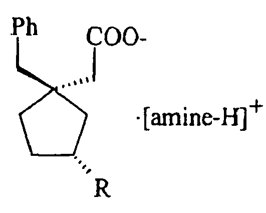 as the amine salt;
e) converting the product of Step d) to a carboxylic acid of formula (6) 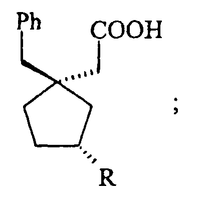
f) adding oxalyl chloride to a mixture of the product of Step e), a solvent, and N,N-dimethylformamide (DMF), and stirring to produce the acid chloride of formula (14) 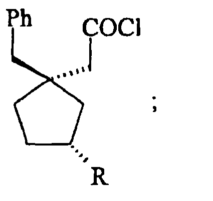
g) adding the product of Step f) to a mixture of *tert*-butyl alcohol, a solvent, and a tertiary amine base, and stirring to produce the ester of formula (15) 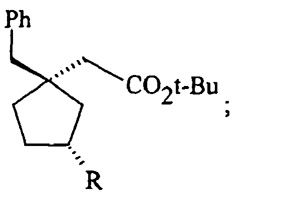
h) adding the product of Step g) to a mixture of carbon tetrachloride or ethyl acetate, and acetonitrile, water, sodium periodate, and ruthenium(III) chloride, and stirring to produce the carboxylic acid of formula (16) 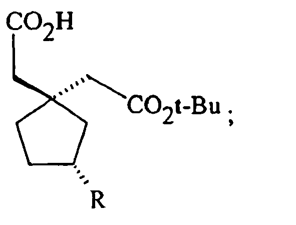
i) adding the product of Step h) to a mixture of a solvent, methanol, and (trimethylsilyl)diazomethane, and stirring to produce the bis ester of formula (17) 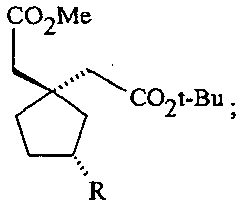 or adding the product of Step h) to a mixture of iodomethane, a solvent, and a base, and stirring to produce the bis ester of formula (17) 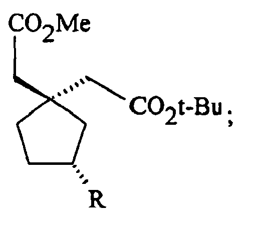
j) adding an acid to a mixture of the product from Step i) and a solvent, and stirring to produce the carboxylic acid of formula (18) 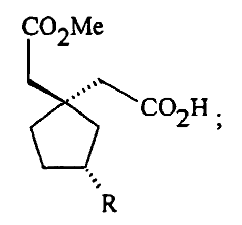
k) adding the product of Step j) to a mixture of a tertiary amine base, a solvent, and diphenylphosphoryl azide (DPPA) is added, and stirring to produce the isocyanate of formula (19) 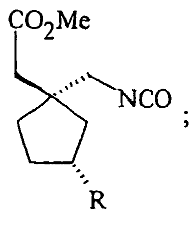 or adding the product of Step j) above to ethyl chloroformate or isobutyl chloroformate and a base in a solvent at a temperature of from -40°C to 78°C, followed by adding a solution of sodium azide in water and tetrahydrofuran or acetone, followed by adding toluene or benzene, and refluxing to produce isocyanate of formula (19) 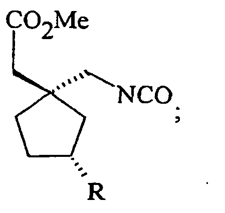
l) adding the product of Step k) to a mixture of a solvent and methanol, and stirring to produce the carbamate of formula (20) 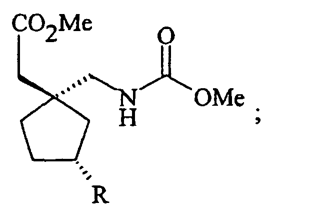
m) adding the product of Step 1) to a mixture of a solvent and aqueous hydrochloric acid is added, and stirring to produce a compound of formula (IIa) 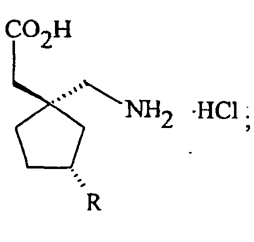 and
n) converting the product of Step m) to a compound of formula (II) 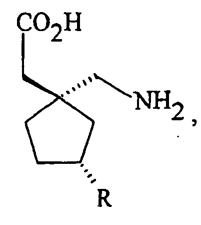 and further converting, if desired, to a pharmaceutically acceptable salt by known means.

10. A process according to Claim 9 which comprises:
a) adding a cyanoacetate of formula (A) 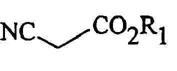 wherein R₁ is selected from methyl, ethyl, *n*-propyl, *iso*-propyl, *n*-butyl, *iso*-butyl, *sec*-butyl, *tert*-butyl, and benzyl to a mixture of a chiral cyclopentanone of formula (1) 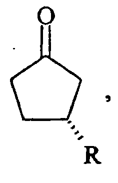 a solvent selected from tetrahydrofuran, 1,4-dioxane, *tert*-butylmethylether, chloroform, dichloromethane, acetonitrile, ethyl ether, ethyl acetate, hexanes, N,N-dimethylformamide, dimethylsulfoxide, ethanol, *tert*-butanol, toluene, benzene, xylenes, and *n*-heptane, acetic acid, and a Knoevenagel reaction catalyst selected from β-alanine, ammonium acetate, and piperidine, and stirring the mixture in the presence of a means of removing water selected from azeotropic distillation, activated molecular sieves, anhydrous magnesium sulfate, anhydrous sodium sulfate, anhydrous sodium carbonate, anhydrous potassium carbonate, anhydrous cesium carbonate, trimethyl orthoformate, and triethyl orthoformate to produce the alkene of formula (2) 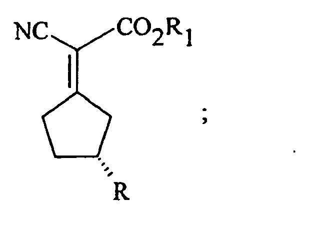
b) adding the product of Step a) above to a mixture of benzylmagnesium chloride, benzylmagnesium bromide, or benzylmagnesium iodide in a solvent selected from tetrahydrofuran, benzene, 1,4-dioxane, hexanes, *n*-heptane, toluene, diethyl ether, and *tert*-butyl methyl ether to produce the addition products of formulas (3a) 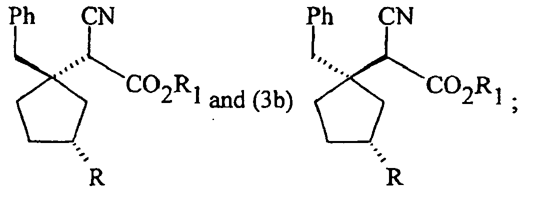
c) adding the products of Step b) above to a mixture of a base selected from potassium hydroxide, sodium hydroxide, lithium hydroxide, and cesium hydroxide in a solvent selected from ethylene glycol, 2-methoxyethyl ether, 1,4-dioxane, and diethylene glycol, and stirring the mixture and then acidifying to produce the carboxylic acids of formulas (4a) 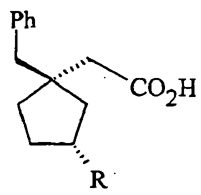 and (4b) 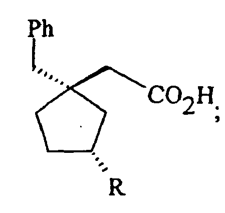 or adding the products of Step b) above to an acid mixture selected from 6-12 M HCl, 12 M H₂SO₄, 10%-48% wt/wt hydrobromic acid, and HBr in aqueous acetic acid, and stirring to produce the carboxylic acids of formulas (4a) 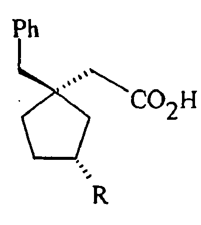 and (4b) 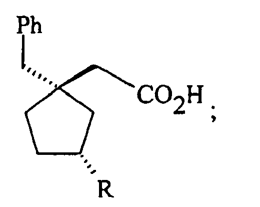
d) contacting the products of Step c) above with an amine selected from (S)-α-methyl-benzylamine, (R)-α-methyl-benzylamine, (R)-(+)-1-(naphthyl)ethylamine, (S)-(+)-1-(naphthyl)ethylamine, triethylamine, diisopropylethylamine, dicyclohexylamine, benzylamine, dibenzylamine, morpholine, N-methylmorpholine, piperidine, N-methylpiperidine, and pyridine in a solvent selected from N,N-dimethylformamide, chloroform, benzene, xylenes, hexanes, acetone, ethanol, methanol, *iso*-propanol, diethyl ether, dichloromethane, benzene, toluene, n-pentane, n-hexane, *n*-heptane, ethyl acetate, acetonitrile, *tert-*butyl methyl ether, tetrahydrofuran, and 1,4-dioxane, and recrystallizing the salt so formed to produce the enriched diastereomer of formula (5) 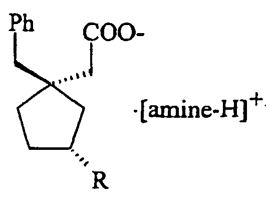 as the amine salt;
e) adding the product of Step d) to a mixture selected from aqueous hydrochloric acid, aqueous sulfuric acid, aqueous acetic acid, hydrochloric acid dissolved in acetic acid, or hydrochloric acid dissolved in acetic acid and water, and stirring to produce the carboxylic acid of formula (6) 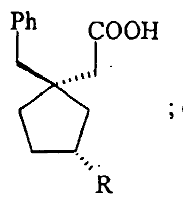 or partitioning the product of Step d) between a mixture of aqueous hydrochloric acid and a solvent selected from chloroform, dichloromethane, ethyl acetate, ethyl ether, tetrahydrofuran, 1,4-dioxane, toluene, and *tert*-butylmethylether, and drying and evaporating the organic layer to produce the carboxylic acid of formula (6) 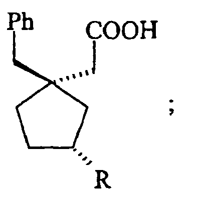
f) adding oxalyl chloride to a mixture of the product of Step e), a solvent selected from dichloromethane, chloroform, ethyl ether, toluene, and *tert-*butyl methyl ether, and 0.01 to 10 mole percent of N,N-dimethylformamide (DMF), and stirring at a temperature from -40°C to 110°C to produce the acid chloride of formula (14) 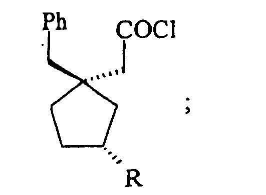
g) adding the product of Step f) to a mixture of *tert*-butyl alcohol, a solvent selected from dichloromethane, chloroform, ethyl ether, toluene, and *tert*-butyl methyl ether, and *N,N-*diisopropylethylamine (DIPEA) or triethylamine, and stirring at a temperature from -40°C to 110°C to produce the ester of formula (15) 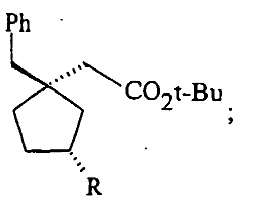
h) adding the product of Step g) to a mixture of carbon tetrachloride or ethyl acetate, and acetonitrile, water, sodium periodate, and ruthenium(III) chloride, and stirring at a temperature from -40°C to 80°C to produce the carboxylic acid of formula (16) 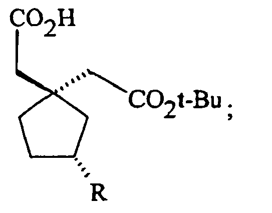
i) adding the product of Step h) to a solvent selected from toluene, benzene, xylenes, and *n*-heptane, methanol, and (trimethylsilyl)diazomethane, and stirring at a temperature from 0°C to 150°C to produce the bis ester of formula (17) 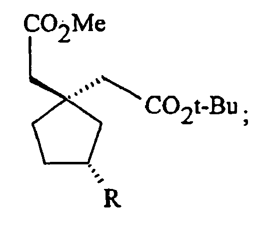 or adding the product of Step h) to a mixture of iodomethane, a solvent selected from dichloromethane, chloroform, tetrahydrofuran, toluene and 1,4-dioxane, and a base selected from 1,8-diazabicyclo[5.4.0]undec-7-ene (DBU), diisopropylethylamine, triethylamine, or 1,5-diazabicyclo[4.3.0]non-5-ene (DBN), and stirring at a temperature of from -40°C to 110°C to produce the bis ester of formula (17) 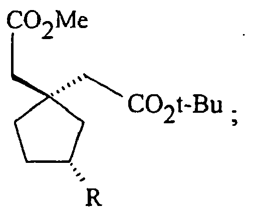
j) adding hydrochloric acid or trifluoroacetic acid (TFA) to a mixture of the product from Step i) and a solvent selected from dichloromethane, chloroform, 1,4-dioxane, tetrahydrofuran, ethyl ether, and *tert*-butyl methyl ether, and stirring at a temperature from -40°C to 110°C to produce the carboxylic acid of formula (18) 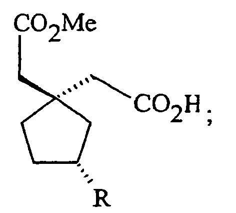
k) adding the product of Step j) to a mixture of a base selected from triethylamine and diisopropylethylamine, a solvent selected from toluene, benzene, xylenes, and *n*-heptane, and diphenylphosphoryl azide (DPPA), and stirring at a temperature from 0°C to 150°C to produce the isocyanate of formula (19) 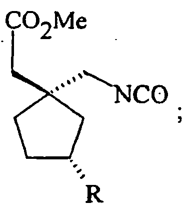 or adding the product of Step j) above to ethyl chloroformate or isobutyl chloroformate, a base selected from triethylamine and diisopropylethylamine, and a solvent selected from tetrahydrofuran, acetone, and diethyl ether at a temperature of from -40°C to 78°C, followed by adding a solution of sodium azide in water and tetrahydrofuran or acetone, followed by adding toluene or benzene, and refluxing to produce isocyanate of formula (19) 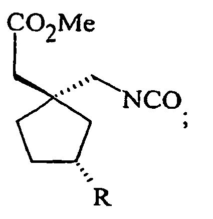
l) adding the product of Step k) to a mixture of a solvent selected from toluene, benzene, xylenes, and *n*-heptane, and methanol, and stirring at a temperature from 0°C to 150°C to produce the carbamate of formula (20) 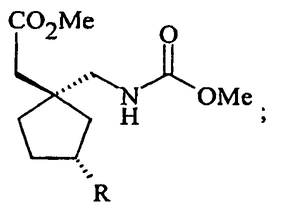
m) adding the product of Step 1) to a mixture of a solvent selected from water, acetic acid, and 1,4-dioxane, and aqueous hydrochloric acid at a concentration of from 0.01 M to 12 M, and stirring at a temperature from 0°C to 115°C to produce a compound of formula IIa 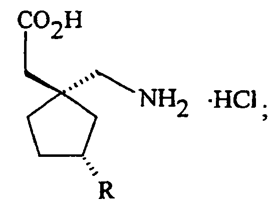 and
n) converting the product of Step m) to a compound of Formula II 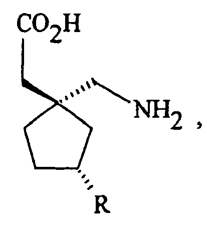 and further converting, if desired, to a pharmaceutically acceptable salt by known means.

11. A process according to Claim 9 which comprises:
a) adding a cyanoacetate of formula (A) 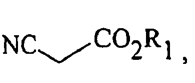 wherein R₁ is ethyl, to a mixture of a chiral cyclopentanone of formula (1) 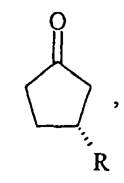 toluene, acetic acid, and a Knoevenagel reaction catalyst which is ammonium acetate, and heating the mixture at reflux over a Dean-Stark trap to produce the alkene of formula (2) 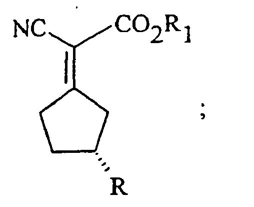
b) adding the product of Step a) above to a mixture of benzylmagnesium chloride in dry tetrahydrofuran at -100°C to 25°C to produce the addition products of formulas (3a) 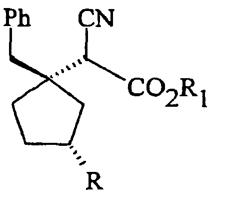 and (3b) 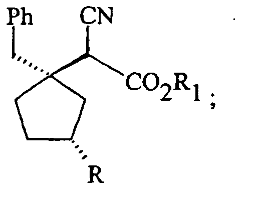
c) adding the products of Step b) above to a mixture of potassium hydroxide in ethylene glycol and heating the mixture at 100°C to 200°C, and then acidifying to produce the hydrolysis products of formulas (4a) 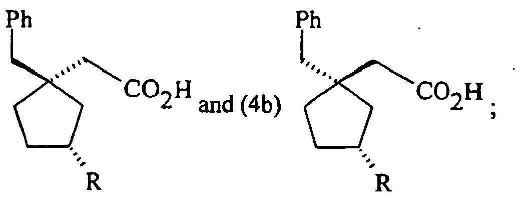
d) contacting the products of Step c) above with (S)-α-methylbenzylamine in ethyl acetate, and recrystallizing the salt so formed from ethyl acetate to produce the enriched diastereomer of formula (5) 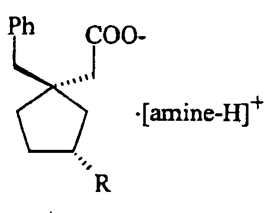 as the (S)-α-methylbenzylamine salt;
e) adding the product of Step d) to aqueous hydrochloric acid and stirring to produce the carboxylic acid of formula (6) 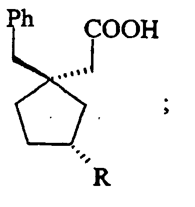
f) adding oxalyl chloride to a mixture of the product of Step e), dichloromethane, and a catalytic amount of N,N-dimethylformamide (DMF), and stirring to produce the acid chloride of formula (14) 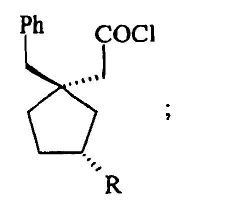
g) adding the product of Step f) to a mixture of *tert*-butyl alcohol, dichloromethane, and *N,N*-diisopropylethylamine (DIPEA), and stirring to produce the ester of formula (15) 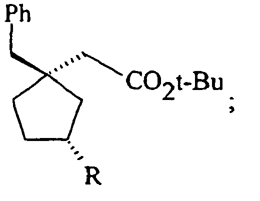
h) adding the product of Step g) to a mixture of carbon tetrachloride or ethyl acetate, and acetonitrile, water, sodium periodate, and ruthenium(III) chloride, and stirring to produce the carboxylic acid of formula (16) 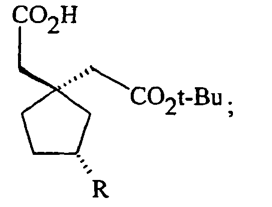
i) adding the product of Step h) to a mixture of methanol, toluene, and (trimethylsilyl)diazomethane, and stirring to produce the bis ester of formula (17) 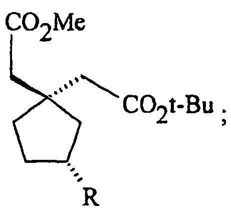 or adding the product of Step h) to a mixture of iodomethane, dichloromethane, triethylamine, and stirring to produce the bis ester of formula (17) 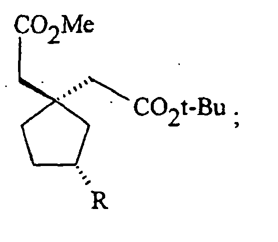
j) adding hydrochloric acid or trifluoroacetic acid (TFA) to a mixture of the product from Step i) and dichloromethane, and stirring to produce the carboxylic acid of formula (18) 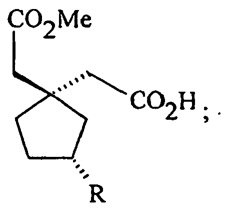
k) adding the product of Step j) to a mixture of triethylamine, toluene, and diphenylphosphoryl azide (DPPA), and refluxing to produce the isocyanate of formula (19) 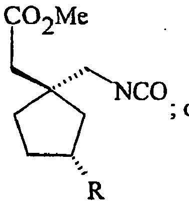 or adding the product of Step j) above to ethyl chloroformate or isobutyl chloroformate, triethylamine, and tetrahydrofuran at a temperature of from -40°C to 78°C, followed by adding a solution of sodium azide in water and tetrahydrofuran or acetone, followed by adding toluene or benzene, and refluxing to produce isocyanate of formula (19) 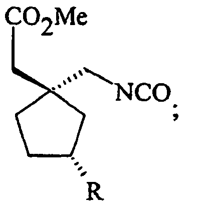
l) adding the product of Step k) to a mixture of methanol and toluene, and refluxing to produce the carbamate of formula (20) 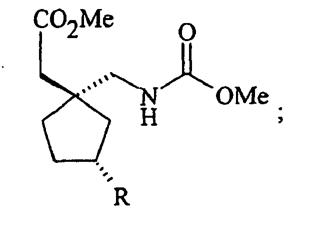
m) adding the product of Step 1) to a mixture of 1,4-dioxane and aqueous hydrochloric acid at a concentration of 6 M, and stirring to produce a compound of formula IIa 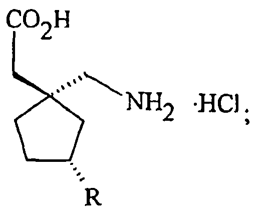
n) converting the product of Step m) to a compound of Formula II 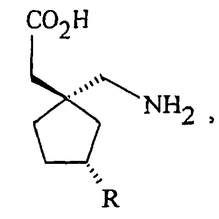 and further converting, if desired, to a pharmaceutically acceptable salt by known means.

12. A process according to Claim 9, further **characterized in that** the intermediate product (14) 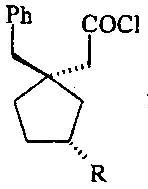 formed is further reacted, without isolation, with *tert*-butyl alcohol to produce the ester of formula (15)

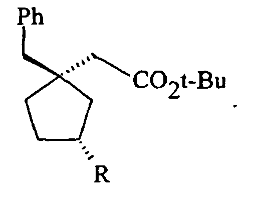

13. A process according to Claim 9, **characterized in that** the intermediate product (19) 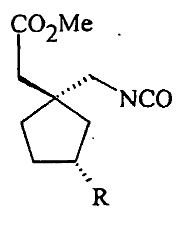 formed is further reacted, without isolation, with methanol to produce the carbamate of formula (20)

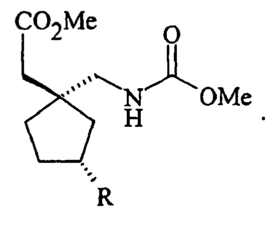

14. A process according to Claim 9, **characterized in that** the intermediate product (14) 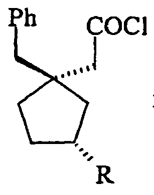 formed is further reacted, without isolation, with *tert*-butyl alcohol to produce the ester of formula (15) 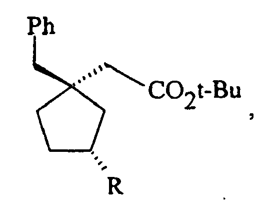 and the intermediate product (19) 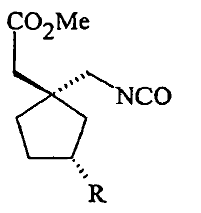 formed is further reacted, without isolation, with methanol to produce the carbamate of formula (20)

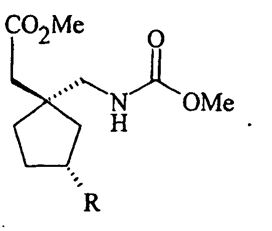

15. A process for the preparation of a compound of Formula III 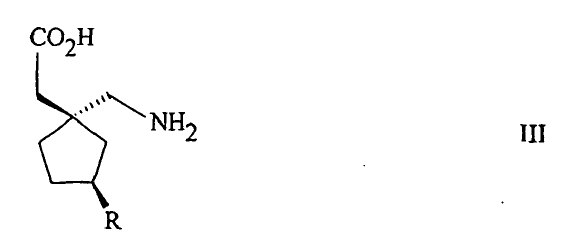 wherein R is C₁-C₁₀ alkyl or C₃-C₁₀ cycloalkyl, and pharmaceutically acceptable salts thereof, which comprises:
a) adding a cyanoacetate of formula (A) 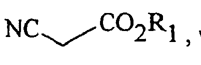 wherein R₁ is alkyl or benzyl, to a mixture of a chiral cyclopentanone of formula (21) 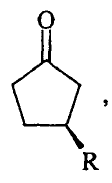 a solvent, a carboxylic acid, and a Knoevenagel reaction catalyst, and stirring the mixture in the presence of a means of removing water to produce the alkene of formula (22) 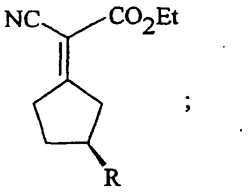
b) adding the product of Step a) above to a mixture of benzylmagnesium chloride or benzylmagnesium iodide, in a solvent to produce the addition of products of formulas (23a) 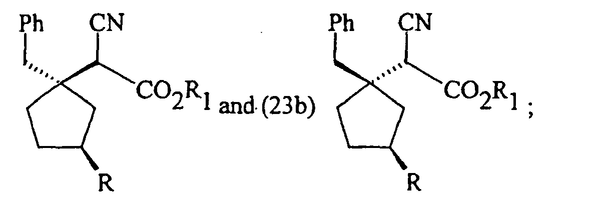
c) adding the products of Step b) above to a mixture of a base selected from potassium hydroxide, sodium hydroxide, lithium hydroxide, and cesium hydroxide, in a solvent, and stirring, and then acidifying to produce the carboxylic acids of formulas (24a) 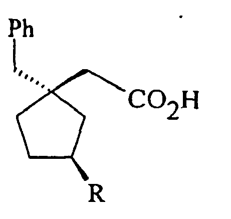 and (24b) 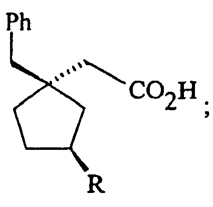 or adding the products of Step b) above to an acid mixture, and stirring to produce the carboxylic acids of formulas (24a) 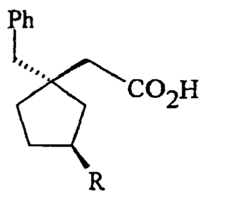 and (24b) 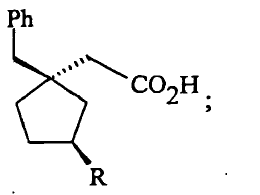
d) contacting the products of Step c) above with an amine in a solvent, and recrystallizing the salt so formed to produce the enriched diastereomer of formula (25) 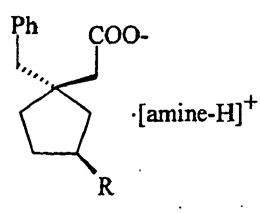 as the amine salt; and
e) converting the product of Step d) to a carboxylic acid of formula (26) 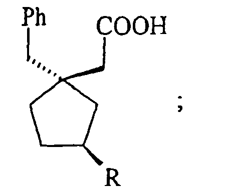
f) adding the product of Step e) to a mixture of iodomethane, a solvent, and 1,8-diazabicyclo[5.4.0]undec-7-ene (DBU), and stirring to produce the ester of formula (27) 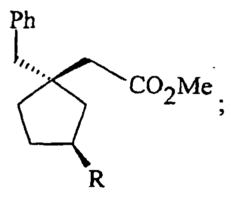 or adding the product of Step e) to methanol and an acid to produce the ester of formula (27) 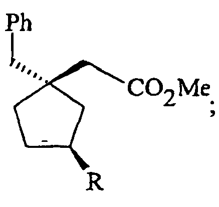 or adding the product of Step e) to a solution of diazomethane or trimethylsilyl-diazomethane in a solvent to produce ester of formula (27) 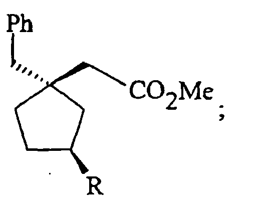
g) adding the product of Step f) to a mixture of carbon tetrachloride or ethyl acetate, and acetonitrile, water, sodium periodate, and ruthenium(III) chloride, and stirring to produce the carboxylic acid of formula (28) 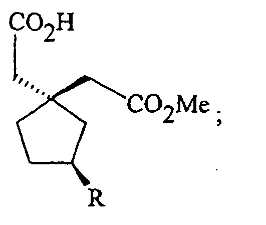
h) adding the product of Step g) to a mixture of a tertiary amine base, a solvent, and diphenylphosphoryl azide (DPPA), and stirring to produce the isocyanate of formula (29) 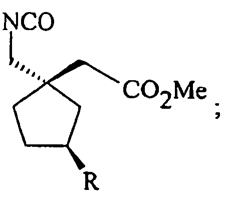 or adding the product of Step g) above to ethyl chloroformate or isobutyl chloroformate and a base in a solvent at a temperature of from -40°C to 78°C, followed by adding a solution of sodium azide in water and tetrahydrofuran or acetone, followed by adding toluene or benzene, and refluxing to produce isocyanate of formula (29) 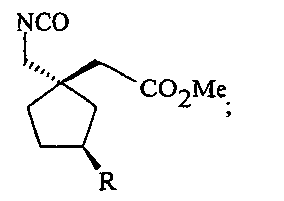
i) adding the product of Step h) to a mixture of a solvent and methanol, and stirring to produce the carbamate of formula (30) 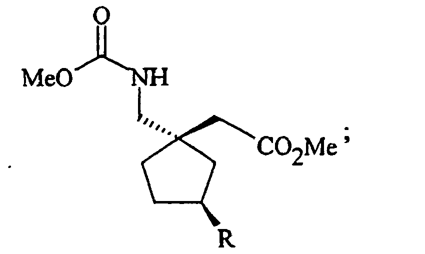
j) adding the product of Step i) to a mixture of a solvent and aqueous hydrochloric acid, and stirring to produce a compound of formula (IIIa) 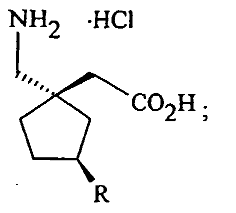
k) converting the product of Step j) to a compound of formula (III) 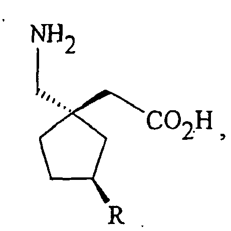 and further converting, if desired, to a pharmaceutically acceptable salt by known means.

16. A process for the preparation of a compound of Formula IV 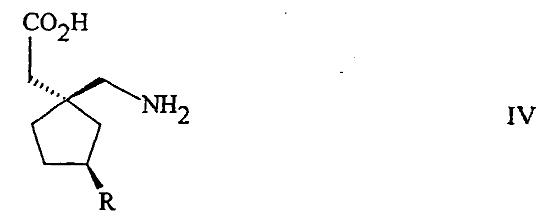 wherein R is C₁-C₁₀ alkyl or C₃-C₁₀ cycloalkyl, and pharmaceutically acceptable salts thereof, which comprises:
a) adding a cyanoacetate of formula (A) 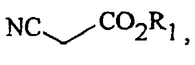 wherein R₁ is alkyl or benzyl, to a mixture of a chiral cyclopentanone of formula (21) 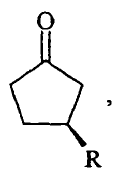 a solvent, a carboxylic acid, and a Knoevenagel reaction catalyst, and stirring the mixture in the presence of a means of removing water to produce the alkene of formula (22) 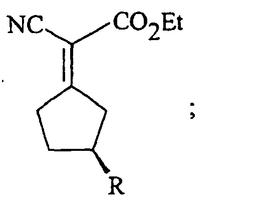
b) adding the product of Step a) above to a mixture of benzylmagnesium chloride or benzylmagnesium iodide, in a solvent to produce the addition of products of formulas (23a) 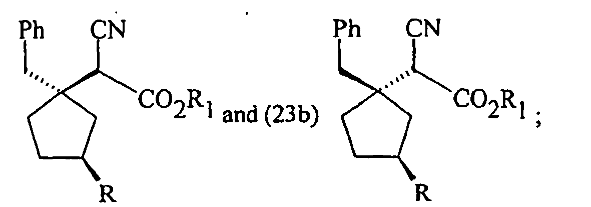
c) adding the products of Step b) above to a mixture of a base selected from potassium hydroxide, sodium hydroxide, lithium hydroxide, and cesium hydroxide, in a solvent, and stirring, and then acidifying to produce the carboxylic acids of formulas (24a) 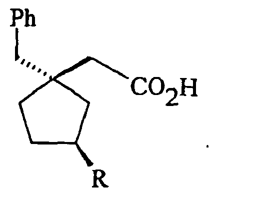 and (24b) 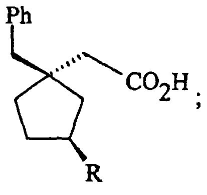 or adding the products of Step b) above to an acid mixture, and stirring to produce the carboxylic acids of formulas (24a) 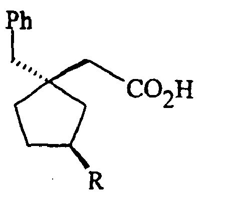 and (24b) 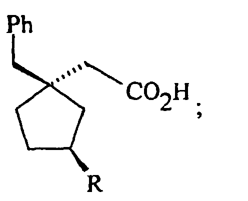
d) contacting the products of Step c) above with an amine in a solvent, and recrystallizing the salt so formed to produce the enriched diastereomer of formula (25) 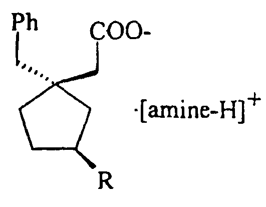 as the amine salt; and
e) converting the product of Step d) to a carboxylic acid of formula (26) 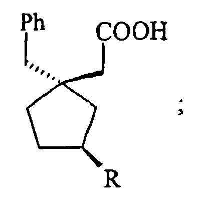
f) adding the product of Step e) to a mixture of a tertiary amine base, a solvent, and diphenylphosphoryl azide (DPPA) is added, and stirring to produce the isocyanate of formula (31) 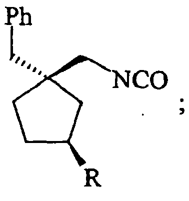 or adding the product of Step g) above to ethyl chloroformate or isobutyl chloroformate and a base in a solvent at a temperature of from -40°C to 78°C, followed by adding a solution of sodium azide in water and tetrahydrofuran or acetone, followed by adding toluene or benzene, and refluxing to produce the isocyanate of formula (31) 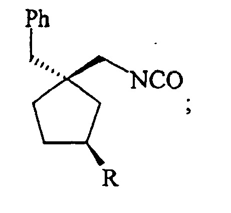
g) adding the product of Step f) to a mixture of a solvent and methanol, and stirring to produce the carbamate of formula (32) 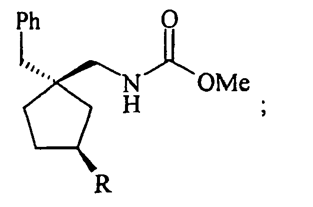
h) adding the product of Step g) to a mixture of carbon tetrachloride or ethyl acetate, and acetonitrile, water, sodium periodate, and ruthenium(III) chloride, and stirring to produce the carboxylic acid of formula (33) 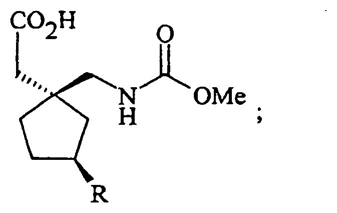
i) adding the product of Step h) to a mixture of a solvent and aqueous hydrochloric acid, and stirring to produce a compound of formula (IVa) 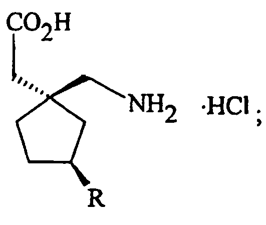 and
j) converting the product of Step i) to a compound of formula (IV) 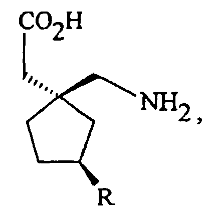 and further converting, if desired, to a pharmaceutically acceptable salt by known means.

17. A process for the preparation of a compound of Formula IV 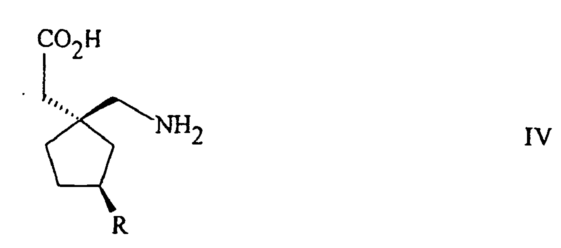 wherein R is C₁-C₁₀ alkyl or C₃-C₁₀ cycloalkyl, and pharmaceutically acceptable salts thereof, which comprises:
a) adding a cyanoacetate of formula (A) 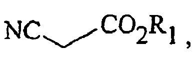 wherein R₁ is alkyl or benzyl, to a mixture of a chiral cyclopentanone of formula (21) 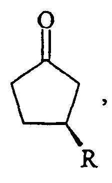 a solvent, a carboxylic acid, and a Knoevenagel reaction catalyst, and stirring the mixture in the presence of a means of removing water to produce the alkene of formula (22) 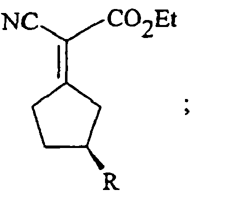
b) adding the product of Step a) above to a mixture of benzylmagnesium chloride or benzylmagnesium iodide, in a solvent to produce the addition of products of formulas (23a) 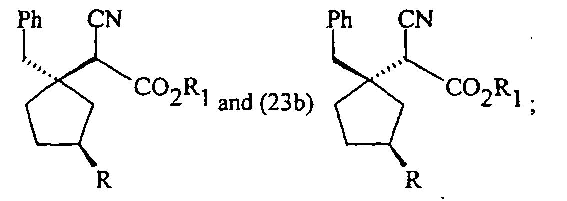
c) adding the products of Step b) above to a mixture of a base selected from potassium hydroxide, sodium hydroxide, lithium hydroxide, and cesium hydroxide, in a solvent, and stirring, and then acidifying to produce the carboxylic acids of formulas (24a) 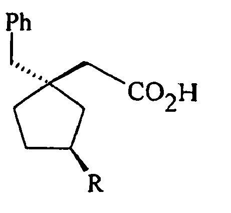 and (24b) 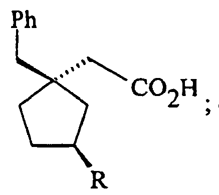 or adding the products of Step b) above to an acid mixture, and stirring to produce the carboxylic acids of formulas (24a) 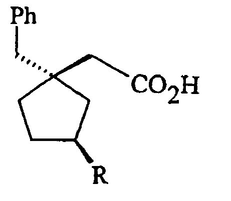 and (24b) 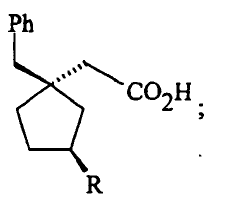
d) contacting the products of Step c) above with an amine in a solvent, and recrystallizing the salt so formed to produce the enriched diastereomer of formula (25) 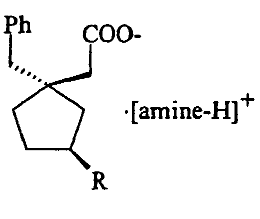 as the amine salt; and
e) converting the product of Step d) to a carboxylic acid of formula (26) 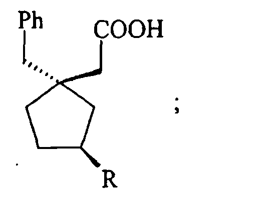
f) adding oxalyl chloride to a mixture of the product of Step e), a solvent, and N,N-dimethylformamide (DMF), and stirring to produce the acid chloride of formula (34) 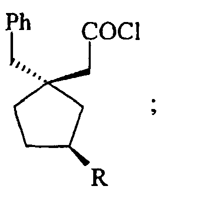
g) adding the product of Step f) to a mixture of *tert-*butyl alcohol, a solvent, and a tertiary amine base, and stirring to produce the ester of formula (35) 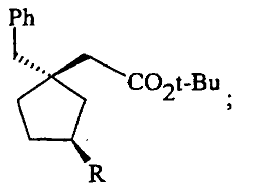
h) adding the product of Step g) to a mixture of carbon tetrachloride or ethyl acetate, and acetonitrile, water, sodium periodate, and ruthenium(III) chloride, and stirring to produce the carboxylic acid of formula (36) 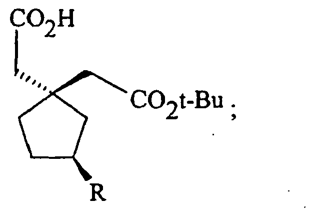
i) adding the product of Step h) to a mixture of a solvent, methanol, and (trimethylsilyl)diazomethane, and stirring to produce the bis ester of formula (37) 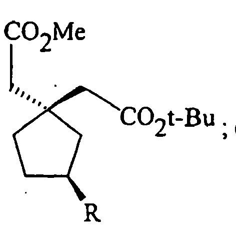 or adding the product of Step h) to a mixture of iodomethane, a solvent, and a base, and stirring to produce the bis ester of formula (37) 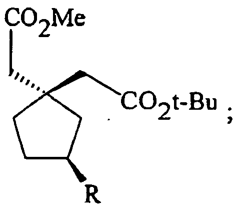
j) adding an acid to a mixture of the product from Step i) and a solvent and stirring to produce the carboxylic acid of formula (38) 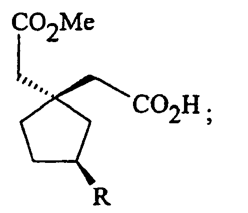
k) adding the product of Step j) to a mixture of a tertiary amine base, a solvent, and diphenylphosphoryl azide (DPPA), and stirring to produce the isocyanate of formula (39) 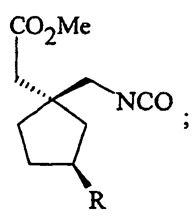 or adding the product of Step j) above to ethyl chloroformate or isobutyl chloroformate and a base in a solvent at a temperature of from -40°C to 78°C, followed by adding a solution of sodium azide in water and tetrahydrofuran or acetone, followed by adding toluene or benzene, and refluxing to produce isocyanate of formula (39) 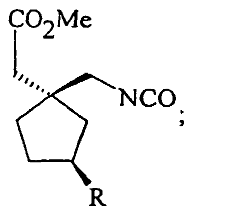
l) adding the product of Step k) to a mixture of a solvent and methanol, and stirring to produce the carbamate of formula (40) 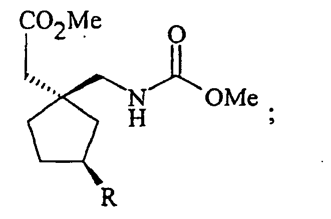
m) adding the product of Step 1) to a mixture of a solvent and hydrochloric acid, and stirring to produce a compound of formula (IVa) 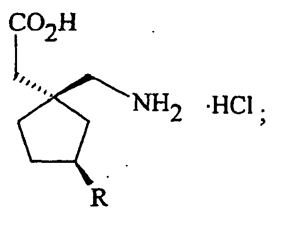
n) converting the product of Step m) to a compound of Formula IV 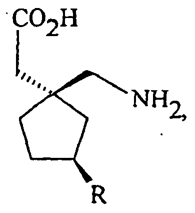 and further converting, if desired, to a pharmaceutically acceptable salt by known means.

18. A process for the preparation of a compound of formula (6) 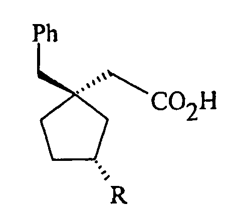 wherein R is C₁-C₁₀alkyl or C₃-C₁₀ cycloalkyl, and pharmaceutically acceptable salts thereof, which comprises:
a) adding a cyanoacetate of formula (A) 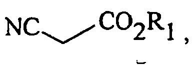 wherein R₁ is alkyl or benzyl, to a mixture of a chiral cyclopentanone of formula (1) 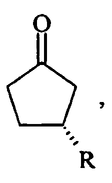 a solvent, a carboxylic acid, and a Knoevenagel reaction catalyst, and stirring the mixture in the presence of a means of removing water to produce the alkene of formula (2) 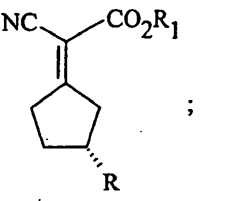
b) adding the product of Step a) above to a mixture of benzylmagnesium chloride, benzylmagnesium bromide, or benzylmagnesium iodide, in a solvent to produce the addition products of formulas (3a) 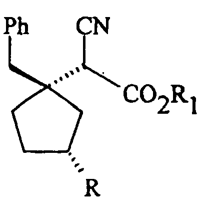 and (3b) 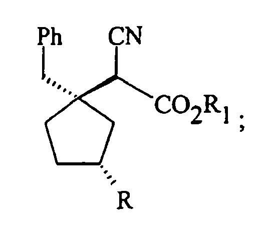
c) adding the products of Step b) above to a mixture of a base selected from potassium hydroxide, sodium hydroxide, lithium hydroxide, and cesium hydroxide, and a solvent, and stirring, and then acidifying to produce the carboxylic acids of formulas (4a) 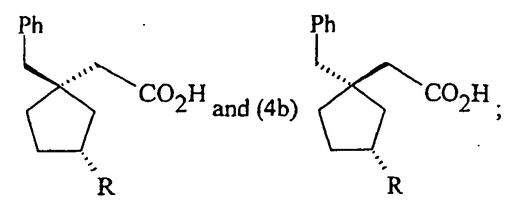 or adding the products of Step b) above to an acid mixture and stirring to produce the carboxylic acids of formulas (4a) 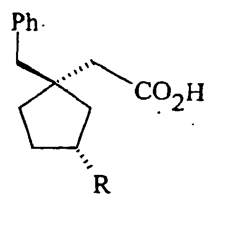 and (4b) 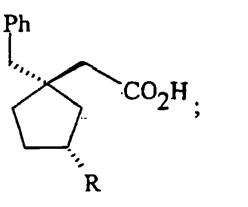
d) contacting the products of Step c) above with an amine in a solvent, and recrystallizing the salt so formed to produce the enriched diastereomer of formula (5) 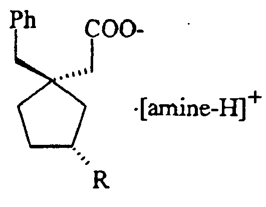 as the amine salt; and
e) converting the product of Step d) to a carboxylic acid of formula (6)

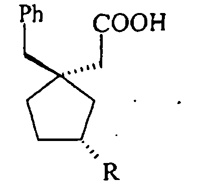

19. A process according to Claim 18 which comprises:
a) adding a cyanoacetate of formula (A) 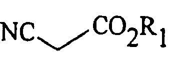 wherein R₁ is selected from methyl, ethyl, *n*-propyl, *iso*-propyl, *n*-butyl, *iso*-butyl, *sec*-butyl, *tert*-butyl, and benzyl to a mixture of a chiral cyclopentanone of formula (1) 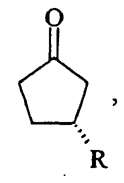 a solvent selected from tetrahydrofuran, 1,4-dioxane, *tert*-butylmethylether, chloroform, dichloromethane, acetonitrile, ethyl ether, ethyl acetate, hexanes, N,N-dimethylformamide, dimethylsulfoxide, ethanol, *tert*-butanol, toluene, benzene, xylenes, and *n*-heptane, acetic acid, and a Knoevenagel reaction catalyst selected from β-alanine, ammonium acetate, and piperidine, and stirring the mixture in the presence of a means of removing water selected from azeotropic distillation, activated molecular sieves, anhydrous magnesium sulfate, anhydrous sodium sulfate, anhydrous sodium carbonate, anhydrous potassium carbonate, anhydrous cesium carbonate, trimethyl orthoformate, and triethyl orthoformate to produce the alkene of formula (2) 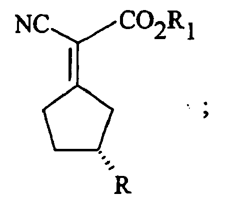
b) adding the product of Step a) above to a mixture of benzylmagnesium chloride, benzylmagnesium bromide, or benzylmagnesium iodide in a solvent selected from tetrahydrofuran, benzene, 1,4-dioxane, hexanes, *n*-heptane, toluene, diethyl ether, and *tert*-butyl methyl ether to produce the addition products of formulas (3a) 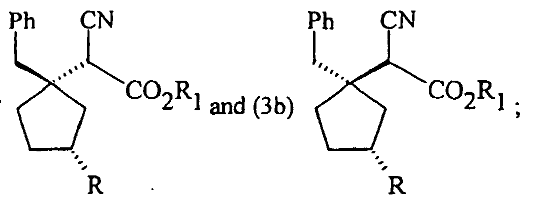
c) adding the products of Step b) above to a mixture of a base selected from potassium hydroxide, sodium hydroxide, lithium hydroxide, and cesium hydroxide in a solvent selected from ethylene glycol, 2-methoxyethyl ether, 1,4-dioxane, and diethylene glycol, and stirring the mixture, and then acidifying to produce the carboxylic acids of formulas (4a) 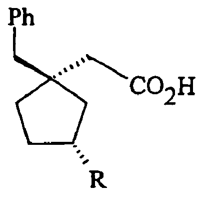 and (4b) 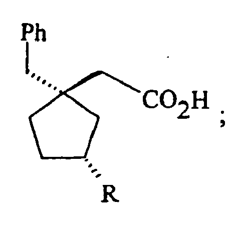 or adding the products of Step b) above to an acid mixture selected from 6-12 M HCl, 12 M H₂SO₄, 10%-48% wt/wt hydrobromic acid, and HBr in aqueous acetic acid, and stirring to produce the carboxylic acids of formulas (4a) 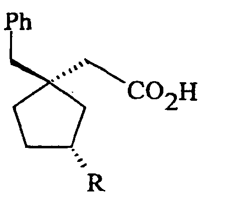 and (4b) 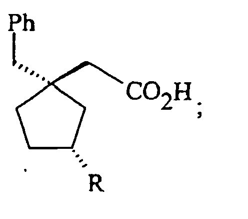
d) contacting the products of Step c) above with an amine selected from (S)-α-methyl-benzylamine, (R)-α-methyl-benzylamine, (R)-(+)-1-(naphthyl)ethylamine, (S)-(+)-1-(naphthyl)ethylamine, triethylamine, diisopropylethylamine, dicyclohexylamine, benzylamine, dibenzylamine, morpholine, N-methylmorpholine, piperidine, N-methylpiperidine, and pyridine in a solvent selected from N,N-dimethylformamide, chloroform, benzene, xylenes, hexanes, acetone, ethanol, methanol, *iso*-propanol, diethyl ether, dichloromethane, benzene, toluene, n-pentane, n-hexane, n-heptane, ethyl acetate, acetonitrile, *tert-*butyl methyl ether, tetrahydrofuran, and 1,4-dioxane, and recrystallizing the salt so formed to produce the enriched diastereomer of formula (5) 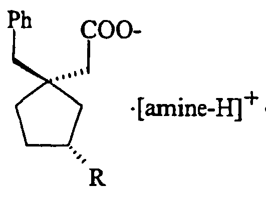 as the amine salt; and
e) adding the product of Step d) to a mixture selected from aqueous hydrochloric acid, aqueous sulfuric acid, aqueous acetic acid, hydrochloric acid dissolved in acetic acid, and hydrochloric acid dissolved in acetic acid and water, and stirring to produce the carboxylic acid of formula (6) 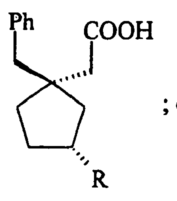 or partitioning the product of Step d) between a mixture of aqueous hydrochloric acid and a solvent selected from chloroform, dichloromethane, ethyl acetate, ethyl ether, tetrahydrofuran, 1,4-dioxane, toluene, and *tert*-butylmethylether, and drying and evaporating the organic layer to produce the carboxylic acid of formula (6)

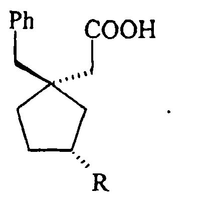

20. A process according to Claim 18 which comprises:
a) adding a cyanoacetate of formula (A) 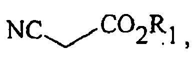 wherein R₁ is ethyl, to a mixture of a chiral cyclopentanone of formula (1) 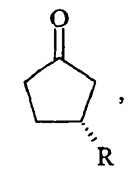 toluene, acetic acid, and a Knoevenagel reaction catalyst which is ammonium acetate, and heating the mixture at reflux over a Dean-Stark trap to produce the alkene of formula (2) 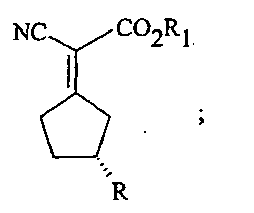
b) adding the product of Step a) above to a mixture of benzylmagnesium chloride in dry tetrahydrofuran at -100°C to 25°C to produce the addition products of formulas (3a) 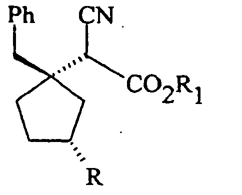 and (3b) 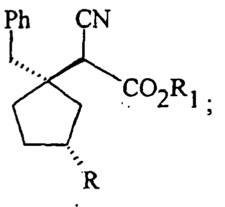
c) adding the products of Step b) above to a mixture of potassium hydroxide in ethylene glycol, and heating the mixture at 100°C to 200°C, and then acidifying to produce the hydrolysis products of formulas (4a) 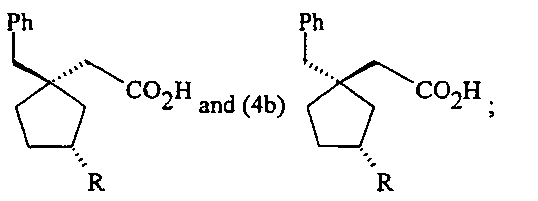
d) contacting the products of Step c) above with (S)-α-methylbenzylamine in ethyl acetate, and recrystallizing the salt so formed from ethyl acetate to produce the enriched diastereomer of formula (5) 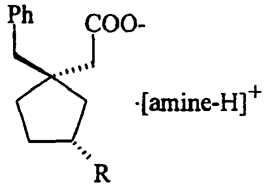 as the (S)-α-methyl-benzylamine salt;
e) adding the product of Step d) to aqueous hydrochloric acid and stirring to produce the carboxylic acid of formula (6)

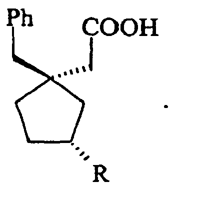

21. A process for the preparation of a compound of formula (26) 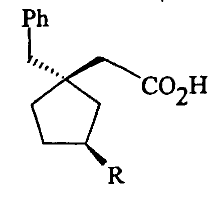 wherein R is C₁-C₁₀ alkyl or C₁-C₁₀ cycloalkyl, and pharmaceutically acceptable salts thereof, which comprises:
a) adding a cyanoacetate of formula (A) 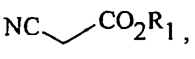 wherein R₁ is alkyl or benzyl, to a mixture of a chiral cyclopentanone of formula (21) 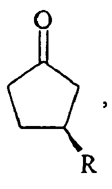 a solvent, a carboxylic acid, and a Knoevenagel reaction catalyst, and stirring the mixture in the presence of a means of removing water to produce the alkene of formula (22) 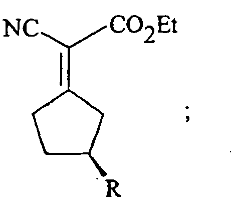
b) adding the product of Step a) above to a mixture of benzylmagnesium chloride or benzylmagnesium iodide, in a solvent to produce the addition of products of formulas (23a) 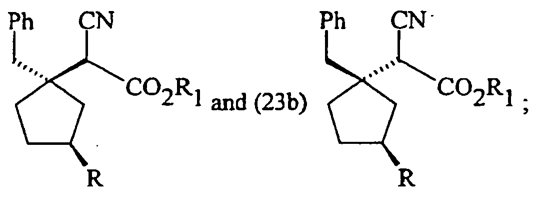
c) adding the products of Step b) above to a mixture of a base selected from potassium hydroxide, sodium hydroxide, lithium hydroxide, and cesium hydroxide, and a solvent, and stirring, and then acidifying to produce the carboxylic acids of formulas (24a) 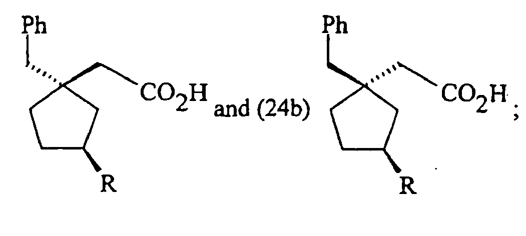 or adding the products of Step b) above to an acid mixture, and stirring to produce the carboxylic acids of formulas (24a) 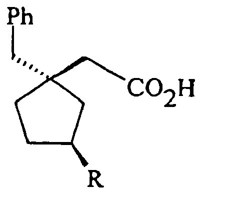 and (24b) 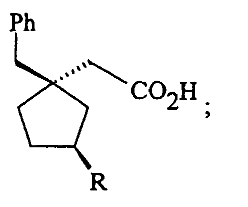
d) contacting the products of Step c) above with an amine in a solvent, and recrystallizing the salt so formed to produce the enriched diastereomer of formula (25) 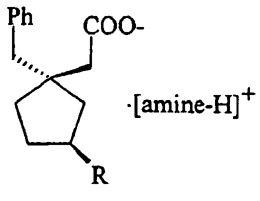 as the amine salt; and
e) converting the product of Step d) to a carboxylic acid of formula (26)

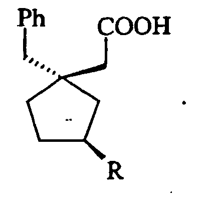

22. A process according to Claim 21 which comprises:
a) adding a cyanoacetate of formula (A) 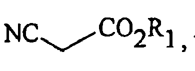 wherein R₁ is selected from methyl, ethyl, *n*-propyl, *iso*-propyl, *n*-butyl, *iso*-butyl, *sec*-butyl, *tert*-butyl, and benzyl, to a mixture of a chiral cyclopentanone of formula (21) 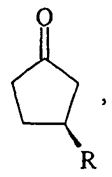 a solvent selected from tetrahydrofuran, 1,4-dioxane, *tert*-butylmethylether, chloroform, dichloromethane, acetonitrile, ethyl ether, ethyl acetate, hexanes, N,N-dimethylformamide, dimethylsulfoxide, ethanol, *tert*-butanol, toluene, benzene, xylenes, and *n*-heptane, acetic acid, and a Knoevenagel reaction catalyst selected from β-alanine, ammonium acetate, and piperidine, and stirring the mixture in the presence of a means of removing water selected from azeotropic distillation, activated molecular sieves, anhydrous magnesium sulfate, anhydrous cesium carbonate, trimethyl orthoformate, and triethyl orthoformate to produce the alkene of formula (22) 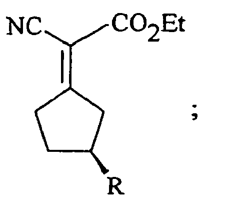
b) adding the product of Step a) above to a mixture of benzylmagnesium chloride, benzylmagnesium bromide, or benzylmagnesium iodide in a solvent selected from tetrahydrofuran, 1,4-dioxane, hexanes, *n-*heptane, toluene, diethyl ether, and *tert*-butyl methyl ether to produce the addition products of formulas (23a) 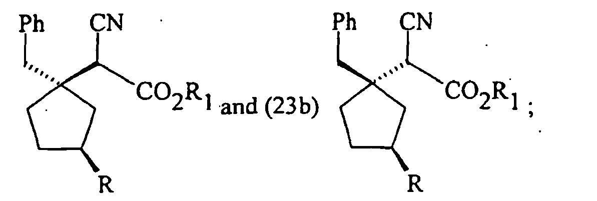
c) adding the products of Step b) above to a mixture of a base selected from potassium hydroxide, sodium hydroxide, lithium hydroxide, and cesium hydroxide in a solvent selected from ethylene glycol, 2-methoxyethyl ether, 1,4-dioxane, and diethylene glycol, and stirring the mixture, and then acidifying to produce the carboxylic acids of formulas (24a) 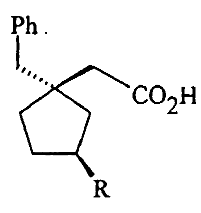 and (24b) 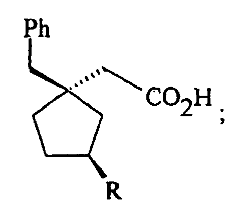 or adding the products of Step b) above to an acid mixture selected from 6-12 M HCl, 12 M H₂SO₄,10%-48% wt/wt hydrobromic acid, and HBr in aqueous acetic acid, and stirring to produce the carboxylic acids of formulas (24a) 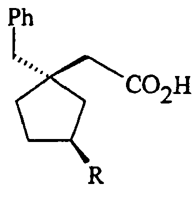 and (24b) 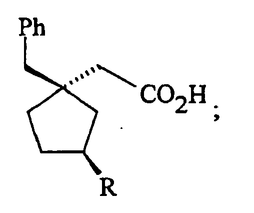
d) contacting the products of Step c) above with an amine selected from (S)-α-methyl-benzylamine, (R)-α-methyl-benzylamine, (R)-(+)-1-(naphthyl)ethylamine, (S)-(+)-1-(naphthyl)ethylamine, triethylamine, diisopropylethylamine, dicyclohexylamine, benzylamine, dibenzylamine, morpholine, N-methylmorpholine, piperidine, N-methylpiperidine, and pyridine in a solvent selected from N,N-dimethylformamide, chloroform, hexanes, acetone, ethanol, methanol, *iso*-propanol, diethyl ether, dichloromethane, benzene, toluene, n-pentane, n-hexane, n-heptane, ethyl acetate, acetonitrile, *tert*-butyl methyl ether, tetrahydrofuran, and 1,4-dioxane, and recrystallizing the salt so formed to produce the enriched diastereomer of formula (25) 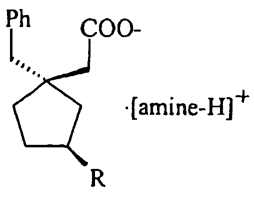 as the amine salt; and
e) adding the product of Step d) to a mixture selected from aqueous hydrochloric acid, aqueous sulfuric acid, aqueous acetic acid, hydrochloric acid dissolved in acetic acid, and hydrochloric acid dissolved in acetic acid and water, and stirring to produce the carboxylic acid of formula (26) 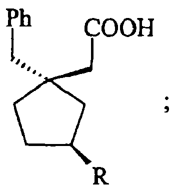 or partitioning the product of Step d) between a mixture of aqueous hydrochloric acid and a solvent selected from chloroform, dichloromethane, ethyl acetate, ethyl ether, tetrahydrofuran, 1,4-dioxane, toluene, and *tert*-butylmethylether, and drying and evaporating the organic layer to produce the carboxylic acid of formula (26)

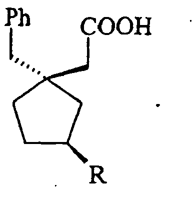

23. A process according to Claim 21 which comprises:
a) adding a cyanoacetate of formula (A) 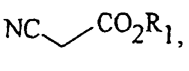 wherein R₁ is ethyl, to a mixture of a chiral cyclopentanone of formula (21) 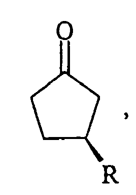 toluene, acetic acid, and a Knoevenagel reaction catalyst which is ammonium acetate, and heating the mixture at reflux over a Dean-Stark trap to produce the alkene of formula (22) 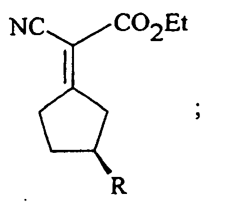
b) adding the product of Step a) above to a mixture of benzylmagnesium chloride in dry tetrahydrofuran at -100°C to -20°C to produce the addition products of formulas (23a) 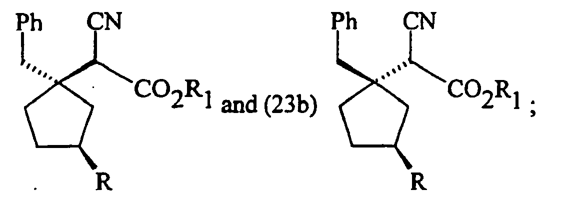
c) adding the products of Step b) above to a mixture of potassium hydroxide in ethylene glycol, and heating the mixture at 100°C to 200°C, and then acidifying to produce the hydrolysis products of formulas (24a) 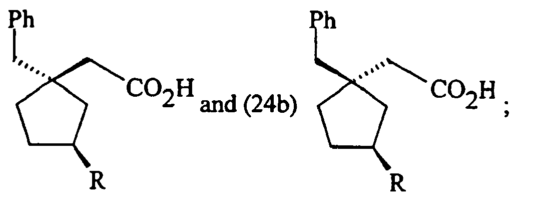
d) contacting the products of Step c) above with (R)-α-methylbenzylamine in ethyl acetate, and recrystallizing the salt so formed from ethyl acetate to produce the enriched diastereomer of formula (25) 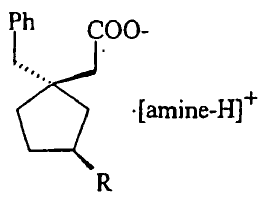 as the (R)-α-methylbenzylamine salt; and
e) adding the product of Step d) to aqueous hydrochloric acid and stirring to produce the carboxylic acid of formula (26)

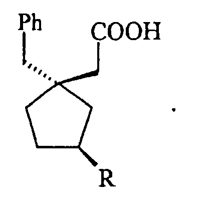

24. A compound of formula (6) 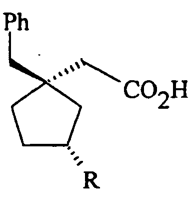 wherein R is C₁-C₁₀ alkyl or C₃-C₁₀ cycloalkyl, and pharmaceutically acceptable salts thereof.

25. A compound according to Claim 24 wherein R is C₁-C₁₀ alkyl.

26. A compound according to Claim 24 wherein R is selected from methyl, ethyl, and *n*-propyl.

27. A compound according to Claim 24 named ((1S,3R)-1-benzyl-3-methylcyclopentyl)-acetic acid.

28. A compound of formula (26) 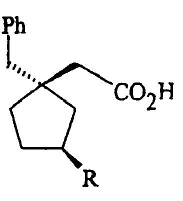 wherein R is C₁-C₁₀ alkyl or C₃-C₁₀ cycloalkyl and pharmaceutically acceptable salts thereof.

29. A compound according to Claim 28 wherein R is C₁-C₁₀ alkyl.

30. A compound according to Claim 28 wherein R is selected from methyl, ethyl, and *n*-propyl.

31. A compound according to Claim 28 named ((1R,3S)-1-benzyl-3-methylcyclopentyl)-acetic acid.

32. A compound of formula (6) 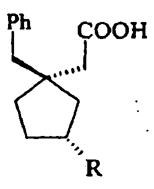 wherein R is C₁-C₁₀ alkyl or C₃-C₁₀ cycloalkyl and pharmaceutically acceptable salts thereof, prepared according to the process of Claim 18.

33. A compound of formula (6) 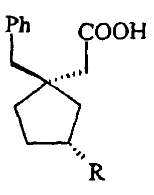 wherein R is C₁-C₁₀ alkyl or C₃-C₁₀ cycloalkyl and pharmaceutically acceptable salts thereof, prepared according to the process of Claim 19.

34. A compound of formula (6) 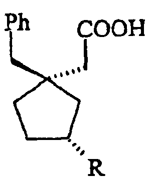 wherein R is C₁-C₁₀ alkyl or C₃-C₁₀ cycloalkyl and pharmaceutically acceptable salts thereof, prepared according to the process of Claim 20.

35. A compound of formula (26) 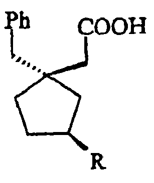 wherein R is C₁-C₁₀ alkyl or C₃-C₁₀ cycloalkyl and pharmaceutically acceptable salts thereof, prepared according to the process of Claim 21.

36. A compound of formula (26) 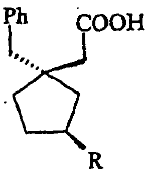 wherein R is C₁-C₁₀ alkyl or C₃-C₁₀ cycloalkyl and pharmaceutically acceptable salts thereof, prepared according to the process of Claim 22.

37. A compound of formula (26) 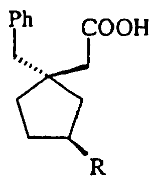 wherein R is C₁-C₁₀ alkyl or C₃-C₁₀ cycloalkyl and pharmaceutically acceptable salts thereof, prepared according to the process of Claim 23.

38. A compound selected from:
E-Cyano-((R)-3-methyl-cyclopentylidene)-acetic acid ethyl ester,
Z-Cyano-((R)-3-methyl-cyclopentylidene)-acetic acid ethyl ester,
(R)-((1S,3R)-1-Benzyl-3-methyl-cyclopentyl)-cyano-acetic acid ethyl ester;
(S)-((1S,3R)-1-Benzyl-3-methyl-cyclopentyl)-cyano-acetic acid ethyl ester;
(R)-((1R,3R)-1-Benzyl-3-methyl-cyclopentyl)-cyano-acetic acid ethyl ester;
(S)-((1R,3R)-1-Benzyl-3-methyl-cyclopentyl)-cyano-acetic acid ethyl ester;
((1S,3R)-1-Isocyanatomethyl-3-methyl-cyclopentylmethyl)-benzene;
((1S,3R)-1-Benzyl-3-methyl-cyclopentylmethyl)-carbamic acid methyl ester;
[(1S,3R)-1-(Methoxycarbonylamino-methyl)-3-methylcyclopentyl]-acetic acid;
((1S,3R)-1-Benzyl-3-methyl-cyclopentyl)-acetic acid methyl ester,
(1S,3R)-1-Methoxycarbonylmethyl-3-methyl-cyclopentyl)-acetic acid;
((1R,3R)-1-Isocyanatomethyl-3-methyl-cyclopentyl)-acetic acid methyl ester,
[(1R,3R)-1-(Methoxycarbonylamino-methyl)-3-methylcyclopentyl]-acetic acid methyl ester;
((1S,3R)-1-Benzyl-3-methyl-cyclopentyl)-acetic acid *tert*-butyl ester;
[(1S,3R)-1-Carboxymethyl-3-methyl-cyclopentyl]-acetic acid *tert-*butyl ester,
[(1S,3R)-1-Methoxycarbonylmethyl-3-methyl-cyclopentyl]-acetic acid *tert*-butyl ester,
((1R,3R)-1-Methoxycarbonylmethyl-3-methyl-cyclopentyl)-acetic acid;
((1S,3R)-1-Isocyanatomethyl-3-methyl-cyclopentyl)-acetic acid methyl ester, and
[(1S,3R)-1-(Methoxycarbonylamino-methyl)-3-methylcyclopentyl]-acetic acid methyl ester.

39. A process for the preparation of a compound of formula (4a) 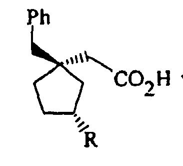 wherein R is C₁-C₁₀ alkyl or C₃-C₁₀ cycloalkyl, comprising, hydrolyzing a compound of formula (3a) 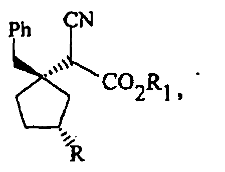 wherein R₁ is H, alkyl, or benzyl.

40. A process for the preparation of a compound of formula (24a) 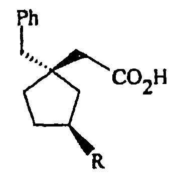 wherein R is C₁-C₁₀ alkyl or C₃-C₁₀ cycloalkyl, comprising, hydrolyzing a compound of formula (23a) 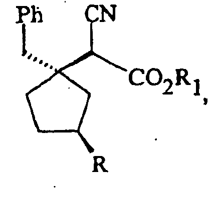 wherein R₁ is H, alkyl, or benzyl.

41. A process for the preparation of a compound of formula (6) 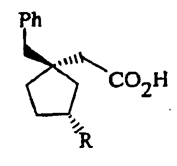 wherein R is C₁-C₁₀ alkyl or C₃-C₁₀ cycloalkyl, comprising, resolving a mixture containing compounds of formulas (4a) 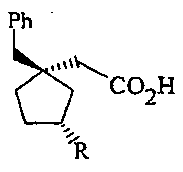 and (4b) 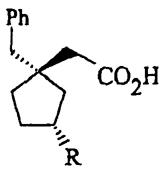 wherein R is C₁-C₁₀ alkyl or C₃-C₁₀ cycloalkyl.

42. A process for the preparation of a compound of formula (26) 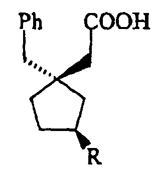 wherein R is C₁-C₁₀ alkyl or C₃-C₁₀cycloalkyl, comprising, resolving a mixture containing compounds of formulas (24a) 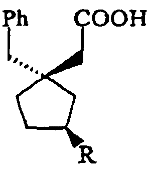 and (24b) 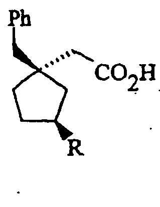 wherein R is C₁-C₁₀ alkyl or C₃-C₁₀ cycloalkyl.

43. A process for the preparation of a compound of Formula I 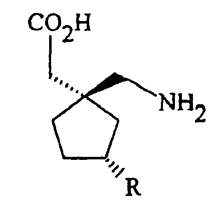 wherein R is C₁-C₁₀ alkyl or C₃-C₁₀ cycloalkyl, and a pharmaceutically acceptable salts thereof, comprising, hydrolyzing a compound of formula (41) 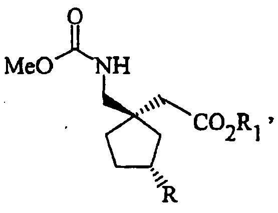 wherein R₁ is H,-alkyl, or benzyl, and contacting the product, if desired, with an acid or a base.

44. A process for the preparation of a compound of Formula II 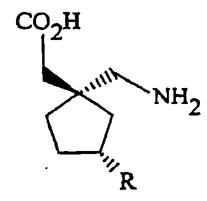 wherein R is C₁-C₁₀ alkyl or C₃-C₁₀ cycloalkyl, and a pharmaceutically acceptable salts thereof, comprising, hydrolyzing a compound of formula (42) 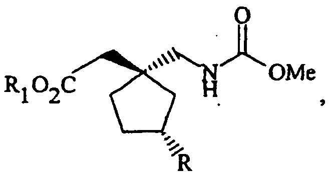 wherein R₁ is H, alkyl, or benzyl, and contacting the product, if desired, with an acid or a base.

45. A process for the preparation of a compound of Formula III 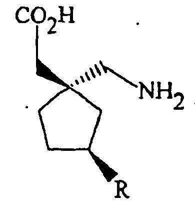 wherein R is C₁-C₁₀ alkyl or C₃-C₁₀ cycloalkyl, and pharmaceutically acceptable salts thereof, comprising, hydrolyzing a compound of formula (43) 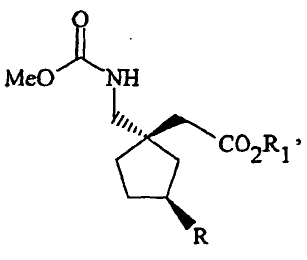 wherein R₁ is H, alkyl, or benzyl, and contacting the product, if desired, with an acid or a base.

46. A process for the preparation of a compound of Formula IV 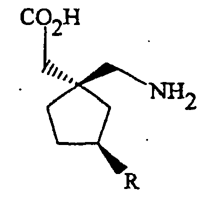 wherein R is C₁-C₁₀ alkyl or C₃-C₁₀ cycloalkyl, and pharmaceutically acceptable salts thereof, comprising, hydrolyzing a compound of formula (44) 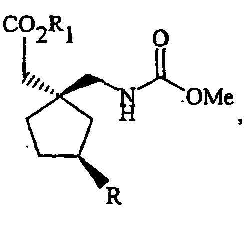 wherein R₁ is H, alkyl, or benzyl, and contacting the product, if desired, with an acid or a base.

## Patentansprüche

1. Verfahren zur Herstellung einer Verbindung der Formel I 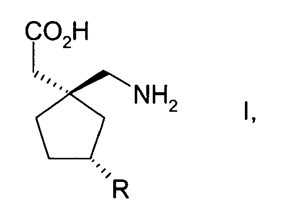 worin R für C₁-C₁₀-Alkyl oder C₃-C₁₀-Cycloalkyl steht, und derer pharmazeutisch akzeptablen Salze, umfassend
a) Zugabe von Cyanoacetat der Formel (A) 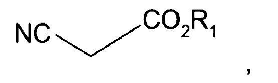 worin R₁ für Alkyl oder Benzyl steht, zu einem Gemisch eines chiralen Cyclopentanons der Formel (1) 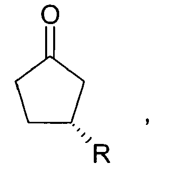 eines Lösungsmittels, einer Carbonsäure und eines Katalysators für die Knoevenagel-Reaktion, und Rühren des Gemischs in Gegenwart eines Mittels zum Entziehen von Wasser, unter Erhalt des Alkens der Formel (2) 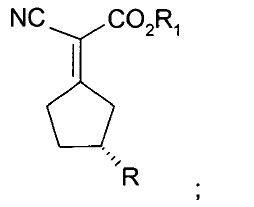
b) Zugabe des Produkts von Stufe a) zu einem Gemisch von Benzylmagnesiumchlorid, Benzylmagnesiumbromid oder Benzylmagnesiumiodid in einem Lösungsmittel unter Erhalt der Additionsprodukte der Formeln 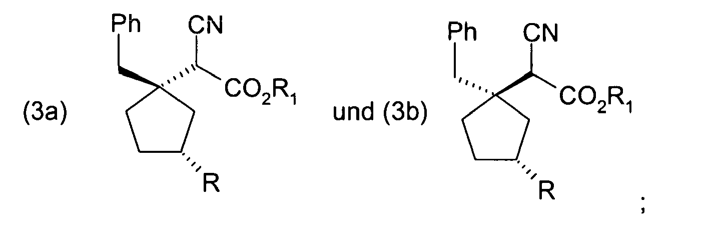
c) Zugabe der Produkte von Stufe b) zu einem Gemisch einer aus Kaliumhydroxid, Natriumhydroxid, Lithiumhydroxid oder Cäsiumhydroxid ausgewählten Base in einem Lösungsmittel, Rühren sowie anschließendes Ansäuern unter Erhalt der Carbonsäuren der Formeln 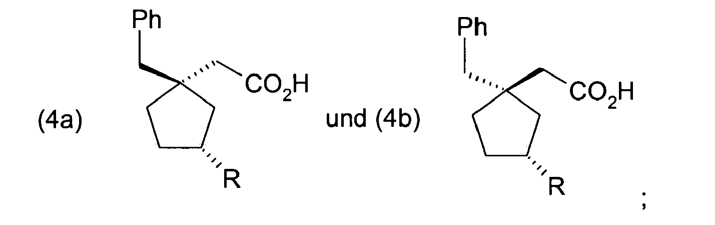 oder Zugabe der Produkte der Stufe b) zu einem Säuregemisch und Rühren unter Erhalt der Carbonsäuren der Formeln 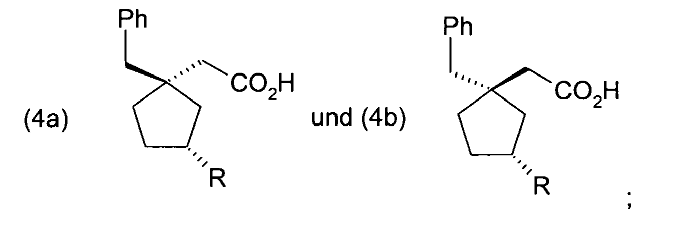
d) Inkontaktbringen der Produkte von Stufe c) mit einem Amin in einem Lösungsmittel und Umkristallisation des so gebildeten Salzes unter Erhalt des angereicherten Diastereomers der Formel (5) 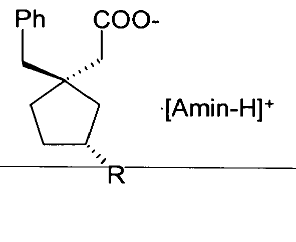 als Aminsalz;
e) Überführen des Produkts von Stufe d) in eine Carbonsäure der Formel (6) 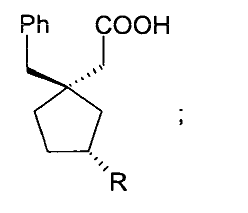
f) Zugabe des Produkts von Stufe e) zu einem Gemisch von Methyliodid, eines Lösungsmittels und einer Base und Rühren unter Erhalt des Esters der Formel (7) 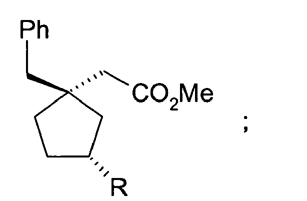 oder Zugabe des Produkts von Stufe e) zu Methanol und einer Säure unter Erhalt des Esters der Formel (7) 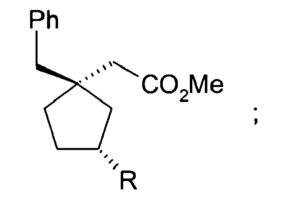 oder Zugabe des Produkts von Stufe e) zu Trimethylsilyldiazomethan und Methanol in einem Lösungsmittel unter Erhalt des Esters der Formel (7) 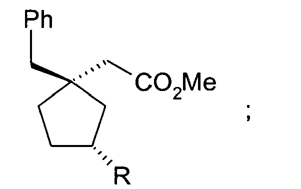 oder Zugabe des Produkts von Stufe e) zu einer Lösung von Diazomethan oder Trimethylsilyl-diazomethan in einem Lösungsmittel unter Erhalt des Esters der Formel (7) 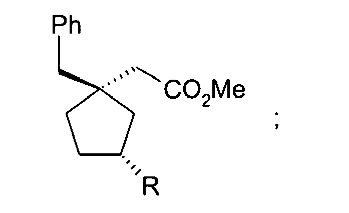
g) Zugabe des Produkts von Stufe f) zu einem Gemisch von Kohlenstofftetrachlorid oder Ethylacetat, Acetonitril, Wasser, Natriumperiodat und Ruthenium(III)chlorid und Rühren unter Erhalt der Carbonsäure der Formel (8) 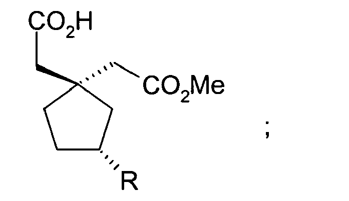
h) Zugabe des Produkts von Stufe g) zu einem Gemisch einer tertiären Aminbase, eines Lösungsmittels und Diphenylphosphorylazid (DPPA) und Rühren unter Erhalt des Isocyanats der Formel (9) 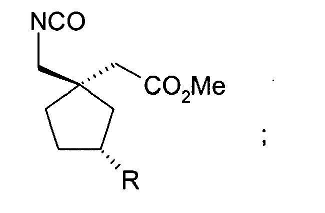 oder Zugabe des Produkts von Stufe g) zu Ethylchlorformiat oder Isobutylchlorformiat und einer Base in einem Lösungsmittel bei einer Temperatur von -40°C bis 78°C, anschließende Zugabe eine Lösung von Natriumazid in Wasser und Tetrahydrofuran oder Aceton, schließlich Zugabe von Toluol oder Benzol und Erhitzen unter Rückfluss unter Erhalt des Isocyanats der Formel (9) 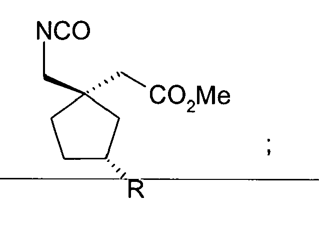
i) Zugabe des Produkts von Stufe h) zu einem Gemisch eines Lösungsmittels und Methanols und Rühren unter Erhalt des Carbamats der Formel (10) 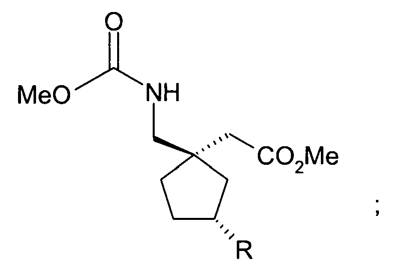
j) Zugabe des Produkts von Stufe i) zu einem Gemisch eines Lösungsmittels und wässriger Salzsäure und Rühren unter Erhalt einer Verbindung der Formel (la) 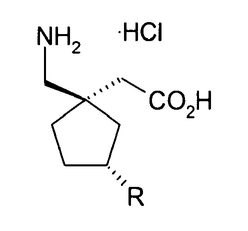 und
k) Überführen des Produkts von Stufe j) in eine Verbindung der Formel (I) 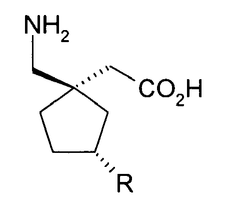 und gegebenenfalls weiteres Überführen in ein pharmazeutisch akzeptables Salz auf bekannte Weise.

2. Verfahren nach Anspruch 1, umfassend
a) Zugabe von Cyanoacetat der Formel (A) 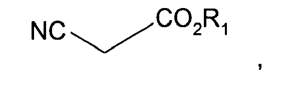 worin R₁ ausgewählt ist aus Methyl, Ethyl, *n*-Propyl, *iso*-Propyl, *n*-Butyl, *iso*-Butyl, *sek*.-Butyl, *tert*.-Butyl und Benzyl, zu einem Gemisch eines chiralen Cyclopentanons der Formel (1) 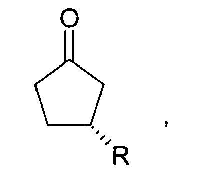 eines Lösungsmittels, das aus Tetrahydrofuran, 1,4-Dioxan, *tert*.-Butylmethylether, Chloroform, Dichlormethan, Acetonitril, Ethylether, Ethylacetat, Hexanen, N,N-Dimethylformamid, Dimethylsulfoxid, Ethanol, *tert*.-Butanol, Toluol, Benzol, Xylolen und *n*-Heptan ausgewählt ist, Essigsäure und eines Katalysators für die Knoevenagel-Reaktion, der aus β-Alanin, Ammoniumacetat und Piperidin ausgewählt ist, und Rühren des Gemischs in Gegenwart eines Mittels zum Entziehen von Wasser, das aus azeotroper Destillation, aktivierten Molekularsieben, wasserfreiem Magnesiumsulfat, wasserfreiem Natriumsulfat, wasserfreiem Natriumcarbonat, wasserfreiem Kaliumcarbonat, wasserfreiem Cäsiumcarbonat, Trimethylorthoformiat und Triethylorthoformiat ausgewählt ist, unter Erhalt des Alkens der Formel (2) 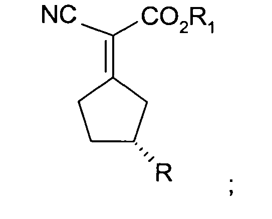
b) Zugabe des Produkts von Stufe a) zu einem Gemisch von Benzylmagnesiumchlorid, Benzylmagnesiumbromid oder Benzylmagnesiumiodid in einem Lösungsmittel, das aus Tetrahydrofuran, Benzol, 1,4-Dioxan, Hexanen, *n*-Heptan, Toluol, Diethylether und *tert*.-Butylmethylether ausgewählt ist, unter Erhalt der Additionsprodukte der Formeln 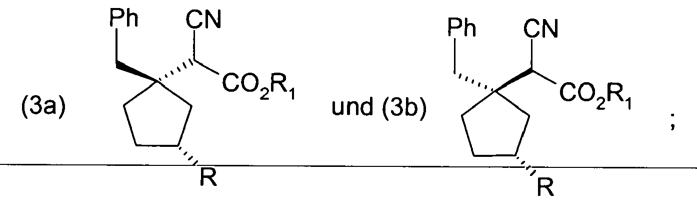
c) Zugabe der Produkte von Stufe b) zu einem Gemisch einer aus Kaliumhydroxid, Natriumhydroxid, Lithiumhydroxid und Cäsiumhydroxid ausgewählten Base in einem Lösungsmittel, das aus Ethylenglycol, 2-Methoxyethylether, 1,4-Dioxan und Diethylenglycol ausgewählt ist, Rühren des Gemischs sowie anschließendes Ansäuern unter Erhalt der Carbonsäuren der Formeln 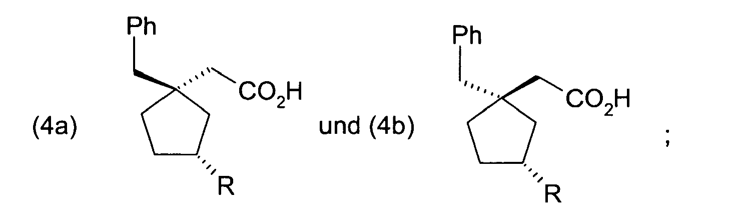 oder Zugabe der Produkte der Stufe b) zu einem Säuregemisch, das aus 6-12 M HCl, 12 M H₂SO₄, 10 - 48 Gew.% Bromwasserstoffsäure und HBr in wässriger Essigsäure ausgewählt ist, und Rühren unter Erhalt der Carbonsäuren der Formeln 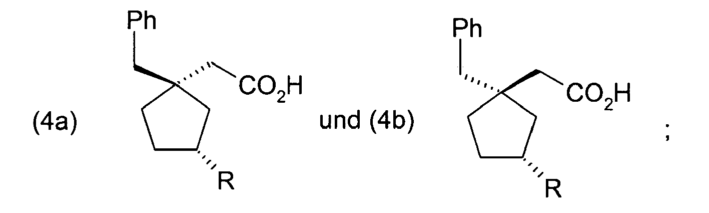
d) Inkontaktbringen der Produkte von Stufe c) mit einem Amin, das aus (S)-α-Methylbenzylamin, (R)-α-Methylbenzylamin, (R)-(+)-1-(Naphthyl)ethylamin, (S)-(+)-1-(Naphthyl)ethylamin, Triethylamin, Diisopropylethylamin, Dicyclohexylamin, Benzylamin, Dibenzylamin, Morpholin, N-Methylmorpholin, Piperidin, N-Methylpiperidin und Pyridin ausgewählt ist, in einem Lösungsmittel, das aus N,N-Dimethylformamid, Chloroform, Benzol, Xylolen, Hexanen, Aceton, Ethanol, Methanol, *iso*-Propanol, Diethylether, Dichlormethan, Benzol, Toluol, *n*-Pentan, *n*-Hexan, *n*-Heptan, Ethylacetat, Acetonitril, *tert.*-Butylmethylether, Tetrahydrofuran und 1,4-Dioxan ausgewählt ist, und Umkristallisation des so gebildeten Salzes unter Erhalt des angereicherten Diastereomers der Formel (5) 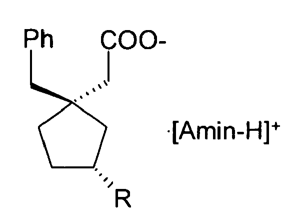 als Aminsalz;
e) Zugabe des Produkts von Stufe d) zu einem Gemisch, das aus wässriger Salzsäure, wässriger Schwefelsäure, wässriger Essigsäure, in Essigsäure gelöster Salzsäure oder in Essigsäure gelöster Salzsäure, dem Wasser zugesetzt wird, ausgewählt ist, und Rühren unter Erhalt der Carbonsäure der Formel (6) 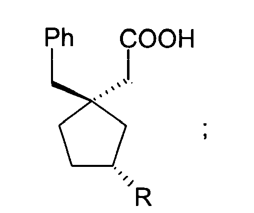 oder Verteilen des Produkts von Stufe d) zwischen einem Gemisch von wässriger Salzsäure und einem Lösungsmittel, das aus Chloroform, Dichlormethan, Ethylacetat, Ethylether, Tetrahydrofuran, 1,4-Dioxan, Toluol und *tert*.-Butylmethylether ausgewählt ist, sowie Trocknen und Eindampfen der organischen Schicht unter Erhalt der Carbonsäure der Formel (6) 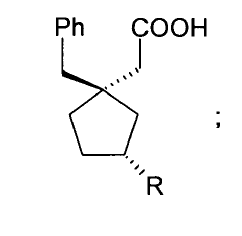
f) Zugabe des Produkts von Stufe e) zu einem Gemisch von Methyliodid, eines Lösungsmittels, das aus Dichlormethan, Chloroform, Tetrahydrofuran, Toluol und 1,4-Dioxan ausgewählt ist, und einer Base, die aus 1,8-Diazabicyclo[5.4.0]undec-7-en (DBU), Diisopropylethylamin, Triethylamin und 1,5-Diazabicyclo[4.3.0]non-5-en (DBN) ausgewählt ist, und Rühren bei einer Temperatur von -40°C bis 110°C unter Erhalt des Esters der Formel (7) 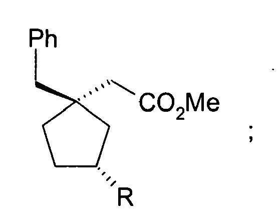 oder Zugabe des Produkts von Stufe e) zu einem Gemisch von Methanol und konzentrierter Schwefelsäure, konzentrierter Salzsäure oder Chlorwasserstoff bei einer Temperatur von 0°C bis 100°C unter Erhalt des Esters der Formel (7) 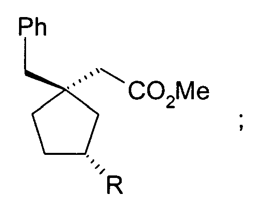 oder Zugabe des Produkts von Stufe e) zu Trimethylsilyldiazomethan und Methanol in Benzol oder Toluol bei einer Temperatur von -40°C bis 100°C unter Erhalt des Esters der Formel (7) 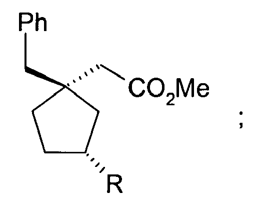 oder Zugabe des Produkts von Stufe e) zu Diazomethan oder Trimethylsilyldiazomethan in einem Lösungsmittel, das aus Benzol, Toluol, Dichlormethan und Diethylether ausgewählt ist, bei einer Temperatur von -40°C bis 40°C unter Erhalt einer Verbindung der Formel (7) 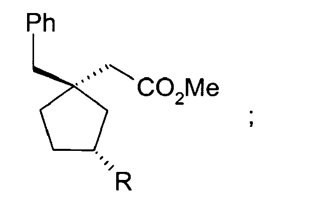
g) Zugabe des Produkts von Stufe f) zu einem Gemisch von Kohlenstofftetrachlorid oder Ethylacetat, Acetonitril, Wasser, Natriumperiodat und Ruthenium(III)chlorid und Rühren bei einer Temperatur von -40°C bis 80°C unter Erhalt der Carbonsäure der Formel (8) 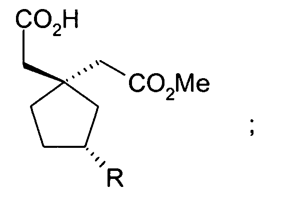
h) Zugabe des Produkts von Stufe g) zu einem Gemisch einer Base, die aus Triethylamin und Diisopropylethylamin ausgewählt ist, eines Lösungsmittels, das aus Toluol, Benzol, Xylolen, Tetrahydrofuran, Diethylether und n-Heptan ausgewählt ist, und Diphenylphosphorylazid (DPPA) und Rühren bei einer Temperatur von 0°C bis 150°C unter Erhalt des Isocyanats der Formel (9) 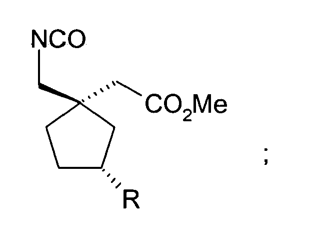 oder Zugabe des Produkts von Stufe g) zu Ethylchlorformiat oder Isobutylchlorformiat und einer Base, die aus Triethylamin und Diisopropylethylamin ausgewählt ist, und einem Lösungsmittel, das aus Tetrahydrofuran, Aceton und Diethylether ausgewählt ist, bei einer Temperatur von -40°C bis 78°C, anschließende Zugabe einer Lösung von Natriumazid in Wasser und Tetrahydrofuran oder Aceton, schließlich Zugabe von Toluol oder Benzol und Erhitzen unter Rückfluss unter Erhalt des Isocyanats der Formel (9) 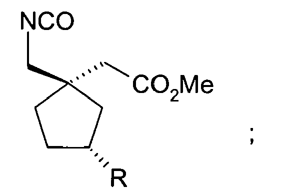
i) Zugabe des Produkts von Stufe h) zu einem Gemisch aus einem Lösungsmittel, das aus Toluol, Benzol, Xylolen und *n*-Heptan ausgewählt ist, und Methanol und Rühren bei einer Temperatur von 0°C bis 150°C unter Erhalt des Carbamats der Formel (10) 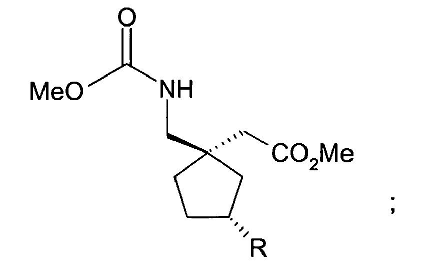
j) Zugabe des Produkts von Stufe i) zu einem Gemisch eines Lösungsmittels, das aus Wasser, Essigsäure und 1,4-Dioxan ausgewählt ist, und wässriger Salzsäure in einer Konzentration von 0,01 M bis 12 M und Rühren bei einer Temperatur von 0°C bis 115°C unter Erhalt einer Verbindung der Formel (Ia) 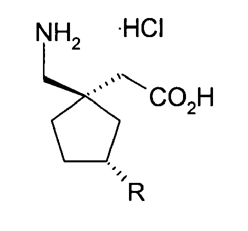 und
k) Überführen des Produkts von Stufe j) in eine Verbindung der Formel (I) 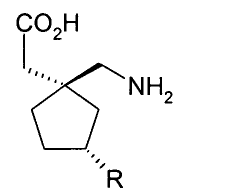 und gegebenenfalls weiteres Überführen in ein pharmazeutisch akzeptables Salz auf bekannte Weise.

3. Verfahren nach Anspruch 1, umfassend
a) Zugabe von Cyanoacetat der Formel (A) 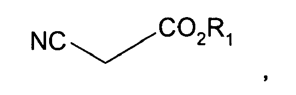 worin R₁ für Ethyl steht, zu einem Gemisch eines chiralen Cyclopentanons der Formel (1) 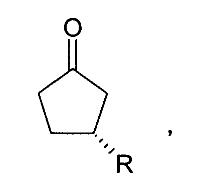 Toluol, Essigsäure und eines Katalysators für die Knoevenagel-Reaktion, der Ammoniumacetat darstellt, und Erhitzen des Gemischs unter Rückfluss an einem Dean-Stark-Abscheider unter Erhalt des Alkens der Formel (2) 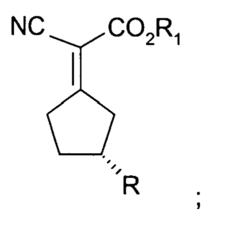
b) Zugabe des Produkts von Stufe a) zu einem Gemisch von Benzylmagnesiumchlorid in trockenem Tetrahydrofuran bei -100°C bis 25° unter Erhalt der Additionsprodukte der Formeln 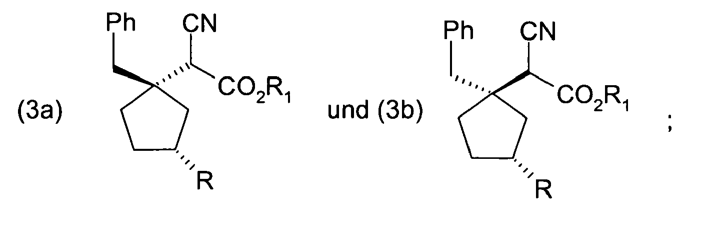
c) Zugabe der Produkte von Stufe b) zu einem Gemisch von Kaliumhydroxid in Ethylenglycol, Erhitzen des Gemischs bei 100°C bis 200°C sowie anschließendes Ansäuern unter Erhalt der Hydrolyseprodukte der Formeln 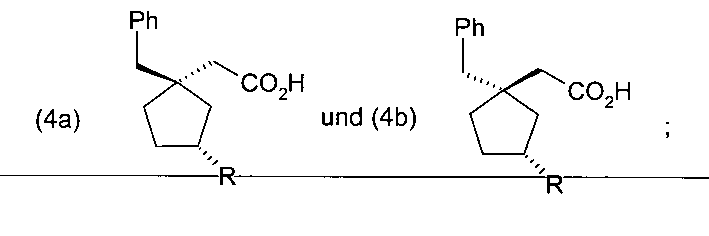
d) Inkontaktbringen der Produkte von Stufe c) mit (S)-α-Methylbenzylamin in Ethylacetat und Umkristallisation des so gebildeten Salzes unter Erhalt des angereicherten Diastereomers der Formel (5) 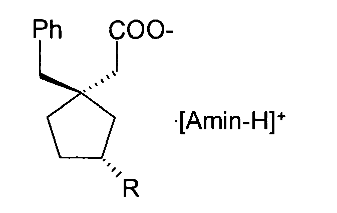 als (S)-α-Methylbenzylaminsalz;
e) Zugabe des Produkts von Stufe d) zu wässriger Salzsäure und Rühren unter Erhalt der Carbonsäure der Formel (6) 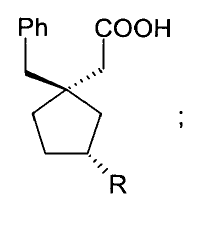
f) Zugabe des Produkts von Stufe e) zu einem Gemisch von Methyliodid, Dichlormethan und 1,8-Diazabicyclo[5.4.0]undec-7-en (DBU) und Rühren unter Erhalt des Esters der Formel (7) 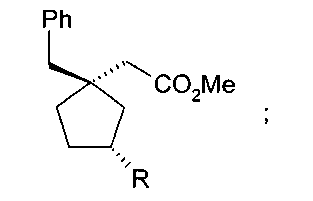 oder Zugabe des Produkts von Stufe e) zu Methanol und konzentrierter Schwefelsäure unter Erhalt des Esters der Formel (7) 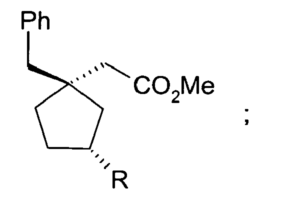 oder Zugabe des Produkts von Stufe e) zu einer Lösung von Diazomethan oder Trimethylsilyldiazomethan in Dichlormethan unter Erhalt des Esters der Formel (7) 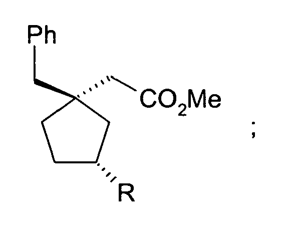
g) Zugabe des Produkts von Stufe f) zu einem Gemisch von Kohlenstofftetrachlorid oder Ethylacetat, Acetonitril, Wasser, Natriumperiodat und Ruthenium(III)chlorid und Rühren unter Erhalt der Carbonsäure der Formel (8) 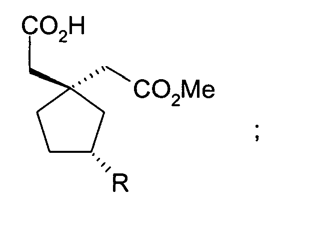
h) Zugabe des Produkts von Stufe g) zu einem Gemisch von Triethylamin, Toluol und Diphenylphosphorylazid (DPPA) und Erhitzen unter Rückfluss unter Erhalt des Isocyanats der Formel (9) 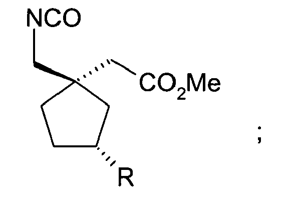 oder Zugabe des Produkts von Stufe g) zu Ethylchlorformiat oder Isobutylchlorformiat und Triethylamin in Tetrahydrofuran bei einer Temperatur von -40°C bis 78°C, anschließende Zugabe einer Lösung von Natriumazid in Wasser und Tetrahydrofuran, schließlich Zugabe von Toluol oder Benzol und Erhitzen unter Rückfluss unter Erhalt des Esters der Formel (9) 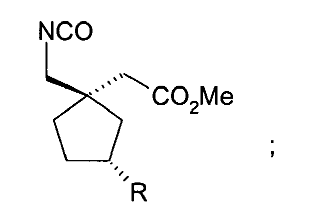
i) Zugabe des Produkts von Stufe h) zu einem Gemisch von Methanol und Toluol und Erhitzen unter Rückfluss unter Erhalt des Carbamats der Formel (10) 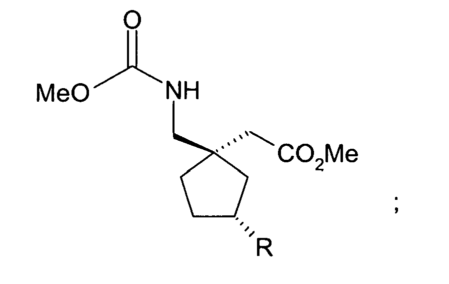
j) Zugabe des Produkts von Stufe i) zu einem Gemisch von 1,4-Dioxan und wässriger Salzsäure in einer Konzentration von 6 M und Rühren unter Erhalt einer Verbindung der Formel (Ia) 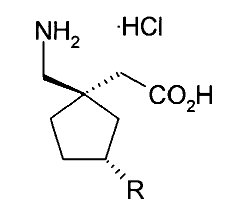 und
k) Überführen des Produkts von Stufe j) in eine Verbindung der Formel (I) 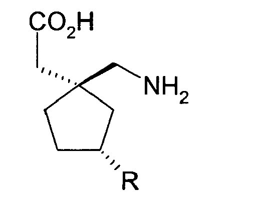 und gegebenenfalls weiteres Überführen in ein pharmazeutisch akzeptables Salz auf bekannte Weise.

4. Verfahren nach Anspruch 1, weiter **dadurch gekennzeichnet, dass** das erhaltene Zwischenprodukt (9) 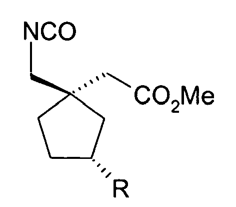 ohne Isolierung mit Methanol umgesetzt wird unter Erhalt des Carbamats der Formel (10)

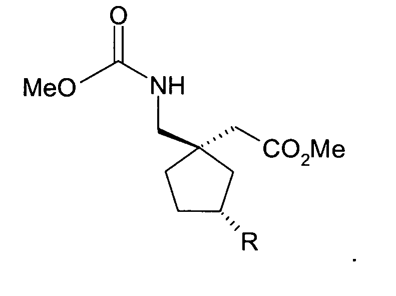

5. Verfahren zur Herstellung einer Verbindung der Formel II 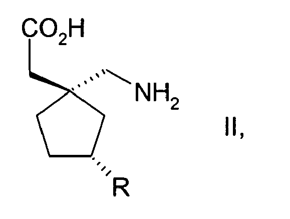 worin R für C₁-C₁₀-Alkyl oder C₃-C₁₀-Cycloalkyl steht, und derer pharmazeutisch akzeptablen Salze, umfassend
a) Zugabe von Cyanoacetat der Formel (A) 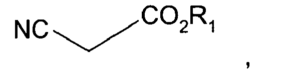 worin R₁ für Alkyl oder Benzyl steht, zu einem Gemisch eines chiralen Cyclopentanons der Formel (1) 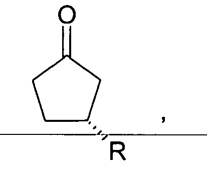 eines Lösungsmittels, einer Carbonsäure und eines Katalysators für die Knoevenagel-Reaktion, und Rühren des Gemischs in Gegenwart eines Mittels zum Entziehen von Wasser, unter Erhalt des Alkens der Formel (2) 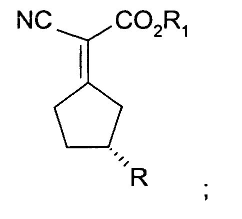
b) Zugabe des Produkts von Stufe a) zu einem Gemisch von Benzylmagnesiumchlorid, Benzylmagnesiumbromid oder Benzylmagnesiumiodid in einem Lösungsmittel unter Erhalt der Additionsprodukte der Formeln 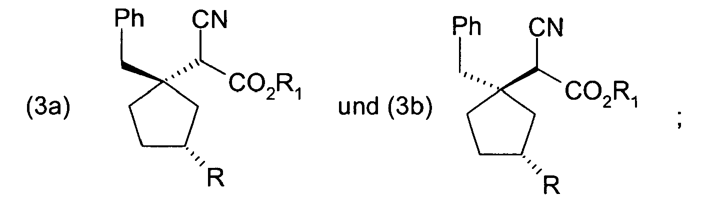
c) Zugabe der Produkte von Stufe b) zu einem Gemisch einer aus Kaliumhydroxid, Natriumhydroxid, Lithiumhydroxid und Cäsiumhydroxid ausgewählten Base und eines Lösungsmittels, Rühren sowie anschließendes Ansäuern unter Erhalt der Carbonsäuren der Formeln 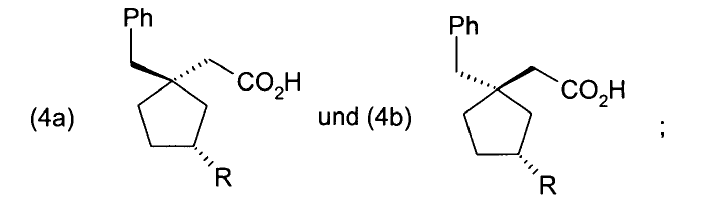 oder Zugabe der Produkte der Stufe b) zu einem Säuregemisch und Rühren unter Erhalt der Carbonsäuren der Formeln 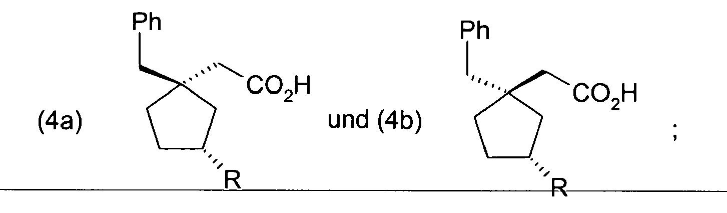
d) Inkontaktbringen der Produkte von Stufe c) mit einem Amin in einem Lösungsmittel und Umkristallisation des so gebildeten Salzes unter Erhalt des angereicherten Diastereomers der Formel (5) 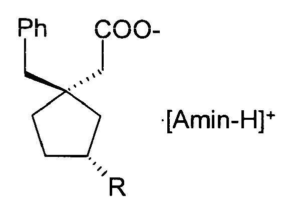 als Aminsalz;
e) Überführen des Produkts von Stufe d) in eine Carbonsäure der Formel (6) 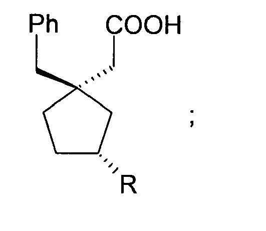
f) Zugabe des Produkts von Stufe e) zu einem Gemisch einer tertiären Aminbase, eines Lösungsmittels und Diphenylphosphorylazid (DPPA) und Rühren unter Erhalt des Isocyanats der Formel (11) 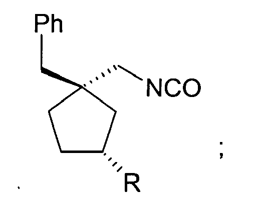 oder Zugabe des Produkts von Stufe e) zu Ethylchlorformiat oder Isobutylchlorformiat und einer Base in einem Lösungsmittel bei einer Temperatur von -40°C bis 78°C, Zugabe einer Lösung von Natriumazid in Wasser und Tetrahydrofuran oder Aceton und anschließende Zugabe von Toluol oder Benzol sowie Erhitzen unter Rückfluss unter Erhalt des Isocyanats der Formel (11) 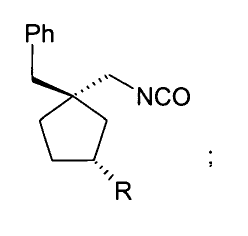
g) Zugabe des Produkts von Stufe f) zu einem Gemisch eines Lösungsmittels und Methanols und Rühren unter Erhalt des Carbamats der Formel (12) 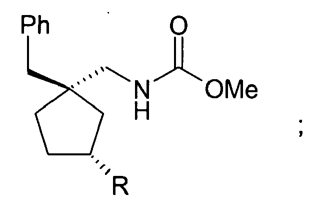
h) Zugabe des Produkts von Stufe g) zu einem Gemisch von Tetrachlormethan oder Ethylacetat, Acetonitril, Wasser, Natriumperiodat und Ruthenium(III)chlorid und Rühren unter Erhalt der Carbonsäure der Formel (13) 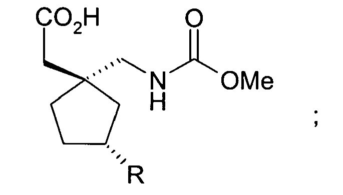
i) Zugabe des Produkts von Stufe h) zu einem Gemisch eines Lösungsmittels und wässriger Salzsäure und Rühren unter Erhalt einer Verbindung der Formel (IIa) 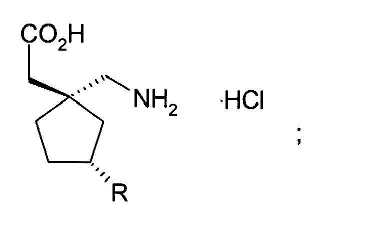
j) Überführen des Produkts von Stufe i) in eine Verbindung der Formel (II) 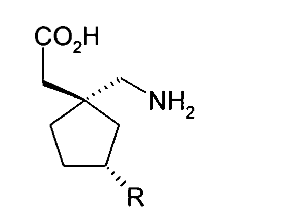 und gegebenenfalls weiteres Überführen in ein pharmazeutisch akzeptables Salz auf bekannte Weise.

6. Verfahren nach Anspruch 5, umfassend
a) Zugabe von Cyanoacetat der Formel (A) 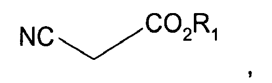 worin R₁ ausgewählt ist aus Methyl, Ethyl, *n*-Propyl, *iso*-Propyl, *n*-Butyl, *iso*-Butyl, *sek*.-Butyl, *tert*.-Butyl und Benzyl, zu einem Gemisch eines chiralen Cyclopentanons der Formel (1) 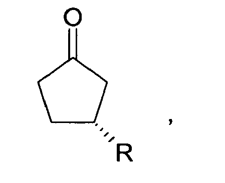 eines Lösungsmittels, das aus Tetrahydrofuran, 1,4-Dioxan, *tert*.-Butylmethylether, Chloroform, Dichlormethan, Acetonitril, Ethylether, Ethylacetat, Hexanen, N,N-Dimethylformamid, Dimethylsulfoxid, Ethanol, *tert*.-Butanol, Toluol, Benzol, Xylolen und *n*-Heptan ausgewählt ist, Essigsäure und eines Katalysators für die Knoevenagel-Reaktion, der aus β-Alanin, Ammoniumacetat und Piperidin ausgewählt ist, und Rühren des Gemischs in Gegenwart eines Mittels zum Entziehen von Wasser, das aus azeotroper Destillation, aktivierten Molekularsieben, wasserfreiem Magnesiumsulfat, wasserfreiem Natriumsulfat, wasserfreiem Natriumcarbonat, wasserfreiem Kaliumcarbonat, wasserfreiem Cäsiumcarbonat, Trimethylorthoformiat und Triethylorthoformiat ausgewählt ist, unter Erhalt des Alkens der Formel (2) 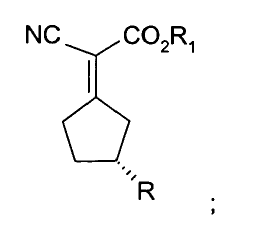
b) Zugabe des Produkts von Stufe a) zu einem Gemisch von Benzylmagnesiumchlorid, Benzylmagnesiumbromid oder Benzylmagnesiumiodid in einem Lösungsmittel, das aus Tetrahydrofuran, Benzol, 1,4-Dioxan, Hexanen, *n*-Heptan, Toluol, Diethylether und t*ert*.-Butylmethylether ausgewählt ist, unter Erhalt der Additionsprodukte der Formeln 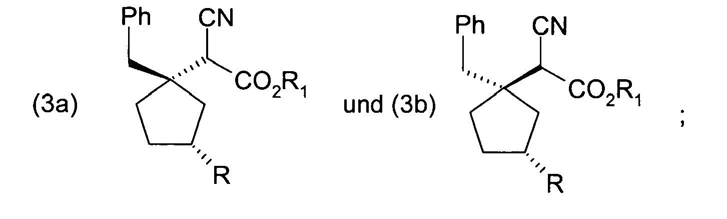
c) Zugabe der Produkte von Stufe b) zu einem Gemisch einer aus Kaliumhydroxid, Natriumhydroxid, Lithiumhydroxid und Cäsiumhydroxid ausgewählten Base in einem Lösungsmittel, das aus Ethylenglycol, 2-Methoxyethylether, 1,4-Dioxan und Diethylenglycol ausgewählt ist, Rühren des Gemischs sowie anschließendes Ansäuern unter Erhalt der Carbonsäuren der Formeln 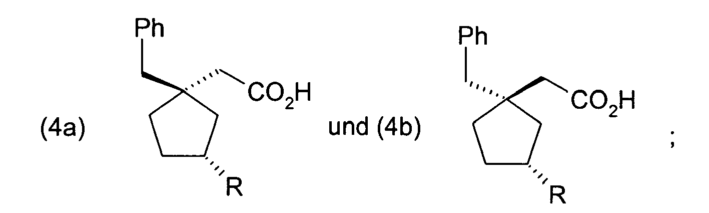 oder Zugabe der Produkte der Stufe b) zu einem Säuregemisch, das aus 6-12 M HCl, 12 M H₂SO₄, 10-48 Gew.% Bromwasserstoffsäure und HBr in wässriger Essigsäure ausgewählt ist, und Rühren unter Erhalt der Carbonsäuren der Formeln 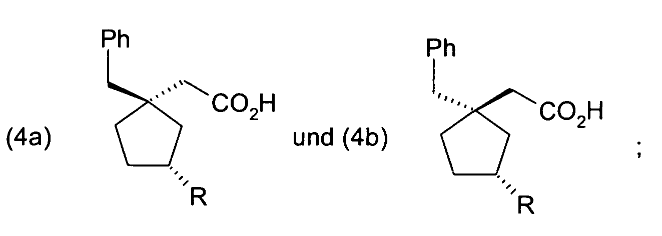
d) Inkontaktbringen der Produkte von Stufe c) mit einem Amin, das aus (S)-α-Methylbenzylamin, (R)-α-Methylbenzylamin, (R)-(+)-1-(Naphthyl)ethylamin, (S)-(+)-1-(Naphthyl)ethylamin, Triethylamin, Diisopropylethylamin, Dicyclohexylamin, Benzylamin, Dibenzylamin, Morpholin, N-Methylmorpholin, Piperidin, N-Methylpiperidin und Pyridin ausgewählt ist, in einem Lösungsmittel, das aus N,N-Dimethylformamid, Chloroform, Benzol, Xylolen, Hexanen, Aceton, Ethanol, Methanol, *iso*-Propanol, Diethylether, Dichlormethan, Benzol, Toluol, *n*-Pentan, *n*-Hexan, *n*-Heptan, Ethylacetat, Acetonitril, *tert*.-Butylmethylether, Tetrahydrofuran und 1,4-Dioxan ausgewählt ist, und Umkristallisation des so gebildeten Salzes unter Erhalt des angereicherten Diastereomers der Formel (5) 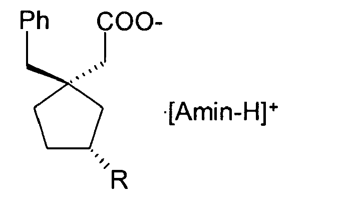 als Aminsalz;
e) Zugabe des Produkts von Stufe d) zu einem Gemisch, das aus wässriger Salzsäure, wässriger Schwefelsäure, wässriger Essigsäure, in Essigsäure gelöster Salzsäure und in Essigsäure und Wasser gelöster Salzsäure ausgewählt ist, und Rühren unter Erhalt der Carbonsäure der Formel (6) 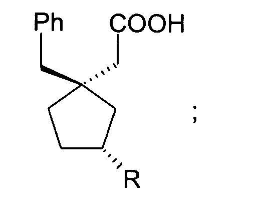 oder Verteilen des Produkts von Stufe d) zwischen einem Gemisch von wässriger Salzsäure und einem Lösungsmittel, das aus Chloroform, Dichlormethan, Ethylacetat, Ethylether, Tetrahydrofuran, 1,4-Dioxan, Toluol und *tert*.-Butylmethylether ausgewählt ist, sowie Trocknen und Eindampfen der organischen Schicht unter Erhalt der Carbonsäure der Formel (6) 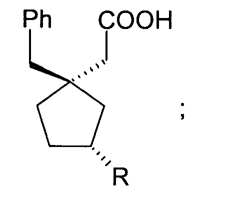
f) Zugabe des Produkts von Stufe e) zu einem Gemisch einer Base, die aus Triethylamin und Diisopropylethylamin ausgewählt ist, eines Lösungsmittels, das aus Toluol, Benzol, Xylolen, Tetrahydrofuran, Diethylether und n-Heptan ausgewählt ist, und Diphenylphosphorylazid (DPPA) und Rühren bei einer Temperatur von 0°C bis 150°C unter Erhalt des Isocyanats der Formel (11) 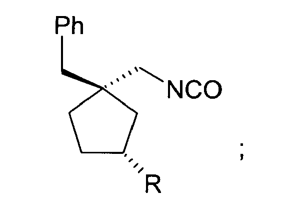 oder Zugabe des Produkts von Stufe e) zu Ethylchlorformiat oder Isobutylchlorformiat und einer Base, die aus Triethylamin und Diisopropylethylamin ausgewählt ist, sowie einem Lösungsmittel, das aus Tetrahydrofuran, Aceton und Diethylether ausgewählt ist, bei einer Temperatur von -40°C bis 78°C, Zugabe einer Lösung von Natriumazid in Wasser und Tetrahydrofuran oder Aceton und anschließende Zugabe von Toluol oder Benzol sowie Erhitzen unter Rückfluss unter Erhalt des Isocyanats der Formel (11) 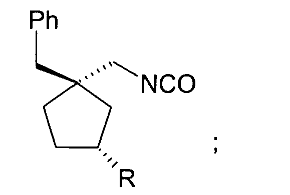
g) Zugabe des Produkts von Stufe f) zu einem Lösungsmittel, das aus Toluol, Benzol, Xylolen und n-Heptan ausgewählt ist, und Methanol und Rühren bei einer Temperatur von 0°C bis 150°C unter Erhalt des Carbamats der Formel (12) 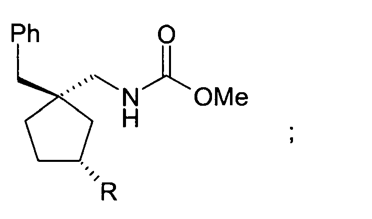
h) Zugabe des Produkts von Stufe g) zu einem Gemisch von Tetrachlormethan oder Ethylacetat, Acetonitril, Wasser, Natriumperiodat und Ruthenium(III)chlorid und Rühren bei einer Temperatur von -40°C bis 80°C unter Erhalt der Carbonsäure der Formel (13) 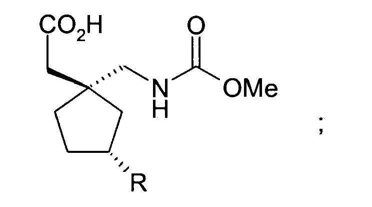
i) Zugabe des Produkts von Stufe h) zu einem Gemisch eines Lösungsmittels, das aus Wasser, Essigsäure und 1,4-Dioxan ausgewählt ist, und wässriger Salzsäure einer Konzentration von 0,01 M bis 12 M und Rühren bei einer Temperatur von 0°C bis 115°C unter Erhalt der Verbindung der Formel (IIa) 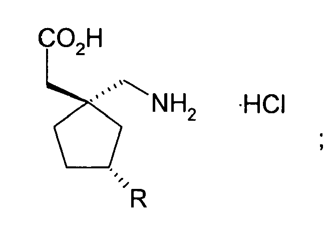 und
j) Überführen des Produkts von Stufe j) in eine Verbindung der Formel (II) 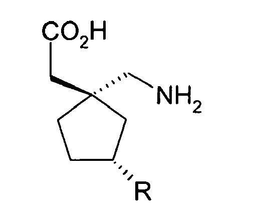 und gegebenenfalls weiteres Überführen in ein pharmazeutisch akzeptables Salz auf bekannte Weise.

7. Verfahren nach Anspruch 5, umfassend
a) Zugabe von Cyanoacetat der Formel (A) 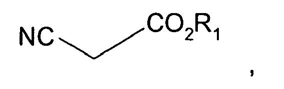 worin R₁ für Ethyl steht, zu einem Gemisch eines chiralen Cyclopentanons der Formel (1) 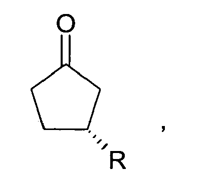 Toluol, Essigsäure und eines Katalysators für die Knoevenagel-Reaktion, der Ammoniumacetat darstellt, und Erhitzen des Gemischs unter Rückfluss an einem Dean-Stark-Abscheider unter Erhalt des Alkens der Formel (2) 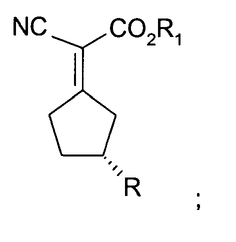
b) Zugabe des Produkts von Stufe a) zu einem Gemisch von Benzylmagnesiumchlorid in trockenem Tetrahydrofuran bei -100°C bis 25°C unter Erhalt der Additionsprodukte der Formeln 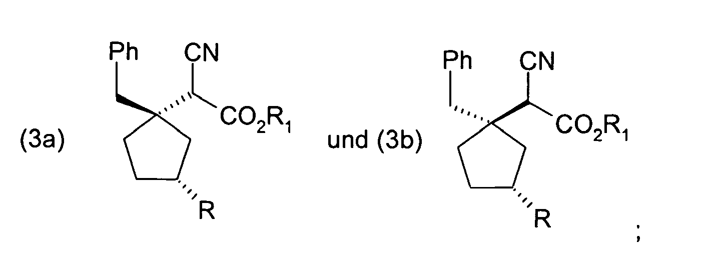
c) Zugabe der Produkte von Stufe b) zu einem Gemisch von Kaliumhydroxid in Ethylenglycol und Erwärmen des Gemischs bei 100°C bis 200°C sowie anschließendes Ansäuern unter Erhalt der Hydrolyseprodukte der Formeln 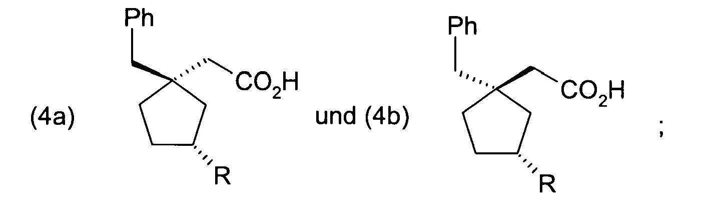
d) Inkontaktbringen der Produkte von Stufe c) mit (S)-α-Methylbenzylamin in Ethylacetat und Umkristallisation des so gebildeten Salzes aus Ethylacetat unter Erhalt des angereicherten Diastereomers der Formel (5) 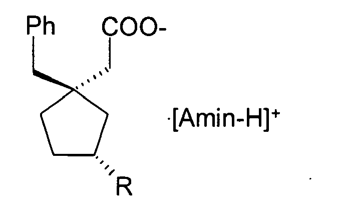 als (S)-α-Methylbenzylaminsalz;
e) Zugabe des Produkts von Stufe d) zu wässriger Salzsäure und Rühren unter Erhalt der Carbonsäure der Formel (6) 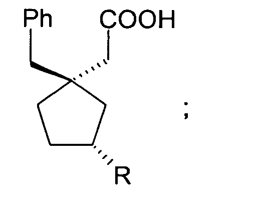
f) Zugabe des Produkts von Stufe e) zu einem Gemisch von Triethylamin, Toluol und Diphenylphosphorylazid (DPPA) und Erhitzen unter Rückfluss unter Erhalt des Isocyanats der Formel (11) 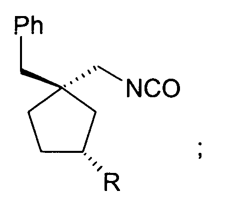 oder Zugabe des Produkts von Stufe e) zu Ethylchlorformiat oder Isobutylchlorformiat und Triethylamin in Tetrahydrofuran bei einer Temperatur von -40°C bis 78°C, Zugabe einer Lösung von Natriumazid in Wasser und Tetrahydrofuran oder Aceton und anschließende Zugabe von Toluol oder Benzol sowie Erhitzen unter Rückfluss unter Erhalt des Isocyanats der Formel (11) 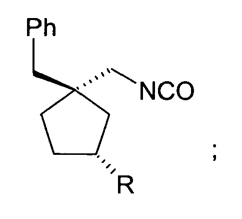
g) Zugabe des Produkts von Stufe f) zu einem Gemisch von Methanol und Toluol und Erhitzen unter Rückfluss unter Erhalt des Carbamats der Formel (12) 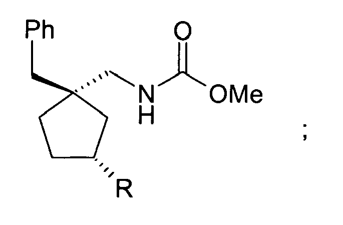
h) Zugabe des Produkts von Stufe g) zu einem Gemisch von Kohlenstofftetrachlorid oder Ethylacetat, Acetonitril, Wasser, Natriumperiodat und Ruthenium(III)chlorid und Rühren unter Erhalt der Carbonsäure der Formel (13) 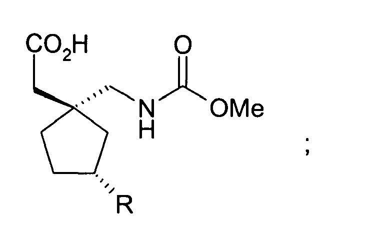
i) Zugabe des Produkts von Stufe h) zu einem Gemisch von 1,4-Dioxan und wässriger Salzsäure einer Konzentration von 6 M und Rühren unter Erhalt der Verbindung der Formel (IIa) 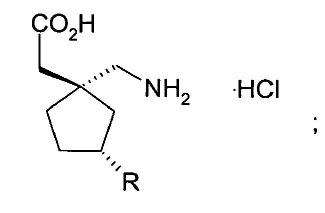 und
j) Überführen des Produkts von Stufe j) in eine Verbindung der Formel (II) 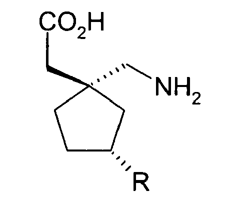 und gegebenenfalls weiteres Überführen in ein pharmazeutisch akzeptables Salz auf bekannte Weise.

8. Verfahren nach Anspruch 5, weiter **dadurch gekennzeichnet, dass** das erhaltene Zwischenprodukt (11) 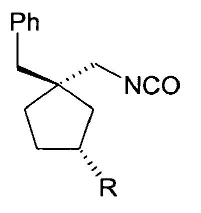 ohne Isolierung mit Methanol umgesetzt wird unter Erhalt des Carbamats der Formel (12)

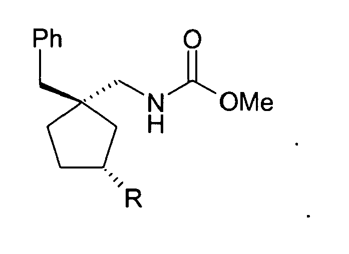

9. Verfahren zur Herstellung einer Verbindung der Formel II 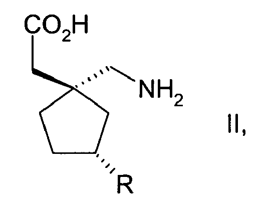 worin R für C₁-C₁₀-Alkyl oder C₃-C₁₀-Cycloalkyl steht, und pharmazeutisch akzeptabler Salze, davon umfassend
a) Zugabe von Cyanoacetat der Formel (A) 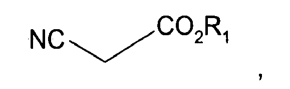 worin R₁ für Alkyl oder Benzyl steht, zu einem Gemisch eines chiralen Cyclopentanons der Formel (1) 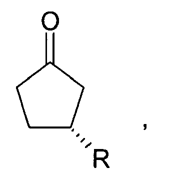 eines Lösungsmittels, einer Carbonsäure und eines Katalysators für die Knoevenagel-Reaktion, und Rühren des Gemischs in Gegenwart eines Mittels zum Entziehen von Wasser, unter Erhalt des Alkens der Formel (2) 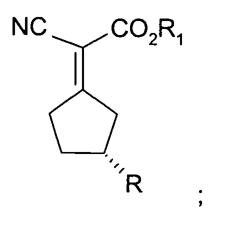
b) Zugabe des Produkts von Stufe a) zu einem Gemisch von Benzylmagnesiumchlorid, Benzylmagnesiumbromid oder Benzylmagnesiumiodid in einem Lösungsmittel unter Erhalt der Additionsprodukte der Formeln 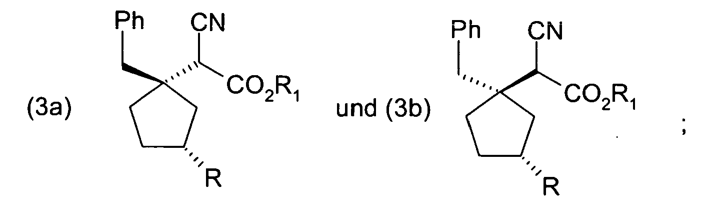
c) Zugabe der Produkte von Stufe b) zu einem Gemisch einer aus Kaliumhydroxid, Natriumhydroxid, Lithiumhydroxid und Cäsiumhydroxid ausgewählten Base und eines Lösungsmittels, Rühren sowie anschließendes Ansäuern unter Erhalt der Carbonsäuren der Formeln 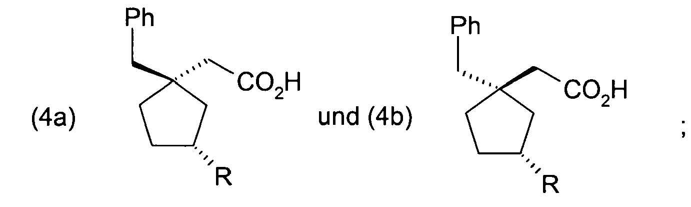 oder Zugabe der Produkte der Stufe b) zu einem Säuregemisch und Rühren unter Erhalt der Carbonsäuren der Formeln 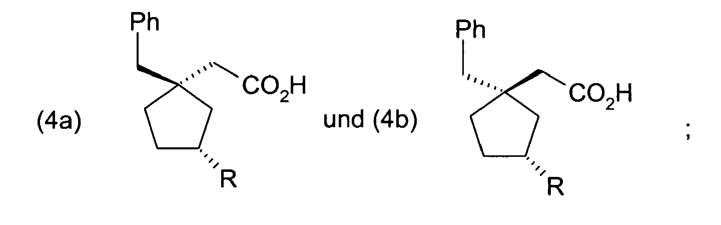
d) Inkontaktbringen der Produkte von Stufe c) mit einem Amin in einem Lösungsmittel und Umkristallisation des so gebildeten Salzes unter Erhalt des angereicherten Diastereomers der Formel (5) 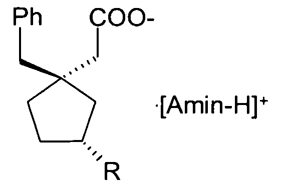 als Aminsalz;
e) Überführen des Produkts von Stufe d) in eine Carbonsäure der Formel (6) 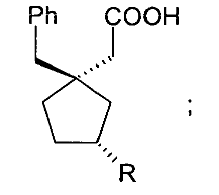
f) Zugabe von Oxalylchlorid zu einem Gemisch des Produkts von Stufe e), eines Lösungsmittels und N,N-Dimethylformamid (DMF) und Rühren unter Erhalt des Säurechlorids der Formel (14) 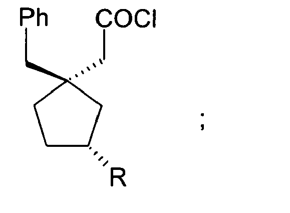
g) Zugabe des Produkts von Stufe f) zu einem Gemisch von *tert*.-Butylalkohol, eines Lösungsmittels und einer tertiären Aminbase und Rühren unter Erhalt des Esters der Formel (15) 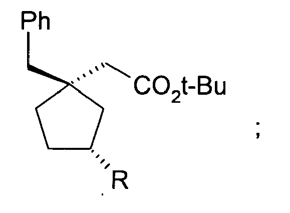
h) Zugabe des Produkts von Stufe g) zu einem Gemisch von Kohlenstofftetrachlorid oder Ethylacetat, Acetonitril, Wasser, Natriumperiodat und Ruthenium(III)chlorid und Rühren unter Erhalt der Carbonsäure der Formel (16) 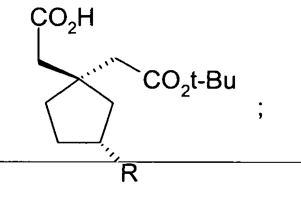
i) Zugabe des Produkts von Stufe h) zu einem Gemisch aus einem Lösungsmittel, Methanol und (Trimethylsilyl)diazomethan und Rühren unter Erhalt des Diesters der Formel (17) 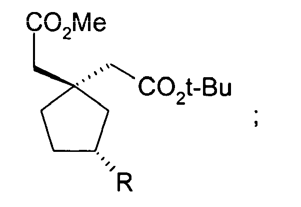 oder Zugabe des Produkts von Stufe h) zu einem Gemisch von Methyliodid, eines Lösungsmittels und einer Base und Rühren unter Erhalt des Diesters der Formel (17) 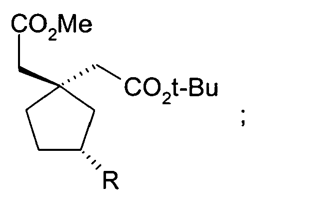
j) Zugabe einer Säure zu einem Gemisch des Produkts von Stufe i) und eines Lösungsmittels und Rühren unter Erhalt der Carbonsäure der Formel (18) 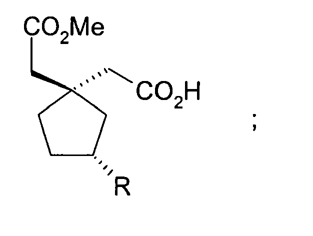
k) Zugabe des Produkts von Stufe j) zu einem Gemisch einer tertiären Aminbase, eines Lösungsmittels und Diphenylphosphorylazid (DPPA) und Rühren unter Erhalt des Isocyanats der Formel (19) 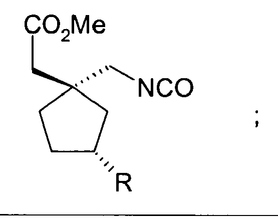 oder Zugabe des Produkts von Stufe j) zu Ethylchlorformiat oder Isobutylchlorformiat und einer Base in einem Lösungsmittel bei einer Temperatur von -40°C bis 78°C, Zugabe einer Lösung von Natriumazid in Wasser und Tetrahydrofuran oder Aceton und anschließende Zugabe von Toluol oder Benzol sowie Erhitzen unter Rückfluss unter Erhalt des Isocyanats der Formel (19) 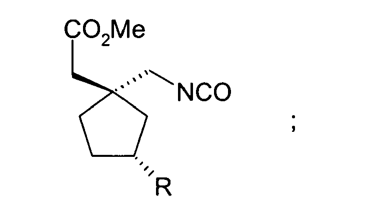
l) Zugabe des Produkts von Stufe k) zu einem Gemisch aus einem Lösungsmittel und Methanol und Rühren unter Erhalt des Carbamats der Formel (20) 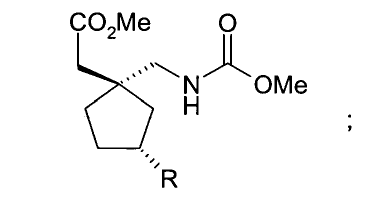
m) Zugabe des Produkts von Stufe I) zu einem Gemisch eines Lösungsmittels und wässriger Salzsäure und Rühren unter Erhalt einer Verbindung der Formel (IIa) 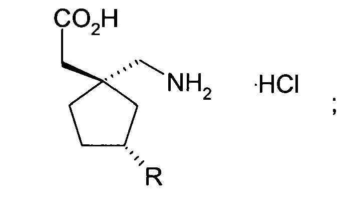 und
n) Überführen des Produkts von Stufe m) in eine Verbindung der Formel (II) 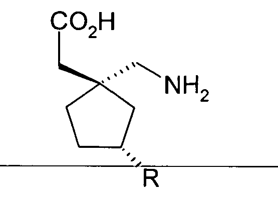 und gegebenenfalls weiteres Überführen in ein pharmazeutisch akzeptables Salz auf bekannte Weise.

10. Verfahren nach Anspruch 9, umfassend
a) Zugabe von Cyanoacetat der Formel (A) 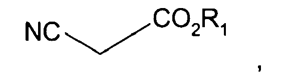 worin R₁ ausgewählt ist aus Methyl, Ethyl, *n*-Propyl, *iso*-Propyl, *n*-Butyl, *iso*-Butyl, *sek*.-Butyl, *tert*.-Butyl und Benzyl, zu einem Gemisch eines chiralen Cyclopentanons der Formel (1) 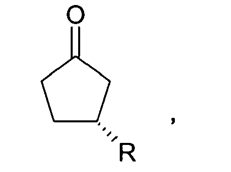 eines Lösungsmittels, das aus Tetrahydrofuran, 1,4-Dioxan, *tert*.-Butylmethylether, Chloroform, Dichlormethan, Acetonitril, Ethylether, Ethylacetat, Hexanen, N,N-Dimethylformamid, Dimethylsulfoxid, Ethanol, *tert.*-Butanol, Toluol, Benzol, Xylolen und n-Heptan ausgewählt ist, Essigsäure und eines Katalysators für die Knoevenagel-Reaktion, der aus β-Alanin, Ammoniumacetat und Piperidin ausgewählt ist, und Rühren des Gemischs in Gegenwart eines Mittels zum Entziehen von Wasser, das aus azeotroper Destillation, aktivierten Molekularsieben, wasserfreiem Magnesiumsulfat, wasserfreiem Natriumsulfat, wasserfreiem Natriumcarbonat, wasserfreiem Kaliumcarbonat, wasserfreiem Cäsiumcarbonat, Trimethylorthoformiat und Triethylorthoformiat ausgewählt ist, unter Erhalt des Alkens der Formel (2) 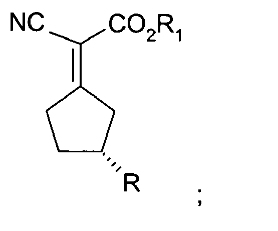
b) Zugabe des Produkts von Stufe a) zu einem Gemisch von Benzylmagnesiumchlorid, Benzylmagnesiumbromid oder Benzylmagnesiumiodid in einem Lösungsmittel, das aus Tetrahydrofuran, Benzol, 1,4-Dioxan, Hexanen, *n*-Heptan, Toluol, Diethylether und *tert*.-Butylmethylether ausgewählt ist, unter Erhalt der Additionsprodukte der Formeln 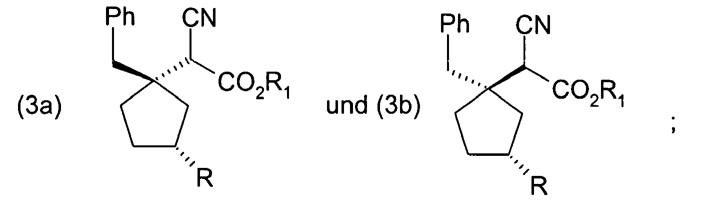
c) Zugabe der Produkte von Stufe b) zu einem Gemisch einer aus Kaliumhydroxid, Natriumhydroxid, Lithiumhydroxid und Cäsiumhydroxid ausgewählten Base in einem Lösungsmittel, das aus Ethylenglycol, 2-Methoxyethylether, 1,4-Dioxan und Diethylenglycol ausgewählt ist, Rühren des Gemischs sowie anschließendes Ansäuern unter Erhalt der Carbonsäuren der Formeln 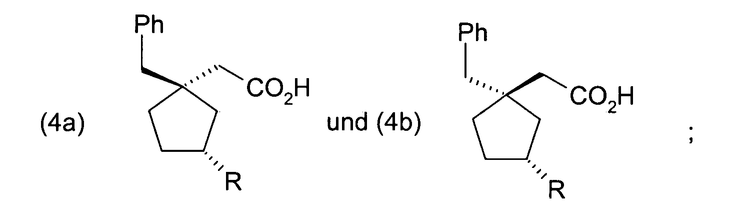 oder Zugabe der Produkte der Stufe b) zu einem Säuregemisch, das aus 6-12 M HCl, 12 M H₂SO₄, 10 - 48 Gew.% Bromwasserstoffsäure und HBr in wässriger Essigsäure ausgewählt ist, und Rühren unter Erhalt der Carbonsäuren der Formeln 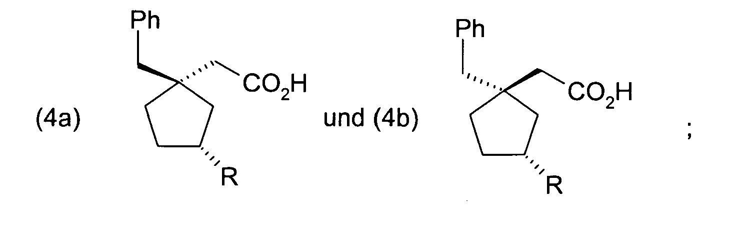
d) Inkontaktbringen der Produkte von Stufe c) mit einem Amin, das aus (S)-α-Methylbenzylamin, (R)-α-Methylbenzylamin, (R)-(+)-1-(Naphthyl)ethylamin, (S)-(+)-1-(Naphthyl)ethylamin, Triethylamin, Diisopropylethylamin, Dicyclohexylamin, Benzylamin, Dibenzylamin, Morpholin, N-Methylmorpholin, Piperidin, N-Methylpiperidin und Pyridin ausgewählt ist, in einem Lösungsmittel, das aus N,N-Dimethylformamid, Chloroform, Benzol, Xylolen, Hexanen, Aceton, Ethanol, Methanol, *iso*-Propanol, Diethylether, Dichlormethan, Benzol, Toluol, *n*-Pentan, *n*-Hexan, *n*-Heptan, Ethylacetat, Acetonitril, *tert.*-Butylmethylether, Tetrahydrofuran und 1,4-Dioxan ausgewählt ist, und Umkristallisation des so gebildeten Salzes unter Erhalt des angereicherten Diastereomers der Formel (5) 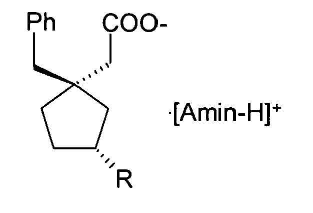 als Aminsalz;
e) Zugabe des Produkts von Stufe d) zu einem Gemisch, das aus wässriger Salzsäure, wässriger Schwefelsäure, wässriger Essigsäure, in Essigsäure gelöster Salzsäure oder in Essigsäure und Wasser gelöster Salzsäure ausgewählt ist, und Rühren unter Erhalt der Carbonsäure der Formel (6) 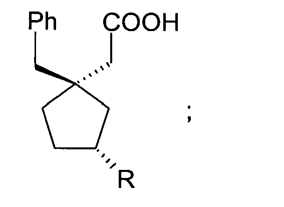 oder Verteilen des Produkts von Stufe d) zwischen einem Gemisch von wässriger Salzsäure und einem Lösungsmittel, das aus Chloroform, Dichlormethan, Ethylacetat, Ethylether, Tetrahydrofuran, 1,4-Dioxan, Toluol und *tert.*-Butylmethylether ausgewählt ist, sowie Trocknen und Eindampfen der organischen Schicht unter Erhalt der Carbonsäure der Formel (6) 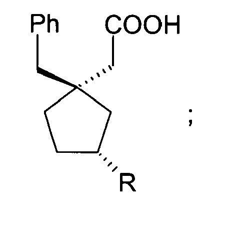
f) Zugabe von Oxalylchlorid zu einem Gemisch des Produkts von Stufe e), eines Lösungsmittels, das aus Dichlormethan, Chloroform, Ethylether, Toluol und *tert*.-Butylmethylether ausgewählt ist, und 0,01 - 10 Mol-% N,N-Dimethylformamid (DMF) und Rühren bei einer Temperatur von -40°C bis 110°C unter Erhalt des Säurechlorids der Formel (14) 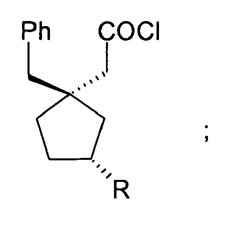
g) Zugabe des Produkts von Stufe f) zu einem Gemisch von *tert*.-Butylalkohol, eines Lösungsmittels, das aus Dichlormethan, Chloroform, Ethylether, Toluol und *tert*.-Butylmethylether ausgewählt ist, und *N,N*-Diisopropylethylamin (DIPEA) oder Triethylamin und Rühren bei einer Temperatur von -40°C bis 110°C unter Erhalt des Esters der Formel (15) 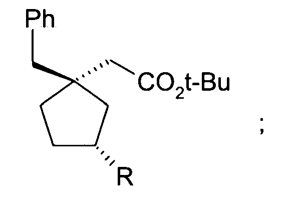
h) Zugabe des Produkts von Stufe g) zu einem Gemisch von Kohlenstofftetrachlorid oder Ethylacetat, Acetonitril, Wasser, Natriumperiodat und Ruthenium(lll)chlorid und Rühren bei einer Temperatur von -40°C bis 80°C unter Erhalt der Carbonsäure der Formel (16) 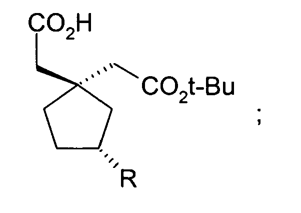
i) Zugabe des Produkts von Stufe h) zu einem Lösungsmittel, das aus Toluol, Benzol, Xylolen und *n*-Heptan ausgewählt ist, Methanol und (Trimethylsilyl)diazomethan und Rühren bei einer Temperatur von 0°C bis 150°C unter Erhalt des Diesters der Formel (17) 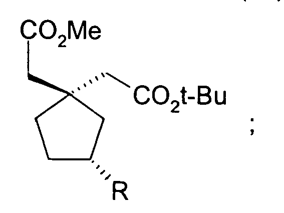 oder Zugabe des Produkts von Stufe h) zu einem Gemisch von Methyliodid, eines Lösungsmittels, das aus Chloroform, Tetrahydrofuran, Toluol und 1,4-Dioxan ausgewählt ist, und einer Base, die aus 1,8-Diazabicyclo[5.4.0]undec-7-en (DBU), Diisopropylethylamin, Triethylamin und 1,5-Diazabicyclo[4.3.0]non-5-en (DBN) ausgewählt ist, und Rühren bei einer Temperatur von -40°C bis 110°C unter Erhalt des Diesters der Formel (17) 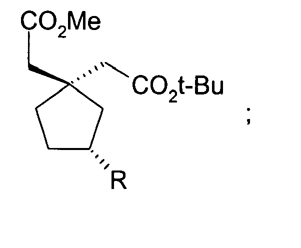
j) Zugabe von Salzsäure oder Trifluoressigsäure (TFA) zu einem Gemisch des Produkts von Stufe i) und eines Lösungsmittels, das aus Dichlormethan, Chloroform, 1,4-Dioxan, Tetrahydrofuran, Ethylether und *tert*.-Butylmethylether ausgewählt ist, und Rühren bei einer Temperatur von -40°C bis 110°C unter Erhalt der Carbonsäure der Formel (18) 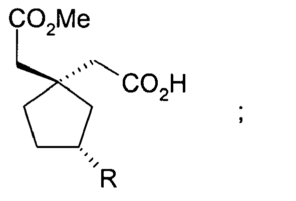
k) Zugabe des Produkts von Stufe j) zu einem Gemisch einer Base, die aus Triethylamin und Diisopropylethylamin ausgewählt ist, eines Lösungsmittels, das aus Toluol, Benzol, Xylolen und *n*-Heptan ausgewählt ist, und Diphenylphosphorylazid (DPPA) und Rühren bei einer Temperatur von 0°C bis 150°C unter Erhalt des Isocyanats der Formel (19) 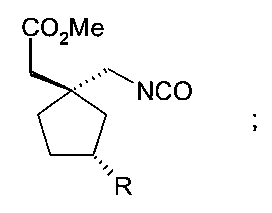 oder Zugabe des Produkts von Stufe j) zu Ethylchlorformiat oder Isobutylchlorformiat, einer Base, die aus Triethylamin und Diisopropylethylamin ausgewählt ist, und einem Lösungsmittel, das aus Tetrahydrofuran, Aceton und Diethylether ausgewählt ist, bei einer Temperatur von -40°C bis 78°C, Zugabe einer Lösung von Natriumazid in Wasser und Tetrahydrofuran oder Aceton und anschließende Zugabe von Toluol oder Benzol sowie Erhitzen unter Rückfluss unter Erhalt des Isocyanats der Formel (19) 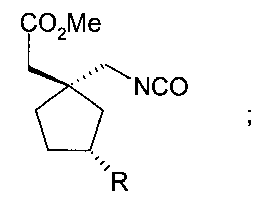
l) Zugabe des Produkts von Stufe k) zu einem Gemisch eines Lösungsmittels, das aus Toluol, Benzol, Xylolen und n-Heptan ausgewählt ist, und Methanol und Rühren bei einer Temperatur von 0°C bis 150°C unter Erhalt des Carbamats der Formel (20) 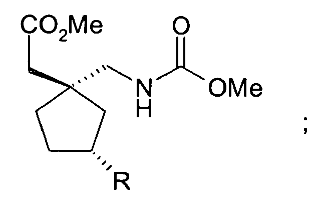
m) Zugabe des Produkts von Stufe I) zu einem Gemisch eines Lösungsmittels, das aus Wasser, Essigsäure und 1,4-Dioxan ausgewählt ist, und wässriger Salzsäure einer Konzentration von 0,01 M bis 12 M und Rühren bei einer Temperatur von 0°C bis 115°C unter Erhalt der Verbindung der Formel (IIa) 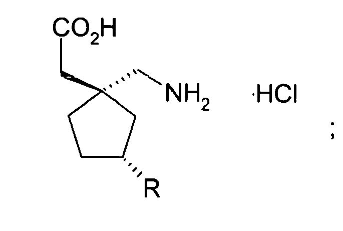 und
n) Überführen des Produkts von Stufe m) in eine Verbindung der Formel (II) 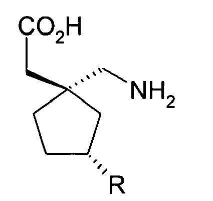 und gegebenenfalls weiteres Überführen in ein pharmazeutisch akzeptables Salz auf bekannte Weise.

11. Verfahren nach Anspruch 9, umfassend
a) Zugabe von Cyanoacetat der Formel (A) 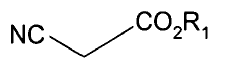 worin R₁ für Ethyl steht, zu einem Gemisch eines chiralen Cyclopentanons der Formel (1) 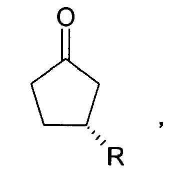 Toluol, Essigsäure und eines Katalysators für die Knoevenagel-Reaktion, der Ammoniumacetat darstellt, und Erhitzen des Gemischs unter Rückfluss an einem Dean-Stark-Abscheider unter Erhalt des Alkens der Formel (2) 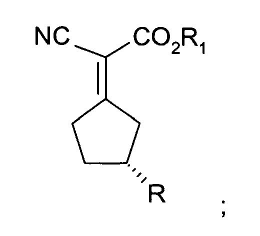
b) Zugabe des Produkts von Stufe a) zu einem Gemisch von Benzylmagnesiumchlorid in trockenem Tetrahydrofuran bei -100°C bis 25°C unter Erhalt der Additionsprodukte der Formeln 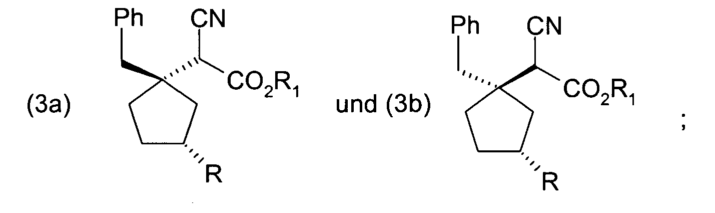
c) Zugabe der Produkte von Stufe b) zu einem Gemisch von Kaliumhydroxid in Ethylenglycol und Erhitzen des Gemischs bei 100°C bis 200°C sowie anschließendes Ansäuern unter Erhalt der Hydrolyseprodukte der Formeln 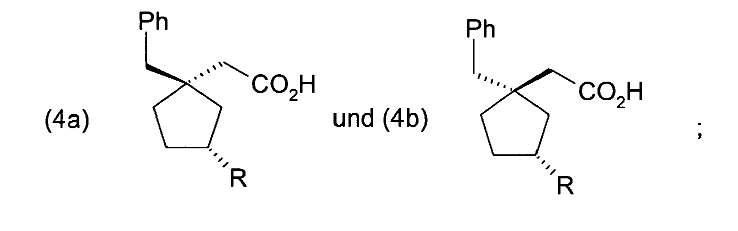
d) Inkontaktbringen der Produkte von Stufe c) mit (S)-α-Methylbenzylamin in Ethylacetat und Umkristallisation des so gebildeten Salzes aus Ethylacetat unter Erhalt des angereicherten Diastereomers der Formel (5) 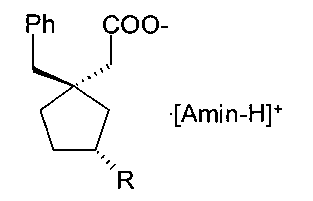 als (S)-α-Methylbenzylaminsalz;
e) Zugabe des Produkts von Stufe d) zu wässriger Salzsäure und Rühren unter Erhalt der Carbonsäure der Formel (6) 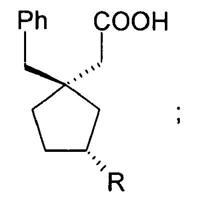
f) Zugabe von Oxalylchlorid zu einem Gemisch des Produkts von Stufe e), Dichlormethan und einer katalytischen Menge N,N-Dimethylformamid (DMF) und Rühren unter Erhalt des Säurechlorids der Formel (14) 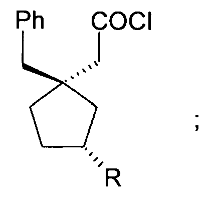
g) Zugabe des Produkts von Stufe f) zu einem Gemisch von *tert.*-Butylalkohol, Dichlormethan und *N,N*-Diisopropylethylamin (DIPEA) und Rühren unter Erhalt des Esters der Formel (15) 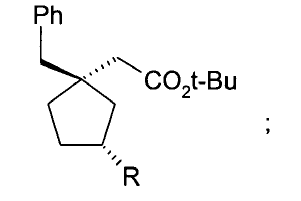
h) Zugabe des Produkts von Stufe g) zu einem Gemisch von Tetrachlormethan oder Ethylacetat, Acetonitril, Wasser, Natriumperiodat und Ruthenium(III)chlorid und Rühren unter Erhalt der Carbonsäure der Formel (16) 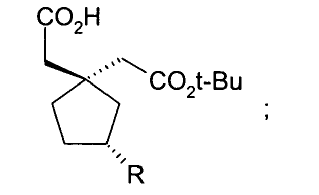
i) Zugabe des Produkts von Stufe h) zu einem Gemisch von Methanol, Toluol und (Trimethylsilyl)diazomethan und Rühren unter Erhalt des Diesters der Formel (17) 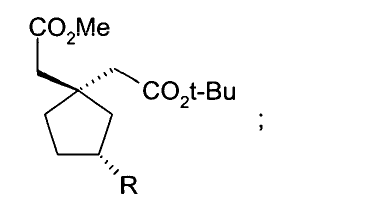 oder Zugabe des Produkts von Stufe h) zu einem Gemisch von Methyliodid, Dichlormethan und Triethylamin und Rühren unter Erhalt des Diesters der Formel (17) 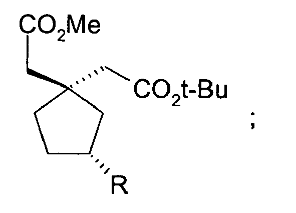
j) Zugabe von Salzsäure oder Trifluoressigsäure (TFA) zu einem Gemisch des Produkts von Stufe i) und Dichlormethan und Rühren unter Erhalt der Carbonsäure der Formel (18) 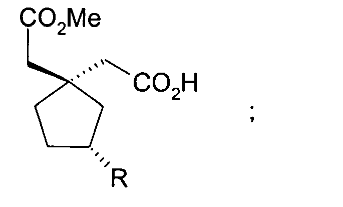
k) Zugabe des Produkts von Stufe j) zu einem Gemisch von Triethylamin, Toluol und Diphenylphosphorylazid (DPPA) und Erhitzen unter Rückfluss unter Erhalt des Isocyanats der Formel (19) 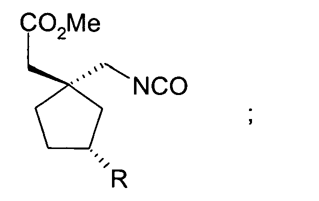 oder Zugabe des Produkts von Stufe j) zu Ethylchlorformiat oder Isobutylchlorformiat, Triethylamin und Tetrahydrofuran bei einer Temperatur von -40°C bis 78°C, Zugabe einer Lösung von Natriumazid in Wasser und Tetrahydrofuran oder Aceton und anschließende Zugabe von Toluol oder Benzol sowie Erhitzen unter Rückfluss unter Erhalt des Isocyanats der Formel (19) 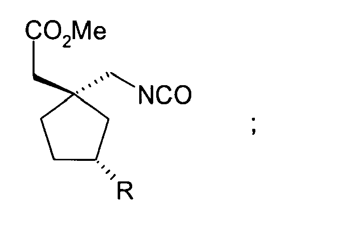
l) Zugabe des Produkts von Stufe k) zu einem Gemisch von Methanol und Toluol und Erhitzen unter Rückfluss unter Erhalt des Carbamats der Formel (20) 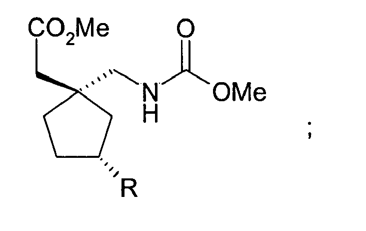
m) Zugabe des Produkts von Stufe I) zu einem Gemisch von 1,4-Dioxan und wässriger Salzsäure einer Konzentration von 6 M und Rühren unter Erhalt der Verbindung der Formel (IIa) 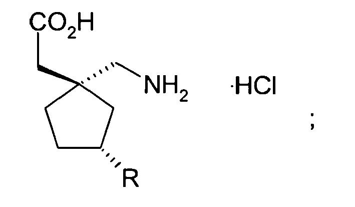
n) Überführen des Produkts von Stufe m) in eine Verbindung der Formel (II) 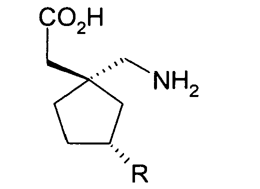 und gegebenenfalls weiteres Überführen in ein pharmazeutisch akzeptables Salz auf bekannte Weise.

12. Verfahren nach Anspruch 9, weiter **dadurch gekennzeichnet, dass** das gebildete Zwischenprodukt (14) 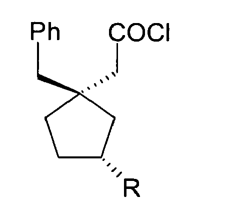 ohne Isolierung weiter mit *tert*.-Butylalkohol umgesetzt wird unter Erhalt des Esters der Formel (15)

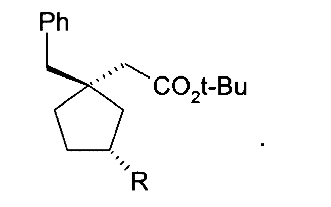

13. Verfahren nach Anspruch 9, **dadurch gekennzeichnet, dass** das gebildete Zwischenprodukt (19) 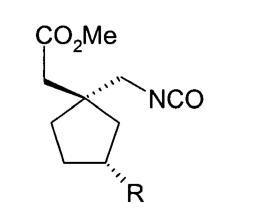 ohne Isolierung weiter mit Methanol umgesetzt wird unter Erhalt des Carbamats der Formel (20)

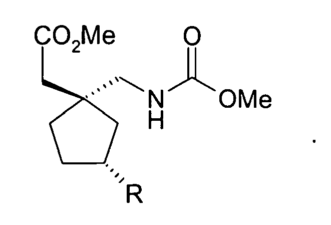

14. Verfahren nach Anspruch 9, **dadurch gekennzeichnet, dass** das gebildete Zwischenprodukt (14) 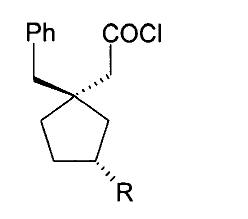 ohne Isolierung weiter mit *tert*.-Butylalkohol umgesetzt wird unter Erhalt des Carbamats der Formel (15) 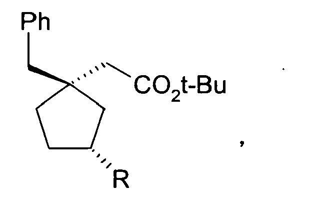 und das gebildete Zwischenprodukt (19) 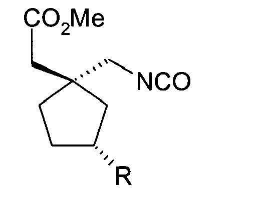 ohne Isolierung weiter mit Methanol umgesetzt wird unter Erhalt des Carbamats der Formel (20)

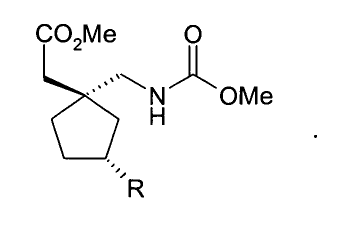

15. Verfahren zur Herstellung einer Verbindung der Formel III 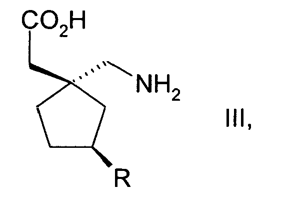 worin R für C₁-C₁₀-Alkyl oder C₃-C₁₀-Cycloalkyl steht, und ihrer pharmazeutisch akzeptabler Salze, umfassend
a) Zugabe eines Cyanoacetats der Formel (A) 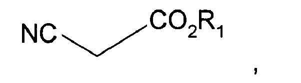 worin R₁ für Alkyl oder Benzyl steht, zu einem Gemisch eines chiralen Cyclopentanons der Formel (21) 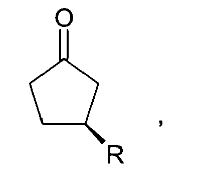 eines Lösungsmittels, einer Carbonsäure und eines Katalysators für die Knoevenagel-Reaktion, und Rühren des Gemischs in Gegenwart eines Mittels zum Entziehen von Wasser, unter Erhalt des Alkens der Formel (22) 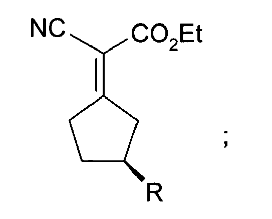
b) Zugabe des Produkts von Stufe a) zu einem Gemisch von Benzylmagnesiumchlorid oder Benzylmagnesiumiodid in einem Lösungsmittel unter Erhalt der Additionsprodukte der Formeln 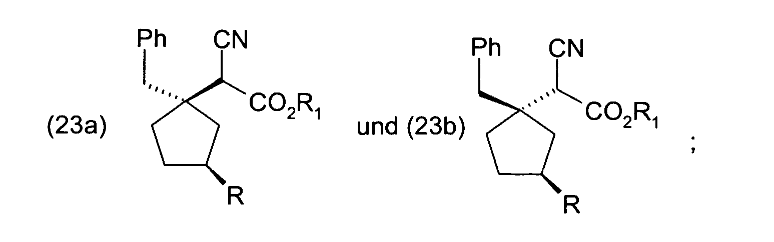
c) Zugabe der Produkte von Stufe b) zu einem Gemisch einer aus Kaliumhydroxid, Natriumhydroxid, Lithiumhydroxid und Cäsiumhydroxid ausgewählten Base und eines Lösungsmittels, Rühren sowie anschließendes Ansäuern unter Erhalt der Carbonsäuren der Formeln 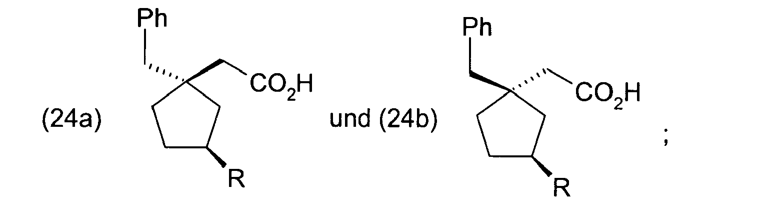 oder Zugabe der Produkte der Stufe b) zu einem Säuregemisch und Rühren unter Erhalt der Carbonsäuren der Formeln 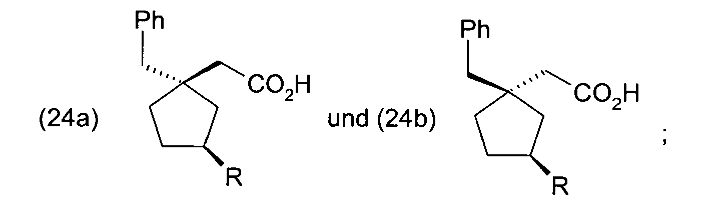
d) Inkontaktbringen der Produkte von Stufe c) mit einem Amin in einem Lösungsmittel und Umkristallisation des so gebildeten Salzes unter Erhalt des angereicherten Diastereomers der Formel (25) 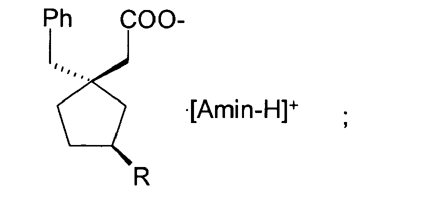 als Aminsalz; und
e) Überführen des Produkts von Stufe d) in eine Carbonsäure der Formel (26) 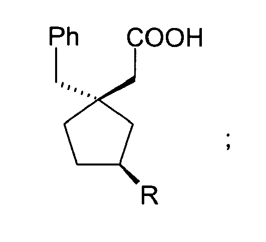
f) Zugabe des Produkts von Stufe e) zu einem Gemisch von Methyliodid, eines Lösungsmittels und 1,8-Diazabicyclo[5.4.0]undec-7-en (DBU) und Rühren unter Erhalt des Esters der Formel (27) 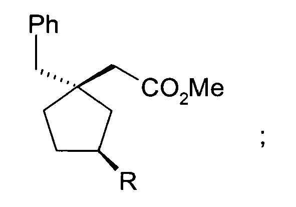 oder Zugabe des Produkts von Stufe e) zu Methanol und einer Säure unter Erhalt des Esters der Formel (27) 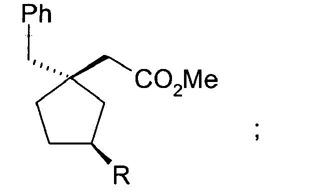 oder Zugabe des Produkts von Stufe e) zu einer Lösung von Diazomethan oder Trimethylsilyl-diazomethan in einem Lösungsmittel unter Erhalt des Esters der Formel (27) 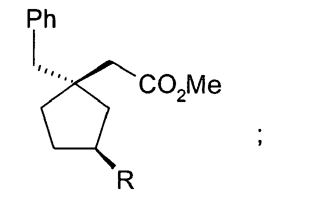
g) Zugabe des Produkts von Stufe f) zu einem Gemisch von Tetrachlormethan oder Ethylacetat und Acetonitril, Wasser, Natriumperiodat und Ruthenium(III)chlorid und Rühren unter Erhalt der Carbonsäure der Formel (28) 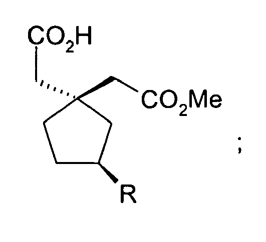
h) Zugabe des Produkts von Stufe g) zu einem Gemisch eines tertiären Amins, eines Lösungsmittels und Diphenylphosphorylazid (DPPA) und Rühren unter Erhalt des Isocyanats der Formel (29) 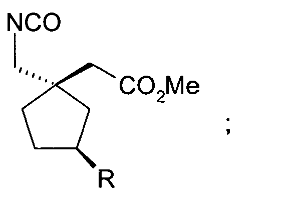 oder Zugabe des Produkts von Stufe g) zu Ethylchlorformiat oder Isobutylchlorformiat und einer Base in einem Lösungsmittel bei einer Temperatur von -40°C bis 78°C, Zugabe einer Lösung von Natriumazid in Wasser und Tetrahydrofuran oder Aceton und anschließende Zugabe von Toluol oder Benzol sowie Erhitzen unter Rückfluss unter Erhalt des Isocyanats der Formel (29) 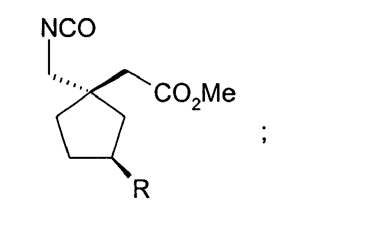
i) Zugabe des Produkts von Stufe h) zu einem Gemisch aus einem Lösungsmittel und Methanol und Rühren unter Erhalt des Carbamats der Formel (30) 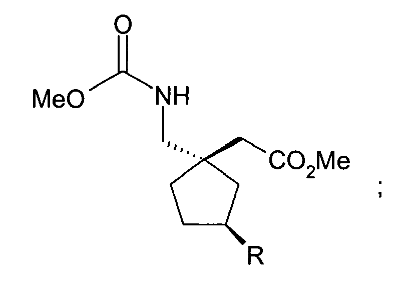
j) Zugabe des Produkts von Stufe i) zu einem Gemisch eines Lösungsmittels und wässriger Salzsäure und Rühren unter Erhalt einer Verbindung der Formel (IIIa) 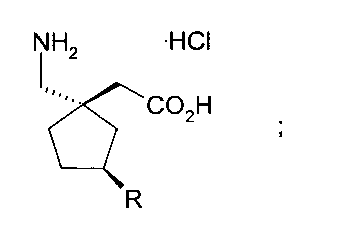
k) Überführen des Produkts von Stufe j) in eine Verbindung der Formel (III) 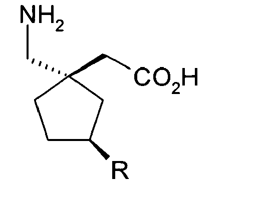 und gegebenenfalls weiteres Überführen in ein pharmazeutisch akzeptables Salz auf bekannte Weise.

16. Verfahren zur Herstellung einer Verbindung der Formel (IV) 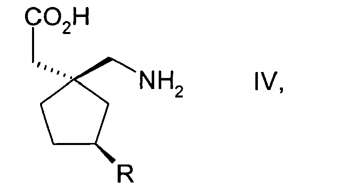 worin R für C₁-C₁₀-Alkyl oder C₃-C₁₀-Cycloalkyl steht, und ihrer pharmazeutisch akzeptabler Salze, umfassend
a) Zugabe eines Cyanoacetats der Formel (A) 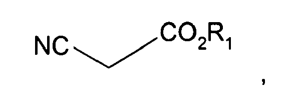 worin R₁ für Alkyl oder Benzyl steht, zu einem Gemisch eines chiralen Cyclopentanons der Formel (21) 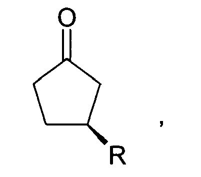 eines Lösungsmittels, einer Carbonsäure und eines Katalysators für die Knoevenagel-Reaktion, und Rühren des Gemischs in Gegenwart eines Mittels zum Entziehen von Wasser, unter Erhalt des Alkens der Formel (22) 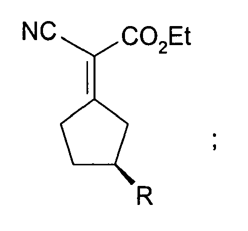
b) Zugabe des Produkts von Stufe a) zu einem Gemisch von Benzylmagnesiumchlorid oder Benzylmagnesiumiodid in einem Lösungsmittel unter Erhalt der Additionsprodukte der Formeln 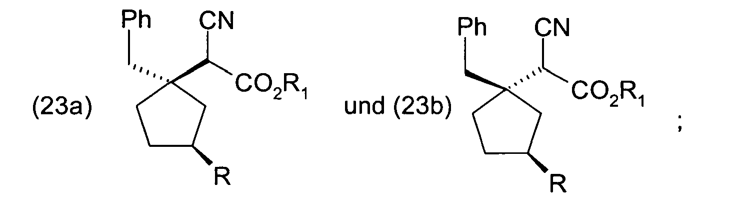
c) Zugabe der Produkte von Stufe b) zu einem Gemisch einer aus Kaliumhydroxid, Natriumhydroxid, Lithiumhydroxid und Cäsiumhydroxid ausgewählten Base und eines Lösungsmittels, Rühren sowie anschließendes Ansäuern unter Erhalt der Carbonsäuren der Formeln 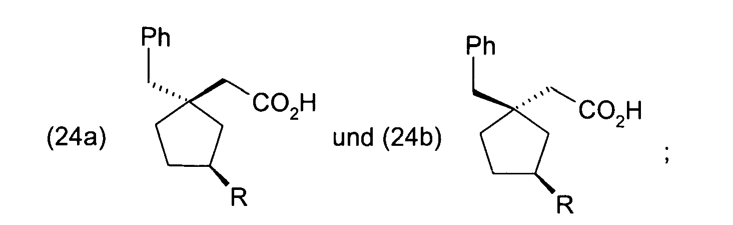 oder Zugabe der Produkte der Stufe b) zu einem Säuregemisch und Rühren unter Erhalt der Carbonsäuren der Formeln 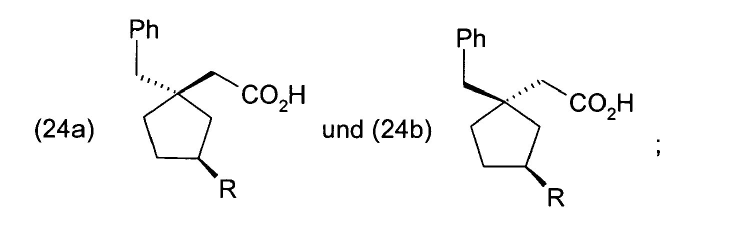
d) Inkontaktbringen der Produkte von Stufe c) mit einem Amin in einem Lösungsmittel und Umkristallisation des so gebildeten Salzes unter Erhalt des angereicherten Diastereomers der Formel (25) 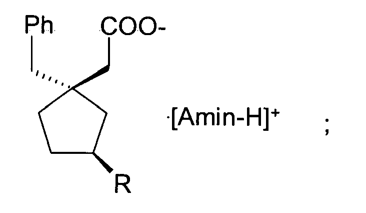 als Aminsalz; und
e) Überführen des Produkts von Stufe d) in eine Carbonsäure der Formel (26) 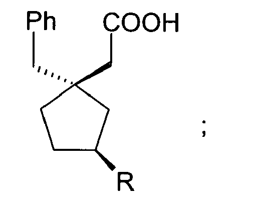
f) Zugabe des Produkts von Stufe e) zu einem Gemisch einer tertiären Aminbase, eines Lösungsmittels und Diphenylphosphorylazid (DPPA) und Rühren unter Erhalt des Isocyanats der Formel (31) 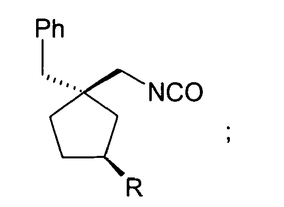 oder Zugabe des Produkts von Stufe g) zu Ethylchlorformiat oder Isobutylchlorformiat und einer Base in einem Lösungsmittel bei einer Temperatur von -40°C bis 78°C, Zugabe einer Lösung von Natriumazid in Wasser und Tetrahydrofuran oder Aceton und anschließende Zugabe von Toluol oder Benzol sowie Erhitzen unter Rückfluss unter Erhalt des Isocyanats der Formel (31) 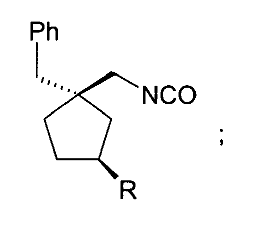
g) Zugabe des Produkts von Stufe f) zu einem Gemisch aus einem Lösungsmittel und Methanol und Rühren unter Erhalt des Carbamats der Formel (32) 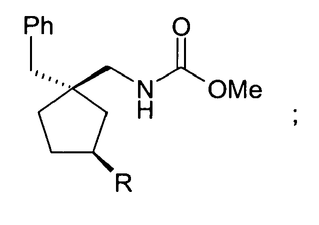
h) Zugabe des Produkts von Stufe g) zu einem Gemisch von Tetrachlormethan oder Ethylacetat und Acetonitril, Wasser, Natriumperiodat und Ruthenium(III)chlorid und Rühren unter Erhalt der Carbonsäure der Formel (33) 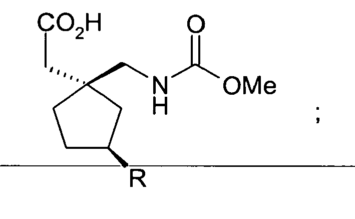
i) Zugabe des Produkts von Stufe h) zu einem Gemisch eines Lösungsmittels und wässriger Salzsäure und Rühren unter Erhalt einer Verbindung der Formel (IVa) 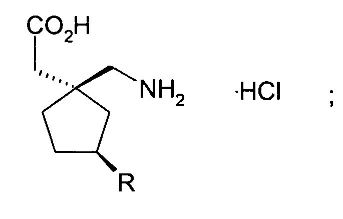 und
j) Überführen des Produkts von Stufe i) in eine Verbindung der Formel (IV) 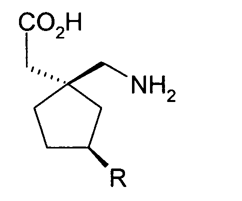 und gegebenenfalls weiteres Überführen in ein pharmazeutisch akzeptables Salz auf bekannte Weise.

17. Verfahren zur Herstellung einer Verbindung der Formel (IV) 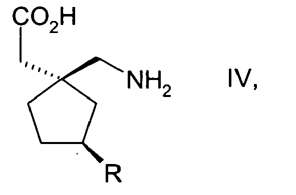 worin R für C₁-C₁₀-Alkyl oder C₃-C₁₀-Cycloalkyl steht, und ihrer pharmazeutisch akzeptabler Salze, umfassend
a) Zugabe von Cyanoacetat der Formel (A) 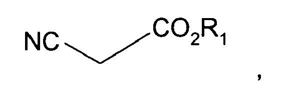 worin R₁ für Alkyl oder Benzyl steht, zu einem Gemisch eines chiralen Cyclopentanons der Formel (21) 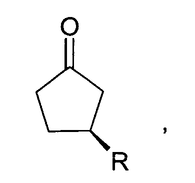 eines Lösungsmittels, einer Carbonsäure und eines Katalysators für die Knoevenagel-Reaktion, und Rühren des Gemischs in Gegenwart eines Mittels zum Entziehen von Wasser, unter Erhalt des Alkens der Formel (22) 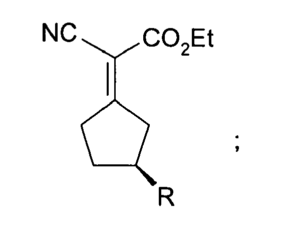
b) Zugabe des Produkts von Stufe a) zu einem Gemisch von Benzylmagnesiumchlorid oder Benzylmagnesiumiodid in einem Lösungsmittel unter Erhalt der Additionsprodukte der Formeln 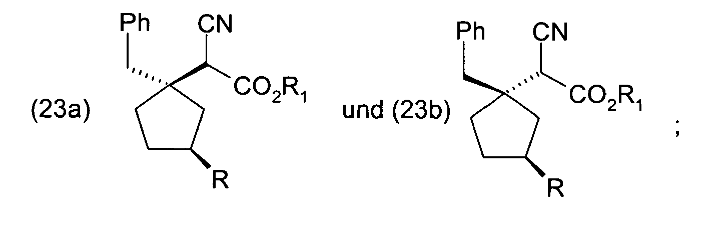
c) Zugabe der Produkte von Stufe b) zu einem Gemisch einer aus Kaliumhydroxid, Natriumhydroxid, Lithiumhydroxid und Cäsiumhydroxid ausgewählten Base und eines Lösungsmittels, Rühren sowie anschließendes Ansäuern unter Erhalt der Carbonsäuren der Formeln 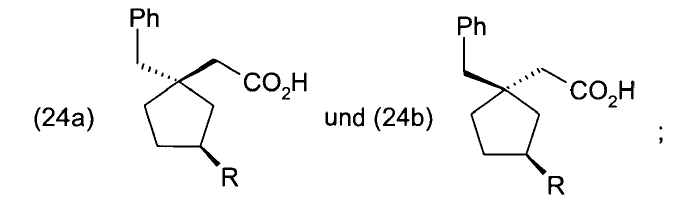 oder Zugabe der Produkte der Stufe b) zu einem Säuregemisch und Rühren unter Erhalt der Carbonsäuren der Formeln 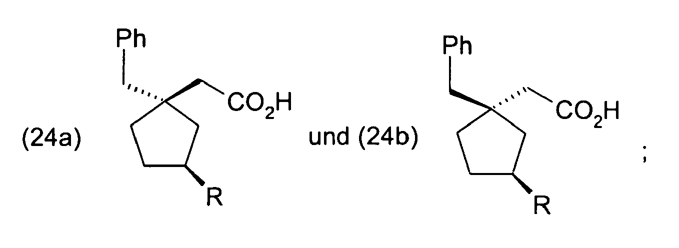
d) Inkontaktbringen der Produkte von Stufe c) mit einem Amin in einem Lösungsmittel und Umkristallisation des so gebildeten Salzes unter Erhalt des angereicherten Diastereomers der Formel (25) 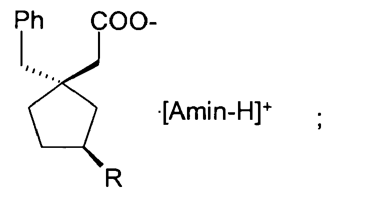 als Aminsalz; und
e) Überführen des Produkts von Stufe d) in eine Carbonsäure der Formel (26) 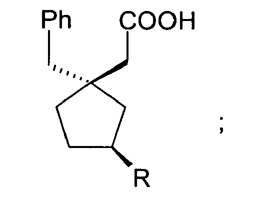
f) Zugabe von Oxalylchlorid zu einem Gemisch des Produkts von Stufe e), eines Lösungsmittels und N,N-Dimethylformamid (DMF) und Rühren unter Erhalt des Säurechlorids der Formel (34) 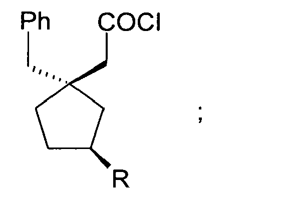
g) Zugabe des Produkts von Stufe f) zu einem Gemisch von *tert*.-Butylalkohol, eines Lösungsmittels und einer tertiären Aminbase und Rühren unter Erhalt des Esters der Formel (35) 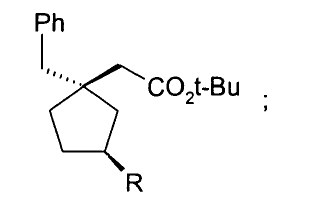
h) Zugabe des Produkts von Stufe g) zu einem Gemisch von Tetrachlormethan oder Ethylacetat und Acetonitril, Wasser, Natriumperiodat und Ruthenium(III)chlorid und Rühren unter Erhalt der Carbonsäure der Formel (36) 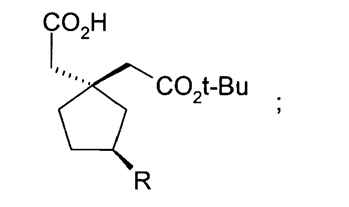
i) Zugabe des Produkts von Stufe h) zu einem Gemisch aus einem Lösungsmittel, Methanol und (Trimethylsilyl)diazomethan und Rühren unter Erhalt des Diesters der Formel (37) 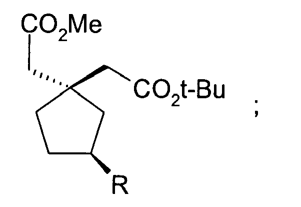 oder Zugabe des Produkts von Stufe h) zu einer Mischung von Methyliodid, eines Lösungsmittels und einer Base und Rühren unter Erhalt des Diesters der Formel (37) 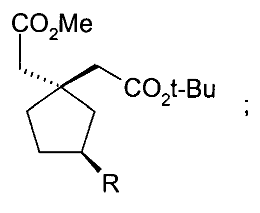
j) Zugabe einer Säure zu einem Gemisch des Produkts von Stufe i) und eines Lösungsmittels und Rühren unter Erhalt der Carbonsäure der Formel (38) 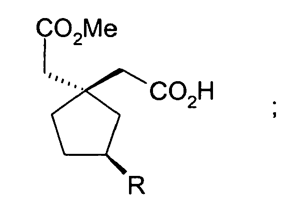
k) Zugabe des Produkts von Stufe j) zu einem Gemisch einer tertiären Aminbase, eines Lösungsmittels und Diphenylphosphorylazid (DPPA) und Rühren unter Erhalt des Isocyanats der Formel (39) 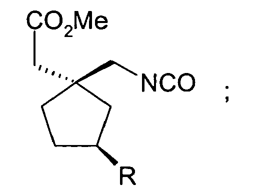 oder Zugabe des Produkts von Stufe j) zu Ethylchlorformiat oder Isobutylchlorformiat und einer Base in einem Lösungsmittel bei einer Temperatur von -40°C bis 78°C, Zugabe einer Lösung von Natriumazid in Wasser und Tetrahydrofuran oder Aceton und anschließende Zugabe von Toluol oder Benzol sowie Erhitzen unter Rückfluss unter Erhalt des Isocyanats der Formel (39) 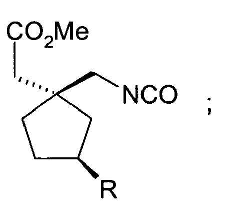
l) Zugabe des Produkts von Stufe k) zu einem Gemisch aus einem Lösungsmittel und Methanol und Rühren unter Erhalt des Carbamats der Formel (40) 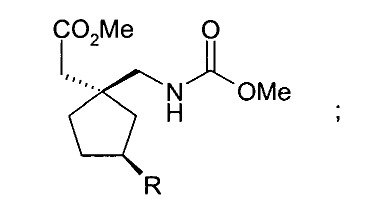
m) Zugabe des Produkts von Stufe I) zu einem Gemisch eines Lösungsmittels und Salzsäure und Rühren unter Erhalt einer Verbindung der Formel (IVa) 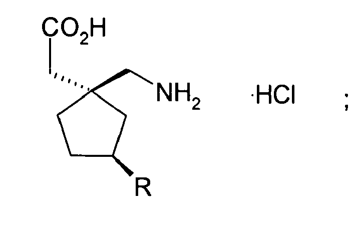 und
n) Überführen des Produkts von Stufe m) in eine Verbindung der Formel (IV) 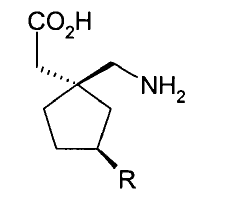 und gegebenenfalls weiteres Überführen in ein pharmazeutisch akzeptables Salz auf bekannte Weise.

18. Verfahren zur Herstellung einer Verbindung der Formel (6) 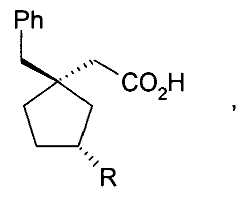 worin R für C₁-C₁₀-Alkyl oder C₃-C₁₀-Cycloalkyl steht, und ihrer pharmazeutisch akzeptabler Salze, umfassend
a) Zugabe von Cyanoacetat der Formel (A) 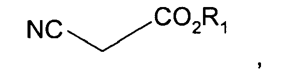 worin R₁ für Alkyl oder Benzyl steht, zu einem Gemisch eines chiralen Cyclopentanons der Formel (1) 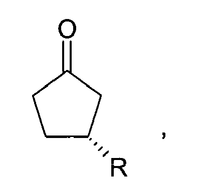 eines Lösungsmittels, einer Carbonsäure und eines Katalysators für die Knoevenagel-Reaktion, und Rühren des Gemischs in Gegenwart eines Mittels zum Entziehen von Wasser, unter Erhalt des Alkens der Formel (2) 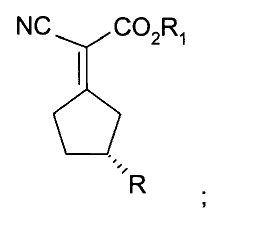
b) Zugabe des Produkts von Stufe a) zu einem Gemisch von Benzylmagnesiumchlorid, Benzylmagnesiumbromid oder Benzylmagnesiumiodid in einem Lösungsmittel unter Erhalt der Additionsprodukte der Formeln 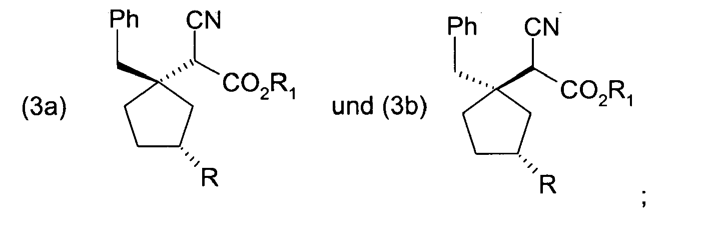
c) Zugabe der Produkte von Stufe b) zu einem Gemisch einer aus Kaliumhydroxid, Natriumhydroxid, Lithiumhydroxid und Cäsiumhydroxid ausgewählten Base in einem Lösungsmittel, Rühren sowie anschließendes Ansäuern unter Erhalt der Carbonsäuren der Formeln 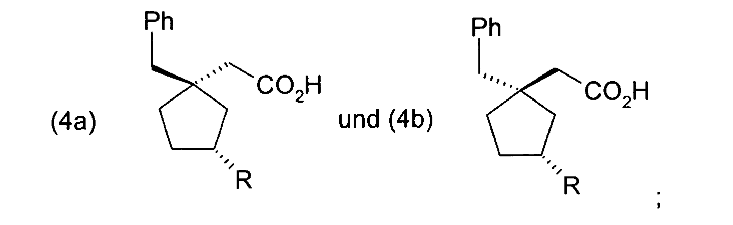 oder Zugabe der Produkte der Stufe b) zu einem Säuregemisch und Rühren unter Erhalt der Carbonsäuren der Formeln 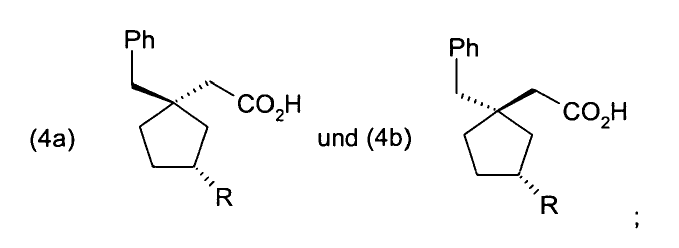
d) Inkontaktbringen der Produkte von Stufe c) mit einem Amin in einem Lösungsmittel und Umkristallisation des so gebildeten Salzes unter Erhalt des angereicherten Diastereomers der Formel (5) 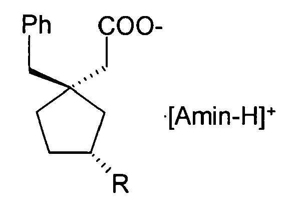 als Aminsalz;
e) Überführen des Produkts von Stufe d) in eine Carbonsäure der Formel (6)

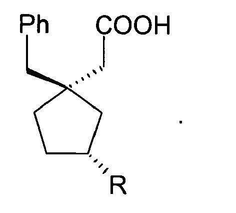

19. Verfahren nach Anspruch 18, umfassend
a) Zugabe von Cyanoacetat der Formel (A) 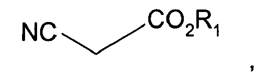 worin R₁ ausgewählt ist aus Methyl, Ethyl, *n*-Propyl, *iso*-Propyl, *n*-Butyl, *iso*-Butyl, *sek*.-Butyl, *tert*.-Butyl und Benzyl, zu einem Gemisch eines chiralen Cyclopentanons der Formel (1) 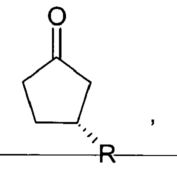 eines Lösungsmittels, das aus Tetrahydrofuran, 1,4-Dioxan, *tert*.-Butylmethylether, Chloroform, Dichlormethan, Acetonitril, Ethylether, Ethylacetat, Hexanen, N,N-Dimethylformamid, Dimethylsulfoxid, Ethanol, *tert*.-Butanol, Toluol, Benzol, Xylolen und *n*-Heptan ausgewählt ist, Essigsäure und eines Katalysators für die Knoevenagel-Reaktion, der aus β-Alanin, Ammoniumacetat und Piperidin ausgewählt ist, und Rühren des Gemischs in Gegenwart eines Mittels zum Entziehen von Wasser, das aus azeotroper Destillation, aktivierten Molekularsieben, wasserfreiem Magnesiumsulfat, wasserfreiem Natriumsulfat, wasserfreiem Natriumcarbonat, wasserfreiem Kaliumcarbonat, wasserfreiem Cäsiumcarbonat, Trimethylorthoformiat und Triethylorthoformiat ausgewählt ist, unter Erhalt des Alkens der Formel (2) 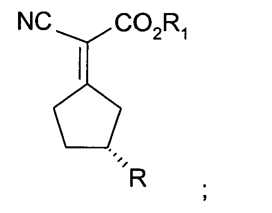
b) Zugabe des Produkts von Stufe a) zu einem Gemisch von Benzylmagnesiumchlorid, Benzylmagnesiumbromid oder Benzylmagnesiumiodid in einem Lösungsmittel, das aus Tetrahydrofuran, Benzol, 1,4-Dioxan, Hexanen, *n*-Heptan, Toluol, Diethylether und *tert*.-Butylmethylether ausgewählt ist, unter Erhalt der Additionsprodukte der Formeln 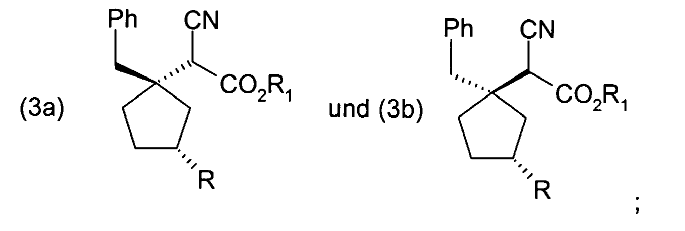
c) Zugabe der Produkte von Stufe b) zu einem Gemisch einer aus Kaliumhydroxid, Natriumhydroxid, Lithiumhydroxid und Cäsiumhydroxid ausgewählten Base in einem Lösungsmittel, das aus Ethylenglycol, 2-Methoxyethylether, 1,4-Dioxan und Diethylenglycol ausgewählt ist, Rühren des Gemischs sowie anschließendes Ansäuern unter Erhalt der Carbonsäuren der Formeln 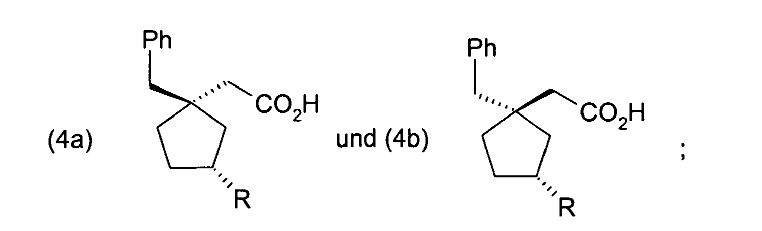 oder Zugabe der Produkte der Stufe b) zu einem Säuregemisch, das aus 6-12 M HCl, 12 M H₂SO₄, 10 - 48 Gew.% Bromwasserstoffsäure und HBr in wässriger Essigsäure ausgewählt ist, und Rühren unter Erhalt der Carbonsäuren der Formeln 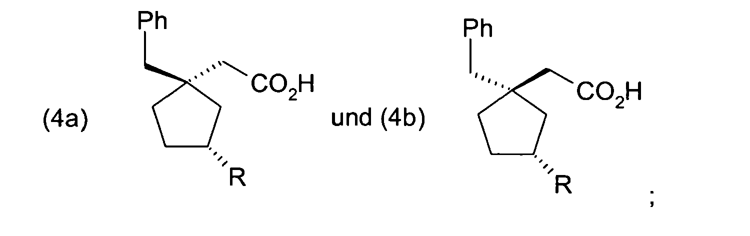
d) Inkontaktbringen der Produkte von Stufe c) mit einem Amin, das aus (S)-α-Methylbenzylamin, (R)-α-Methylbenzylamin, (R)-(+)-1-(Naphthyl)ethylamin, (S)-(+)-1-(Naphthyl)ethylamin, Triethylamin, Diisopropylethylamin, Dicyclohexylamin, Benzylamin, Dibenzylamin, Morpholin, N-Methylmorpholin, Piperidin, N-Methylpiperidin und Pyridin ausgewählt ist, in einem Lösungsmittel, das aus N,N-Dimethylformamid, Chloroform, Benzol, Xylolen, Hexanen, Aceton, Ethanol, Methanol, *iso*-Propanol, Diethylether, Dichlormethan, Benzol, Toluol, n-Pentan, n-Hexan, n-Heptan, Ethylacetat, Acetonitril, *tert*.-Butylmethylether, Tetrahydrofuran und 1,4-Dioxan ausgewählt ist, und Umkristallisation des so gebildeten Salzes unter Erhalt des angereicherten Diastereomers der Formel (5) 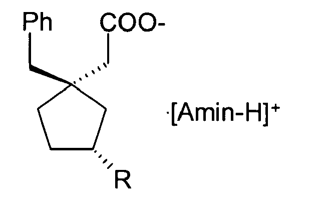 als Aminsalz; und
e) Zugabe des Produkts von Stufe d) zu einem Gemisch, das aus wässriger Salzsäure, wässriger Schwefelsäure, wässriger Essigsäure, in Essigsäure gelöster Salzsäure oder in Essigsäure und Wasser gelöster Salzsäure ausgewählt ist, und Rühren unter Erhalt der Carbonsäure der Formel (6) 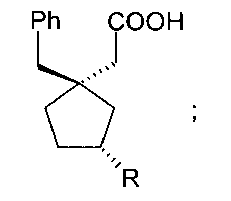 oder Verteilen des Produkts von Stufe d) zwischen einem Gemisch von wässriger Salzsäure und einem Lösungsmittel, das aus Chloroform, Dichlormethan, Ethylacetat, Ethylether, Tetrahydrofuran, 1,4-Dioxan, Toluol und *tert*.-Butylmethylether ausgewählt ist, sowie Trocknen und Eindampfen der organischen Schicht unter Erhalt der Carbonsäure der Formel (6)

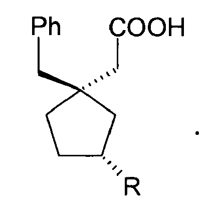

20. Verfahren nach Anspruch 18, umfassend
a) Zugabe von Cyanoacetat der Formel (A) 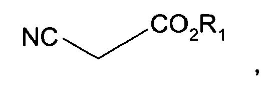 worin R₁ für Ethyl steht, zu einem Gemisch eines chiralen Cyclopentanons der Formel (1) 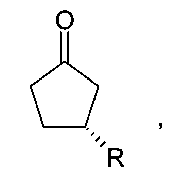 Toluol, Essigsäure und eines Katalysators für die Knoevenagel-Reaktion, der Ammoniumacetat darstellt, und Erwärmen des Gemischs unter Rückfluss an einem Dean-Stark-Abscheider unter Erhalt des Alkens der Formel (2) 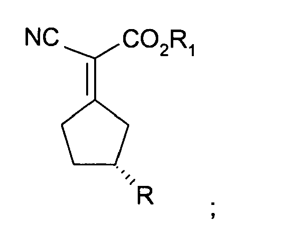
b) Zugabe des Produkts von Stufe a) zu einem Gemisch von Benzylmagnesiumchlorid in trockenem Tetrahydrofuran bei -100°C bis 25° unter Erhalt der Additionsprodukte der Formeln 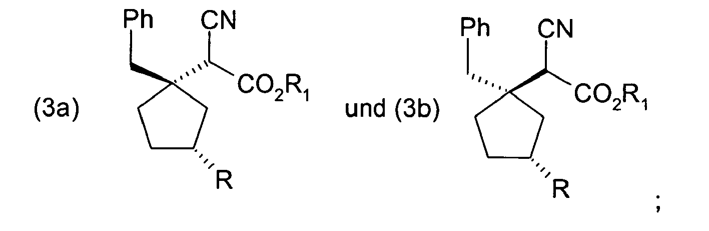
c) Zugabe der Produkte von Stufe b) zu einem Gemisch von Kaliumhydroxid in Ethylenglycol, Erhitzen des Gemischs bei 100°C bis 200°C sowie anschließendes Ansäuern unter Erhalt der Hydrolyseprodukte der Formeln 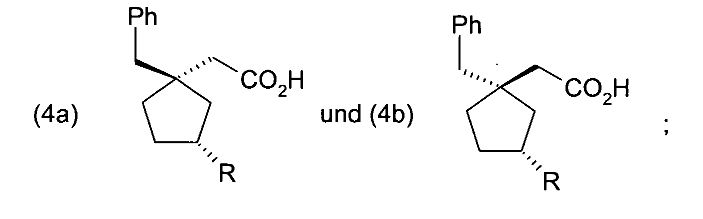
d) Inkontaktbringen der Produkte von Stufe c) mit (S)-α-Methylbenzylamin in Ethylacetat und Umkristallisation des so gebildeten Salzes aus Ethylacetat unter Erhalt des angereicherten Diastereomers der Formel (5) 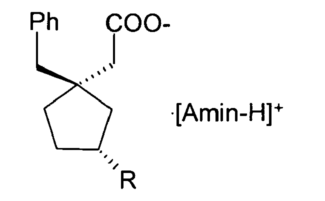 als (S)-α-Methylbenzylaminsalz;
e) Zugabe des Produkts von Stufe d) zu wässriger Salzsäure und Rühren unter Erhalt der Carbonsäure der Formel (6)

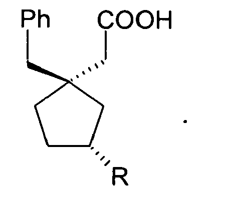

21. Verfahren zur Herstellung einer Verbindung der Formel (26) 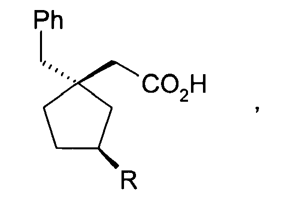 worin R für C₁-C₁₀-Alkyl oder C₁-C₁₀-Cycloalkyl steht, und ihrer pharmazeutisch akzeptabler Salze, umfassend
a) Zugabe von Cyanoacetat der Formel (A) 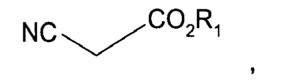 worin R₁ für Alkyl oder Benzyl steht, zu einem Gemisch eines chiralen Cyclopentanons der Formel (21) 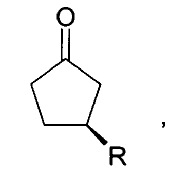 eines Lösungsmittels, einer Carbonsäure und eines Katalysators für die Knoevenagel-Reaktion, und Rühren des Gemischs in Gegenwart eines Mittels zum Entziehen von Wasser, unter Erhalt des Alkens der Formel (22) 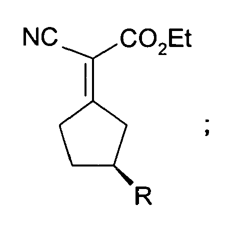
b) Zugabe des Produkts von Stufe a) zu einem Gemisch von Benzylmagnesiumchlorid oder Benzylmagnesiumiodid in einem Lösungsmittel unter Erhalt der Additionsprodukte der Formeln 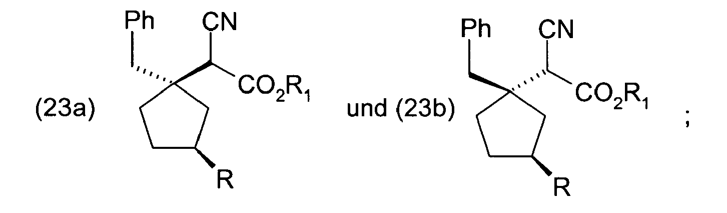
c) Zugabe der Produkte von Stufe b) zu einem Gemisch einer aus Kaliumhydroxid, Natriumhydroxid, Lithiumhydroxid und Cäsiumhydroxid ausgewählten Base in einem Lösungsmittel, Rühren sowie anschließendes Ansäuern unter Erhalt der Carbonsäuren der Formeln 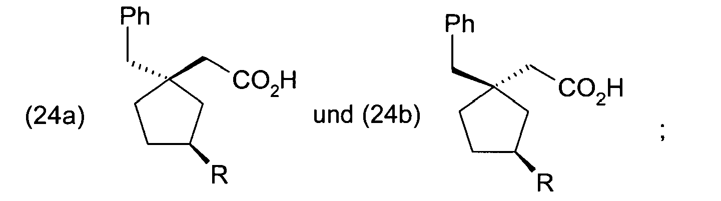 oder Zugabe der Produkte der Stufe b) zu einem Säuregemisch und Rühren unter Erhalt der Carbonsäuren der Formeln 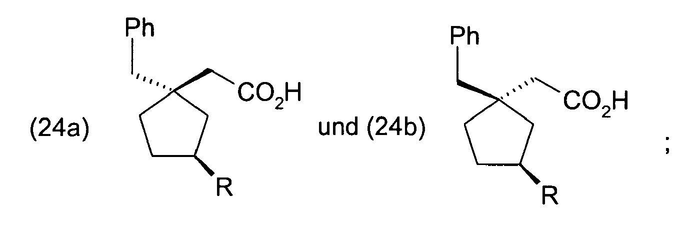
d) Inkontaktbringen der Produkte von Stufe c) mit einem Amin in einem Lösungsmittel und Umkristallisation des so gebildeten Salzes unter Erhalt des angereicherten Diastereomers der Formel (25) 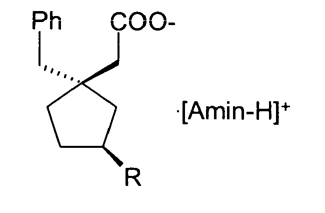 als Aminsalz; und
e) Überführen des Produkts von Stufe d) in eine Carbonsäure der Formel (26)

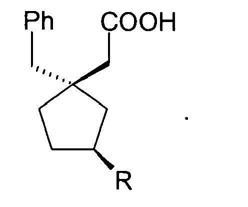

22. Verfahren nach Anspruch 21, umfassend
a) Zugabe von Cyanoacetat der Formel (A) 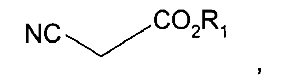 worin R₁ aus Methyl, Ethyl, *n*-Propyl, *iso*-Propyl, *n*-Butyl, *iso*-Butyl, *sek.-*Butyl, *tert*.-Butyl und Benzyl ausgewählt ist, zu einem Gemisch eines chiralen Cyclopentanons der Formel (21) 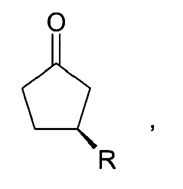 eines Lösungsmittels, das aus Tetrahydrofuran, 1,4-Dioxan, *tert*.-Butylmethylether, Chloroform, Dichlormethan, Acetonitril, Ethylether, Ethylacetat, Hexanen, N,N-Dimethylformamid, Dimethylsulfoxid, Ethanol, *tert*.-Butanol, Toluol, Benzol, Xylolen und *n*-Heptan ausgewählt ist, Essigsäure und eines Katalysators für die Knoevenagel-Reaktion, der aus β-Alanin, Ammoniumacetat und Piperidin ausgewählt ist, und Rühren des Gemischs in Gegenwart eines Mittels zum Entziehen von Wasser, das aus azeotroper Destillation, aktivierten Molekularsieben, wasserfreiem Magnesiumsulfat, wasserfreiem Cäsiumcarbonat, Trimethylorthoformiat und Triethylorthoformiat ausgewählt ist, unter Erhalt des Alkens der Formel (22) 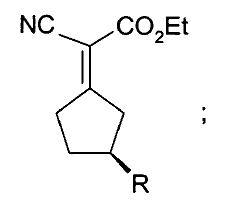
b) Zugabe des Produkts von Stufe a) zu einem Gemisch von Benzylmagnesiumchlorid, Benzylmagnesiumbromid oder Benzylmagnesiumiodid in einem Lösungsmittel, das aus Tetrahydrofuran, 1,4-Dioxan, Hexanen, *n-*Heptan, Toluol, Diethylether und *tert.*-Butylmethylether ausgewählt ist, unter Erhalt der Additionsprodukte der Formeln 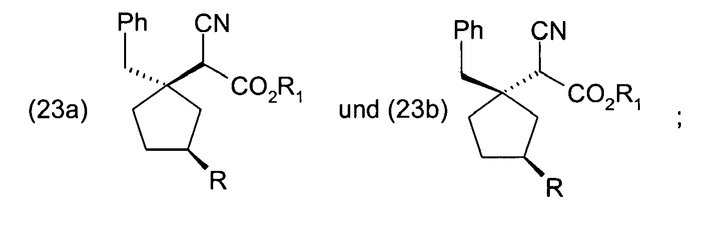
c) Zugabe der Produkte von Stufe b) zu einem Gemisch einer aus Kaliumhydroxid, Natriumhydroxid, Lithiumhydroxid und Cäsiumhydroxid ausgewählten Base in einem Lösungsmittel, das aus Ethylenglycol, 2-Methoxyethylether, 1,4-Dioxan und Diethylenglycol ausgewählt ist, Rühren des Gemischs sowie anschließendes Ansäuern unter Erhalt der Carbonsäuren der Formeln 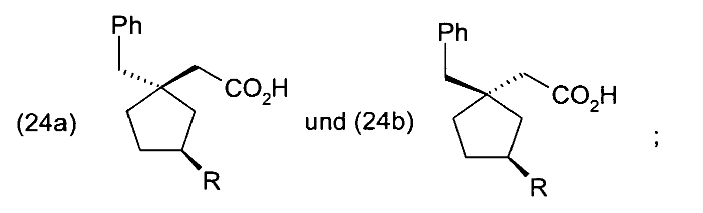 oder Zugabe der Produkte der Stufe b) zu einem Säuregemisch, das aus 6 - 12 M HCl, 12 M H₂SO₄,10-48 Gew.% Bromwassertoffsäure und HBr in wässriger Essigsäure ausgewählt ist, und Rühren unter Erhalt der Carbonsäuren der Formeln 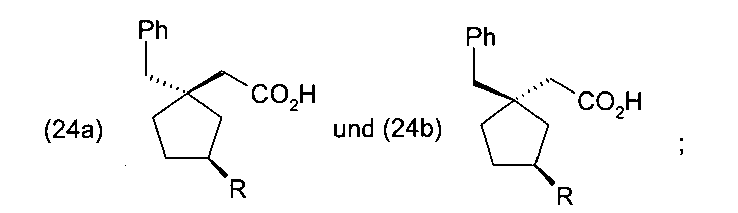
d) Inkontaktbringen der Produkte von Stufe c) mit einem Amin, das aus (S)-α-Methylbenzylamin, (R)-α-Methylbenzylamin, (R)-(+)-1-(Naphthyl)ethylamin, (S)-(+)-1-(Naphthyl)ethylamin, Triethylamin, Diisopropylethylamin, Dicyclohexylamin, Benzylamin, Dibenzylamin, Morpholin, N-Methylmorpholin, Piperidin, N-Methylpiperidin und Pyridin ausgewählt ist, in einem Lösungsmittel, das aus N,N-Dimethylformamid, Chloroform, Hexanen, Aceton, Ethanol, Methanol, *iso*-Propanol, Diethylether, Dichlormethan, Benzol, Toluol, *n*-Pentan, *n*-Hexan, *n*-Heptan, Ethylacetat, Acetonitril, *tert.-*Butylmethylether, Tetrahydrofuran und 1,4-Dioxan ausgewählt ist, und Umkristallisation des so gebildeten Salzes unter Erhalt des angereicherten Diastereomers der Formel (25) 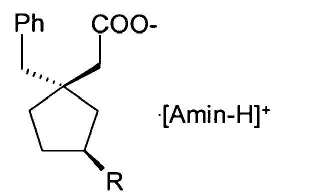 als Aminsalz; und
e) Zugabe des Produkts von Stufe d) zu einem Gemisch, das aus wässriger Salzsäure, wässriger Schwefelsäure, wässriger Essigsäure, in Essigsäure gelöster Salzsäure oder in Essigsäure und Wasser gelöster Salzsäure ausgewählt ist, und Rühren unter Erhalt der Carbonsäure der Formel (26) 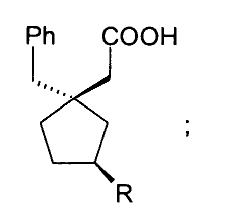 oder Verteilen des Produkts von Stufe d) zwischen einem Gemisch von wässriger Salzsäure und einem Lösungsmittel, das aus Chloroform, Dichlormethan, Ethylacetat, Ethylether, Tetrahydrofuran, 1,4-Dioxan, Toluol und *tert*.-Butylmethylether ausgewählt ist, sowie Trocknen und Eindampfen der organischen Schicht unter Erhalt der Carbonsäure der Formel (26)

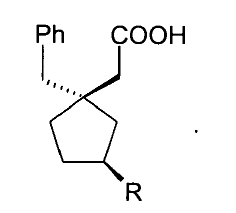

23. Verfahren nach Anspruch 21, umfassend
a) Zugabe von Cyanoacetat der Formel (A) 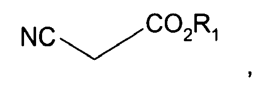 worin R₁ für Ethyl steht, zu einem Gemisch eines chiralen Cyclopentanons der Formel (21) 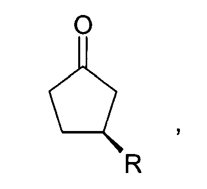 Toluol, Essigsäure und eines Katalysators für die Knoevenagel-Reaktion, der Ammoniumacetat darstellt, und Erhitzen des Gemischs unter Rückfluss an einem Dean-Stark-Abscheider unter Erhalt des Alkens der Formel (22) 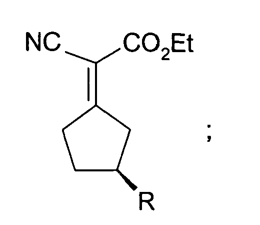
b) Zugabe des Produkts von Stufe a) zu einem Gemisch von Benzylmagnesiumchlorid in trockenem Tetrahydrofuran bei -100°C bis -20°C unter Erhalt der Additionsprodukte der Formeln 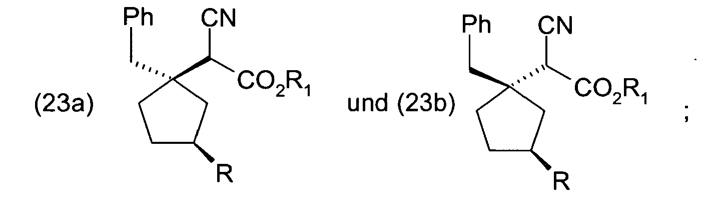
c) Zugabe der Produkte von Stufe b) zu einem Gemisch von Kaliumhydroxid in Ethylenglycol und Erhitzen des Gemischs bei 100°C bis 200°C sowie anschließendes Ansäuern unter Erhalt der Hydrolyseprodukte der Formeln 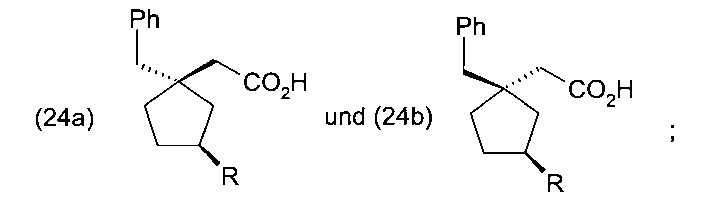
d) Inkontaktbringen der Produkte von Stufe c) mit (R)-α-Methylbenzylamin in Ethylacetat und Umkristallisation des so gebildeten Salzes aus Ethylacetat unter Erhalt des angereicherten Diastereomers der Formel (25) 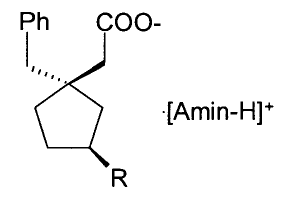 als (R)-α-Methylbenzylaminsalz; und
e) Zugabe des Produkts von Stufe d) zu wässriger Salzsäure und Rühren unter Erhalt der Carbonsäure der Formel (26)

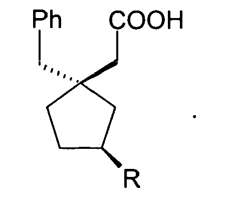

24. Verbindung der Formel (6) 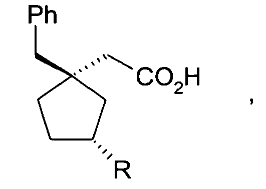 worin R für C₁-C₁₀-Alkyl oder C₃-C₁₀-Cycloalkyl steht, und ihre pharmazeutisch akzeptablen Salze.

25. Verbindung nach Anspruch 24, worin R für C₁-C₁₀-Alkyl steht.

26. Verbindung nach Anspruch 24, worin R aus Methyl, Ethyl und *n*-Propyl ausgewählt ist.

27. Verbindung nach Anspruch 24, die ((1S,3R)-1-Benzyl-3-methyl-cyclopentyl)-essigsäure darstellt.

28. Verbindung der Formel (26) 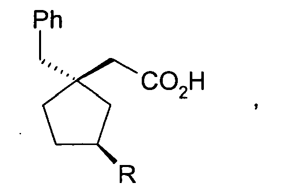 worin R für C₁-C₁ₒ-Alkyl oder C₃-C₁₀-Cycloalkyl steht, und ihre pharmazeutisch akzeptablen Salze.

29. Verbindung nach Anspruch 28, worin R für C₁-C₁₀-Alkyl steht.

30. Verbindung nach Anspruch 28, worin R aus Methyl, Ethyl und n-Propyl ausgewählt ist.

31. Verbindung nach Anspruch 28, die ((1R,3S)-1-Benzyl-3-methyl-cyclopentyl)-essigsäure darstellt.

32. Verbindung der Formel (6) 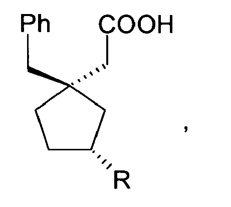 worin R für C₁-C₁₀-Alkyl oder C₃-C₁₀-Cycloalkyl steht, und ihre pharmazeutisch akzeptablen Salze, erhalten nach dem Verfahren gemäß Anspruch 18.

33. Verbindung der Formel (6) 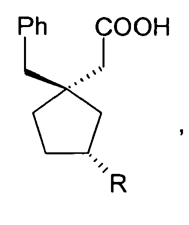 worin R für C₁-C₁₀-Alkyl oder C₃-C₁₀-Cycloalkyl steht, und ihre pharmazeutisch akzeptablen Salze, erhalten nach dem Verfahren gemäß Anspruch 19.

34. Verbindung der Formel (6) 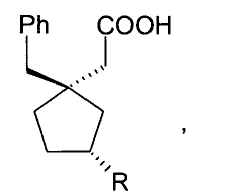 worin R für C₁-C₁₀-Alkyl oder C₃-C₁₀-Cycloalkyl steht, und ihre pharmazeutisch akzeptablen Salze, erhalten nach dem Verfahren gemäß Anspruch 20.

35. Verbindung der Forme! (26) 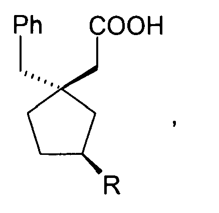 worin R für C₁-C₁₀-Alkyl oder C₃-C₁₀-Cycloalkyl steht, und ihre pharmazeutisch akzeptablen Salze, erhalten nach dem Verfahren gemäß Anspruch 21.

36. Verbindung der Formel (26) 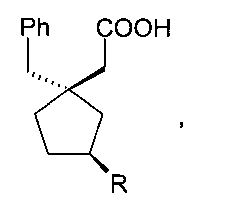 worin R für C₁-C₁₀-Alkyl oder C₃-C₁₀-Cycloalkyl steht, und ihre pharmazeutisch akzeptablen Salze, erhalten nach dem Verfahren gemäß Anspruch 22.

37. Verbindung der Formel (26) 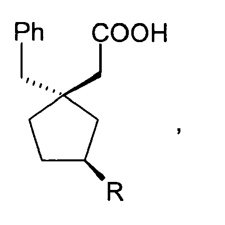 worin R für C₁-C₁₀-Alkyl oder C₃-C₁₀-Cycloalkyl steht, und ihre pharmazeutisch akzeptablen Salze, erhalten nach dem Verfahren gemäß Anspruch 23.

38. Verbindung, ausgewählt aus:
E-Cyano-((R)-3-methyl-cyclopentyliden)-essigsäureethylester;
Z-Cyano-((R)-3-methyl-cyclopentyliden)-essigsäureethylester;
(R)-((1S,3R)-1-Benzyl-3-methyl-cyclopentyl)-cyano-essigsäureethylester ;
(S)-((1S,3R)-1-Benzyl-3-methyl-cyclopentyl)-cyano-essigsäureethylester;
(R)-((1R,3R)-1-Benzyl-3-methyl-cyclopentyl)-cyano-essigsäureethylester;
(S)-((1R,3R)-1-Benzyl-3-methyl-cyclopentyl)-cyano-essigsäureethylester;
((1S,3R)-1-Isocyanatomethyl-3-methyl-cyclopentylmethyl)-benzol;
((1S,3R)-1-Benzyl-3-methyl-cyclopentylmethyl)-carbamidsäuremethylester;
[(1S,3R)-1-(Methoxycarbonylamino-methyl)-3-methyl-cyclopentyl]-essigsäure;
((1S,3R)-1-Benzyl-3-methyl-cyclopentyl)-essigsäuremethylester;
(1S,3R)-1-Methoxycarbonylmethyl-3-methyl-cyclopentyl)-essigsäure;
((1R,3R)-1-Isocyanatomethyl-3-methyl-cyclopentyl)-essigsäuremethylester;
[(1R,3R)-1-(Methoxycarbonylamino-methyl)-3-methyl-cyclopentyl]-essigsäuremethylester;
((1S,3R)-1-Benzyl-3-methyl-cyclopentyl)-essigsäure-*tert*.-butylester;
[(1S,3R)-1-Carboxymethyl-3-methyl-cyclopentyl)-essigsäure-*tert*.-butylester;
[(1S,3R)-1-Methoxycarbonylmethyl-3-methyl-cyclopentyl)-essigsäure-*tert*.-butylester;
((1R,3R)-1-Methoxycarbonylmethyl-3-methyl-cyclopentyl)-essigsäure;
((1S,3R)-1-Isocyanatomethyl-3-methyl-cyclopentyl)-essigsäuremethylester und
[(1S,3R)-1-(Methoxycarbonylamino-methyl)-3-methyl-cyclopentyl]-essigsäuremethylester.

39. Verfahren zur Herstellung einer Verbindung der Formel (4a) 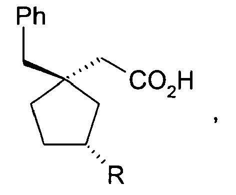 worin R für C₁-C₁₀-Alkyl oder C₃-C₁₀-Cycloalkyl steht, umfassend Hydrolyse einer Verbindung der Formel (3a) 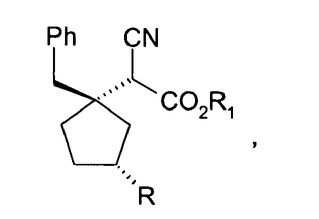 worin R₁ für Wasserstoff, Alkyl oder Benzyl steht.

40. Verfahren zur Herstellung einer Verbindung der Formel (24a) 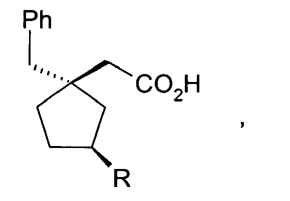 worin R für C₁-C₁₀-Alkyl oder C₃-C₁₀-Cycloalkyl steht, umfassend Hydrolyse einer Verbindung der Formel (23a) 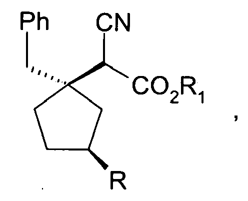 worin R₁ für Wasserstoff, Alkyl oder Benzyl steht.

41. Verfahren zur Herstellung einer Verbindung der Formel (6) 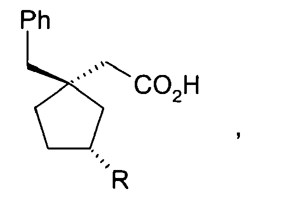 worin R für C₁-C₁₀-Alkyl oder C₃-C₁₀-Cycloalkyl steht, umfassend Auftrennung eines Gemischs, das die Verbindungen der Formeln 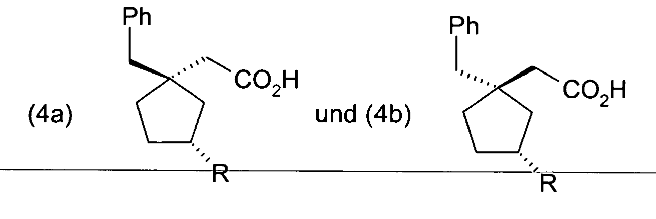 enthält, worin R für C₁-C₁₀-Alkyl oder C₃-C₁₀-Cycloalkyl steht.

42. Verfahren zur Herstellung einer Verbindung der Formel (26) 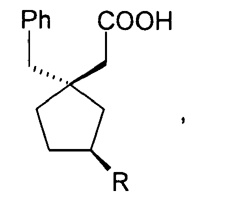 worin R für C₁-C₁₀-Alkyl oder C₃-C₁₀-Cycloalkyl steht, umfassend Auftrennung eines Gemischs, das die Verbindungen der Formeln 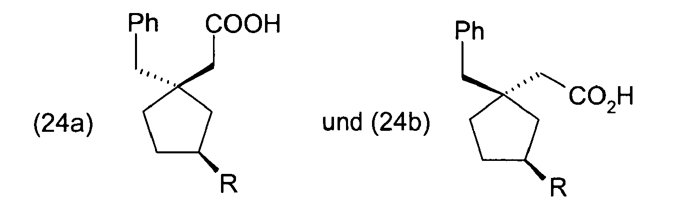 enthält, worin R für C₁-C₁₀-Alkyl oder C₃-C₁₀-Cycloalkyl steht.

43. Verfahren zur Herstellung einer Verbindung der Formel I 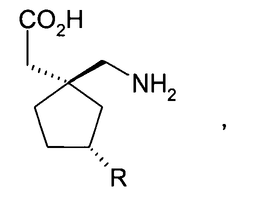 worin R für C₁-C₁₀-Alkyl oder C₃-C₁₀-Cycloalkyl steht, und ihrer pharmazeutisch akzeptablen Salze, umfassend Hydrolyse einer Verbindung der Formel (41) 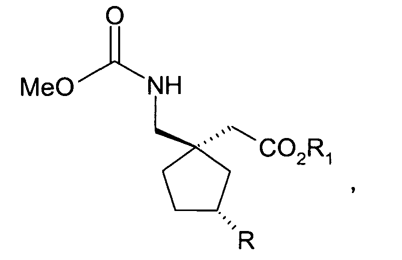 worin R₁ für Wasserstoff, Alkyl oder Benzyl steht, und gegebenenfalls Inkontaktbringen des erhaltenen Produkts mit einer Säure oder einer Base.

44. Verfahren zur Herstellung einer Verbindung der Formel II 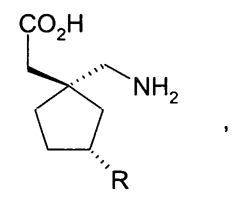 worin R für C₁-C₁₀-Alkyl oder C₃-C₁₀-Cycloalkyl steht, und ihrer pharmazeutisch akzeptablen Salze, umfassend Hydrolyse einer Verbindung der Formel (42) 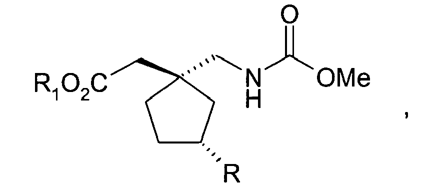 worin R₁ für Wasserstoff, Alkyl oder Benzyl steht, und gegebenenfalls Inkontaktbringen des erhaltenen Produkts mit einer Säure oder einer Base.

45. Verfahren zur Herstellung einer Verbindung der Formel III 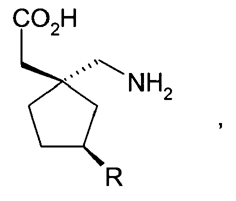 worin R für C₁-C₁₀-Alkyl oder C₃-C₁₀-Cycloalkyl steht, und ihrer pharmazeutisch akzeptablen Salze, umfassend Hydrolyse einer Verbindung der Formel (43) 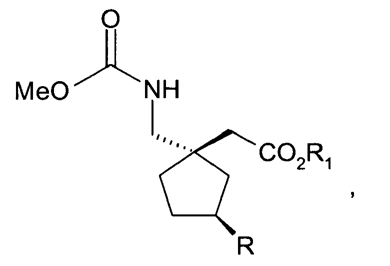 worin R₁ für Wasserstoff, Alkyl oder Benzyl steht, und gegebenenfalls Inkontaktbringen des erhaltenen Produkts mit einer Säure oder einer Base.

46. Verfahren zur Herstellung einer Verbindung der Formel IV 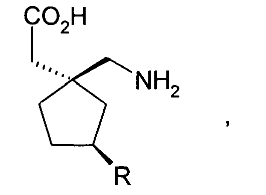 worin R für C₁-C₁₀-Alkyl oder C₃-C₁₀-Cycloalkyl steht, und ihrer pharmazeutisch akzeptablen Salze, umfassend Hydrolyse einer Verbindung der Formel (44) 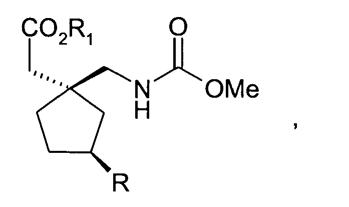 worin R₁ für Wasserstoff, Alkyl oder Benzyl steht, und gegebenenfalls Inkontaktbringen des erhaltenen Produkts mit einer Säure oder einer Base.

## Revendications

1. Procédé pour la préparation d'un composé répondant à la formule I 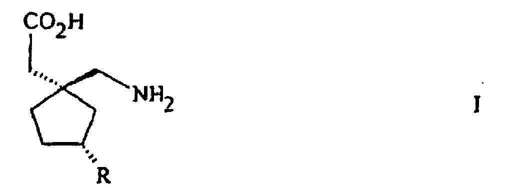 dans laquelle R représente un groupe alkyle en C₁-C₁₀ ou un groupe cycloalkyle en C₃-C₁₀, et de sels pharmaceutiquement acceptables de celui-ci, qui comprend les étapes :
a) ajouter un cyanoacétate répondant à la formule (A) 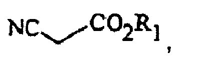 dans laquelle R₁ représente un groupe alkyle ou un groupe benzyle, à un mélange d'une cyclopentanone chirale répondant à la formule (1) 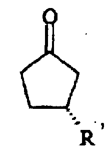 d'un solvant, d'un acide carboxylique, et d'un catalyseur de la réaction de Knoevenagel, et agiter le mélange en présence d'un moyen pour éliminer l'eau, afin de produire l'alcène répondant à la formule (2) 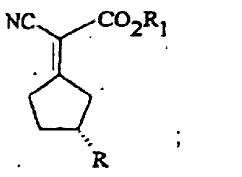
b) ajouter le produit de l'étape a) ci-dessus à un mélange de chlorure de benzylmagnésium, de bromure de benzylmagnésium, ou d'iodure de benzylmagnésium, dans un solvant pour produire les produits d'addition répondant aux formules (3a) 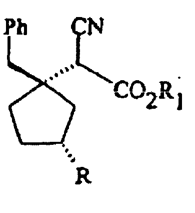 et (3b) 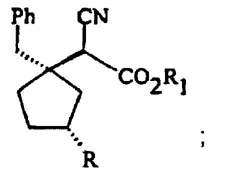
c) ajouter les produits de l'étape b) ci-dessus à un mélange d'une base choisie parmi l'hydroxyde de potassium, l'hydroxyde de sodium, l'hydroxyde de lithium, et l'hydroxyde de césium, dans un solvant, et agiter, puis acidifier pour produire les acides carboxyliques répondant aux formules (4a) 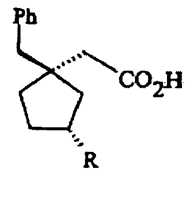 et (4b) 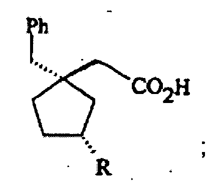 ou
ajouter les produits de l'étape b) ci-dessus à un mélange acide, et agiter pour produire les acides carboxyliques répondant aux formules (4a) 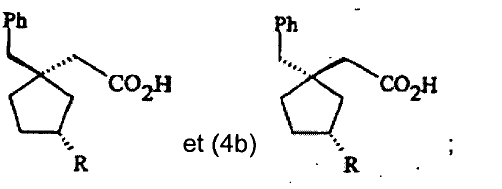
d) mettre en contact les produits de l'étape c) ci-dessus avec une amine dans un solvant, et recristalliser le sel ainsi formé pour produire le diastéréoisomère enrichi répondant à la formule (5) 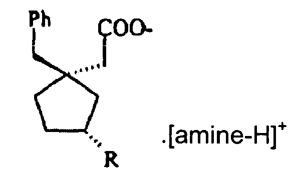 en tant que sel d'amine ;
e) convertir le produit de l'étape d) en acide carboxylique répondant à la formule (6) 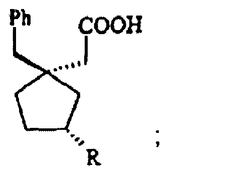
f) ajouter le produit de l'étape e) à un mélange d'iodométhane, d'un solvant, et d'une base, et agiter pour produire l'ester répondant à la formule (7) 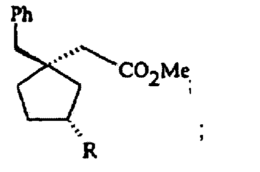 ou ajouter le produit de l'étape e) à du méthanol et un acide pour produire l'ester répondant à la formule (7) 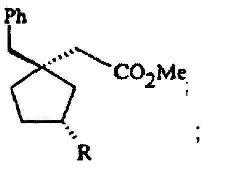 ou ajouter le produit de l'étape e) ci-dessus à du triméthylsilyldiazométhane et du méthanol dans un solvant pour produire l'ester répondant à la formule (7) 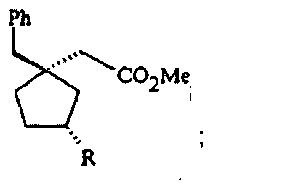 ou ajouter le produit de l'étape e) à une solution de diazométhane ou de triméthylsilyldiazométhane dans un solvant pour produire l'ester répondant à la formule (7) 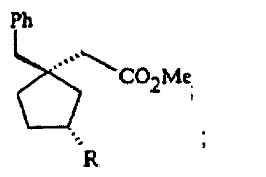
g) ajouter le produit de l'étape f) à un mélange de tétrachlorure de carbone ou d'acétate d'éthyle, et d'acétonitrile, d'eau, de périodate de sodium, et de chlorure de ruthénium (III), et agiter pour produire l'acide carboxylique répondant à la formule (8) 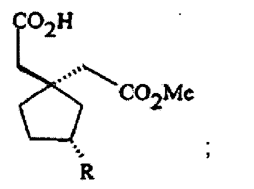
h) ajouter le produit de l'étape g) à un mélange d'une amine tertiaire, d'un solvant, et d'azide de diphénylphosphoryle (DPPA), et agiter pour produire l'isocyanate répondant à la formule (9) 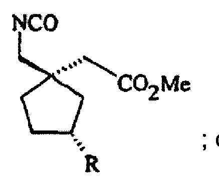 ou ajouter le produit de l'étape g) ci-dessus à du chloroformiate d'éthyle ou du chloroformiate d'isobutyle et une base dans un solvant à une température de -40 °C à 78 °C, puis ajouter une solution d'azide de sodium dans de l'eau et de tétrahydrofurane ou d'acétone, puis ajouter du toluène ou du benzène, et porter au reflux pour produire l'isocyanate répondant à la formule (9) 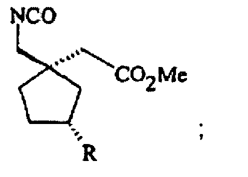
i) ajouter le produit de l'étape h) à un mélange d'un solvant et de méthanol, et agiter pour produire le carbamate répondant à la formule (10) 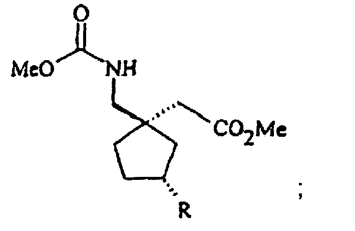
j) ajouter le produit de l'étape i) à un mélange d'un solvant et d'acide chlorhydrique aqueux, et agiter pour produire un composé répondant à la formule (Ia) 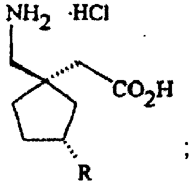 et
k) convertir le produit de l'étape j) en composé répondant à la formule
(I) 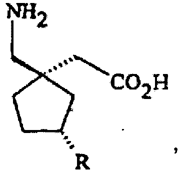 et en outre convertir, si souhaité, en sel pharmaceutiquement acceptable par des moyens connus.

2. Procédé selon la revendication 1, qui comprend les étapes:
a) ajouter un cyanoacétate répondant à la formule (A) 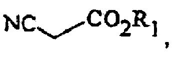 dans laquelle R₁ est choisi parmi les groupes méthyle, éthyle, n-propyle, isopropyle, n-butyle, isobutyle, sec-butyle, tert-butyle, et benzyle, à un mélange d'une cyclopentanone chirale répondant à la formule (1) 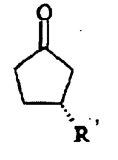 d'un solvant choisi parmi le tétrahydrofurane, le 1,4-dioxane, l'éther tert-butylméthylique, le chloroforme, le dichlorométhane, l'acétonitrile, l'éther éthylique, l'acétate d'éthyle, les hexanes, le N,N-diméthylformamide, le diméthylsulfoxyde, l'éthanol, le tert-butanol, le toluène, le benzène, les xylènes, et le n-heptane, d'acide acétique, et d'un catalyseur de la réaction de Knoevenagel choisi parmi la β-alanine, l'acétate d'ammonium, et la pipéridine, et agiter le mélange en présence d'un moyen pour éliminer l'eau choisi parmi la distillation azéotropique, les tamis moléculaires activés, le sulfate de magnésium anhydre, le sulfate de sodium anhydre, le carbonate de sodium anhydre, le carbonate de potassium anhydre, le carbonate de césium anhydre, l'orthoformiate de triméthyle, et l'orthoformiate de triéthyle, afin de produire l'alcène répondant à la formule (2) 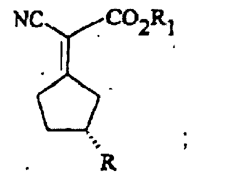
b) ajouter le produit de l'étape a) ci-dessus à un mélange de chlorure de benzylmagnésium, de bromure de benzylmagnésium, ou d'iodure de benzylmagnésium, dans un solvant choisi parmi le tétrahydrofurane, le benzène, le 1,4-dioxane, les hexanes, le n-heptane, le toluène, l'éther diéthylique, et l'éther tert-butylméthylique pour produire les produits d'addition répondant aux formules (3a) 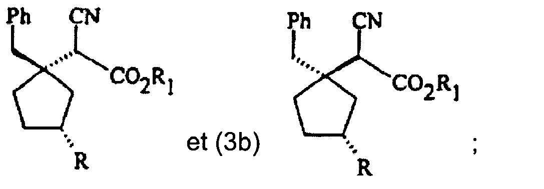
c) ajouter les produits de l'étape b) ci-dessus à un mélange d'une base choisie parmi l'hydroxyde de potassium, l'hydroxyde de sodium, l'hydroxyde de lithium, et l'hydroxyde de césium, dans un solvant choisi parmi l'éthylène glycol, l'éther 2-méthoxyéthylique, le 1,4-dioxane, et le diéthylène glycol, et agiter le mélange, puis l'acidifier pour produire les acides carboxyliques répondant aux formules (4a) 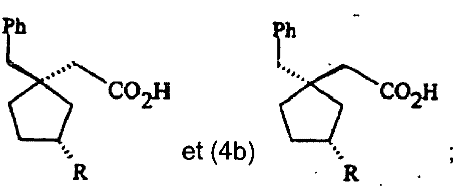 ou
ajouter les produits de l'étape b) ci-dessus à un mélange acide choisi parmi de l'HCl à 6 à 12 M, de l'H₂SO₄ à 12 M, 10 % à 48 % poids/poids d'acide bromhydrique, et de l'HBr dans de l'acide acétique aqueux, et agiter pour produire les acides carboxyliques répondant aux formules (4a) 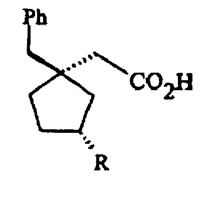 et (4b) 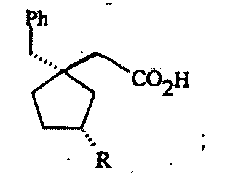
d) mettre en contact les produits de l'étape c) ci-dessus avec une amine choisie parmi la (S)-α-méthyl-benzylamine, la (R)-α-méthyl-benzylamine, la (R)-(+)-1-(naphtyl)éthylamine, la (S)-(+)-1-(naphtyl)éthylamine, la triéthylamine, la diisopropyléthylamine, la dicylohexylamine, la benzylamine, la dibenzylamine, la morpholine, la N-méthylmorpholine, la pipéridine, la N-méthylpipéridine, et la pyridine dans un solvant choisi parmi le N,N-diméthylformamide, le chloroforme, le benzène, les xylènes, les hexanes, l'acétone, l'éthanol, le méthanol, l'isopropanol, l'éther diéthylique, le dichlorométhane, le benzène, le toluène, le n-pentane, le n-hexane, le n-heptane, l'acétate d'éthyle, l'acétonitrile, l'éther tert-butylméthylique, le tétrahydrofurane, et le 1,4-dioxane, et recristalliser le sel ainsi formé pour produire le diastéréoisomère enrichi répondant à la formule (5) 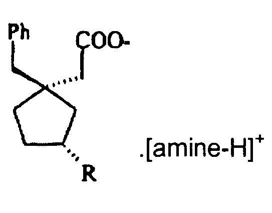 en tant que sel d'amine ;
e) ajouter le produit de l'étape d) à un mélange choisi parmi l'acide chlorhydrique aqueux, l'acide sulfurique aqueux, l'acide acétique aqueux, l'acide chlorhydrique dissous dans de l'acide acétique, ou l'acide chlorhydrique dissous dans de l'acide acétique auquel de l'eau est ajoutée, et agiter pour produire l'acide carboxylique répondant à la formule (6) 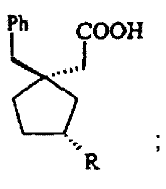 ou diviser le produit de l'étape d) entre un mélange d'acide chlorhydrique aqueux et d'un solvant choisi parmi le chloroforme, le dichlorométhane, l'acétate d'éthyle, l'éther éthylique, le tétrahydrofurane, le 1,4-dioxane, le toluène, et l'éther tert-butylméthylique, et sécher et évaporer la phase organique pour produire l'acide carboxylique répondant à la formule (6) 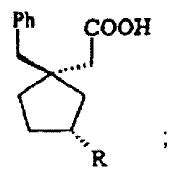
f) ajouter le produit de l'étape e) ci-dessus à un mélange d'iodométhane, d'un solvant choisi parmi le dichlorométhane, le chloroforme, le tétrahydrofurane, le toluène, et le 1,4-dioxane, et d'une base choisie parmi le 1,8-diazabicyclo[5.4.0]undéc-7-ène (DBU), la diisopropyléthylamine, la triéthylamine, et le 1,5-diazabicyclo[4.3.0]non-5-ène (DBN), et agiter à une température de -40 °C à 110°C pour produire l'ester répondant à la formule (7) 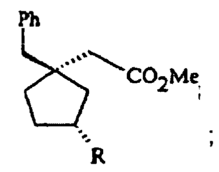 ou ajouter le produit de l'étape e) ci-dessus à un mélange de méthanol et d'acide sulfurique concentré, d'acide chlorhydrique concentré, ou de chlorure d'hydrogène à une température de 0 °C à 100 °C pour produire l'ester répondant à la formule (7) 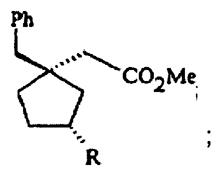 ou ajouter le produit de l'étape e) ci-dessus à du triméthylsilyldiazométhane et du méthanol dans du benzène ou du toluène à une température de -40 °C à 100 °C pour produire l'ester répondant à la formule (7) 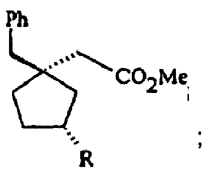 ou ajouter le produit de l'étape e) ci-dessus à du diazométhane ou du triméthylsilyldiazométhane dans un solvant choisi parmi le benzène, le toluène, le dichlorométhane, et l'éther diéthylique à une température de -40 °C à 40 °C pour produire un composé répondant à la formule (7) 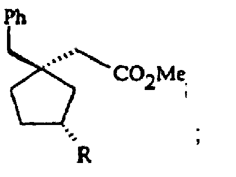
g) ajouter le produit de l'étape f) à un mélange de tétrachlorure de carbone ou d'acétate d'éthyle, et d'acétonitrile, d'eau, de périodate de sodium, et de chlorure de ruthénium (III), et agiter à une température de -40 °C à 80 °C pour produire l'acide carboxylique répondant à la formule (8) 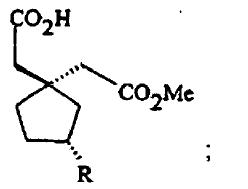
h) ajouter le produit de l'étape g) ci-dessus à un mélange d'une base choisie parmi la triéthylamine et la diisopropyléthylamine, d'un solvant choisi parmi le toluène, le benzène, les xylènes, le tétrahydrofurane, l'éther diéthylique et le n-heptane, et d'azide de diphénylphosphoryle (DPPA), et agiter à une température de 0 °C à 150 °C pour produire l'isocyanate répondant à la formule (9) 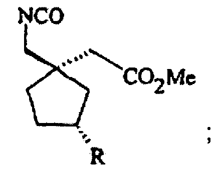 ou ajouter le produit de l'étape g) ci-dessus à du chloroformiate d'éthyle ou du chloroformiate d'isobutyle, une base choisie parmi la triéthylamine et la diisopropyléthylamine, et un solvant choisi parmi le tétrahydrofurane, l'acétone, et l'éther diéthylique à une température de -40 °C à 78 °C, puis ajouter une solution d'azide de sodium dans de l'eau et de tétrahydrofurane ou d'acétone, puis ajouter du toluène ou du benzène, et porter au reflux pour produire l'isocyanate répondant à la formule (9) 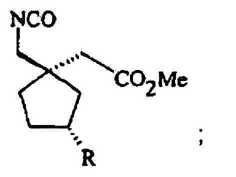
i) ajouter le produit de l'étape h) à un mélange d'un solvant choisi parmi le toluène, le benzène, les xylènes et le n-heptane, et de méthanol, et agiter à une température de 0 °C à 150 °C pour produire le carbamate répondant à la formule (10) 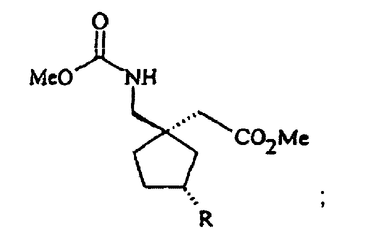
j) ajouter le produit de l'étape i) à un mélange d'un solvant choisi parmi l'eau, l'acide acétique, et le 1,4-dioxane, et d'acide chlorhydrique aqueux à une concentration de 0,01 M à 12 M, et agiter à une température de 0 °C à 115 °C pour produire un composé répondant à la formule (Ia) 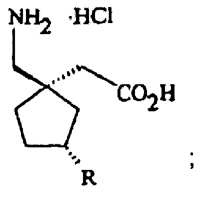 et
k) convertir le produit de l'étape j) en composé répondant à la formule
(I) 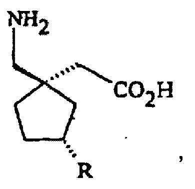 et en outre convertir, si souhaité, en sel pharmaceutiquement acceptable par des moyens connus.

3. Procédé selon la revendication 1, qui comprend les étapes :
a) ajouter un cyanoacétate répondant à la formule (A) 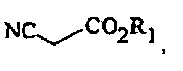 dans laquelle R₁ représente un groupe éthyle, à un mélange d'une cyclopentanone chirale répondant à la formule (1) 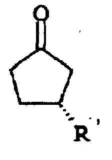 de toluène, d'acide acétique, et d'un catalyseur de la réaction de Knoevenagel qui est l'acétate d'ammonium, et chauffer le mélange au reflux sur un piège Dean-Stark, pour produire l'alcène répondant à la formule (2) 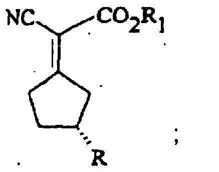
b) ajouter le produit de l'étape a) ci-dessus à un mélange de chlorure de benzylmagnésium dans du tétrahydrofurane anhydre à une température de -100 °C à 25 °C pour produire les produits d'addition répondant aux formules (3a) 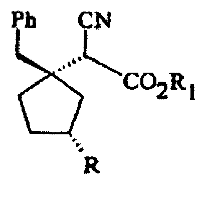 et (3b) 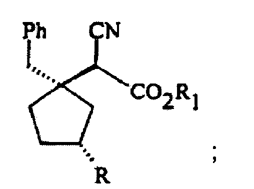
c) ajouter les produits de l'étape b) ci-dessus à un mélange d'hydroxyde de potassium dans de l'éthylène glycol, et chauffer le mélange à une température de 100 °C à 200 °C, puis l'acidifier pour produire les produits d'hydrolyse répondant aux formules (4a) 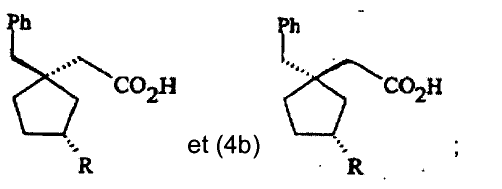
d) mettre en contact les produits de l'étape c) ci-dessus avec de la (S)-α-méthyl-benzylamine dans de l'acétate d'éthyle, et recristalliser le sel ainsi formé à partir d'acétate d'éthyle pour produire le diastéréoisomère enrichi répondant à la formule (5) 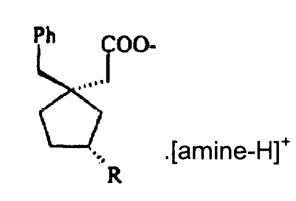 en tant que sel de (S)-α-méthyl-benzylamine ;
e) ajouter le produit de l'étape d) à de l'acide chlorhydrique aqueux, et agiter pour produire l'acide carboxylique répondant à la formule (6) 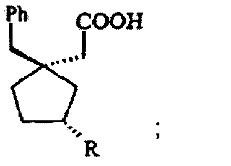
f) ajouter le produit de l'étape e) à un mélange d'iodométhane, de dichlorométhane, et de 1,8-diazabicyclo[5.4.0]undéc-7-ène (DBU), et agiter pour produire l'ester répondant à la formule (7) 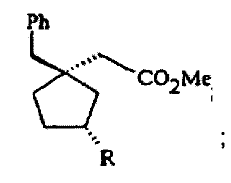 ou
ajouter le produit de l'étape e) à du méthanol et de l'acide sulfurique concentré, pour produire l'ester répondant à la formule (7) 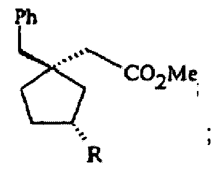 ou
ajouter le produit de l'étape e) à une solution de diazométhane ou de triméthylsilyldiazométhane dans du dichlorométhane , pour produire l'ester répondant à la formule (7) 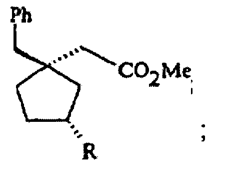
g) ajouter le produit de l'étape f) à un mélange de tétrachlorure de carbone ou d'acétate d'éthyle, et d'acétonitrile, d'eau, de périodate de sodium, et de chlorure de ruthénium (III), et agiter pour produire l'acide carboxylique répondant à la formule (8) 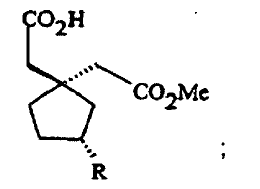
h) ajouter le produit de l'étape g) à un mélange de triéthylamine, de toluène, et d'azide de diphénylphosphoryle (DPPA), et porter au reflux pour produire l'isocyanate répondant à la formule (9) 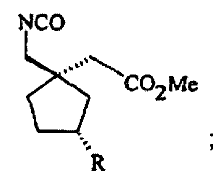 ou
ajouter le produit de l'étape g) ci-dessus à du chloroformiate d'éthyle ou du chloroformiate d'isobutyle et de la triéthylamine dans du tétrahydrofurane à une température de -40 °C à 78 °C, puis ajouter une solution d'azide de sodium dans de l'eau et de tétrahydrofurane, puis ajouter du toluène ou du benzène, et porter au reflux pour produire l'ester répondant à la formule (9) 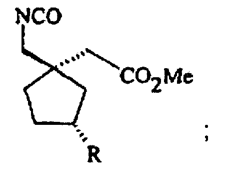
i) ajouter le produit de l'étape h) à un mélange de méthanol et de toluène, et porter au reflux pour produire le carbamate répondant à la formule (10) 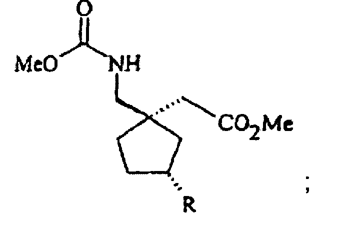
j) ajouter le produit de l'étape i) à un mélange de 1,4-dioxane et d'acide chlorhydrique aqueux à une concentration de 6 M, et agiter pour produire un composé répondant à la formule (Ia) 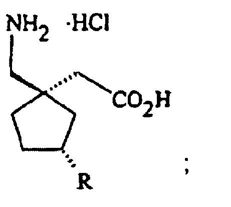
k) convertir le produit de l'étape j) en composé répondant à la formule (I) 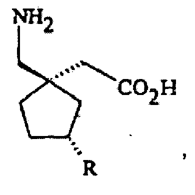 et en outre convertir, si souhaité, en sel pharmaceutiquement acceptable par des moyens connus.

4. Procédé selon la revendication 1, **caractérisé en outre en ce que** le produit intermédiaire (9) 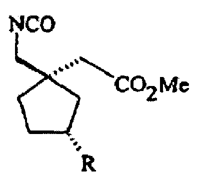 formé est mis à réagir, sans isolement, avec du méthanol pour produire le carbamate répondant à la formule (10)

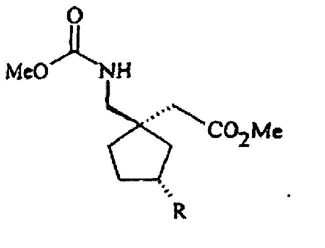

5. Procédé pour la préparation d'un composé répondant à la formule II 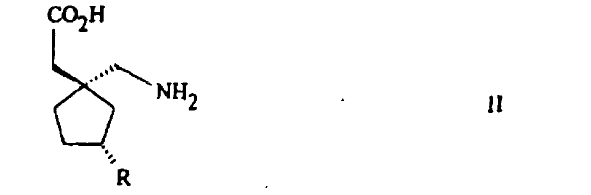 dans laquelle R représente un groupe alkyle en C₁-C₁₀ ou un groupe cycloalkyle en C₃-C₁₀, et de sels pharmaceutiquement acceptables de celui-ci, qui comprend les étapes :
a) ajouter un cyanoacétate répondant à la formule (A) 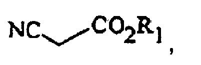 dans laquelle R₁ représente un groupe alkyle ou un groupe benzyle, à un mélange d'une cyclopentanone chirale répondant à la formule (1) 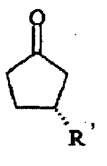 d'un solvant, d'un acide carboxylique, et d'un catalyseur de la réaction de Knoevenagel, et agiter le mélange en présence d'un moyen pour éliminer l'eau, afin de produire l'alcène répondant à la formule (2) 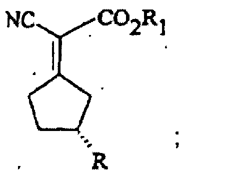
b) ajouter le produit de l'étape a) ci-dessus à un mélange de chlorure de benzylmagnésium, de bromure de benzylmagnésium, ou d'iodure de benzylmagnésium, dans un solvant pour produire les produits d'addition répondant aux formules (3a) 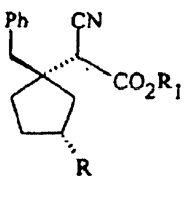 et (3b) 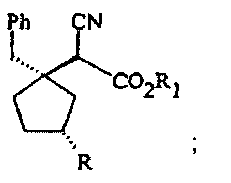
c) ajouter les produits de l'étape b) ci-dessus à un mélange d'une base choisie parmi l'hydroxyde de potassium, l'hydroxyde de sodium, l'hydroxyde de lithium, et l'hydroxyde de césium, et d'un solvant, et agiter, puis acidifier pour produire les acides carboxyliques répondant aux formules (4a) 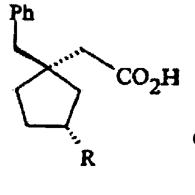 et (4b) 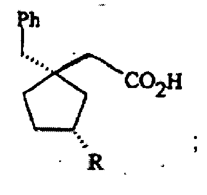 ou
ajouter les produits de l'étape b) ci-dessus à un mélange acide, et agiter pour produire les acides carboxyliques répondant aux formules (4a) 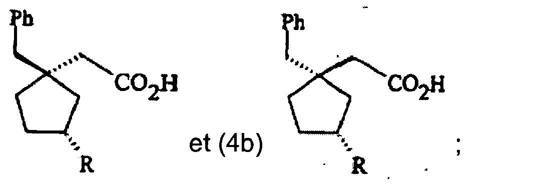
d) mettre en contact les produits de l'étape c) ci-dessus avec une amine dans un solvant, et recristalliser le sel ainsi formé pour produire le diastéréoisomère enrichi répondant à la formule (5) 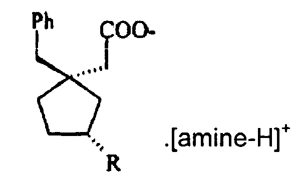 en tant que sel d'amine ; et
e) convertir le produit de l'étape d) en acide carboxylique répondant à la formule (6) 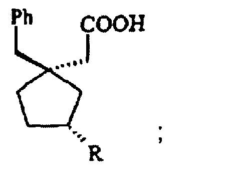
f) ajouter le produit de l'étape e) à un mélange d'une amine tertiaire, d'un solvant, et d'azide de diphénylphosphoryle (DPPA), et agiter pour produire l'isocyanate répondant à la formule (11) 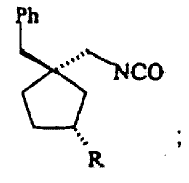 ou ajouter le produit de l'étape e) ci-dessus à du chloroformiate d'éthyle ou du chloroformiate d'isobutyle et une base dans un solvant à une température de -40 °C à 78 °C, puis ajouter une solution d'azide de sodium dans de l'eau et de tétrahydrofurane ou d'acétone, puis ajouter du toluène ou du benzène, et porter au reflux pour produire l'isocyanate répondant à la formule (11) 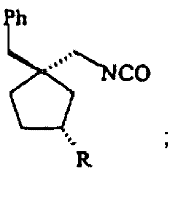
g) ajouter le produit de l'étape f) à un mélange d'un solvant et de méthanol, et agiter pour produire le carbamate répondant à la formule (12) 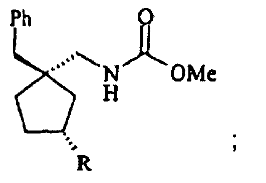
h) ajouter le produit de l'étape g) à un mélange de tétrachlorure de carbone ou d'acétate d'éthyle, et d'acétonitrile, d'eau, de périodate de sodium, et de chlorure de ruthénium (III), et agiter pour produire l'acide carboxylique répondant à la formule (13) 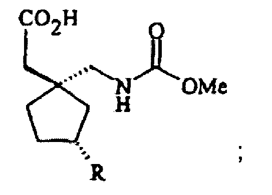
i) ajouter le produit de l'étape h) à un mélange d'un solvant et d'acide chlorhydrique aqueux, et agiter pour produire un composé répondant à la formule (IIa) 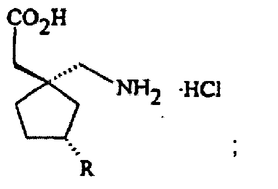
j) convertir le produit de l'étape i) en composé répondant à la formule (II) 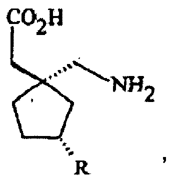 et en outre convertir, si souhaité, en sel pharmaceutiquement acceptable par des moyens connus.

6. Procédé selon la revendication 5, qui comprend les étapes :
a) ajouter un cyanoacétate répondant à la formule (A) 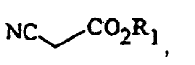 dans laquelle R₁ est choisi parmi les groupes méthyle, éthyle, n-propyle, isopropyle, n-butyle, isobutyle, sec-butyle, tert-butyle, et benzyle, à un mélange d'une cyclopentanone chirale répondant à la formule (1) 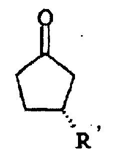 d'un solvant choisi parmi le tétrahydrofurane, le 1,4-dioxane, l'éther tert-butylméthylique, le chloroforme, le dichlorométhane, l'acétonitrile, l'éther éthylique, l'acétate d'éthyle, les hexanes, le N,N-diméthylformamide, le diméthylsulfoxyde, l'éthanol, le tert-butanol, le toluène, le benzène, les xylènes, et le n-heptane, d'acide acétique, et d'un catalyseur de la réaction de Knoevenagel choisi parmi la β-alanine, l'acétate d'ammonium, et la pipéridine, et agiter le mélange en présence d'un moyen pour éliminer l'eau choisi parmi la distillation azéotropique, les tamis moléculaires activés, le sulfate de magnésium anhydre, le sulfate de sodium anhydre, le carbonate de sodium anhydre, le carbonate de potassium anhydre, le carbonate de césium anhydre, l'orthoformiate de triméthyle, et l'orthoformiate de triéthyle, afin de produire l'alcène répondant à la formule (2) 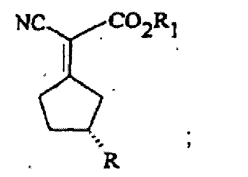
b) ajouter le produit de l'étape a) ci-dessus à un mélange de chlorure de benzylmagnésium, de bromure de benzylmagnésium, ou d'iodure de benzylmagnésium, dans un solvant choisi parmi le tétrahydrofurane, le benzène, le 1,4-dioxane, les hexanes, le n-heptane, le toluène, l'éther diéthylique, et l'éther tert-butylméthylique pour produire les produits d'addition répondant aux formules (3a) 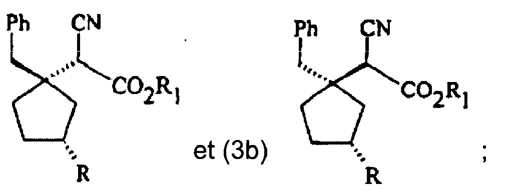
c) ajouter les produits de l'étape b) ci-dessus à un mélange d'une base choisie parmi l'hydroxyde de potassium, l'hydroxyde de sodium, l'hydroxyde de lithium, et l'hydroxyde de césium, dans un solvant choisi parmi l'éthylène glycol, l'éther 2-méthoxyéthylique, le 1,4-dioxane, et le diéthylène glycol, et agiter le mélange, puis l'acidifier pour produire les acides carboxyliques répondant aux formules (4a) 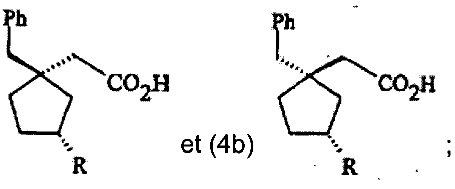 ou ajouter les produits de l'étape b) ci-dessus à un mélange acide choisi parmi de l'HCl à 6 à 12 M, de l'H₂SO₄ à 12 M, 10 % à 48 % poids/poids d'acide bromhydrique, et de l'HBr dans de l'acide acétique aqueux, et agiter pour produire les acides carboxyliques répondant aux formules (4a) 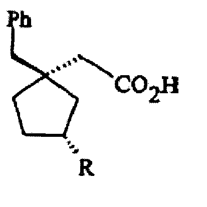 et (4b) 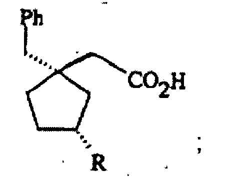
d) mettre en contact les produits de l'étape c) ci-dessus avec une amine choisie parmi la (S)-α-méthyl-benzylamine, la (R)-α-méthyl-benzylamine, la (R)-(+)-1-(naphtyl)éthylamine, la (S)-(+)-1-(naphtyl)éthylamine, la triéthylamine, la diisopropyléthylamine, la dicylohexylamine, la benzylamine, la dibenzylamine, la morpholine, la N-méthylmorpholine, la pipéridine, la N-méthylpipéridine, et la pyridine dans un solvant choisi parmi le N,N-diméthylformamide, le chloroforme, le benzène, les xylènes, les hexanes, l'acétone, l'éthanol, le méthanol, l'isopropanol, l'éther diéthylique, le dichlorométhane, le benzène, le toluène, le n-pentane, le n-hexane, le n-heptane, l'acétate d'éthyle, l'acétonitrile, l'éther tert-butylméthylique, le tétrahydrofurane, et le 1,4-dioxane, et recristalliser le sel ainsi formé pour produire le diastéréoisomère enrichi répondant à la formule (5) 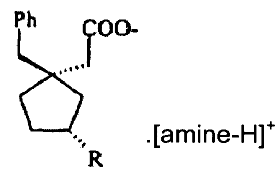 en tant que sel d'amine ;
e) ajouter le produit de l'étape d) à un mélange choisi parmi l'acide chlorhydrique aqueux, l'acide sulfurique aqueux, l'acide acétique aqueux, l'acide chlorhydrique dissous dans de l'acide acétique, et l'acide chlorhydrique dissous dans de l'acide acétique et de l'eau, et agiter pour produire l'acide carboxylique répondant à la formule (6) 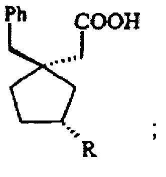 ou
diviser le produit de l'étape d) entre un mélange d'acide chlorhydrique aqueux et d'un solvant choisi parmi le chloroforme, le dichlorométhane, l'acétate d'éthyle, l'éther éthylique, le tétrahydrofurane, le 1,4-dioxane, le toluène, et l'éther tert-butylméthylique, et sécher et évaporer la phase organique pour produire l'acide carboxylique répondant à la formule (6) 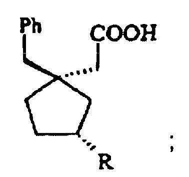
f) ajouter le produit de l'étape e) ci-dessus à un mélange d'une base choisie parmi la triéthylamine et la diisopropyléthylamine, d'un solvant choisi parmi le toluène, le benzène, les xylènes, le tétrahydrofurane, l'éther diéthylique et le n-heptane, et d'azide de diphénylphosphoryle (DPPA), et agiter à une température de 0 °C à 150 °C pour produire l'isocyanate répondant à la formule (11) 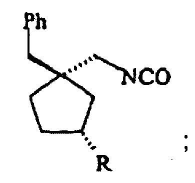 ou ajouter le produit de l'étape e) ci-dessus à du chloroformiate d'éthyle ou du chloroformiate d'isobutyle et une base choisie parmi la triéthylamine et la diisopropyléthylamine, et un solvant choisi parmi le tétrahydrofurane, l'acétone, et l'éther diéthylique à une température de -40 °C à 78 °C, puis ajouter une solution d'azide de sodium dans de l'eau et de tétrahydrofurane ou d'acétone, puis ajouter du toluène ou du benzène, et porter au reflux pour produire l'isocyanate répondant à la formule (11) 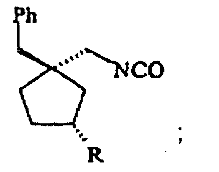
g) ajouter le produit de l'étape f) à un solvant choisi parmi le toluène, le benzène, les xylènes et le n-heptane, et du méthanol, et agiter à une température de 0 °C à 150 °C pour produire le carbamate répondant à la formule (12) 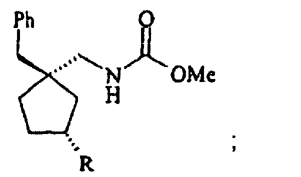
h) ajouter le produit de l'étape g) à un mélange de tétrachlorure de carbone ou d'acétate d'éthyle, et d'acétonitrile, d'eau, de périodate de sodium, et de chlorure de ruthénium (III), et agiter à une température de -40 °C à 80 °C pour produire l'acide carboxylique répondant à la formule (13) 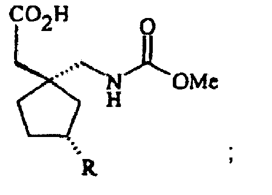
i) ajouter le produit de l'étape h) à un mélange d'un solvant choisi parmi l'eau, l'acide acétique, et le 1,4-dioxane, et d'acide chlorhydrique aqueux à une concentration de 0,01 M à 12 M, et agiter à une température de 0°C à 115°C pour produire un composé répondant à la formule (IIa) 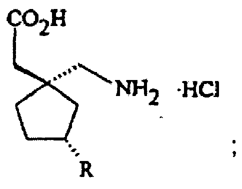 et
j) convertir le produit de l'étape i) en composé répondant à la formule
II 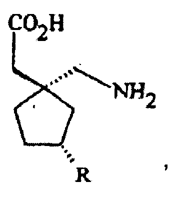 et en outre convertir, si souhaité, en sel pharmaceutiquement acceptable par des moyens connus.

7. Procédé selon la revendication 5, qui comprend les étapes :
a) ajouter un cyanoacétate répondant à la formule (A) 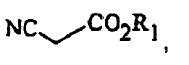 dans laquelle R₁ représente un groupe éthyle, à un mélange d'une cyclopentanone chirale répondant à la formule (1) 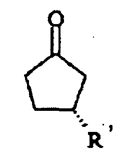 de toluène, d'acide acétique, et d'un catalyseur de la réaction de Knoevenagel qui est l'acétate d'ammonium, et chauffer le mélange au reflux sur un piège Dean-Stark, pour produire l'alcène répondant à la formule (2) 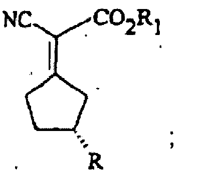
b) ajouter le produit de l'étape a) ci-dessus à un mélange de chlorure de benzylmagnésium dans du tétrahydrofurane anhydre à une température de -100 °C à 25 °C pour produire les produits d'addition répondant aux formules (3a) 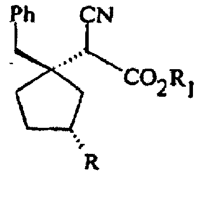 et (3b) 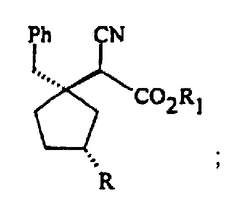
c) ajouter les produits de l'étape b) ci-dessus à un mélange d'hydroxyde de potassium dans de l'éthylène glycol, et chauffer le mélange à une température de 100 °C à 200 °C, puis l'acidifier pour produire les produits d'hydrolyse répondant aux formules (4a) 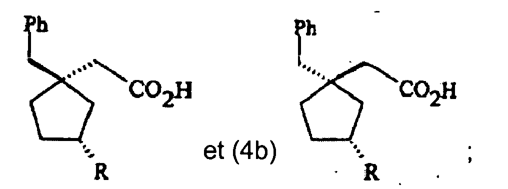
d) mettre en contact les produits de l'étape c) ci-dessus avec de la (S)-α-méthyl-benzylamine dans de l'acétate d'éthyle, et recristalliser le sel ainsi formé à partir d'acétate d'éthyle pour produire le diastéréoisomère enrichi répondant à la formule (5) 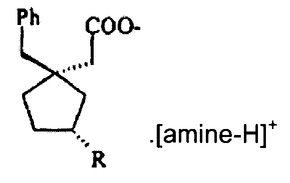 en tant que sel de (S)-α-méthyl-benzylamine ;
e) ajouter le produit de l'étape d) à de l'acide chlorhydrique aqueux, et agiter pour produire l'acide carboxylique répondant à la formule (6) 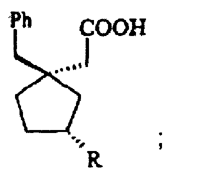
f) ajouter le produit de l'étape e) ci-dessus à un mélange de triéthylamine, de toluène, et d'azide de diphénylphosphoryle (DPPA), et porter au reflux pour produire l'isocyanate répondant à la formule (11) 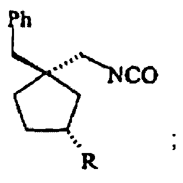 ou ajouter le produit de l'étape e) ci-dessus à du chloroformiate d'éthyle ou du chloroformiate d'isobutyle et de la triéthylamine dans du tétrahydrofurane à une température de -40 °C à 78 °C, puis ajouter une solution d'azide de sodium dans de l'eau et de tétrahydrofurane ou d'acétone, puis ajouter du toluène ou du benzène, et porter au reflux pour produire l'isocyanate répondant à la formule (11) 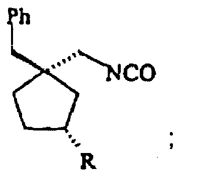
g) ajouter le produit de l'étape f) à un mélange de méthanol et de toluène, et porter au reflux pour produire le carbamate répondant à la formule (12) 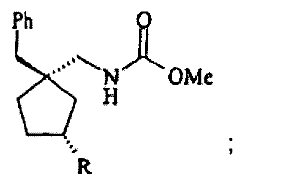
h) ajouter le produit de l'étape g) à un mélange de tétrachlorure de carbone ou d'acétate d'éthyle, et d'acétonitrile, d'eau, de périodate de sodium, et de chlorure de ruthénium (III), et agiter pour produire l'acide carboxylique répondant à la formule (13) 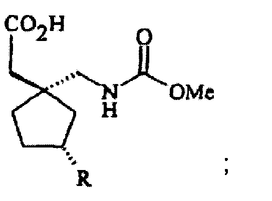
i) ajouter le produit de l'étape h) à un mélange de 1,4-dioxane et d'acide chlorhydrique aqueux à une concentration de 6 M, et agiter pour produire un composé répondant à la formule (IIa) 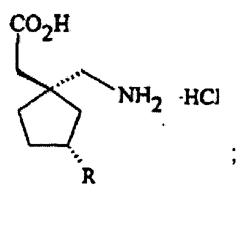 et
j) convertir le produit de l'étape i) en composé répondant à la formule (II) 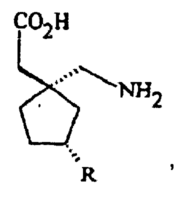 et en outre convertir, si souhaité, en sel pharmaceutiquement acceptable par des moyens connus.

8. Procédé selon la revendication 5, **caractérisé en outre en ce que** le produit intermédiaire (11) 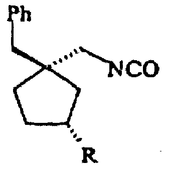 formé est en outre mis à réagir, sans isolement, avec du méthanol pour produire le carbamate répondant à la formule (12)

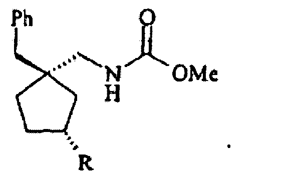

9. Procédé pour la préparation d'un composé répondant à la formule Il 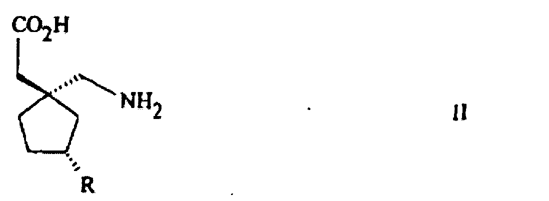 dans laquelle R représente un groupe alkyle en C₁-C₁₀ ou un groupe cycloalkyle en C₃-C₁₀, et de sels pharmaceutiquement acceptables de celui-ci, qui comprend les étapes :
a) ajouter un cyanoacétate répondant à la formule (A) 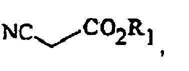 dans laquelle R₁ représente un groupe alkyle ou un groupe benzyle, à un mélange d'une cyclopentanone chirale répondant à la formule (1) 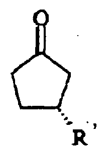 d'un solvant, d'un acide carboxylique, et d'un catalyseur de la réaction de Knoevenagel, et agiter le mélange en présence d'un moyen pour éliminer l'eau, afin de produire l'alcène répondant à la formule (2) 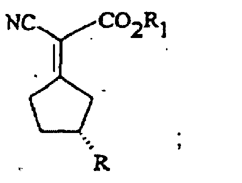
b) ajouter le produit de l'étape a) ci-dessus à un mélange de chlorure de benzylmagnésium, de bromure de benzylmagnésium, ou d'iodure de benzylmagnésium, dans un solvant pour produire les produits d'addition répondant aux formules (3a) 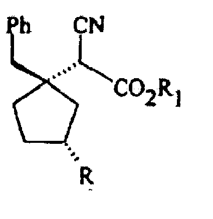 et (3b) 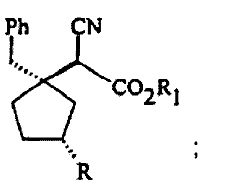
c) ajouter les produits de l'étape b) ci-dessus à un mélange d'une base choisie parmi l'hydroxyde de potassium, l'hydroxyde de sodium, l'hydroxyde de lithium, et l'hydroxyde de césium, dans un solvant, et agiter, puis acidifier pour produire les acides carboxyliques répondant aux formules (4a) 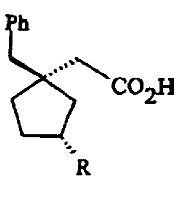 et (4b) 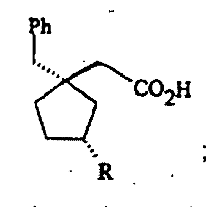 ou
ajouter les produits de l'étape b) ci-dessus à un mélange acide, et agiter pour produire les acides carboxyliques répondant aux formules (4a) 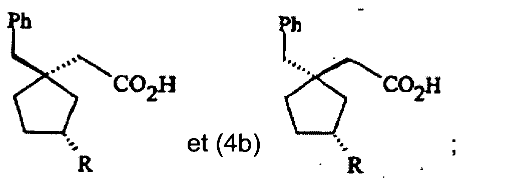
d) mettre en contact les produits de l'étape c) ci-dessus avec une amine dans un solvant, et recristalliser le sel ainsi formé pour produire le diastéréoisomère enrichi répondant à la formule (5) 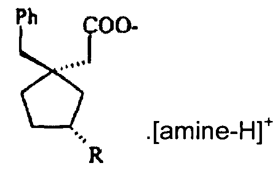 en tant que sel d'amine ;
e) convertir le produit de l'étape d) en acide carboxylique répondant à la formule (6) 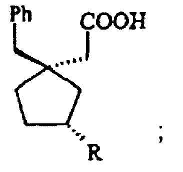
f) ajouter du chlorure d'oxalyle à un mélange du produit de l'étape e), d'un solvant, et de N,N-diméthylformamide (DMF), et agiter pour produire le chlorure d'acide répondant à la formule (14) 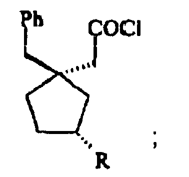
g) ajouter le produit de l'étape f) à un mélange d'alcool tert-butylique, d'un solvant, et d'une amine tertiaire, et agiter pour produire l'ester répondant à la formule (15) 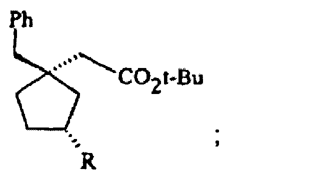
h) ajouter le produit de l'étape g) à un mélange de tétrachlorure de carbone ou d'acétate d'éthyle, et d'acétonitrile, d'eau, de périodate de sodium, et de chlorure de ruthénium (III), et agiter pour produire l'acide carboxylique répondant à la formule (16) 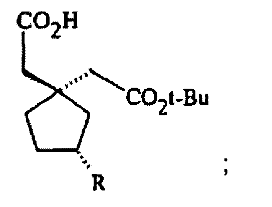
i) ajouter le produit de l'étape h) à un mélange d'un solvant, de méthanol, et de (triméthylsilyl)diazométhane, et agiter pour produire le bis-ester répondant à la formule (17) 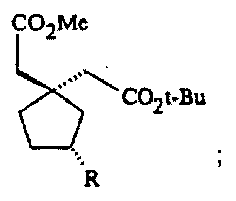 ou ajouter le produit de l'étape h) à un mélange d'iodométhane, d'un solvant, et d'une base, et agiterpour produire le bis-ester répondant à la formule (17) 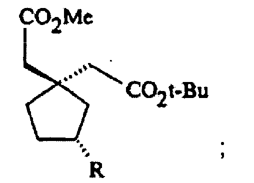
j) ajouter un acide à un mélange du produit de l'étape i) et d'un solvant, et agiter pour produire l'acide carboxylique répondant à la formule (18) 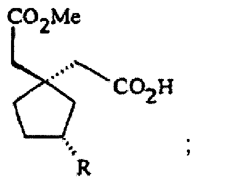
k) ajouter le produit de l'étape j) à un mélange d'une amine tertiaire, d'un solvant, et d'azide de diphénylphosphoryle (DPPA), et agiter pour produire l'isocyanate répondant à la formule (19) 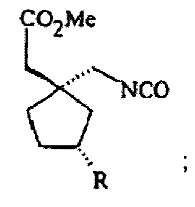 ou
ajouter le produit de l'étape j) ci-dessus à du chloroformiate d'éthyle ou du chloroformiate d'isobutyle et une base dans un solvant à une température de -40 °C à 78 °C, puis ajouter une solution d'azide de sodium dans de l'eau et de tétrahydrofurane ou d'acétone, puis ajouter du toluène ou du benzène, et porter au reflux pour produire l'isocyanate répondant à la formule (19) 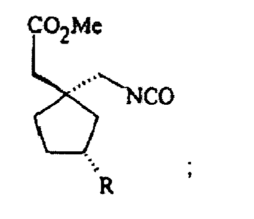
l) ajouter le produit de l'étape k) à un mélange d'un solvant et de méthanol, et agiter pour produire le carbamate répondant à la formule (20) 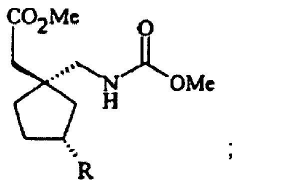
m) ajouter le produit de l'étape I) à un mélange d'un solvant et d'acide chlorhydrique aqueux, et agiter pour produire un composé répondant à la formule (IIa) 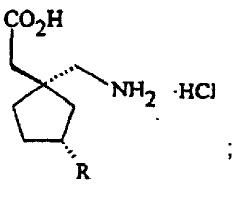 et
n) convertir le produit de l'étape m) en composé répondant à la formule (II) 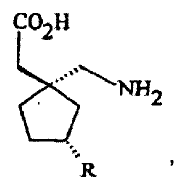 et en outre convertir, si souhaité, en sel pharmaceutiquement acceptable par des moyens connus.

10. Procédé selon la revendication 9, qui comprend les étapes :
a) ajouter un cyanoacétate répondant à la formule (A) 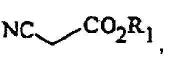 dans laquelle R₁ est choisi parmi les groupes méthyle, éthyle, n-propyle, isopropyle, n-butyle, isobutyle, sec-butyle, tert-butyle, et benzyle, à un mélange d'une cyclopentanone chirale répondant à la formule (1) 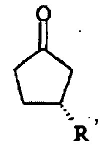 d'un solvant choisi parmi le tétrahydrofurane, le 1,4-dioxane, l'éther tert-butylméthylique, le chloroforme, le dichlorométhane l'acétonitrile, l'éther éthylique, l'acétate d'éthyle, les hexanes, le N,N-diméthylformamide, le diméthylsulfoxyde, l'éthanol, le tert-butanol, le toluène, le benzène, les xylènes, et le n-heptane, d'acide acétique, et d'un catalyseur de la réaction de Knoevenagel choisi parmi la β-alanine, l'acétate d'ammonium, et la pipéridine, et agiter le mélange en présence d'un moyen pour éliminer l'eau choisi parmi la distillation azéotropique, les tamis moléculaires activés, le sulfate de magnésium anhydre, le sulfate de sodium anhydre, le carbonate de sodium anhydre, le carbonate de potassium anhydre, le carbonate de césium anhydre, l'orthoformiate de triméthyle, et l'orthoformiate de triéthyle, afin de produire l'alcène répondant à la formule (2) 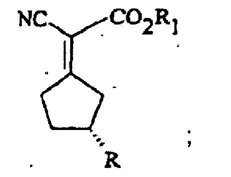
b) ajouter le produit de l'étape a) ci-dessus à un mélange de chlorure de benzylmagnésium, de bromure de benzylmagnésium, ou d'iodure de benzylmagnésium, dans un solvant choisi parmi le tétrahydrofurane, le benzène, le 1,4-dioxane, les hexanes, le n-heptane, le toluène, l'éther diéthylique, et l'éther tert-butylméthylique pour produire les produits d'addition répondant aux formules (3a) 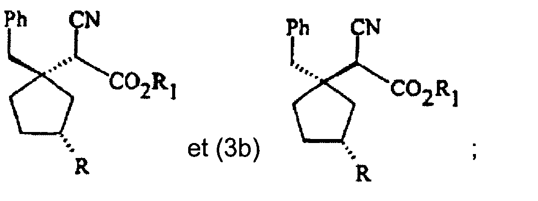
c) ajouter les produits de l'étape b) ci-dessus à un mélange d'une base choisie parmi l'hydroxyde de potassium, l'hydroxyde de sodium, l'hydroxyde de lithium, et l'hydroxyde de césium, dans un solvant choisi parmi l'éthylène glycol, l'éther 2-méthoxyéthylique, le 1,4-dioxane, et le diéthylène glycol, et agiter le mélange, puis l'acidifier pour produire les acides carboxyliques répondant aux formules (4a) 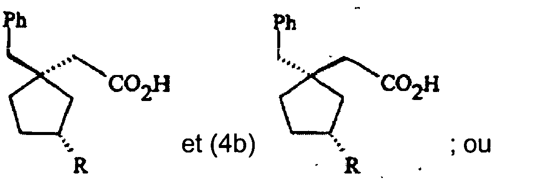 ajouter les produits de l'étape b) ci-dessus à un mélange acide choisi parmi de l'HCl à 6 à 12 M, de l'H₂SO₄ à 12 M, 10 % à 48 % poids/poids d'acide bromhydrique, et de l'HBr dans de l'acide acétique aqueux, et agiter pour produire les acides carboxyliques répondant aux formules (4a) 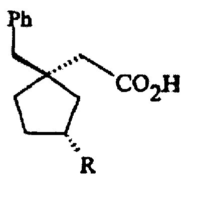 et (4b) 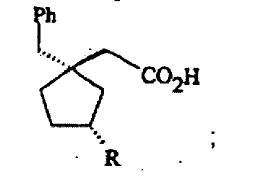
d) mettre en contact les produits de l'étape c) ci-dessus avec une amine choisie parmi la (S)-α-méthyl-benzylamine, la (R)-α-méthyl-benzylamine, la (R)-(+)-1-(naphtyl)éthylamine, la (S)-(+)-1-(naphtyl)éthylamine, la triéthylamine, la diisopropyléthylamine, la dicylohexylamine, la benzylamine, la dibenzylamine, la morpholine, la N-méthylmorpholine, la pipéridine, la N-méthylpipéridine, et la pyridine dans un solvant choisi parmi le N,N-diméthylformamide, le chloroforme, le benzène, les xylènes, les hexanes, l'acétone, l'éthanol, le méthanol, l'isopropanol, l'éther diéthylique, le dichlorométhane, le benzène, le toluène, le n-pentane, le n-hexane, le n-heptane, l'acétate d'éthyle, l'acétonitrile, l'éther tert-butylméthylique, le tétrahydrofurane, et le 1,4-dioxane, et recristalliser le sel ainsi formé pour produire le diastéréoisomère enrichi répondant à la formule (5) 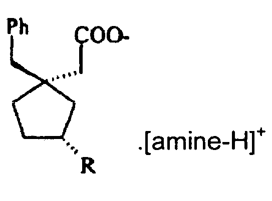 en tant que sel d'amine ;
e) ajouter le produit de l'étape d) à un mélange choisi parmi l'acide chlorhydrique aqueux, l'acide sulfurique aqueux, l'acide acétique aqueux, l'acide chlorhydrique dissous dans de l'acide acétique, et l'acide chlorhydrique dissous dans de l'acide acétique auquel de l'eau est ajoutée, et agiter pour produire l'acide carboxylique répondant à la formule (6) 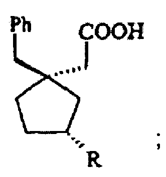 ou
diviser le produit de l'étape d) entre un mélange d'acide chlorhydrique aqueux et d'un solvant choisi parmi le chloroforme, le dichlorométhane, l'acétate d'éthyle, l'éther éthylique, le tétrahydrofurane, le 1,4-dioxane, le toluène, et l'éther tert-butylméthylique, et sécher et évaporer la phase organique pour produire l'acide carboxylique répondant à la formule (6) 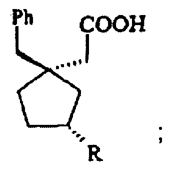
f) ajouter du chlorure d'oxalyle à un mélange du produit de l'étape e), d'un solvant choisi parmi le dichlorométhane, le chloroforme, l'éther éthylique, le toluène, et l'éther tert-butylméthylique, et de 0,01 à 10 % en mole de N,N-diméthylformamide (DMF), et agiter à une température de -40 °C à 110 °C pour produire le chlorure d'acide répondant à la formule (14) 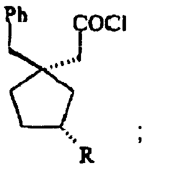
g) ajouter le produit de l'étape f) à un mélange d'alcool tert-butylique, d'un solvant choisi parmi le dichlorométhane, le chloroforme, l'éther éthylique, le toluène et l'éther tert-butylméthylique, et de N,N-diisopropyléthylamine (DIPEA) ou de triéthylamine, et agiter à une température de -40 °C à 110 °C pour produire l'ester répondant à la formule (15) 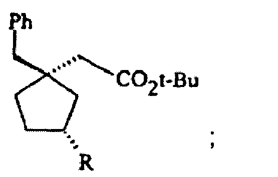
h) ajouter le produit de l'étape g) ci-dessus à un mélange de tétrachlorure de carbone ou d'acétate d'éthyle, et d'acétonitrile, d'eau, de périodate de sodium, et de chlorure de ruthénium (III), et agiter à une température de -40 °C à 80 °C pour produire l'acide carboxylique répondant à la formule (16) 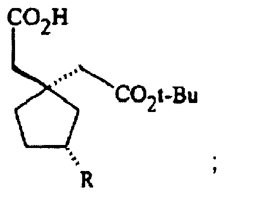
i) ajouter le produit de l'étape h) à un solvant choisi parmi le toluène, le benzène, les xylènes, et le n-heptane, du méthanol, et du (triméthylsilyl)diazométhane, et agiter à une température de 0 °C à 150 °C pour produire le bis-ester répondant à la formule (17) 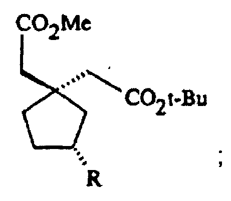 ou ajouter le produit de l'étape h) à un mélange d'iodométhane, un solvant choisi parmi le dicfilorométhane, le
chloroforme, le tétrahydrofurane, le toluène et le 1,4-dioxane, et d'une base choisie parmi le 1,8-diazabicyclo[5.4.0]undéc-7-ène (DBU), la diisopropyléthylamine, la triéthylamine, et la 1,5-diazabicyclo[4.3.0]non-5-ène, et agiter à une température de -40 °C à 110 °C pour produire le bis-ester répondant à la formule (17) 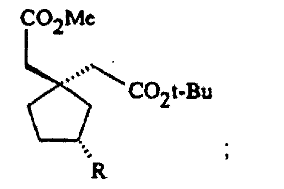
j) ajouter de l'acide chlorhydrique ou de l'acide trifluoroacétique (TFA) à un mélange du produit de l'étape i) et d'un solvant choisi parmi le dichlorométhane, le chloroforme, le 1,4-dioxane, le tétrahydrofurane, l'éther diéthylique, et l'éther tert-butylméthylique, et agiter à une température de -40 °C à 110°C pour produire l'acide carboxylique répondant à la formule (18) 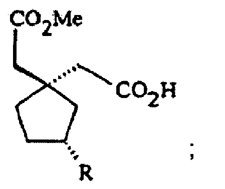
k) ajouter le produit de l'étape j) à un mélange d'une base choisie parmi la triéthylamine et la diisopropyléthylamine, d'un solvant choisi parmi le toluène, le benzène, les xylènes et le n-heptane, et d'azide de diphénylphosphoryle (DPPA), et agiter à une température de 0 °C à 150 °C pour produire l'isocyanate répondant à la formule (19) 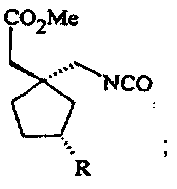 ou ajouter le produit de l'étape j) ci-dessus à du chloroformiate d'éthyle ou du chloroformiate d'isobutyle, une base choisie parmi la triéthylamine et la diisopropyléthylamine, et un solvant choisi parmi le tétrahydrofurane, l'acétone, et l'éther diéthytique à une température de -40 °C à 78 °C, puis ajouter une solution d'azide de sodium dans de l'eau et de tétrahydrofurane ou d'acétone, puis ajouter du toluène ou du benzène, et porter au reflux pour produire l'isocyanate répondant à la formule (19) 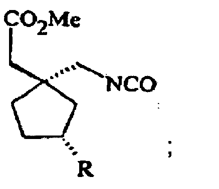
l) ajouter le produit de l'étape k) à un mélange d'un solvant choisi parmi le toluène, le benzène, les xylènes et le n-heptane, et de méthanol, et agiter à une température de 0 °C à 150 °C pour produire le carbamate répondant à la formule (20) 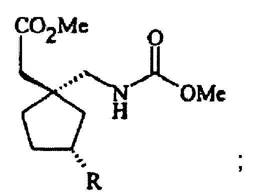
m) ajouter le produit de l'étape I) à un mélange d'un solvant choisi parmi l'eau, l'acide acétique, et le 1,4-dioxane, et d'acide chlorhydrique aqueux à une concentration de 0,01 M à 12 M, et agiter à une température de 0°C à 115°C pour produire un composé répondant à la formule (IIa) 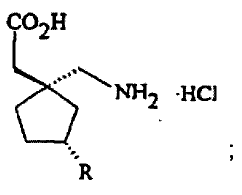 et
n) convertir le produit de l'étape m) en composé répondant à la formule II 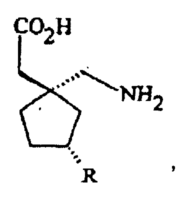 et en outre convertir, si souhaité, en sel pharmaceutiquement acceptable par des moyens connus.

11. Procédé selon la revendication 9, qui comprend les étapes :
a) ajouter un cyanoacétate répondant à la formule (A) 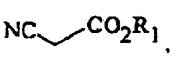 dans laquelle R₁ représente un groupe éthyle, à un mélange d'une cyclopentanone chirale répondant à la formule (1) 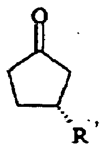 de toluène, d'acide acétique, et d'un catalyseur de la réaction de Knoevenagel qui est l'acétate d'ammonium, et chauffer le mélange au reflux sur un piège Dean-Stark, pour produire l'alcène répondant à la formule (2) 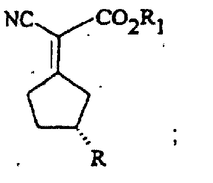
b) ajouter le produit de l'étape a) ci-dessus à un mélange de chlorure de benzylmagnésium dans du tétrahydrofurane anhydre à une température de -100 °C à 25 °C pour produire les produits d'addition répondant aux formules (3a) 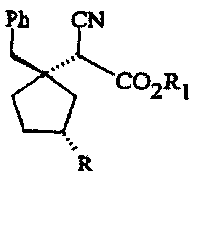 et (3b) 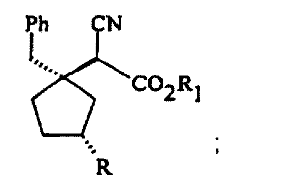
c) ajouter les produits de l'étape b) ci-dessus à un mélange d'hydroxyde de potassium dans de l'éthylène glycol, et chauffer le mélange à une température de 100 °C à 200 °C, puis l'acidifier pour produire les produits d'hydrolyse répondant aux formules (4a) 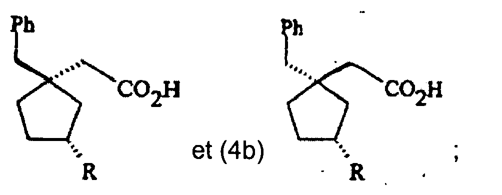
d) mettre en contact les produits de l'étape c) ci-dessus avec de la (S)-α-méthyl-benzylamine dans de l'acétate d'éthyle, et recristalliser le sel ainsi formé à partir d'acétate d'éthyle pour produire le diastéréoisomère enrichi répondant à la formule (5) 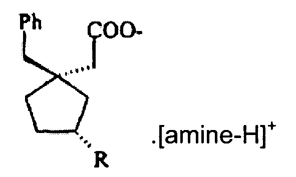 en tant que sel de (S)-α-méthyl-benzylamine ;
e) ajouter le produit de l'étape d) à de l'acide chlorhydrique aqueux, et agiter pour produire l'acide carboxylique répondant à la formule (6) 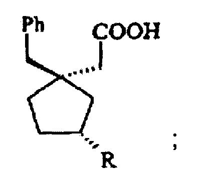
f) ajouter du chlorure d'oxalyle à un mélange du produit de l'étape e), de dichlorométhane, et d'une quantité catalytique de N,N-diméthylformamide (DMF), et agiter pour produire le chlorure d'acide répondant à la formule (14) 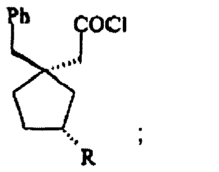
g) ajouter le produit de l'étape f) à un mélange d'alcool tert-butylique, de dichlorométhane, et de N,N-diisopropyléthylamine (DIPEA), et agiter pour produire l'ester répondant à la formule (15) 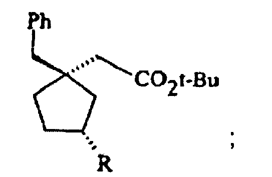
h) ajouter le produit de l'étape g) à un mélange de tétrachlorure de carbone ou d'acétate d'éthyle, et d'acétonitrile, d'eau, de périodate de sodium, et de chlorure de ruthénium (III), et agiter pour produire l'acide carboxylique répondant à la formule (16) 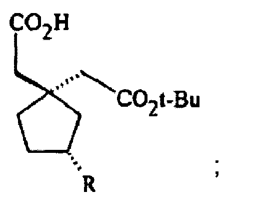
i) ajouter le produit de l'étape h) à un mélange de méthanol, de toluène, et de (triméthylsilyl)diazométhane, et agiter pour produire le bis-ester répondant à la formule (17) 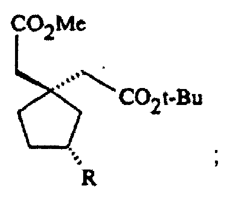 ou ajouter le produit de l'étape h) à un mélange d'iodométhane, de dichlorométhane, de triéthylamine, et agiter pour produire le bis-ester répondant à la formule (17) 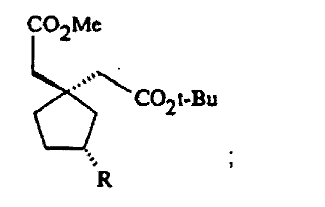
j) ajouter de l'acide chlorhydrique ou de l'acide trifluoroacétique (TFA) à un mélange du produit de l'étape i) et de dichlorométhane, et agiter pour produire l'acide carboxylique répondant à la formule (18) 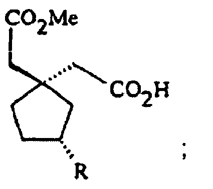
k) ajouter le produit de l'étape j) à un mélange de triéthylamine, de toluène, et d'azide de diphénylphosphoryle (DPPA), et porter au reflux pour produire l'isocyanate répondant à la formule (19) 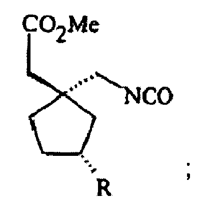 ou ajouter le produit de l'étape j) ci-dessus à du chloroformiate d'éthyle ou du chloroformiate d'isobutyle, de la triéthylamine et du tétrahydrofurane à une température de -40 °C à 78 °C, puis ajouter une solution d'azide de sodium dans de l'eau et de tétrahydrofurane ou d'acétone, puis ajouter du toluène ou du benzène, et porter au reflux pour produire l'isocyanate répondant à la formule (19) 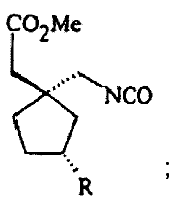
l) ajouter le produit de l'étape k) à un mélange de méthanol et de toluène, et porter au reflux pour produire le carbamate répondant à la formule (20) 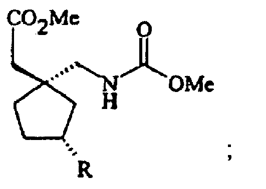
m) ajouter le produit de l'étape l) à un mélange de 1,4-dioxane et d'acide chlorhydrique aqueux à une concentration de 6 M, et agiter pour produire un composé répondant à la formule (IIa) 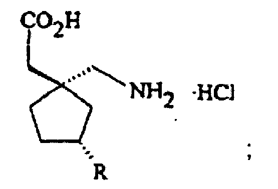
n) convertir le produit de l'étape m) en composé répondant à la formule (11) 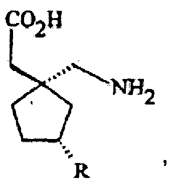 et en outre convertir, si souhaité, en sel pharmaceutiquement acceptable par des moyens connus.

12. Procédé selon la revendication 9, **caractérisé en outre en ce que** le produit intermédiaire (14) 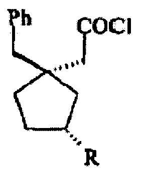 formé est en outre mis à réagir, sans isolement, avec de l'alcool tert-butylique, pour produire l'ester répondant à la formule (15)

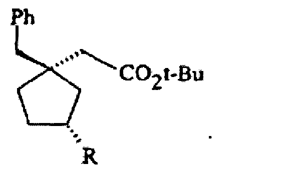

13. Procédé selon la revendication 9, **caractérisé en ce que** le produit intermédiaire (19) 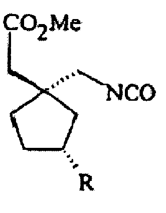 formé est en outre mis à réagir, sans isolement, avec du méthanol, pour produire le carbamate répondant à la formule (20)

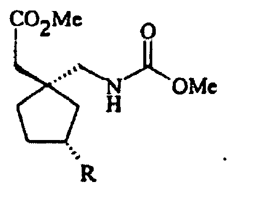

14. Procédé selon la revendication 9, **caractérisé en ce que** le produit intermédiaire (14) 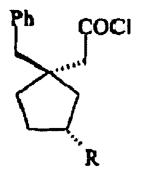 formé est en outre mis à réagir, sans isolement, avec de l'alcool tert-butylique, pour produire l'ester répondant à la formule (15) 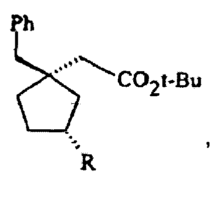 et le produit intermédiaire (19) 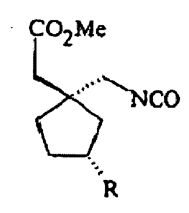 formé est en outre mis à réagir, sans isolement, avec du méthanol, pour produire le carbamate répondant à la formule (20)

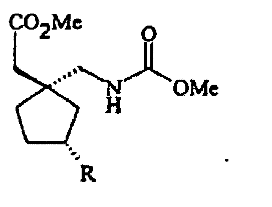

15. Procédé pour la préparation d'un composé répondant à la formule III 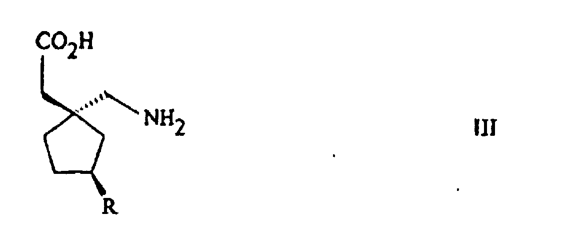 dans laquelle R représente un groupe alkyle en C₁-C₁₀ ou un groupe cycloalkyle en C₃-C₁₀, et de sels pharmaceutiquement acceptables de celui-ci, qui comprend les étapes :
a) ajouter un cyanoacétate répondant à la formule (A) 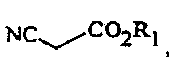 dans laquelle R₁ représente un groupe alkyle ou un groupe benzyle, à un mélange d'une cyclopentanone chirale répondant à la formule (21) 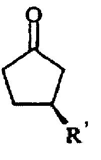 d'un solvant, d'un acide carboxylique, et d'un catalyseur de la réaction de Knoevenagel, et agiter le mélange en présence d'un moyen pour éliminer l'eau, afin de produire l'alcène répondant à la formule (22) 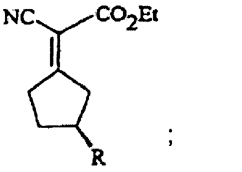
b) ajouter le produit de l'étape a) ci-dessus à un mélange de chlorure de benzylmagnésium ou d'iodure de benzylmagnésium, dans un solvant pour produire les produits d'addition répondant aux formules (23a) 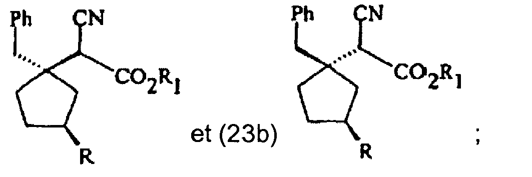
c) ajouter les produits de l'étape b) ci-dessus à un mélange d'une base choisie parmi l'hydroxyde de potassium, l'hydroxyde de sodium, l'hydroxyde de lithium, et l'hydroxyde de césium, dans un solvant, et agiter, puis acidifier pour produire les acides carboxyliques répondant aux formules (24a) 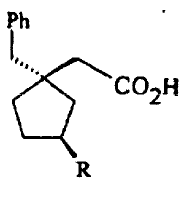 et (24b) 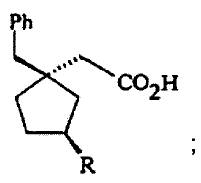 ou
ajouter les produits de l'étape b) ci-dessus à un mélange acide, et agiter pour produire les acides carboxyliques répondant aux formules (24a) 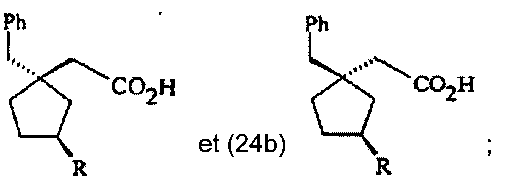
d) mettre en contact les produits de l'étape c) ci-dessus avec une amine dans un solvant, et recristalliser le sel ainsi formé pour produire le diastéréoisomère enrichi répondant à la formule (25) 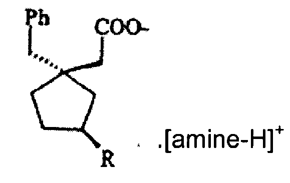 en tant que sel d'amine ; et
e) convertir le produit de l'étape d) en acide carboxylique répondant à la formule (26) 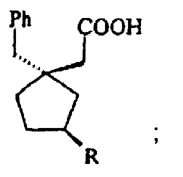
f) ajouter le produit de l'étape e) à un mélange d'iodométhane, d'un solvant, et de 1,8-diazabicyclo[5.4.0]undéc-7-ène (DBU), et agiter pour produire l'ester répondant à la formule (27) 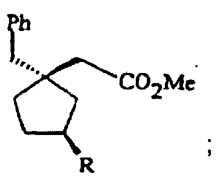 ou ajouter le produit de l'étape e) à du méthanol et un acide pour produire l'ester répondant à la formule (27) 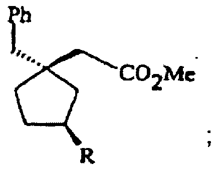 ou ajouter le produit de l'étape e) à une solution de diazométhane ou de triméthylsilyldiazométhane dans un solvant pour produire l'ester répondant à la formule (27) 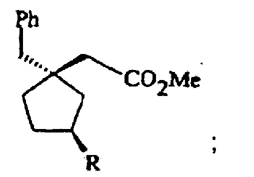
g) ajouter le produit de l'étape f) à un mélange de tétrachlorure de carbone ou d'acétate d'éthyle, et d'acétonitrile, d'eau, de périodate de sodium, et de chlorure de ruthénium (III), et agiter pour produire l'acide carboxylique répondant à la formule (28) 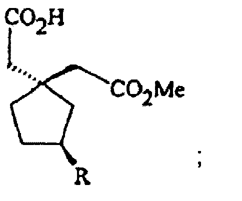
h) ajouter le produit de l'étape g) ci-dessus à un mélange d'une amine tertiaire, d'un solvant, et d'azide de diphénylphosphoryle (DPPA), et agiter pour produire l'isocyanate répondant à la formule (29) 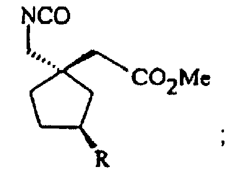 ou
ajouter le produit de l'étape g) ci-dessus à du chloroformiate d'éthyle ou du chloroformiate d'isobutyle et une base dans un solvant à une température de -40 °C à 78 °C, puis ajouter une solution d'azide de sodium dans de l'eau et de tétrahydrofurane ou d'acétone, puis ajouter du toluène ou du benzène, et porter au reflux pour produire l'isocyanate répondant à la formule (29) 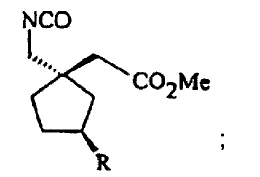
i) ajouter le produit de l'étape h) à un mélange d'un solvant et de méthanol, et agiter pour produire le carbamate répondant à la formule (30) 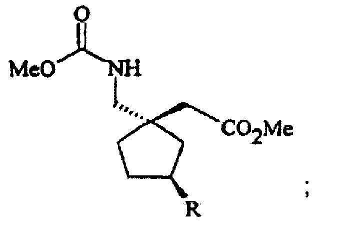
j) ajouter le produit de l'étape i) à un mélange d'un solvant et d'acide chlorhydrique aqueux, et agiter pour produire un composé répondant à la formule (IIIa) 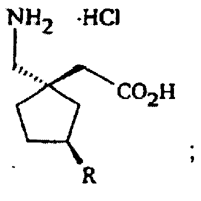 et
k) convertir le produit de l'étape j) en composé répondant à la formule (III) 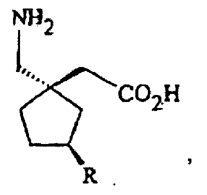 et en outre convertir, si souhaité, en sel pharmaceutiquement acceptable par des moyens connus.

16. Procédé pour la préparation d'un composé répondant à la formule IV 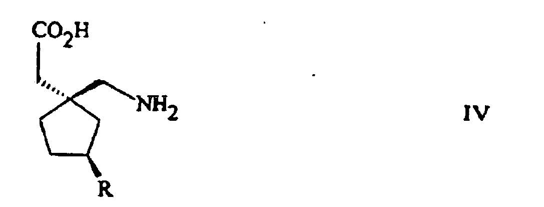 dans laquelle R représente un groupe alkyle en C₁-C₁₀ ou un groupe cycloalkyle en C₃-C₁₀, et de sels pharmaceutiquement acceptables de celui-ci, qui comprend les étapes :
a) ajouter un cyanoacétate répondant à la formule (A) 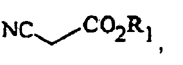 dans laquelle R₁ représente un groupe alkyle ou un groupe benzyle, à un mélange d'une cyclopentanone chirale répondant à la formule (21) 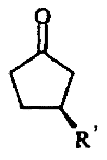 d'un solvant, d'un acide carboxylique, et d'un catalyseur de la réaction de Knoevenagel, et agiter le mélange en présence d'un moyen pour éliminer l'eau, afin de produire l'alcène répondant à la formule (22) 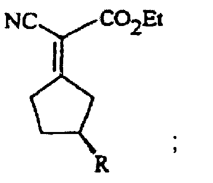
b) ajouter le produit de l'étape a) ci-dessus à un mélange de chlorure de benzylmagnésium ou d'iodure de benzylmagnésium, dans un solvant pour produire les produits d'addition répondant aux formules (23a) 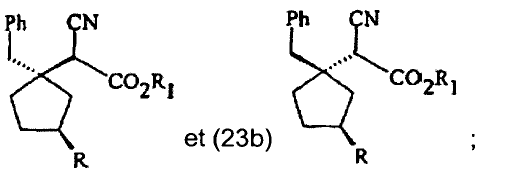
c) ajouter les produits de l'étape b) ci-dessus à un mélange d'une base choisie parmi l'hydroxyde de potassium, l'hydroxyde de sodium, l'hydroxyde de lithium, et l'hydroxyde de césium, dans un solvant, et agiter, puis acidifier pour produire les acides carboxyliques répondant aux formules (24a) 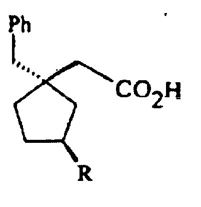 et (24b) 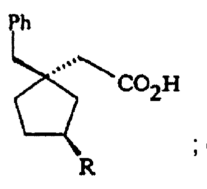 ou ajouter les produits de l'étape b) ci-dessus à un mélange acide, et agiter pour produire les acides carboxyliques répondant aux formules (24a) 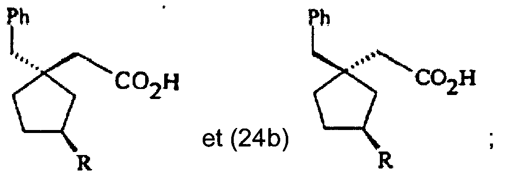
d) mettre en contact les produits de l'étape c) ci-dessus avec une amine dans un solvant, et recristalliser le sel ainsi formé pour produire le diastéréoisomère enrichi répondant à la formule (25) 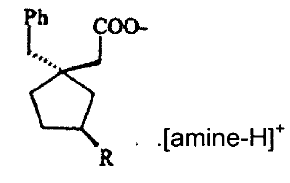 en tant que sel d'amine ; et
e) convertir le produit de l'étape d) en acide carboxylique répondant à la formule (26) 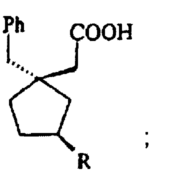
f) ajouter le produit de l'étape e) ci-dessus à un mélange d'une amine tertiaire, d'un solvant, et d'azide de diphénylphosphoryle (DPPA), et agiter pour produire l'isocyanate répondant à la formule (31) 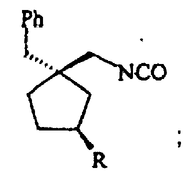 ou
ajouter le produit de l'étape e) ci-dessus à du chloroformiate d'éthyle ou du chloroformiate d'isobutyle et une base dans un solvant à une température de -40 °C à 78 °C, puis ajouter une solution d'azide de sodium dans de l'eau et de tétrahydrofurane ou d'acétone, puis ajouter du toluène ou du benzène, et porter au reflux pour produire l'isocyanate répondant à la formule (31) 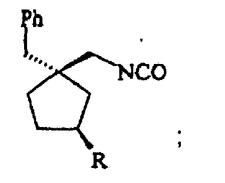
g) ajouter le produit de l'étape f) à un mélange d'un solvant et de méthanol, et agiter pour produire le carbamate répondant à la formule (32) 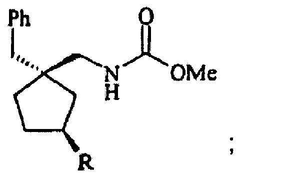
h) ajouter le produit de l'étape g) à un mélange de tétrachlorure de carbone ou d'acétate d'éthyle, et d'acétonitrile, d'eau, de périodate de sodium, et de chlorure de ruthénium (III), et agiter pour produire l'acide carboxylique répondant à la formule (33) 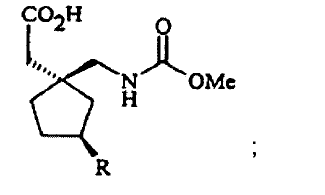
i) ajouter le produit de l'étape h) à un mélange d'un solvant et d'acide chlorhydrique aqueux, et agiter pour produire un composé répondant à la formule (IVa) 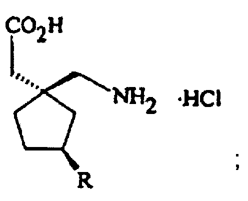 et
j) convertir le produit de l'étape i) en composé répondant à la formule (IV) 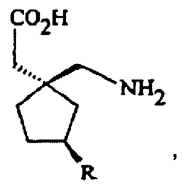 et en outre convertir, si souhaité, en sel pharmaceutiquement acceptable par des moyens connus.

17. Procédé pour la préparation d'un composé répondant à la formule IV 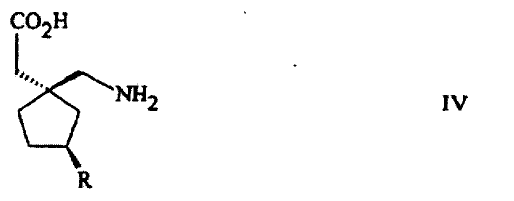 dans laquelle R représente un groupe alkyle en C₁-C₁₀ ou un groupe cycloalkyle en C₃-C₁₀, et de sels pharmaceutiquement acceptables de celui-ci, qui comprend les étapes :
a) ajouter un cyanoacétate répondant à la formule (A) 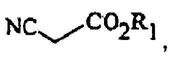 dans laquelle R₁ représente un groupe alkyle ou un groupe benzyle, à un mélange d'une cyclopentanone chirale répondant à la formule (21) 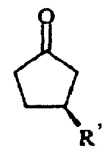 d'un solvant, d'un acide carboxylique, et d'un catalyseur de la réaction de Knoevenagel, et agiter le mélange en présence d'un moyen pour éliminer l'eau, afin de produire l'alcène répondant à la formule (22) 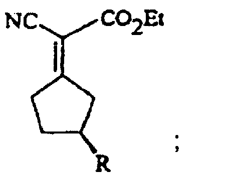
b) ajouter le produit de l'étape a) ci-dessus à un mélange de chlorure de benzylmagnésium ou d'iodure de benzylmagnésium, dans un solvant pour produire les produits d'addition répondant aux formules (23a) 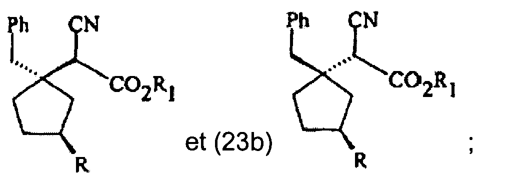
c) ajouter les produits de l'étape b) ci-dessus à un mélange d'une base choisie parmi l'hydroxyde de potassium, l'hydroxyde de sodium, l'hydroxyde de lithium, et l'hydroxyde de césium, dans un solvant, et agiter, puis acidifier pour produire les acides carboxyliques répondant aux formules (24a) 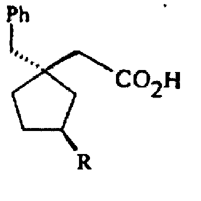 et (24b) 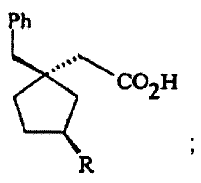 ou
ajouter les produits de l'étape b) ci-dessus à un mélange acide, et agiter pour produire les acides carboxyliques répondant aux formules (24a) 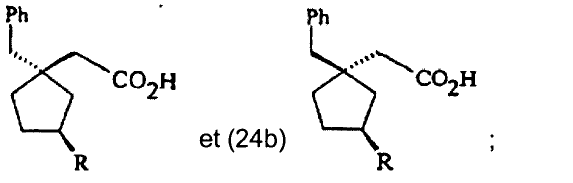
d) mettre en contact les produits de l'étape c) ci-dessus avec une amine dans un solvant, et recristalliser le sel ainsi formé pour produire le diastéréoisomère enrichi répondant à la formule (25) 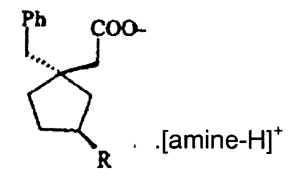 en tant que sel d'amine ; et
e) convertir le produit de l'étape d) en acide carboxylique répondant à la formule (26) 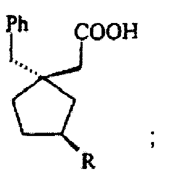
f) ajouter du chlorure d'oxalyle à un mélange du produit de l'étape e), d'un solvant, et de N,N-diméthylformamide (DMF), et agiter pour produire le chlorure d'acide répondant à la formule (34) 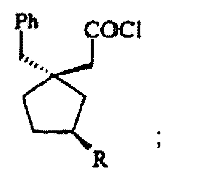
g) ajouter le produit de l'étape f) à un mélange d'alcool tert-butylique, d'un solvant, et d'une amine tertiaire, et agiter pour produire l'ester répondant à la formule (35) 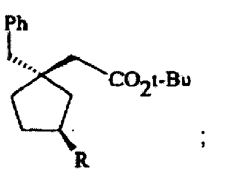
h) ajouter le produit de l'étape g) ci-dessus à un mélange de tétrachlorure de carbone ou d'acétate d'éthyle, et d'acétonitrile, d'eau, de périodate de sodium, et de chlorure de ruthénium (lll), et agiter pour produire l'acide carboxylique répondant à la formule (36) 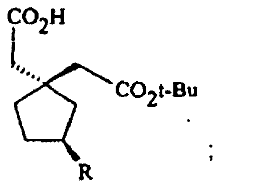
i) ajouter le produit de l'étape h) à un mélange d'un solvant, de méthanol, et de (triméthylsilyl)diazométhane, et agiter pour produire le bis-ester répondant à la formule (37) 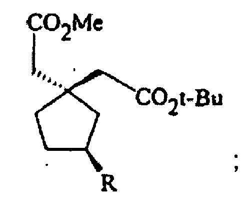 ou ajouter le produit de l'étape h) à un mélange d'iodométhane, d'un solvant, et d'une base, et agiter pour produire le bis-ester répondant à la formule (37) 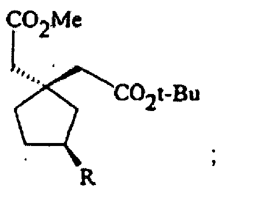
j) ajouter un acide à un mélange du produit de l'étape i) et d'un solvant, et agiter pour produire l'acide carboxylique répondant à la formule (38) 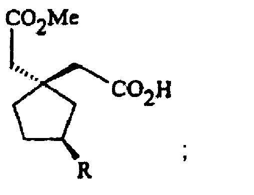
k) ajouter le produit de l'étape j) à un mélange d'une amine tertiaire, d'un solvant, et d'azide de diphénylphosphoryle (DPPA), et agiter pour produire l'isocyanate répondant à la formule (39) 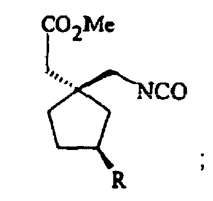 ou ajouter le produit de l'étape j) ci-dessus à du chloroformiate d'éthyle ou du chloroformiate d'isobutyle et une base dans un solvant à une température de -40 °C à 78 °C, puis ajouter une solution d'azide de sodium dans de l'eau et de tétrahydrofurane ou d'acétone, puis ajouter du toluène ou du benzène, et porter au reflux pour produire l'isocyanate répondant à la formule (39) 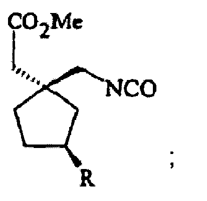
l) ajouter le produit de l'étape k) à un mélange d'un solvant et de méthanol, et agiter pour produire le carbamate répondant à la formule (40) 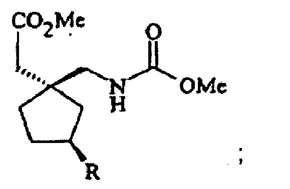
m) ajouter le produit de l'étape 1) à un mélange d'un solvant et d'acide chlorhydrique, et agiter pour produire un composé répondant à la formule (IVa) 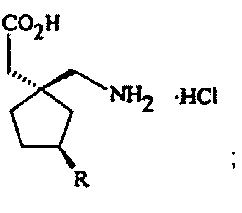 et
n) convertir le produit de l'étape m) en composé répondant à la formule (IV) 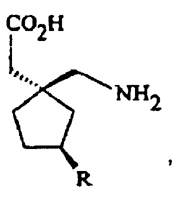 et en outre convertir, si souhaité, en sel pharmaceutiquement acceptable par des moyens connus.

18. Procédé pour la préparation d'un composé répondant à la formule (6) 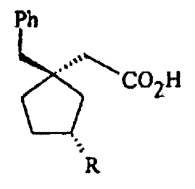 dans laquelle R représente un groupe alkyle en C₁-C₁₀ ou un groupe cycloalkyle en C₃-C₁₀, et de sels pharmaceutiquement acceptables de celui-ci, qui comprend les étapes :
a) ajouter un cyanoacétate répondant à la formule (A) 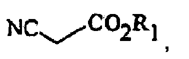 dans laquelle R₁ représente un groupe alkyle ou un groupe benzyle, à un mélange d'une cyclopentanone chirale répondant à la formule (1) 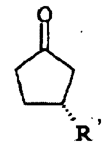 d'un solvant, d'un acide carboxylique, et d'un catalyseur de la réaction de Knoevenagel, et agiter le mélange en présence d'un moyen pour éliminer l'eau, afin de produire l'alcène répondant à la formule (2) 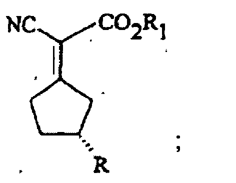
b) ajouter le produit de l'étape a) ci-dessus à un mélange de chlorure de benzylmagnésium, de bromure de benzylmagnésium, ou d'iodure de benzylmagnésium, dans un solvant pour produire les produits d'addition répondant aux formules (3a) 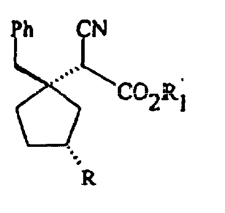 et (3b) 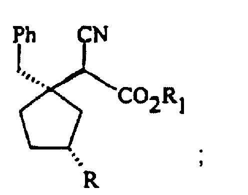
c) ajouter les produits de l'étape b) ci-dessus à un mélange d'une base choisie parmi l'hydroxyde de potassium, l'hydroxyde de sodium, l'hydroxyde de lithium, et l'hydroxyde de césium, et d'un solvant, et agiter, puis acidifier pour produire les acides carboxyliques répondant aux formules (4a) 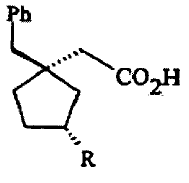 et (4b) 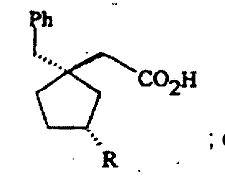 ou
ajouter les produits de l'étape b) ci-dessus à un mélange acide, et agiter pour produire les acides carboxyliques répondant aux formules (4a) 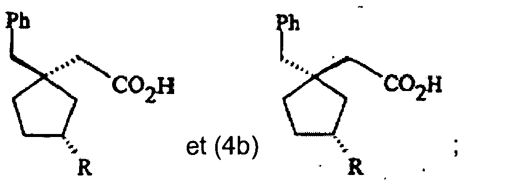
d) mettre en contact les produits de l'étape c) ci-dessus avec une amine dans un solvant, et recristalliser le sel ainsi formé pour produire le diastéréoisomère enrichi répondant à la formule (5) 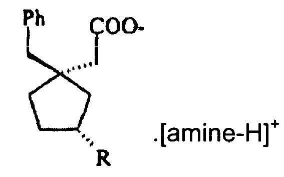 en tant que sel d'amine ; et
e) convertir le produit de l'étape d) en acide carboxylique répondant à la formule (6)

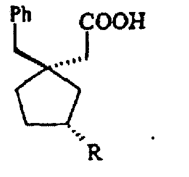

19. Procédé selon la revendication 18, qui comprend les étapes :
a) ajouter un cyanoacétate répondant à la formule (A) 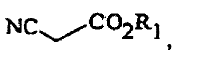 dans laquelle R₁ est choisi parmi les groupes méthyle, éthyle, n-propyle, isopropyle, n-butyle, isobutyle, sec-butyle, tert-butyle, et benzyle, à un mélange d'une cyclopentanone chirale répondant à la formule (1) 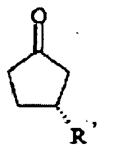 d'un solvant choisi parmi le tétrahydrofurane, le 1,4-dioxane, l'éther tert-butylméthylique, le chloroforme, le dichlorométhane, l'acétonitrile, l'éther éthylique, l'acétate d'éthyle, les hexanes, le N,N-diméthylformamide, le diméthylsulfoxyde, l'éthanol, le tert-butanol, le toluène, le benzène, les xylènes, et le n-heptane, d'acide acétique, et d'un catalyseur de la réaction de Knoevenagel choisi parmi la β-alanine, l'acétate d'ammonium, et la pipéridine, et agiter le mélange en présence d'un moyen pour éliminer l'eau choisi parmi la distillation azéotropique, les tamis moléculaires activés, le sulfate de magnésium anhydre, le sulfate de sodium anhydre, le carbonate de sodium anhydre, le carbonate de potassium anhydre, le carbonate de césium anhydre, l'orthoformiate de triméthyle, et l'orthoformiate de triéthyle, afin de produire l'alcène répondant à la formule (2) 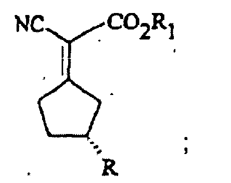
b) ajouter le produit de l'étape a) ci-dessus à un mélange de chlorure de benzylmagnésium, de bromure de benzylmagnésium, ou d'iodure de benzylmagnésium, dans un solvant choisi parmi le tétrahydrofurane, le benzène, le 1,4-dioxane, les hexanes, le n-heptane, le toluène, l'éther diéthylique, et l'éther tert-butylméthylique pour produire les produits d'addition répondant aux formules (3a) 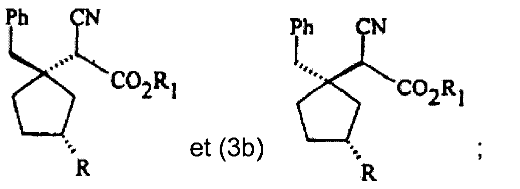
c) ajouter les produits de l'étape b) ci-dessus à un mélange d'une base choisie parmi l'hydroxyde de potassium, l'hydroxyde de sodium, l'hydroxyde de lithium, et l'hydroxyde de césium, dans un solvant choisi parmi l'éthylène glycol, l'éther 2-méthoxyéthylique, le 1,4-dioxane, et le diéthylène glycol, et agiter le mélange, puis l'acidifier pour produire les acides carboxyliques répondant aux formules (4a) 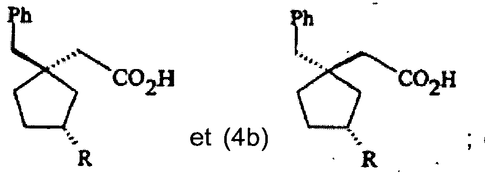 ou ajouter les produits de l'étape b) ci-dessus à un mélange acide choisi parmi de l'HCl à 6 à 12 M, de l'H₂SO₄ à 12 M, 10 % à 48 % poids/poids d'acide bromhydrique, et de l'HBr dans de l'acide acétique aqueux, et agiter pour produire les acides carboxyliques répondant aux formules (4a) 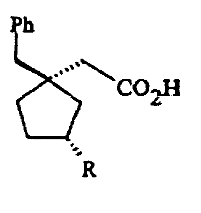 et (4b) 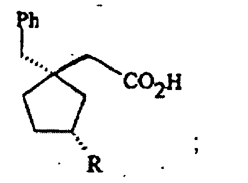
d) mettre en contact les produits de l'étape c) ci-dessus avec une amine choisie parmi la (S)-α-méthyl-benzylamine, la (R)-α-méthyl-benzylamine, la (R)-(+)-1-(naphtyl)éthylamine, la (S)-(+)-1-(naphtyl)éthylamine, la triéthylamine, la diisopropyléthylamine, la dicylohexylamine, la benzylamine, la dibenzylamine, la morpholine, la N-méthylmorpholine, la pipéridine, la N-méthylpipéridine, et la pyridine dans un solvant choisi parmi le N,N-diméthylformamide, le chloroforme, le benzène, les xylènes, les hexanes, l'acétone, l'éthanol, le méthanol, l'isopropanol, l'éther diéthylique, le dichlorométhane, le benzène, le toluène, le n-pentane, le n-hexane, le n-heptane, l'acétate d'éthyle, l'acétonitrile, l'éther tert-butylméthylique, le tétrahydrofurane, et le 1,4-dioxane, et recristalliser le sel ainsi formé pour produire le diastéréoisomère enrichi répondant à la formule (5) 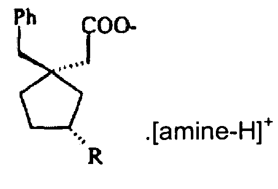 en tant que sel d'amine ; et
e) ajouter le produit de l'étape d) à un mélange choisi parmi l'acide chlorhydrique aqueux, l'acide sulfurique aqueux, l'acide acétique aqueux, l'acide chlorhydrique dissous dans de l'acide acétique, et l'acide chlorhydrique dissous dans de l'acide acétique et de l'eau, et agiter pour produire l'acide carboxylique répondant à la formule (6) 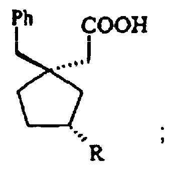 ou
diviser le produit de l'étape d) entre un mélange d'acide chlorhydrique aqueux et d'un solvant choisi parmi le chloroforme, le dichlorométhane, l'acétate d'éthyle, l'éther éthylique, le tétrahydrofurane, le 1,4-dioxane, le toluène, et l'éther tert-butylméthylique, et sécher et évaporer la phase organique pour produire l'acide carboxylique répondant à la formule (6)

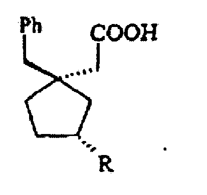

20. Procédé selon la revendication 18, qui comprend les étapes :
a) ajouter un cyanoacétate répondant à la formule (A) 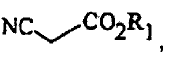 dans laquelle R₁ représente un groupe éthyle, à un mélange d'une cyclopentanone chirale répondant à la formule (1) 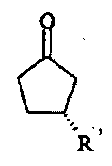 de toluène, d'acide acétique, et d'un catalyseur de la réaction de Knoevenagel qui est l'acétate d'ammonium, et chauffer le mélange au reflux sur un piège Dean-Stark, pour produire l'alcène répondant à la formule (2) 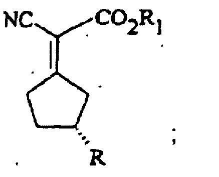
b) ajouter le produit de l'étape a) ci-dessus à un mélange de chlorure de benzylmagnésium dans du tétrahydrofurane anhydre à une température de -100 °C à 25 °C pour produire les produits d'addition répondant aux formules (3a) 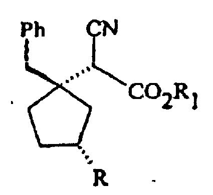 et (3b) 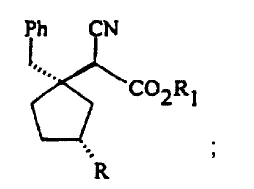
c) ajouter les produits de l'étape b) ci-dessus à un mélange d'hydroxyde de potassium dans de l'éthylène glycol, et chauffer le mélange à une température de 100 °C à 200 °C, puis l'acidifier pour produire les produits d'hydrolyse répondant aux formules (4a) 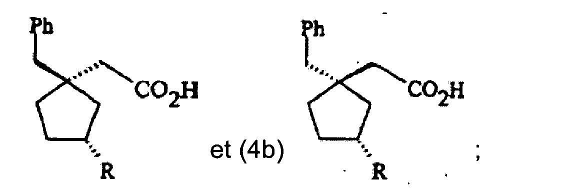
d) mettre en contact les produits de l'étape c) ci-dessus avec de la (S)-α-méthyl-benzylamine dans de l'acétate d'éthyle, et recristalliser le sel ainsi formé à partir d'acétate d'éthyle pour produire le diastéréoisomère enrichi répondant à la formule (5) 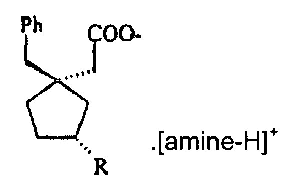 en tant que sel de (S)-α-méthyl-benzylamine ;
e) ajouter le produit de l'étape d) à de l'acide chlorhydrique aqueux, et agiter pour produire l'acide carboxylique répondant à la formule (6)

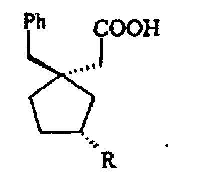

21. Procédé pour la préparation d'un composé répondant à la formule (26) 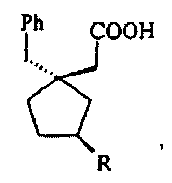 dans laquelle R représente un groupe alkyle en C₁-C₁₀ ou un groupe cycloalkyle en C₃-C₁₀, et de sels pharmaceutiquement acceptables de celui-ci, qui comprend les étapes :
a) ajouter un cyanoacétate répondant à la formule (A) 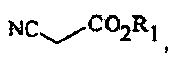 dans laquelle R₁ représente un groupe alkyle ou un groupe benzyle, à un mélange d'une cyclopentanone chirale répondant à la formule (21) 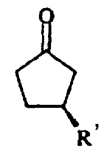 d'un solvant, d'un acide carboxylique, et d'un catalyseur de la réaction de Knoevenagel, et agiter le mélange en présence d'un moyen pour éliminer l'eau, afin de produire l'alcène répondant à la formule (22) 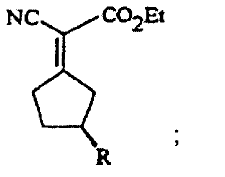
b) ajouter le produit de l'étape a) ci-dessus à un mélange de chlorure de benzylmagnésium ou d'iodure de benzylmagnésium, dans un solvant pour produire les produits d'addition répondant aux formules (23a) 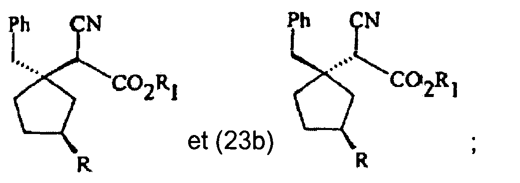
c) ajouter les produits de l'étape b) ci-dessus à un mélange d'une base choisie parmi l'hydroxyde de potassium, l'hydroxyde de sodium, l'hydroxyde de lithium, et l'hydroxyde de césium, et d'un solvant, et agiter, puis acidifier pour produire les acides carboxyliques répondant aux formules (24a) 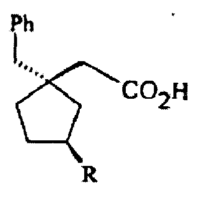 et (24b) 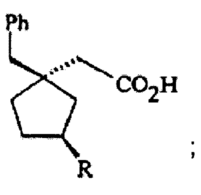 ou
ajouter les produits de l'étape b) ci-dessus à un mélange acide, et agiter pour produire les acides carboxyliques répondant aux formules (24a) 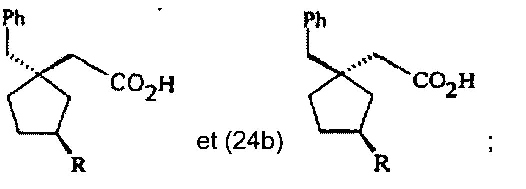
d) mettre en contact les produits de l'étape c) ci-dessus avec une amine dans un solvant, et recristalliser le sel ainsi formé pour produire le diastéréoisomère enrichi répondant à la formule (25) 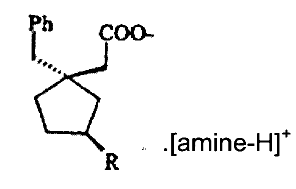 en tant que sel d'amine ; et
e) convertir le produit de l'étape d) en acide carboxylique répondant à la formule (26)

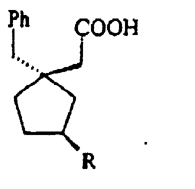

22. Procédé selon la revendication 21, qui comprend les étapes :
a) ajouter un cyanoacétate répondant à la formule (A) 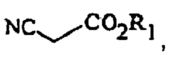 dans laquelle R₁ est choisi parmi les groupes méthyle, éthyle, n-propyle, isopropyle, n-butyle, isobutyle, sec-butyle, tert-butyle, et benzyle, à un mélange d'une cyclopentanone chirale répondant à la formule (21) 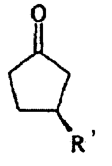 d'un solvant choisi parmi le tétrahydrofurane, le 1,4-dioxane, l'éther tert-butylméthylique, le chloroforme, le dichlorométhane, l'acétonitrile, l'éther éthylique, l'acétate d'éthyle, les hexanes, le N,N-diméthylformamide, le diméthylsulfoxyde, l'éthanol, le tert-butanol, le toluène, le benzène, les xylènes, et le n-heptane, d'acide acétique, et d'un catalyseur de la réaction de Knoevenagel choisi parmi la β-alanine, l'acétate d'ammonium, et la pipéridine, et agiter le mélange en présence d'un moyen pour éliminer l'eau choisi parmi la distillation azéotropique, les tamis moléculaires activés, le sulfate de magnésium anhydre, le carbonate de césium anhydre, l'orthoformiate de triméthyle, et l'orthoformiate de triéthyle, afin de produire l'alcène répondant à la formule (22) 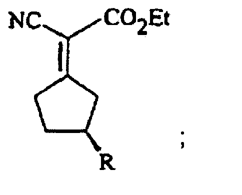
b) ajouter le produit de l'étape a) ci-dessus à un mélange de chlorure de benzylmagnésium, le bromure de benzylmagnesium ou l'iodure de benzylmagnésium, dans un solvant choisi parmi le tétrahydrofurane, le 1,4-dioxane, les hexanes, le n-heptane, le toluène, l'éther diéthylique, et l'éther tert-butylméthylique pour produire les produits d'addition répondant aux formules (23a) 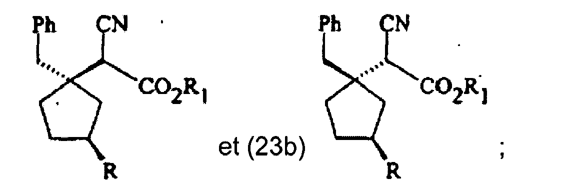
c) ajouter les produits de l'étape b) ci-dessus à un mélange d'une base choisie parmi l'hydroxyde de potassium, l'hydroxyde de sodium, l'hydroxyde de lithium, et l'hydroxyde de césium, dans un solvant choisi parmi l'éthylène glycol, l'éther 2-méthoxyéthylique, le 1,4-dioxane, et le diéthylène glycol, et agiter le mélange, puis l'acidifier pour produire les acides carboxyliques répondant aux formules (24a) 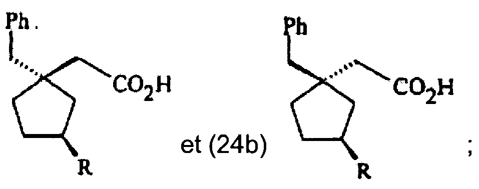 ou
ajouter les produits de l'étape b) ci-dessus à un mélange acide choisi parmi de l'HCl à 6 à 12 M, de l'H₂SO₄ à 12 M, 10 à 48 % poids/poids d'acide bromhydrique, et de l'HBr dans de l'acide acétique aqueux, et agiter pour produire les acides carboxyliques répondant aux formules (24a) 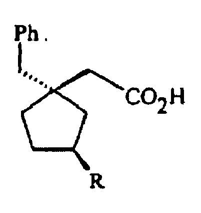 et (24b) 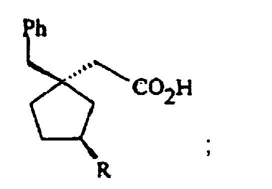
d) mettre en contact les produits de l'étape c) ci-dessus avec une amine choisie parmi la (S)-α-méthyl-benzylamine, la (R)-α-méthyl-benzylamine, la (R)-(+)-1-(naphtyl)éthylamine, la (S)-(+)-1-(naphtyl)éthylamine, la triéthylamine, la diisopropyléthylamine, la dicylohexylamine, la benzylamine, la dibenzylamine, la morpholine, la N-méthylmorpholine, la pipéridine, la N-méthylpipéridine, et la pyridine dans un solvant choisi parmi le N,N-diméthylformamide, le chloroforme, les hexanes, l'acétone, l'éthanol, le méthanol, l'isopropanol, l'éther diéthylique, le dichlorométhane, le benzène, le toluène, le n-pentane, le n-hexane, le n-heptane, l'acétate d'éthyle, l'acétonitrile, l'éther tert-butylméthylique, le tétrahydrofurane, et le 1,4-dioxane, et recristalliser le sel ainsi formé pour produire le diastéréoisomère enrichi répondant à la formule (25) 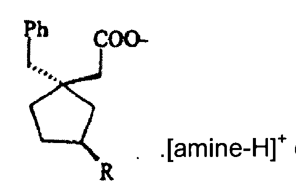 en tant que sel d'amine ; et
e) ajouter le produit de l'étape d) à un mélange choisi parmi l'acide chlorhydrique aqueux, l'acide sulfurique aqueux, l'acide acétique aqueux, l'acide chlorhydrique dissous dans de l'acide acétique, et l'acide chlorhydrique dissous dans de l'acide acétique et de l'eau, et agiter pour produire l'acide carboxylique répondant à la formule (26) 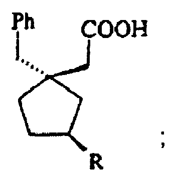 ou
diviser le produit de l'étape d) entre un mélange d'acide chlorhydrique aqueux et d'un solvant choisi parmi le chloroforme, le dichlorométhane, l'acétate d'éthyle, l'éther éthylique, le tétrahydrofurane, le 1,4-dioxane, le toluène, et l'éther tert-butylméthylique, et sécher et évaporer la phase organique pour produire l'acide carboxylique répondant à la formule (26)

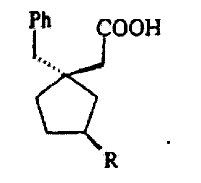

23. Procédé selon la revendication 21, qui comprend les étapes :
a) ajouter un cyanoacétate répondant à la formule (A) 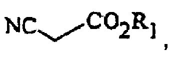 dans laquelle R₁ représente un groupe éthyle, à un mélange d'une cyclopentanone chirale répondant à la formule (21) 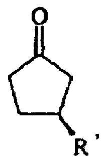 de toluène, d'acide acétique, et d'un catalyseur de la réaction de Knoevenagel qui est l'acétate d'ammonium, et chauffer le mélange au reflux sur un piège Dean-Stark, pour produire l'alcène répondant à la formule (22) 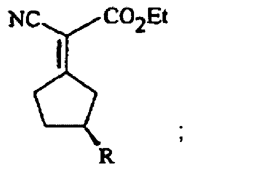
b) ajouter le produit de l'étape a) ci-dessus à un mélange de chlorure de benzylmagnésium dans du tétrahydrofurane anhydre à une température de -100 °C à -20 °C pour produire les produits d'addition répondant aux formules (23a) 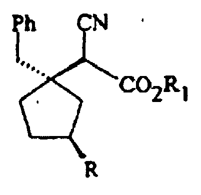 et (23b) 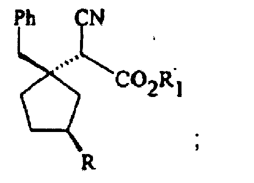
c) ajouter les produits de l'étape b) ci-dessus à un mélange d'hydroxyde de potassium dans de l'éthylène glycol, et chauffer le mélange à une température de 100 °C à 200 °C, puis l'acidifier pour produire les produits d'hydrolyse répondant aux formules (24a) 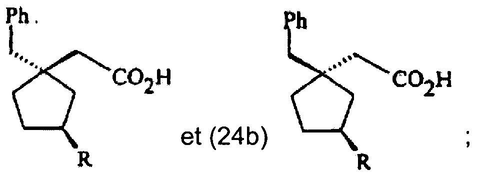
d) mettre en contact les produits de l'étape c) ci-dessus avec de la (R)-α-méthyl-benzylamine dans de l'acétate d'éthyle, et recristalliser le sel ainsi formé à partir d'acétate d'éthyle pour produire le diastéréoisomère enrichi répondant à la formule (25) 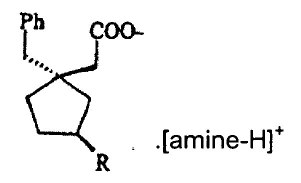 en tant que sel de (R)-α-méthyl-benzylamine ; et
e) ajouter le produit de l'étape d) à de l'acide chlorhydrique aqueux, et agiter pour produire l'acide carboxylique répondant à la formule (26)

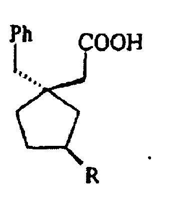

24. Composé répondant à la formule (6) 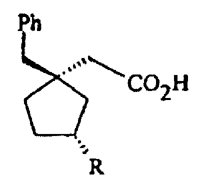 dans laquelle R représente un groupe alkyle en C₁-C₁₀ ou un groupe cycloalkyle en C₃₋C₁₀, et les sels pharmaceutiquement acceptables de celui-ci.

25. Composé selon la revendication 24, dans lequel R représente un groupe alkyle en C₁-C₁₀.

26. Composé selon la revendication 24, dans lequel R est choisi parmi les groupes méthyle, éthyle et n-propyle.

27. Composé selon la revendication 24, nommé acide ((1S,3R)-1-benzyl-3-méthyl-cyclopentyl)acétique.

28. Composé répondant à la formule (26) 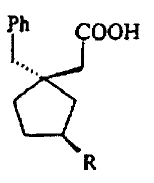 dans laquelle R représente un groupe alkyle en C₁-C₁₀ ou un groupe cycloalkyle en C₃₋C₁₀, et les sels pharmaceutiquement acceptables de celui-ci.

29. Composé selon la revendication 28, dans lequel R représente un groupe alkyle en C₁-C₁₀.

30. Composé selon la revendication 28, dans lequel R est choisi parmi les groupes méthyle, éthyle et n-propyle.

31. Composé selon la revendication 28, nommé acide ((1R,3S)-1-benzyl-3-méthyl-cyclopentyl)acétique.

32. Composé répondant à la formule (6) 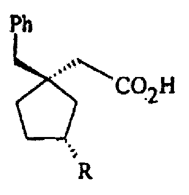 dans laquelle R représente un groupe alkyle en C₁-C₁₀ ou un groupe cycloalkyle en C₃₋C₁₀, et les sels pharmaceutiquement acceptables de celui-ci, préparé selon le procédé de la revendication 18.

33. Composé répondant à la formule (6) 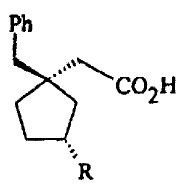 dans laquelle R représente un groupe alkyle en C₁-C₁₀ ou un groupe cycloalkyle en C₃₋C₁₀, et les sels pharmaceutiquement acceptables de celui-ci, préparé selon le procédé de la revendication 19.

34. Composé répondant à la formule (6) 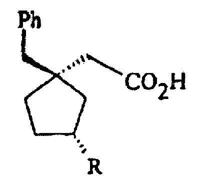 dans laquelle R représente un groupe alkyle en C₁-C₁₀ ou un groupe cycloalkyle en C₃₋C₁₀, et les sels pharmaceutiquement acceptables de celui-ci, préparé selon le procédé de la revendication 20.

35. Composé répondant à la formule (26) 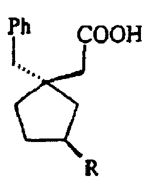 dans laquelle R représente un groupe alkyle en C₁-C₁₀ ou un groupe cycloalkyle en C₃₋C₁₀. et les sels pharmaceutiquement acceptables de celui-ci, préparé selon le procédé de la revendication 21.

36. Composé répondant à la formule (26) 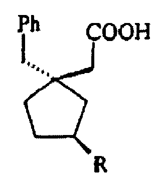 dans laquelle R représente un groupe alkyle en C₁-C₁₀ ou un groupe cycloalkyle en C₃₋C₁₀, et les sels pharmaceutiquement acceptables de celui-ci, préparé selon le procédé de la revendication 22.

37. Composé répondant à la formule (26) 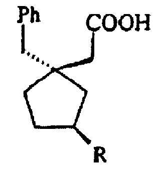 dans laquelle R représente un groupe alkyle en C₁-C₁₀ ou un groupe cycloalkyle en C₃₋C₁₀, et les sels pharmaceutiquement acceptables de celui-ci, préparé selon le procédé de la revendication 23.

38. Composé choisi parmi :
l'ester éthylique de l'acide E-cyano-((R)-3-méthyl-cyclopentylidène)acétique ;
l'ester éthylique de l'acide Z-cyano-((R)-3-méthyl-cyclopentylidène)acétique ;
l'ester éthylique de l'acide (R)-((1S,3R)-1-benzyl-3-méthyl-cyclopentyl)cyanoacétique ;
l'ester éthylique de l'acide (S)-((1S,3R)-1-benzyl-3-méthyl-cyclopentyl)cyanoacétique ;
l'ester éthylique de l'acide (R)-((1R,3R)-1-benzyl-3-méthyl-cyclopentyl)cyanoacétique ;
l'ester éthylique de l'acide (S)-((1R,3R)-1-benzyl-3-méthyl-cyclopentyl)cyanoacétique ;
le ((1S,3R)-1-isocyanatométhyl-3-méthyl-cyclopentylméthyl)benzène ;
l'ester méthylique de l'acide ((1S,3R)-1-benzyl-3-méthyl-cyclopentylméthyl)carbamique ;
l'acide [((1S,3R)-1-(méthoxycarbonylaminométhyl)-3-méthyl-cyclopentyl]acétique ;
l'ester méthylique de l'acide ((1S,3R)-1-benzyl-3-méthyl-cyclopentyl)acétique ;
l'acide ((1S,3R)-1-méthoxycarbonylméthyl-3-méthyl-cyclopentyl)acétique ;
l'ester méthylique de l'acide ((1R,3R)-1-isocyanatométhyl-3-méthyl-cyclopentyl)acétique ;
l'ester méthylique de l'acide [(1R,3R)-1-(méthoxycarbonylamino-méthyl)-3-méthyl-cyclopentyl]acétique ;
l'ester tert-butylique de l'acide ((1S,3R)-1-benzyl-3-méthyl-cyclopentyl)acétique ;
l'ester tert-butylique de l'acide [(1S,3R)-1-carboxyméthyl-3-méthyl-cyclopentyl]acétique ;
l'ester tert-butylique de l'acide [(1S,3R)-1-méthoxycarbonylméthyl-3-méthyl-cyclopentyl]acétique ;
l'acide ((1R,3R)-1-méthoxycarbonylméthyl-3-méthyl-cyclopentyl)acétique ;
l'ester méthylique de l'acide ((1S,3R)-1-isocyanatométhyl-3-méthyl-cyclopentyl)acétique ; et
l'ester méthylique de l'acide [(1S,3R)-1-(méthoxycarbonylaminométhyl)-3-méthyl-cyclopentyl]acétique.

39. Procédé pour la préparation d'un composé répondant à la formule (4a) 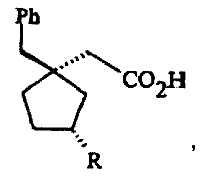 dans laquelle R représente un groupe alkyle en C₁-C₁₀ ou un groupe cycloalkyle en C₃-C₁₀, comprenant l'hydrolyse d'un composé répondant à la formule (3a) 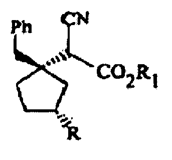 dans laquelle R₁ représente H, un groupe alkyle ou un groupe benzyle.

40. Procédé pour la préparation d'un composé répondant à la formule (24a) 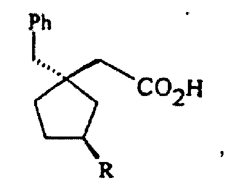 dans laquelle R représente un groupe alkyle en C₁-C₁₀ ou un groupe cycloalkyle en C₃-C₁₀, comprenant l'hydrolyse d'un composé répondant à la formule (23a) 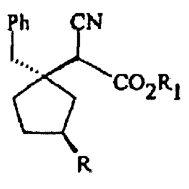 dans laquelle R₁ représente H, un groupe alkyle ou un groupe benzyle.

41. Procédé pour la préparation d'un composé répondant à la formule (6) 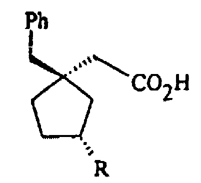 dans laquelle R représente un groupe alkyle en C₁-C₁₀ ou un groupe cycloalkyle en C₃-C₁₀, comprenant la résolution d'un mélange contenant les composés répondant aux formules (4a) 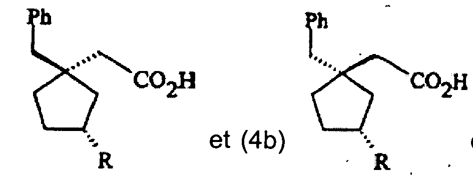 dans lesquelles R représente un groupe alkyle en C₁-C₁₀ ou un groupe cycloalkyle en C₃-C₁₀.

42. Procédé pour la préparation d'un composé répondant à la formule (26) 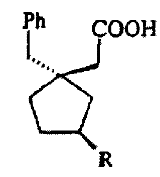 dans laquelle R représente un groupe alkyle en C₁-C₁₀ ou un groupe cycloalkyle en C₃-C₁₀, comprenant la résolution d'un mélange contenant les composés répondant aux formules (24a) 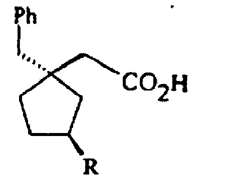 et (24b) 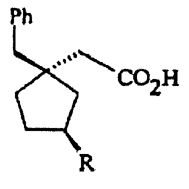 dans lesquelles R représente un groupe alkyle en C₁-C₁₀ ou un groupe cycloalkyle en C₃-C₁₀.

43. Procédé pour la préparation d'un composé répondant à la formule I 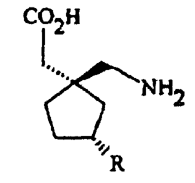 dans laquelle R représente un groupe alkyle en C₁-C₁₀ ou un groupe cycloalkyle en C₃-C₁₀, et de sels pharmaceutiquement acceptables de celui-ci, comprenant l'hydrolyse d'un composé répondant à la formule (41) 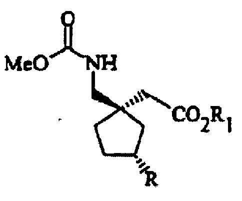 dans laquelle R₁ représente H, un groupe alkyle ou un groupe benzyle, et la mise en contact du produit, si souhaité, avec un acide ou une base.

44. Procédé de préparation d'un composé répondant à la formule II 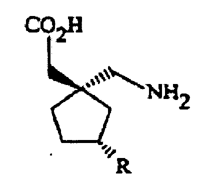 dans laquelle R représente un groupe alkyle en C₁-C₁₀ ou un groupe cycloalkyle en C₃-C₁₀, et de sels pharmaceutiquement acceptables de celui-ci, comprenant l'hydrolyse d'un composé répondant à la formule (42) 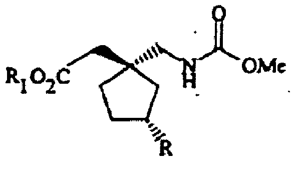 dans laquelle R₁ représente H, un groupe alkyle ou un groupe benzyle, et la mise en contact du produit, si souhaité, avec un acide ou une base.

45. Procédé pour la préparation d'un composé répondant à la formule III 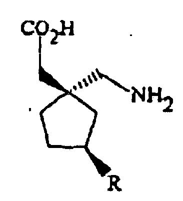 dans laquelle R représente un groupe alkyle en C₁-C₁₀ ou un groupe cycloalkyle en C₃-C₁₀, et de sels pharmaceutiquement acceptables de celui-ci, comprenant l'hydrolyse d'un composé répondant à la formule (43) 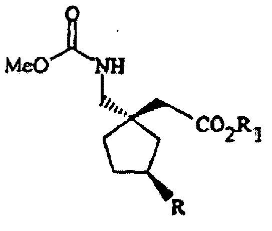 dans laquelle R₁ représente H, un groupe alkyle ou un groupe benzyle, et la mise en contact du produit, si souhaité, avec un acide ou une base.

46. Procédé pour la préparation d'un composé répondant à la formule IV 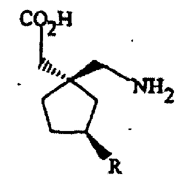 dans laquelle R représente un groupe alkyle en C₁-C₁₀ ou un groupe cycloalkyle en C₃-C₁₀, et de sels pharmaceutiquement acceptables de celui-ci, comprenant l'hydrolyse d'un composé répondant à la formule (44) 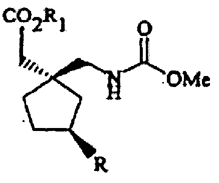 dans laquelle R₁ représente H, un groupe alkyle ou un groupe benzyle, et la mise en contact du produit, si souhaité, avec un acide ou une base.
